# EUROPEAN PATENT APPLICATION

(11) **EP 4 516 794 A1**
(43) Date of publication of application: **05.03.2025**
(21) Application number: 23796309.5
(22) Date of filing: 24.04.2023
(51) Int. Cl.: C07D 513/04, C08G 61/12, H01L 29/786, H10K 85/10

(54) **NOVEL COMPOUND, CONJUGATED POLYMER AND METHOD FOR PRODUCING SAME, FILM FORMATION COMPOSITION, ORGANIC THIN FILM, AND ORGANIC SEMICONDUCTOR ELEMENT**

(30) Priority: 26.04.2022 JP 2022072654
(71) Applicant: Tosoh Corporation, Yamaguchi 746-8501 (JP); Sagami Chemical Research Institute, Ayase-shi Kanagawa 252-1193 (JP)
(72) Inventor: YAMAGATA, Takuya, Ayase-shi, Kanagawa 252-1193 (JP); MIKAMI, Koichiro, Ayase-shi, Kanagawa 252-1193 (JP); HANAMURA, Hitoshi, Ayase-shi, Kanagawa 252-1193 (JP); WAKIOKA, Masayuki, Ayase-shi, Kanagawa 252-1193 (JP); MIYASHITA, Masato, Yokkaichi-shi, Mie 510-8540 (JP); WATANABE, Makoto, Yokkaichi-shi, Mie 510-8540 (JP); OKU, Shinya, Yokkaichi-shi, Mie 510-8540 (JP)
(74) Representative: Novagraaf International SA
(86) International application number: PCT/JP2023/016079
(87) International publication number: WO 2023/210569

(57) **Abstract**

The compound according to the present disclosure is a compound represented by general formula (1) below.

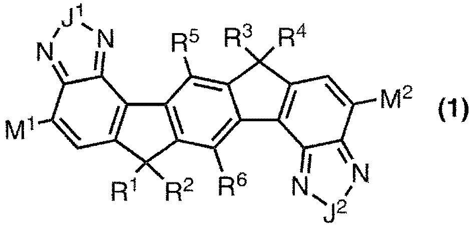

## Description

### Technical Field

The present invention relates to a novel compound having an indenofluorenochalcogenadiazole structure, a conjugated polymer including the structure as its structural unit and a method for producing it and a film formation composition, an organic thin film and an organic semiconductor element.

### Background Art

Conjugated organic compounds are well known as organic semiconductors used in applications such as organic thin-film solar cells, organic thin-film transistors and OLEDs. Conjugated organic compounds have features not found in inorganic compounds, such as energy saving, low cost, solubility in organic solvents, light weight and flexibility, and can also be used as coating materials applied to printed electronics (PTL 1).

In particular, there are many examples of donor-acceptor (D-A) polymers developed for the purpose of increasing intermolecular interactions through electrostatic interactions or extending the absorption spectrum. In NPL 1, a D-A polymer having a naphthobisthiadiazole backbone is described. In NPL 2, a D-A polymer having an anthrabisthiadiazole backbone is described. In PTL 2, a D-A polymer having a bis(thienocyclopenta)benzothiadiazole backbone is described.

### Citation List

### Patent Literature

PTL 1: Japanese Unexamined Patent Application Publication No. 2017-59668
PTL 2: Japanese Unexamined Patent Application Publication No. 2012-503045

### Non Patent Literature

NPL 1: Journal of the American Chemical Society, vol. 134, pp. 3498-3507, 2012.
NPL 2: Macromolecules, vol. 51, pp. 5473-5484, 2018.

### Summary of Invention

### Technical Problem

Conjugated polymers used as materials for organic semiconductors are required to have characteristics such as high carrier mobility, high heat resistance, high atmospheric stability and high solubility. Among known conjugated polymers, however, there is no compound that combines all such characteristics.

An object of the present invention, therefore, is to provide a new conjugated polymer combining high carrier mobility, high heat resistance, high atmospheric stability and high solubility, a method for producing the polymer, a film formation composition containing the polymer, an organic thin film containing the polymer and an organic semiconductor element and an organic thin-film transistor element containing the polymer.

### Solution to Problem

After conducting extensive research to solve the above problem, the inventors found that a conjugated polymer containing an indenofluorenochalcogenadiazole structure as its structural unit exhibits high carrier mobility, high solubility, high heat resistance, high atmospheric stability and high film-forming properties. The conjugated polymer containing an indenofluorenochalcogenadiazole structure as its structural unit and a monomer that gives the polymer are novel substances, and their production methods have yet to be reported either. There are, furthermore, no reports on the physical characteristics, such as solubility and heat resistance, and the carrier mobility of the polymer. The inventors also found that an organic thin film can be easily and conveniently formed using a film formation composition containing the polymer, and that an organic thin-film transistor element made using the organic thin film operates in a stable manner, thereby completing the present invention.

The present invention, therefore, is composed of the following gists.

### [Gist 1]

A compound represented by general formula (1) below.

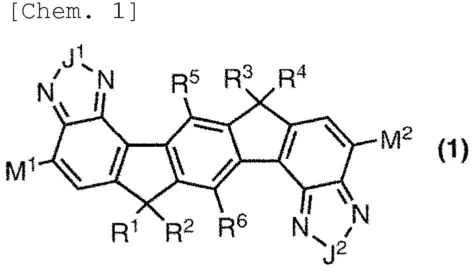

(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom. M¹ and M² each independently represent one group selected from the group consisting of a hydrogen atom, a halogen atom, a boron-containing group and a tin-containing group.)

### [Gist 2]

A conjugated polymer comprising a structural unit represented by general formula (2) below

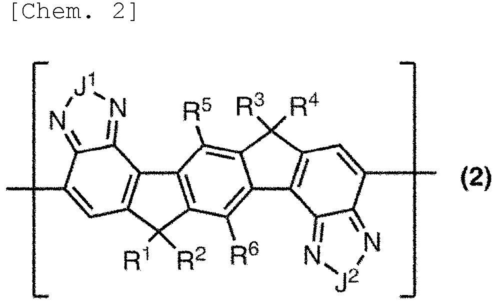

(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom.) and a structural unit represented by general formula (3) below.

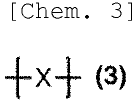

(In the formula, X represents a divalent heteroaromatic ring linking group optionally substituted with a C1 to C50 alkyl group or a C1 to C50 alkoxy group.)

### [Gist 3]

A method for producing a conjugated polymer composed of a structural unit represented by general formula (2) below and a structural unit represented by general formula (3) below, the method comprising:
allowing a monomer represented by general formula (mono-hal) below to react with a monomer represented by general formula (mono-X-B) below in the presence of a transition metal catalyst.

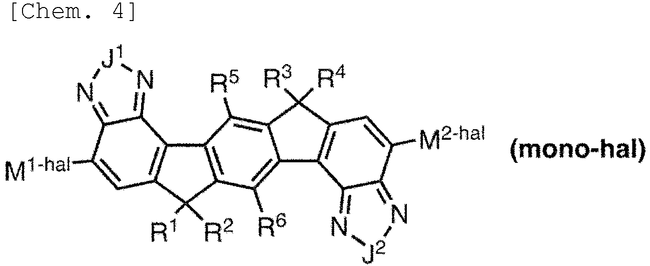

(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom. M^{1-hal} and M^{2-hal} each independently represent a halogen atom.)

[Chem. 5] M^{3-B}-X-M^{4-B} **(mono-X-B)**

(In the formula, X represents a divalent heteroaromatic ring linking group optionally substituted with a C1 to C50 alkyl group or a C1 to C50 alkoxy group. M^{3-B} and M^{4-B} each independently represent a boron-containing group.)

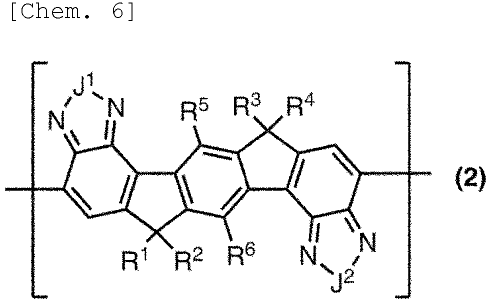

(In the formula, R¹, R², R³, R⁴, R⁵, R⁶, J¹ and J² have the same meanings as described above.)

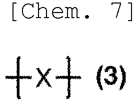

(In the formula, X has the same meaning as described above.)

### [Gist 4]

A method for producing a conjugated polymer composed of a structural unit represented by general formula (2) below and a structural unit represented by general formula (3) below, the method comprising:
allowing a monomer represented by general formula (mono-hal) below to react with a monomer represented by general formula (mono-X-Sn) below in the presence of a transition metal catalyst.

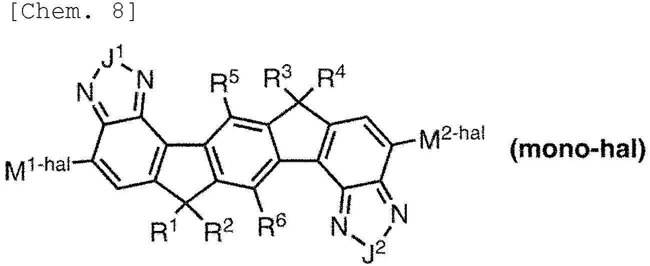

(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom. M^{1-hal} and M^{2-hal} each independently represent a halogen atom.)

[Chem. 9] M^{3-Sn}-X-M^{4-Sn} (**mono-X-Sn**)

(In the formula, X represents a divalent heteroaromatic ring linking group optionally substituted with a C1 to C50 alkyl group or a C1 to C50 alkoxy group. M^{3-Sn} and M^{4-Sn} each independently represent a tin-containing group.)

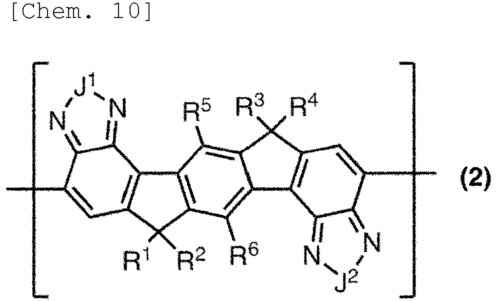

(In the formula, R¹, R², R³, R⁴, R⁵, R⁶, J¹ and J² have the same meanings as described above.)

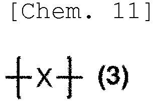

(In the formula, X has the same meaning as described above.)

### [Gist 5]

A method for producing a conjugated polymer composed of a structural unit represented by general formula (2) below and a structural unit represented by general formula (3) below, the method comprising:
allowing a monomer represented by general formula (mono-B) below to react with a monomer represented by general formula (mono-X-hal) below in the presence of a transition metal catalyst.

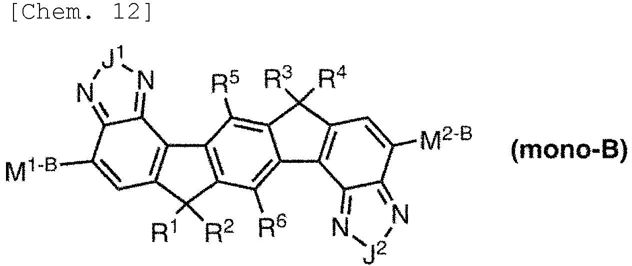

(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom. M^{1-B} and M^{2-B} each independently represent a boron-containing group.)

[Chem. 13] M^{3-hal}-X-M^{4-hal} **(mono-X-hal)**

(In the formula, X represents a divalent heteroaromatic ring linking group optionally substituted with a C1 to C50 alkyl group or a C1 to C50 alkoxy group. M^{3-hal} and M^{4-hal} each independently represent a halogen atom.)

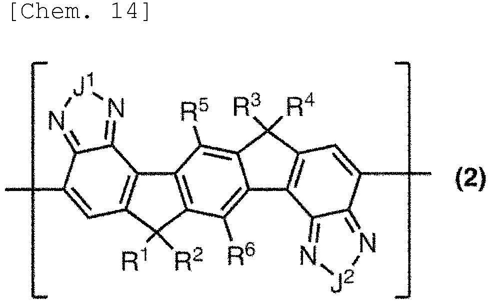

(In the formula, R¹, R², R³, R⁴, R⁵, R⁶, J¹ and J² have the same meanings as described above.)

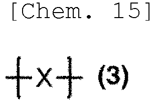

(In the formula, X has the same meaning as described above.)

### [Gist 6]

A method for producing a conjugated polymer composed of a structural unit represented by general formula (2) below and a structural unit represented by general formula (3) below, the method comprising:
allowing a monomer represented by general formula (mono-Sn) below to react with a monomer represented by general formula (mono-X-hal) below in the presence of a transition metal catalyst.

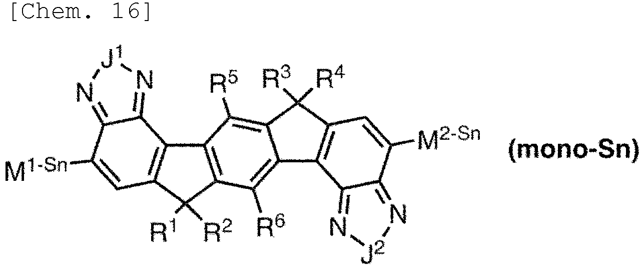

(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom. M^{1-Sn} and M^{2-Sn} each independently represent a tin-containing group.)

[Chem. 17] M^{3-hal}-X-M^{4-hal} **(mono-X-hal)**

(In the formula, X represents a divalent heteroaromatic ring linking group optionally substituted with a C1 to C50 alkyl group or a C1 to C50 alkoxy group. M^{3-hal} and M^{4-hal} each independently represent a halogen atom.)

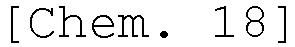

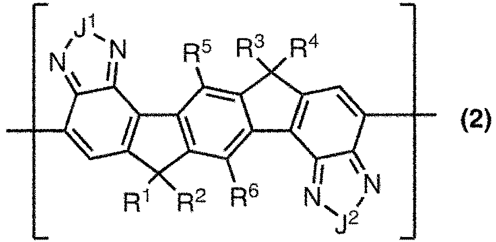

(In the formula, R¹, R², R³, R⁴, R⁵, R⁶, J¹ and J² have the same meanings as described above.)

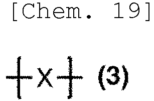

(In the formula, X has the same meaning as described above.)

### [Gist 7]

A method for producing a conjugated polymer composed of a divalent structural unit represented by general formula (2) below and a structural unit represented by general formula (3) below, the method comprising:
allowing a monomer represented by general formula (mono-hal) below to react with a monomer represented by general formula (mono-X-H) below in the presence of a transition metal catalyst.

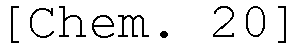

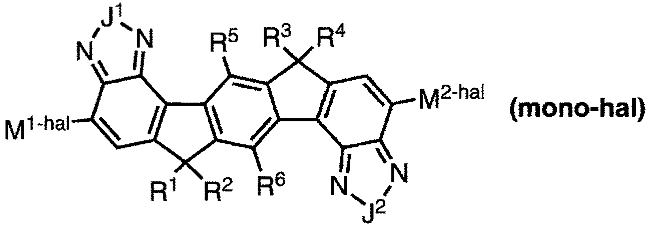

(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom. M^{1-hal} and M^{2-hal} each independently represent a halogen atom.)

[Chem. 21] **H-X-H (mono-X-H)**

(In the formula, X represents a divalent heteroaromatic ring linking group optionally substituted with a C1 to C50 alkyl group or a C1 to C50 alkoxy group.)

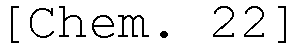

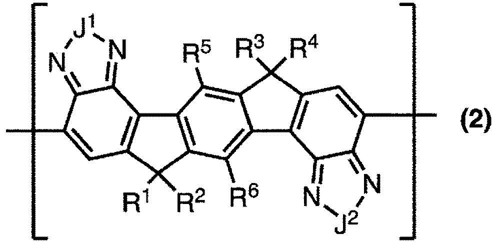

(In the formula, R¹, R², R³, R⁴, R⁵, R⁶, J¹ and J² have the same meanings as described above.)

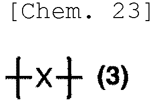

(In the formula, X has the same meaning as described above.)

### Advantageous Effects of Invention

The conjugated polymer according to the present invention is an organic semiconductor combining high carrier mobility, high heat resistance, high atmospheric stability and high solubility and allows an organic thin-film transistor element having it as its active layer to operate efficiently.

### Brief Description of Drawings

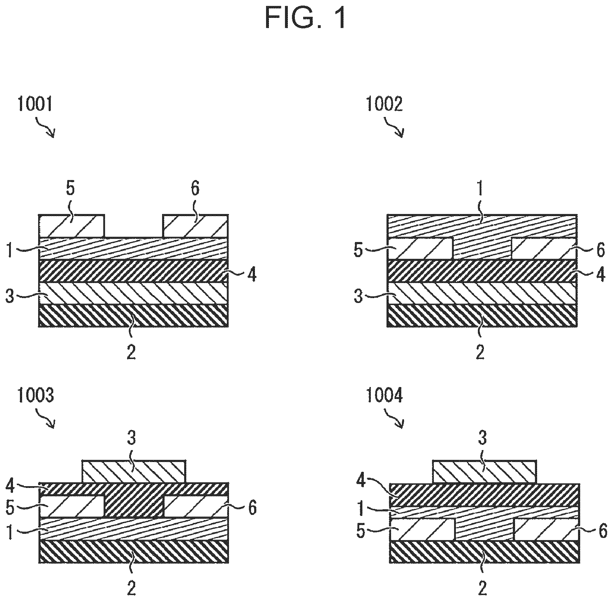

[Fig. 1] Diagrams illustrating the structure of organic thin-film transistor elements based on its cross-sectional shape.

### Description of Embodiments

Embodiments of the present invention will now be described in detail. The present invention, however, is not limited to these; various modifications are possible within the scope of the description, and embodiments obtained by combining technical means disclosed in different embodiments as necessary are also included in the technical scope of the present invention. It should be noted that as mentioned herein, "P to Q" representing a numerical range means "P or more and Q or less."

### [Compound]

A compound according to an embodiment of the present invention (Also referred to as "a compound in this embodiment" herein.) is a compound represented by general formula (1).

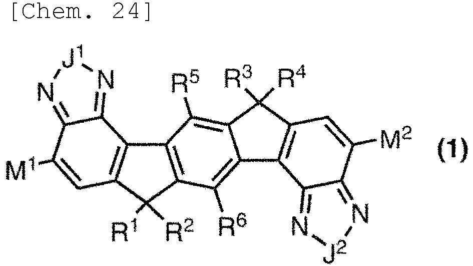

(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom. M¹ and M² each independently represent one group selected from the group consisting of a hydrogen atom, a halogen atom, a boron-containing group and a tin-containing group.)

C1 to C50 alkyl groups represented by R¹, R², R³, R⁴, R⁵ and R⁶ may be any of linear-chain, branched or cyclic. Examples include linear-chain alkyl groups, such as a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a heneicosyl group, a docosyl group, a tricosyl group, a tetracosyl group, a pentacosyl group, a hexacosyl group, a heptacosyl group, an octacosyl group, a nonacosyl group, a triacontyl group, a hentriacontyl group, a dotriacontyl group, a tritriacontyl group, a tetratriacontyl group, a pentatriacontyl group, a hexatriacontyl group, a tetracontyl group, a hentetracontyl group, a dotetracontyl group, a tritetracontyl group, a tetratetracontyl group and a pentacontyl group; branched alkyl groups, such as an isopropyl group, an isobutyl group, a sec-butyl group, a tert-butyl group, a 2-ethylhexyl group, a 3,7-dimethyloctyl group, a 2-hexyloctyl group, a 2-hexyldecyl group, a 2-octyldodecyl group, a 2-decyltetradecyl group, a 2-dodecyltetradecyl group, a 2-dodecylhexadecyl group, a 2-tetradecylhexadecyl group, a 3-decylpentadecyl group, a 3-dodecylheptadecyl group, a 3-tetradecylnonacosyl group, a 4-decylhexadecyl group, a 4-dodecyloctadecyl group and a 4-tetradecylicosyl group; and cyclic alkyl groups, such as a cyclopentyl group and a cyclohexyl group.

The alkyl groups represented by R¹, R², R³ and R⁴ are preferably C1 to C34 alkyl groups because this increases the solubility of the compound in this embodiment. More preferably, the alkyl groups are C1 to C20 alkyl groups, even more preferably methyl groups, ethyl groups, propyl groups, butyl groups, pentyl groups, hexyl groups, heptyl groups, octyl groups, nonyl groups, decyl groups, undecyl groups, dodecyl groups, tridecyl groups, tetradecyl groups, pentadecyl groups, hexadecyl groups, heptadecyl groups, octadecyl groups, nonadecyl groups, icosyl groups, 2-ethylhexyl groups, 3,7-dimethyloctyl groups, 2-hexyloctyl groups, 2-hexyldecyl groups, 2-octyldodecyl groups or 12-butyloctadecyl groups, especially preferably decyl groups or hexadecyl groups.

R¹ and R² may be bound to each other to form a ring together with the carbon atom to which they are bound. Examples of such rings include a cyclopropan-1,1-diyl group, a cyclobutan-1,1-diyl group, a cyclopentan-1,1-diyl group, a cyclohexan-1,1-diyl group, a cycloheptan-1,1-diyl group, a cyclooctan-1,1-diyl group, an inden-1,1-diyl group and a fluoren-9,9-diyl group. Preferably, this ring is a cyclopentan-1,1-diyl group, a cyclohexan-1,1-diyl group, an inden-1,1-diyl group or a fluoren-9,9-diyl group because this leads to higher solubility of the compound in this embodiment. More preferably, the ring is a cyclohexan-1,1-diyl group or a fluoren-9,9-diyl group.

R³ and R⁴ may be bound to each other to form a ring together with the carbon atom to which they are bound. Examples of such rings include a cyclopropan-1,1-diyl group, a cyclobutan-1,1-diyl group, a cyclopentan-1,1-diyl group, a cyclohexan-1,1-diyl group, a cycloheptan-1,1-diyl group, a cyclooctan-1,1-diyl group, an inden-1,1-diyl group and a fluoren-9,9-diyl group. Preferably, this ring is a cyclopentan-1,1-diyl group, a cyclohexan-1,1-diyl group, an inden-1,1-diyl group or a fluoren-9,9-diyl group because this leads to higher solubility of the compound in this embodiment. More preferably, the ring is a cyclohexan-1,1-diyl group or a fluoren-9,9-diyl group.

R⁵ and R⁶ are preferably hydrogen atoms, fluorine atoms or C1 to C34 alkyl groups because this leads to higher carrier mobility of conjugated polymers made using the compound in this embodiment. More preferably, R⁵ and R⁶ are hydrogen atoms, fluorine atoms or C1 to C20 alkyl groups, even more preferably hydrogen atoms, fluorine atoms, methyl groups, ethyl groups, propyl groups, butyl groups, pentyl groups, hexyl groups, heptyl groups, octyl groups, nonyl groups, decyl groups, undecyl groups, dodecyl groups, tridecyl groups, tetradecyl groups, pentadecyl groups, hexadecyl groups, heptadecyl groups, octadecyl groups, nonadecyl groups, icosyl groups, 2-ethylhexyl groups, 3,7-dimethyloctyl groups, 2-hexyloctyl groups, 2-hexyldecyl groups or 2-octyldodecyl groups. Of these, it is especially preferred that R⁵ and R⁶ be hydrogen atoms because this leads to even higher carrier mobility.

The chalcogen atoms represented by J¹ and J² are preferably oxygen atoms, sulfur atoms or selenium atoms because this increases the solubility of the compound in this embodiment. More preferably, the chalcogen atoms are oxygen atoms or sulfur atoms, even more preferably sulfur atoms.

Examples of halogen atoms represented by M¹ and M² include fluorine atoms, chlorine atoms, bromine atoms and iodine atoms.

Examples of boron-containing groups represented by M¹ and M² include dihydroboryl groups and dialkoxyboryl groups.

Examples of tin-containing groups represented by M¹ and M² include trialkylstannyl groups, dialkylarylstannyl groups, alkyldiarylstannyl groups and triarylstannyl groups.

M¹ and M² are preferably hydrogen atoms, bromine atoms, iodine atoms, boron-containing groups or tin-containing groups because this makes the compound in this embodiment a monomer with good reactivity. More preferably, M¹ and M² are hydrogen atoms, bromine atoms, iodine atoms, dihydroboryl groups, dialkoxyboryl groups or trialkylstannyl groups, even more preferably bromine atoms.

Examples of compounds in this embodiment include the following compounds. The present invention, however, is not limited to these. In the formulae, the ns represent natural numbers of 1 to 50 that may be the same or different from one another. The xs represent natural numbers of 2 to 24 that may be the same or different from one another. The ys represent natural numbers of 1 to 24 that may be the same or different from one another. The zs represent natural numbers of 1 to 20 that may be the same or different from one another. Examples of specific compounds included in them are also presented below.

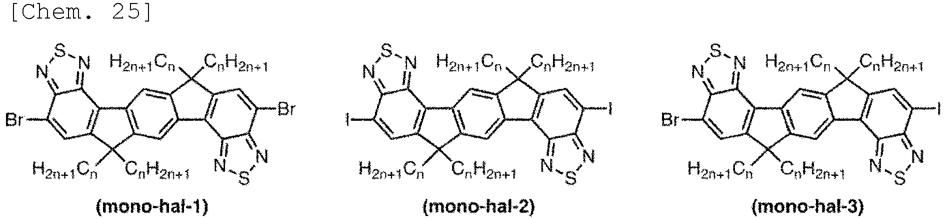

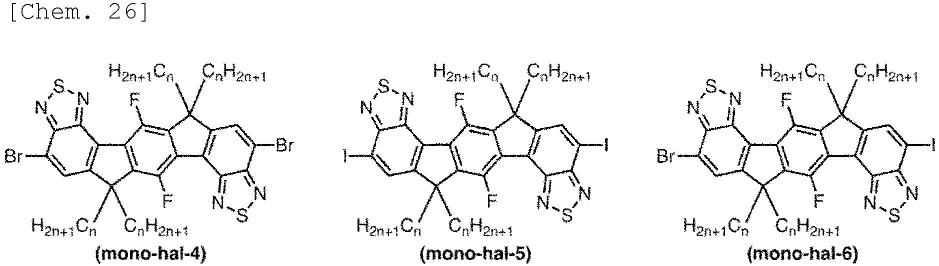

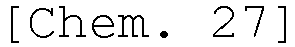

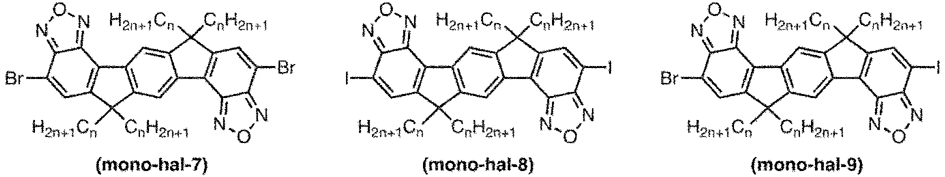

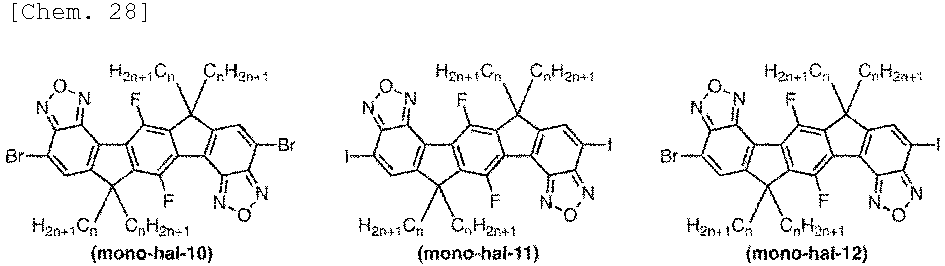

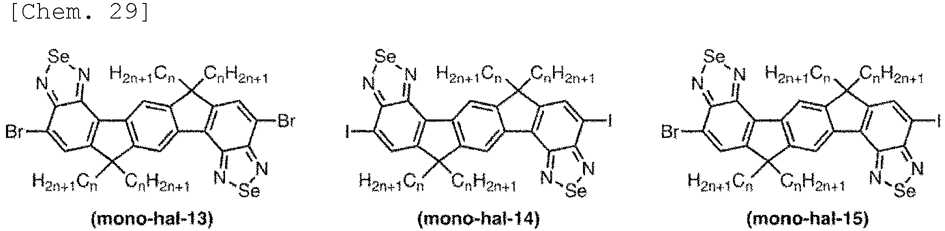

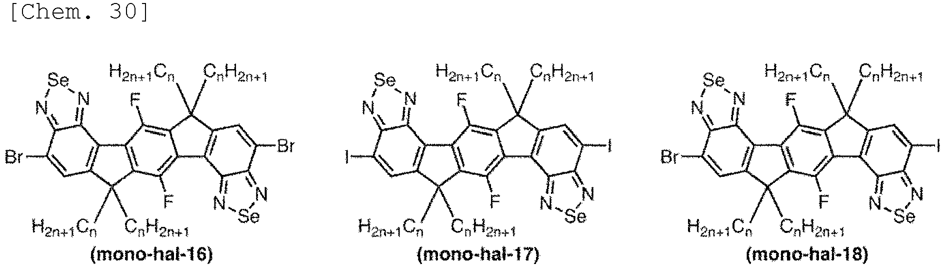

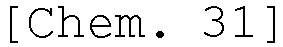

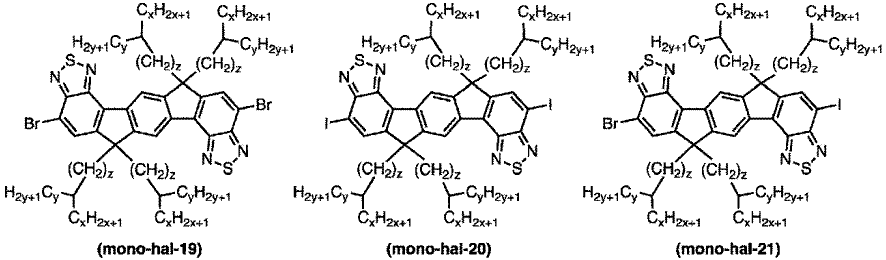

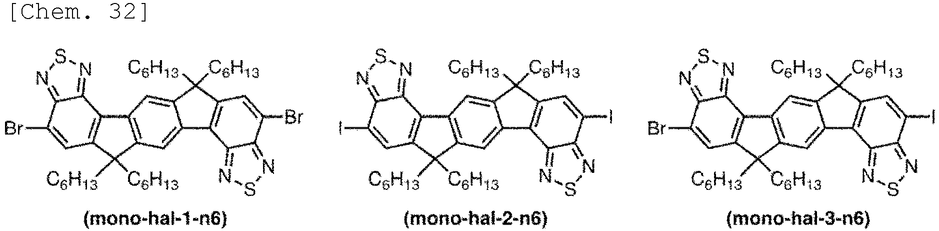

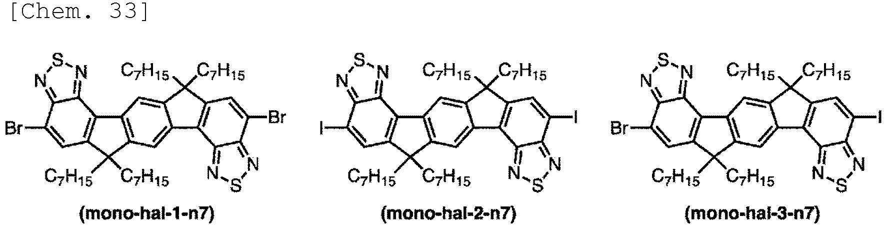

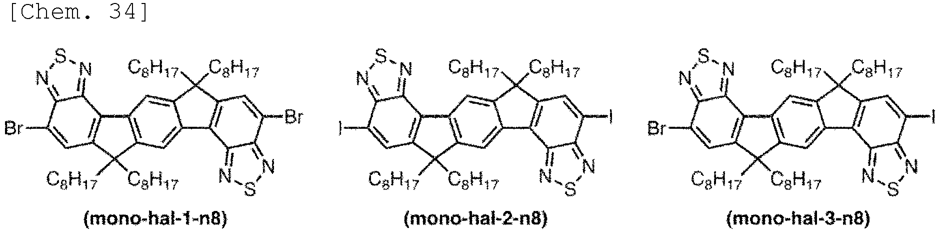

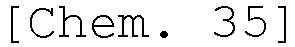

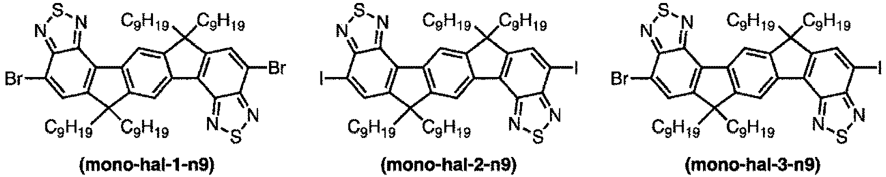

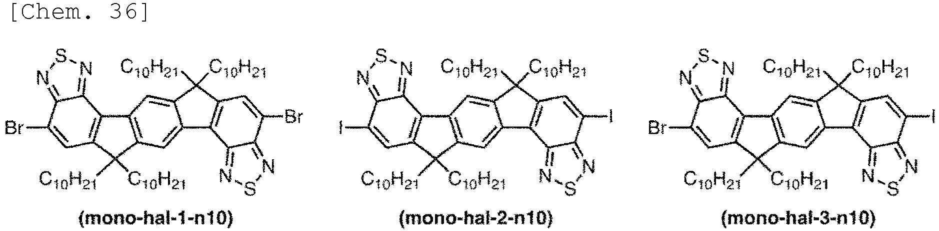

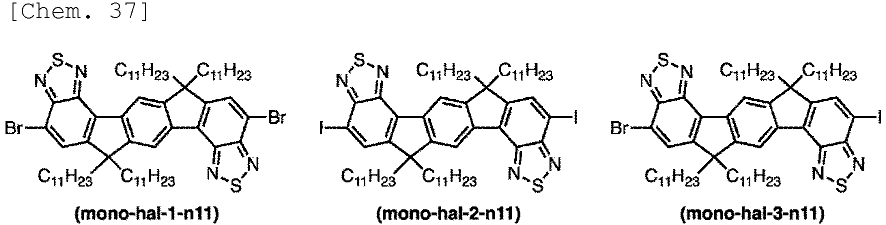

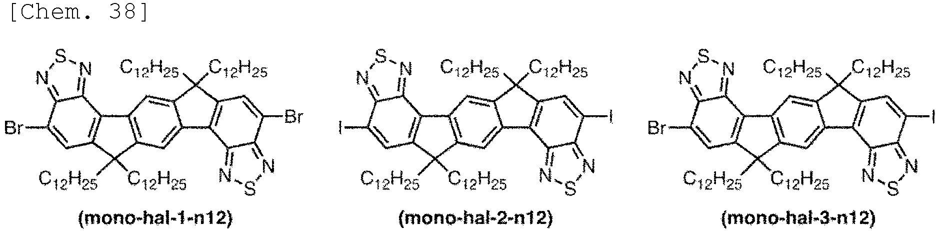

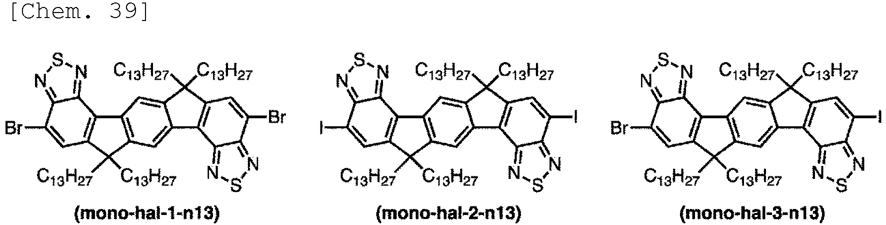

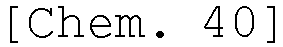

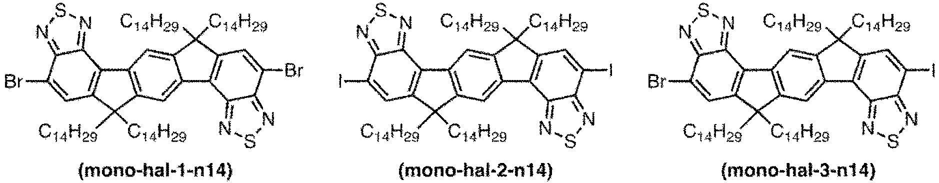

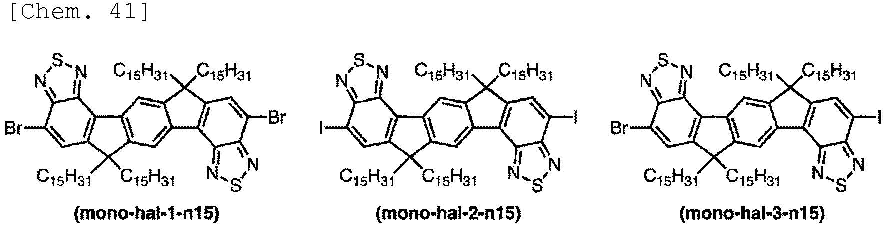

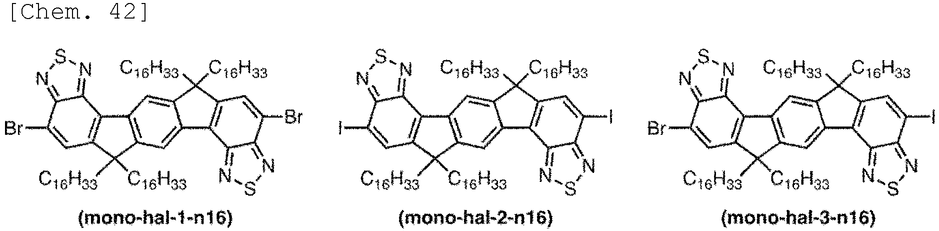

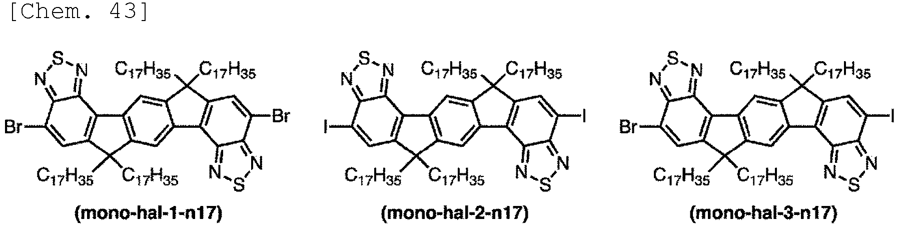

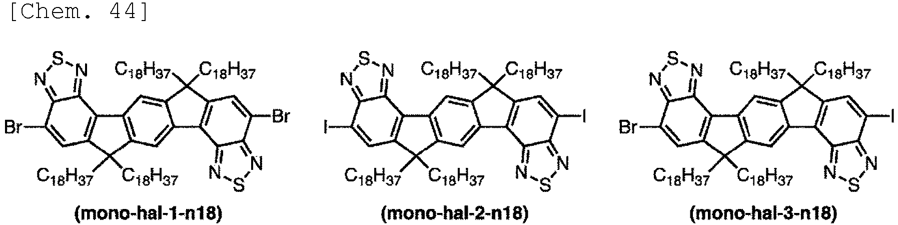

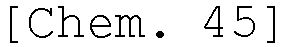

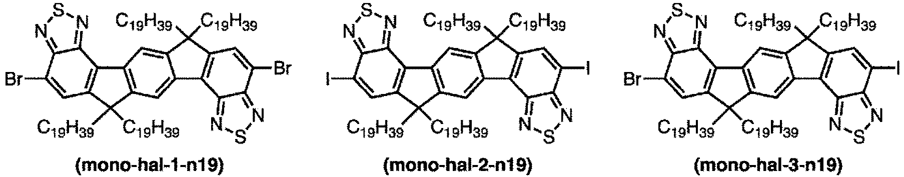

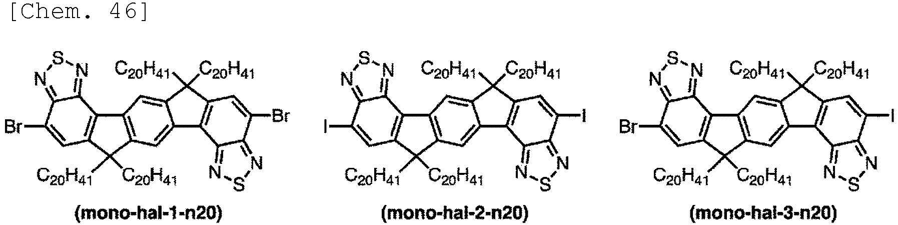

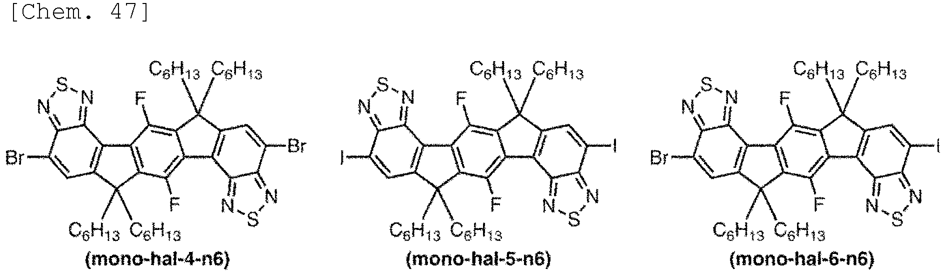

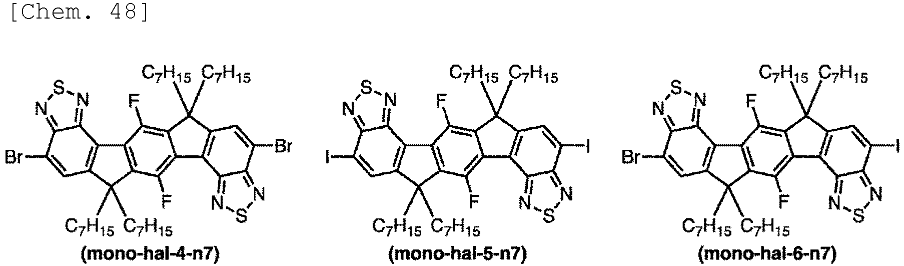

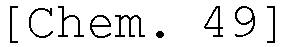

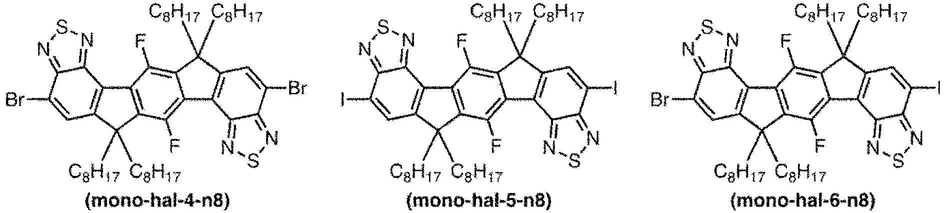

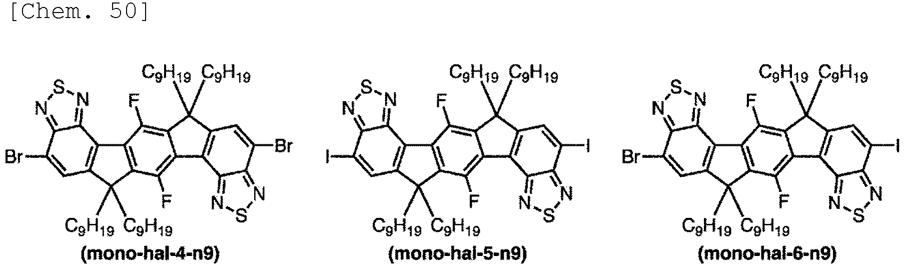

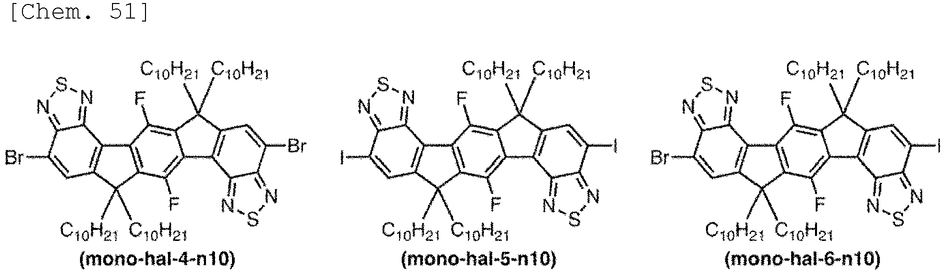

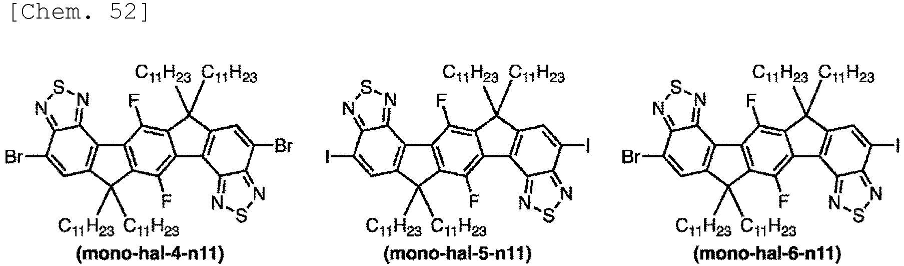

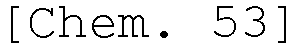

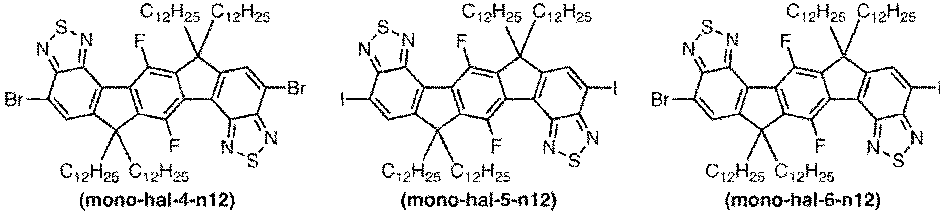

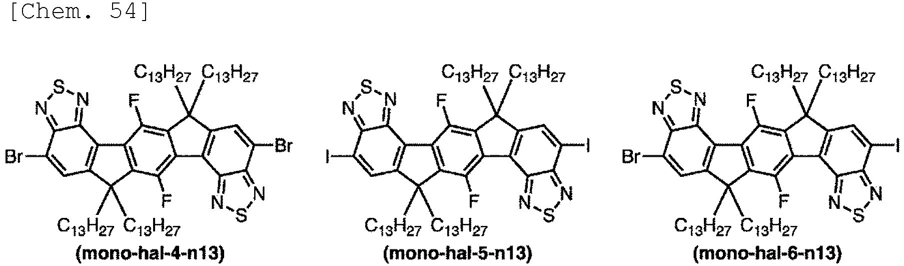

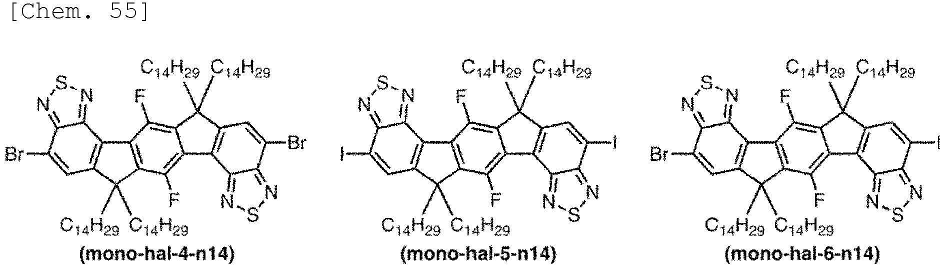

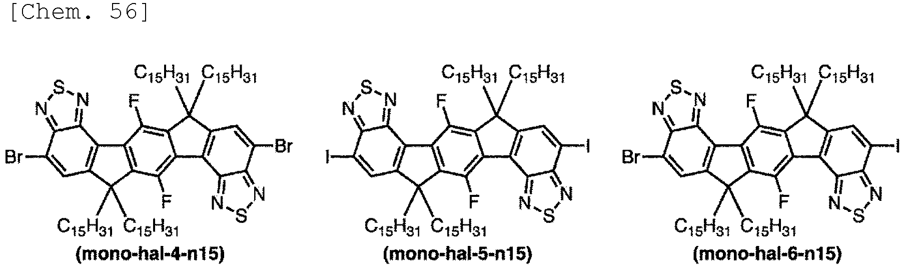

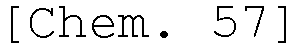

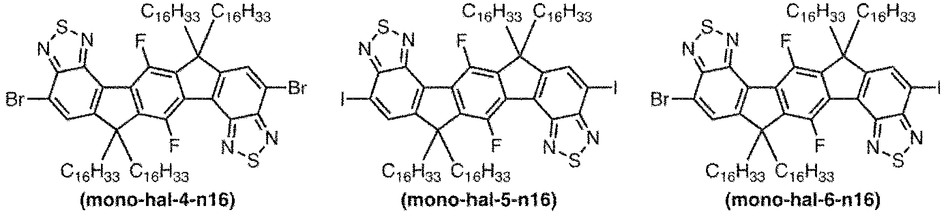

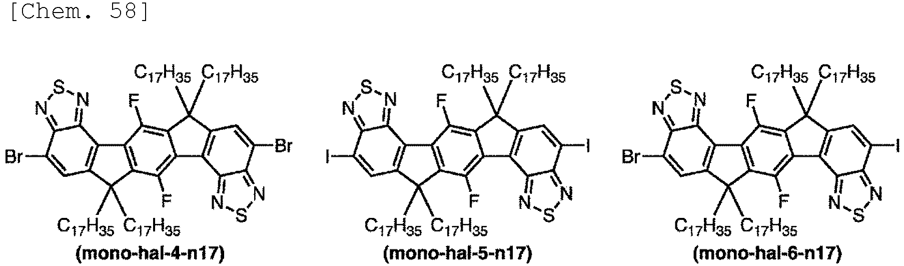

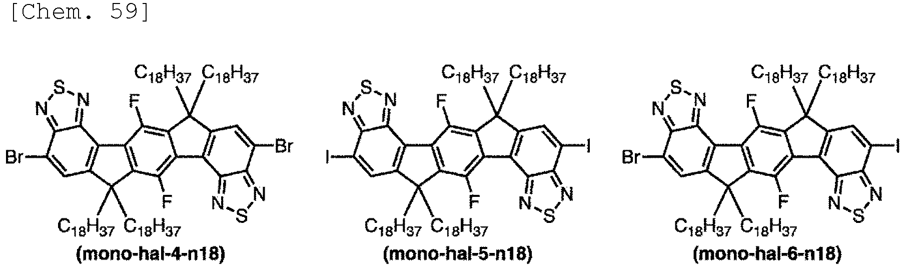

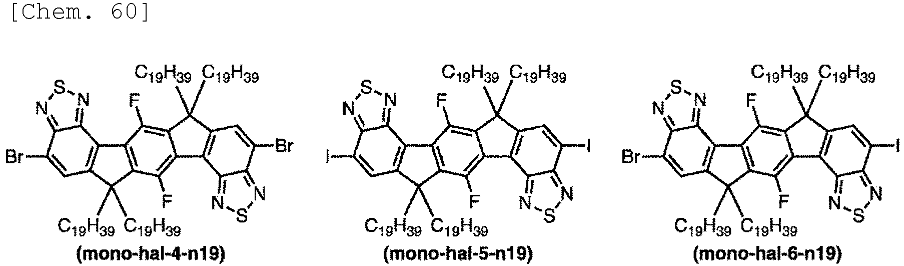

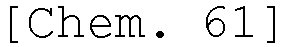

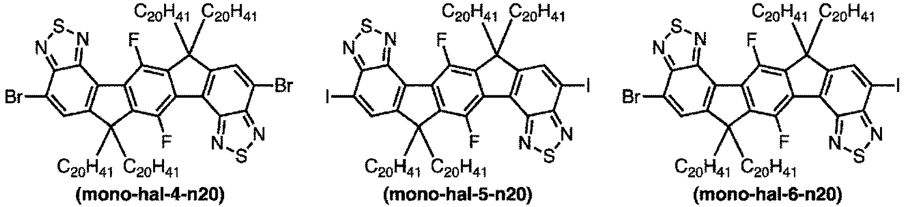

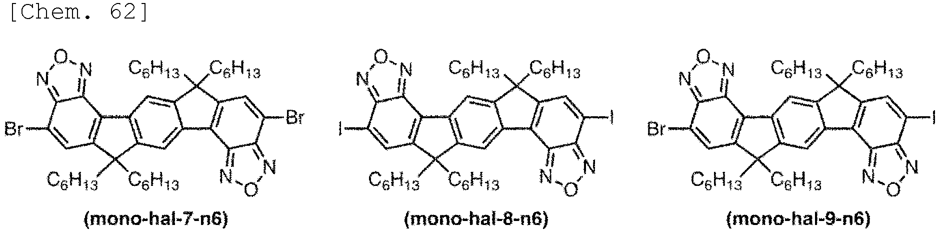

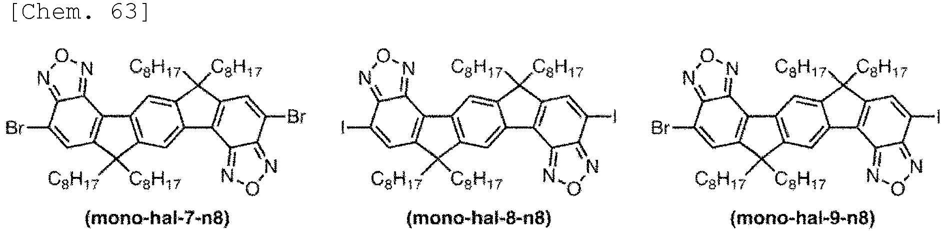

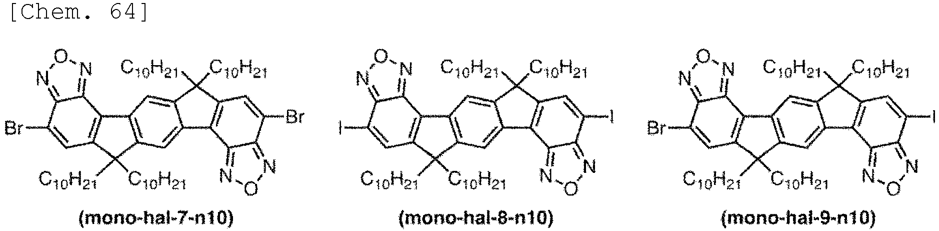

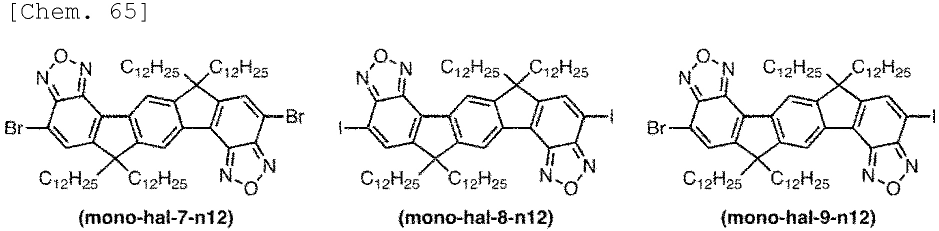

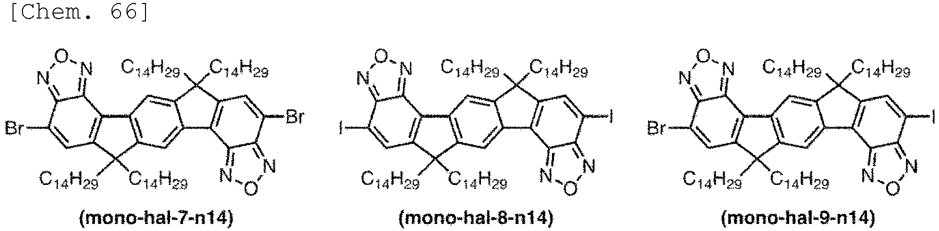

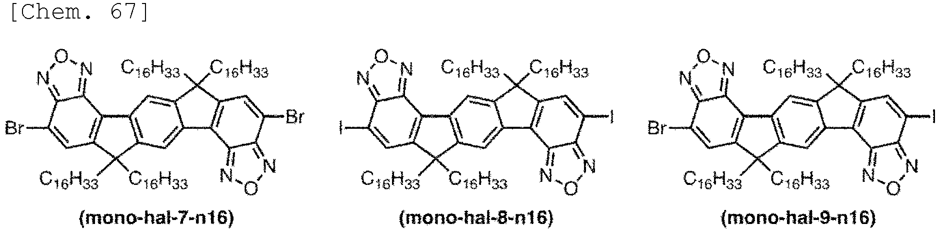

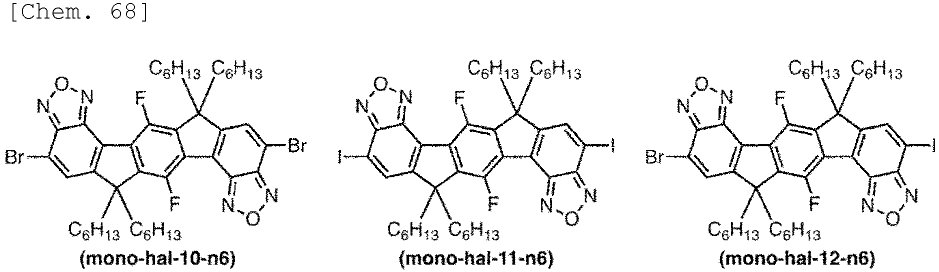

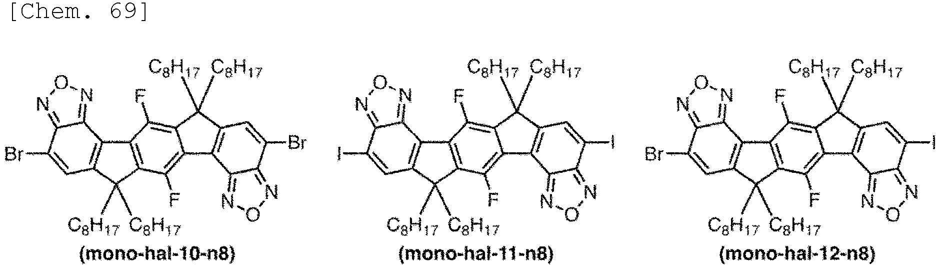

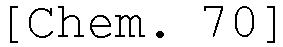

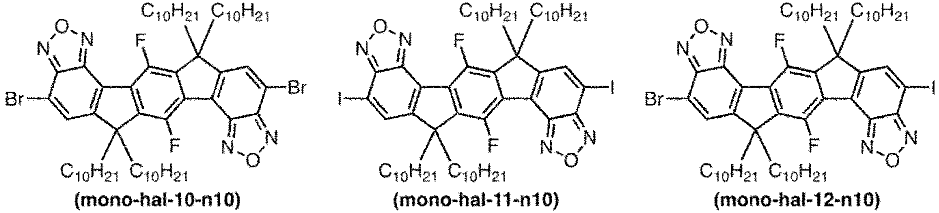

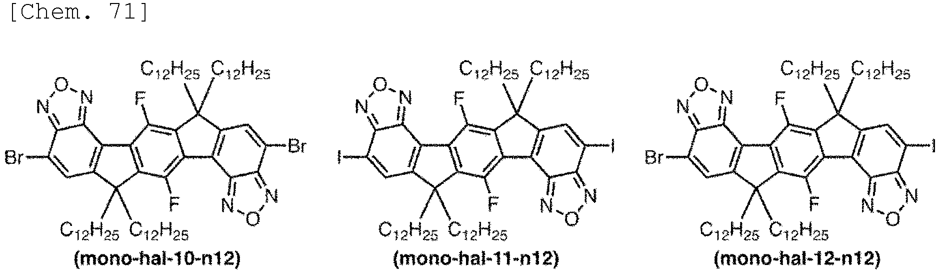

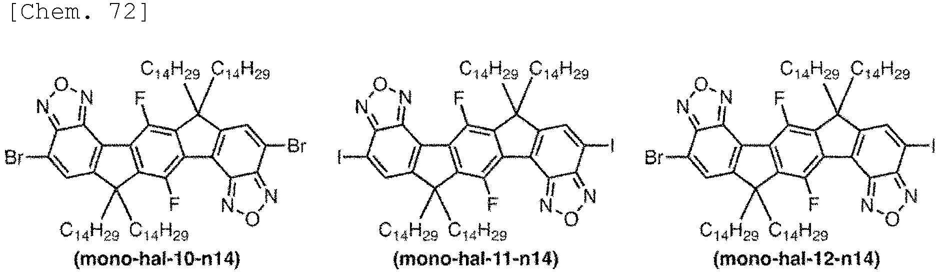

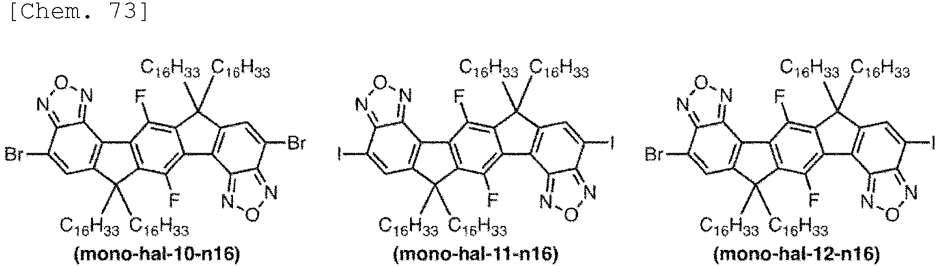

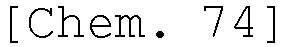

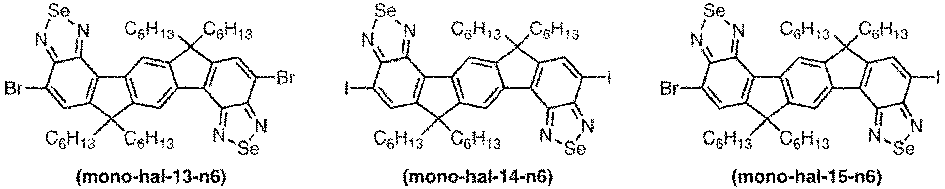

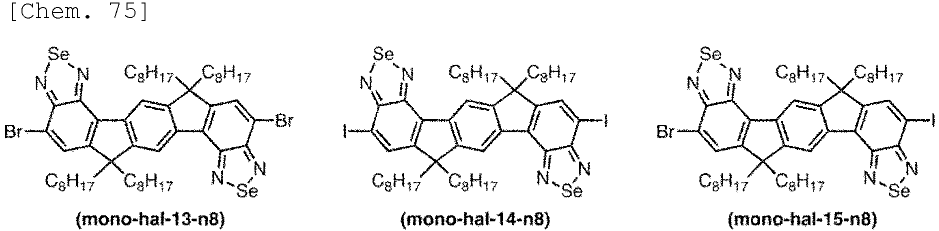

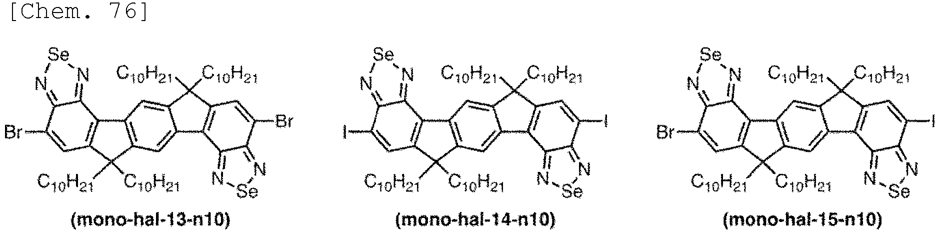

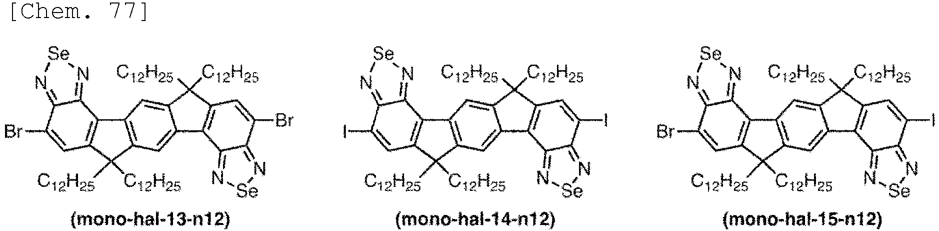

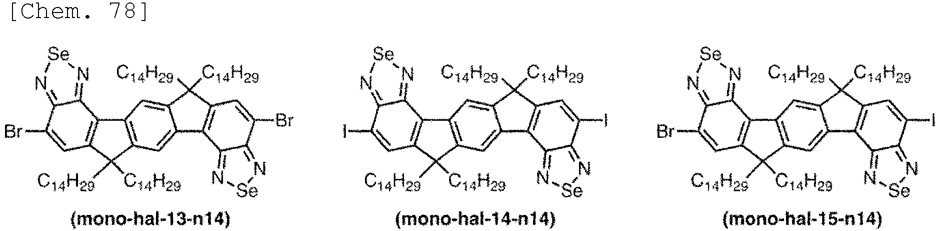

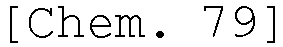

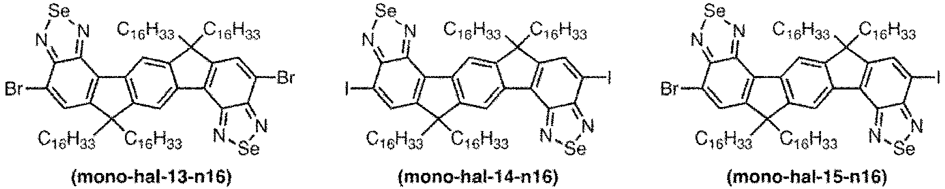

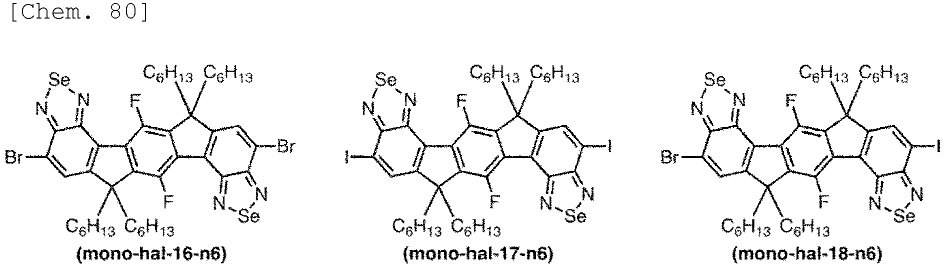

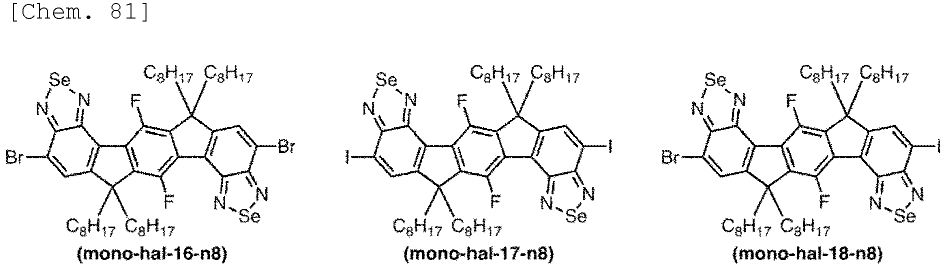

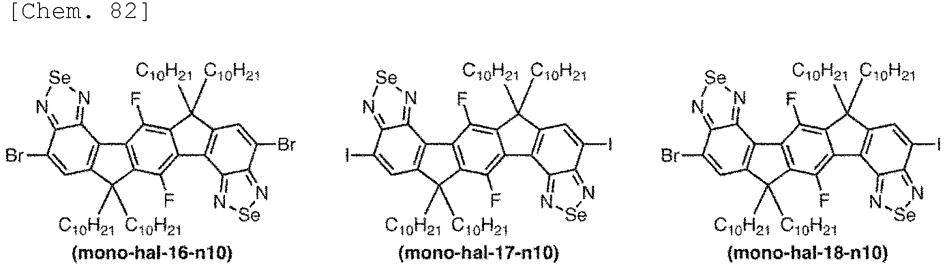

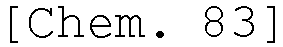

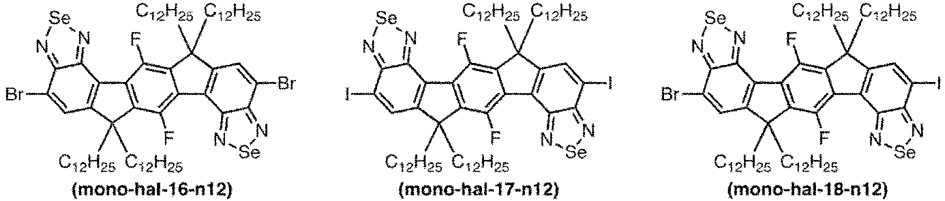

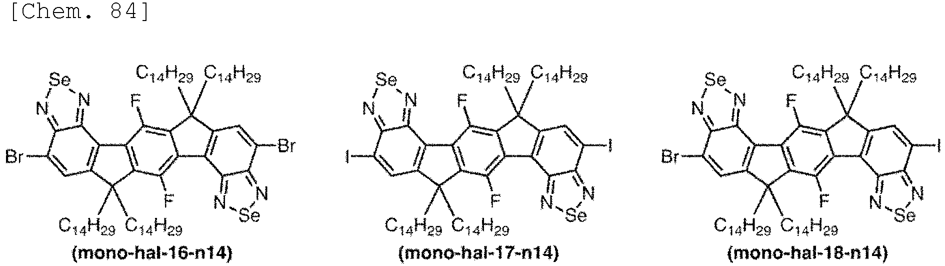

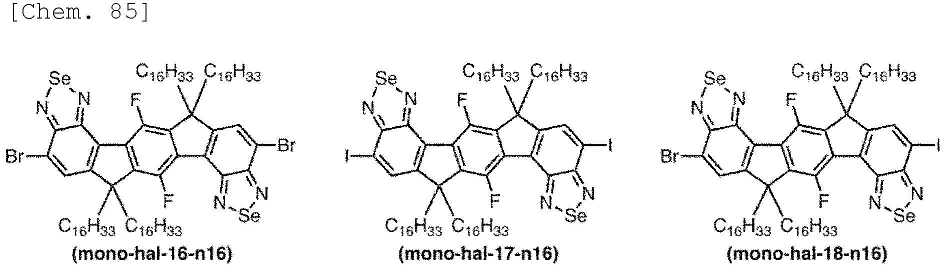

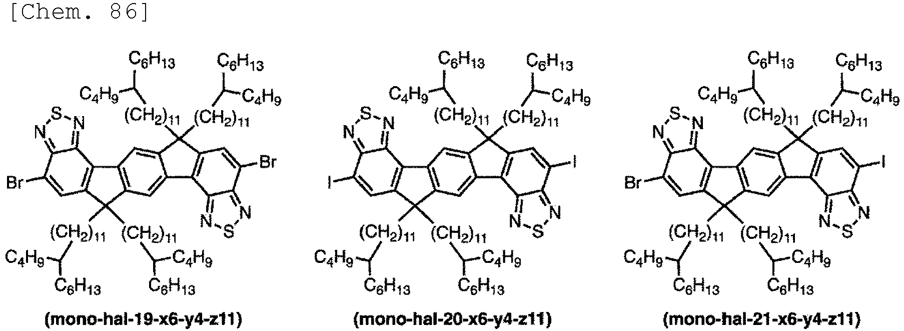

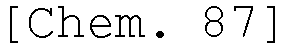

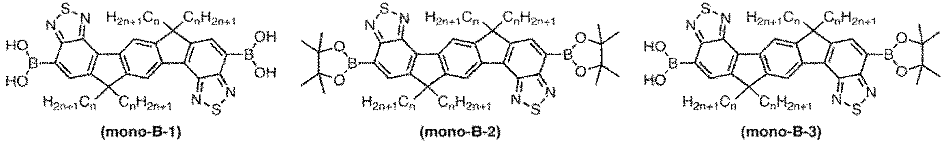

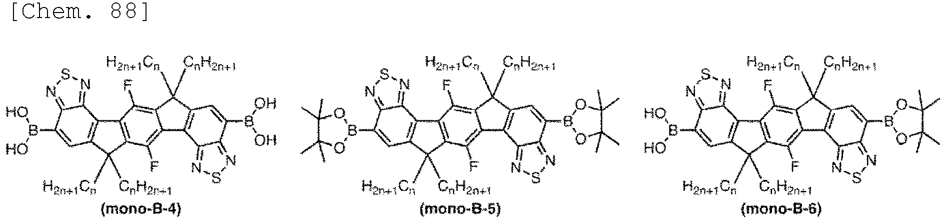

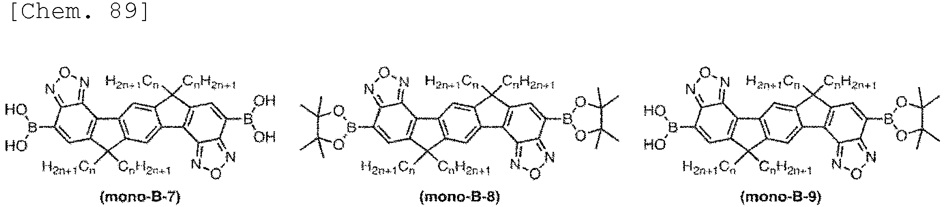

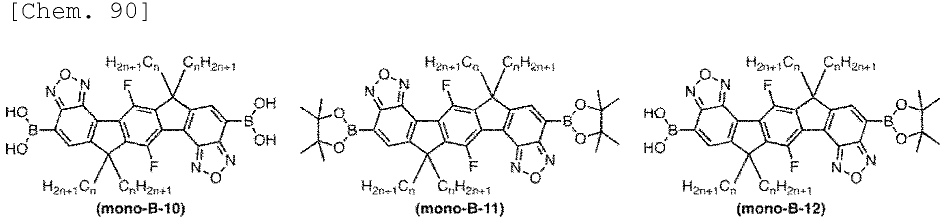

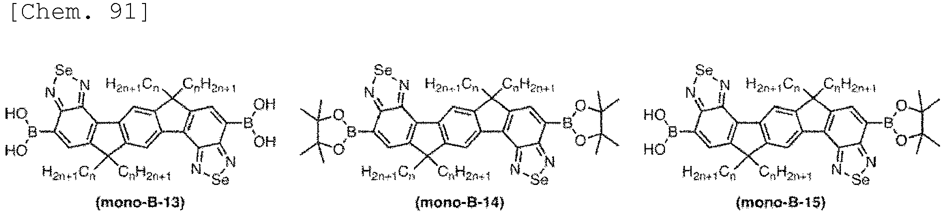

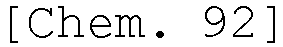

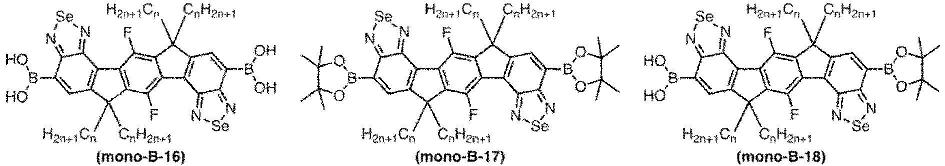

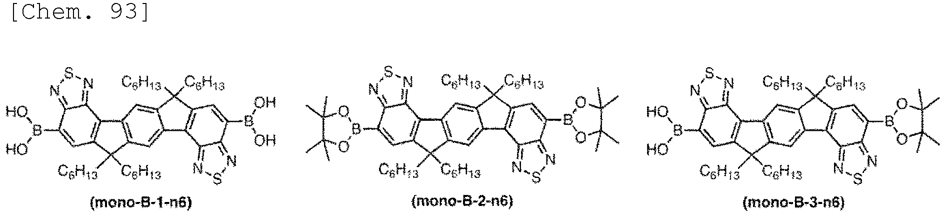

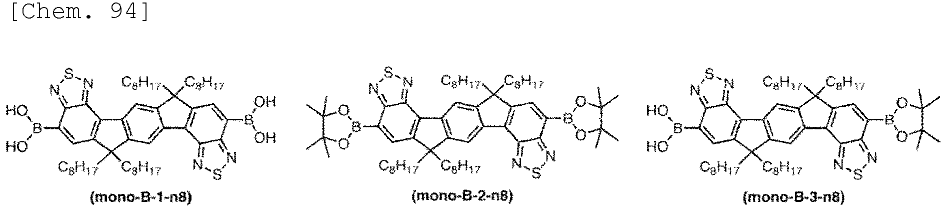

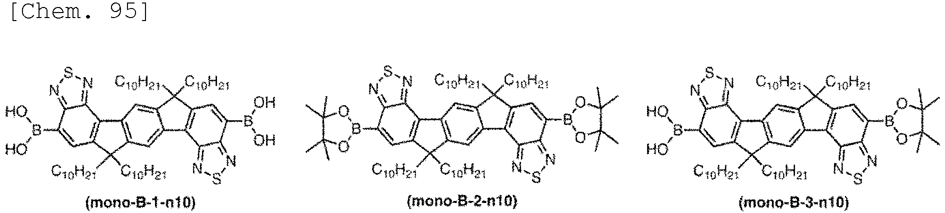

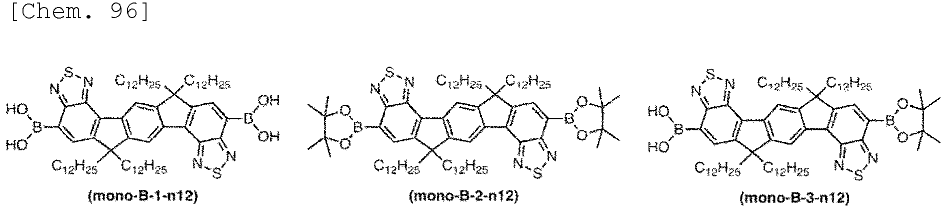

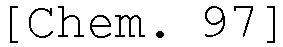

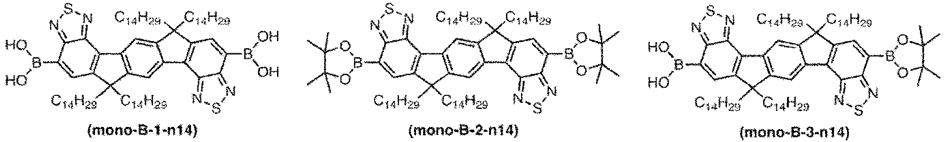

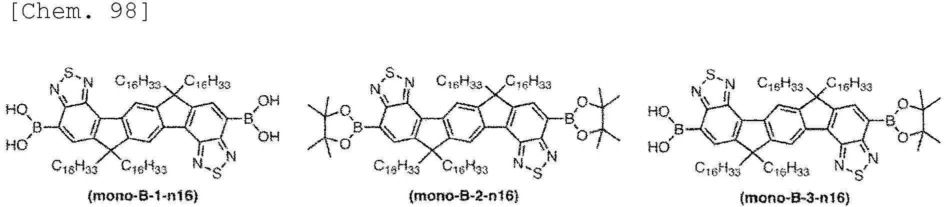

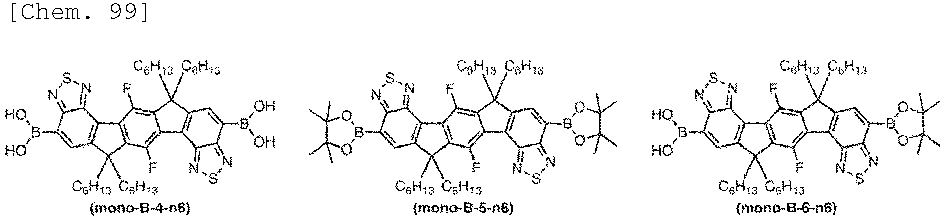

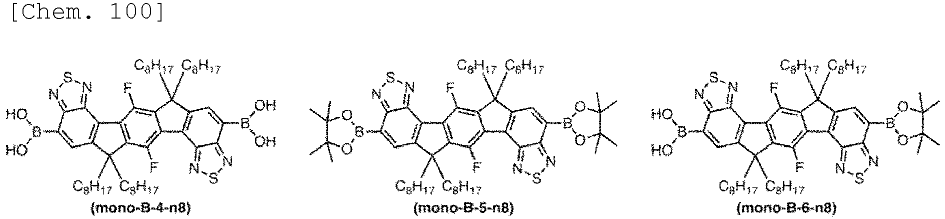

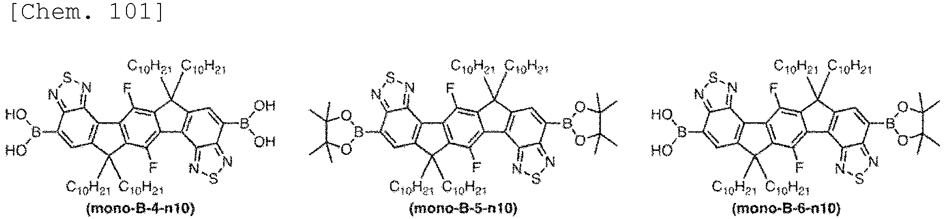

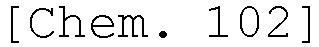

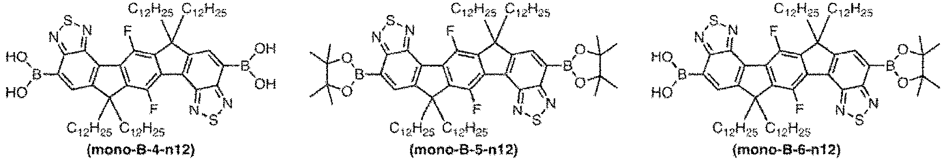

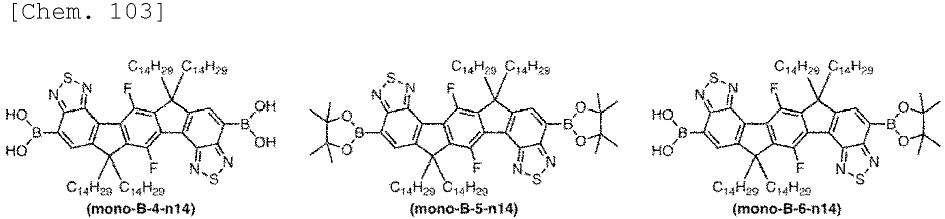

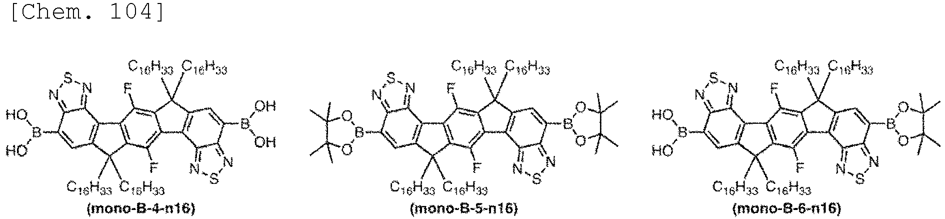

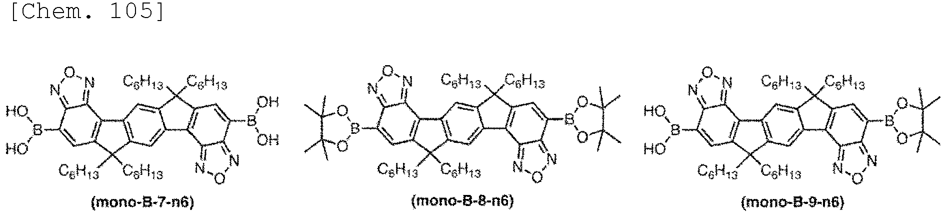

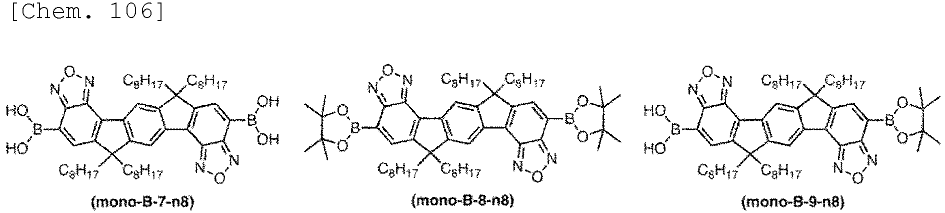

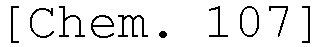

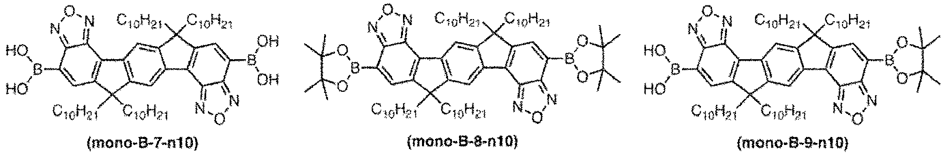

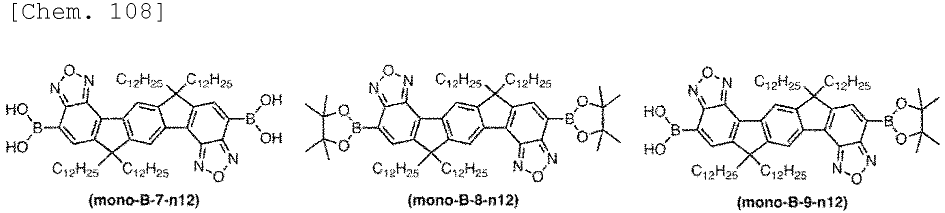

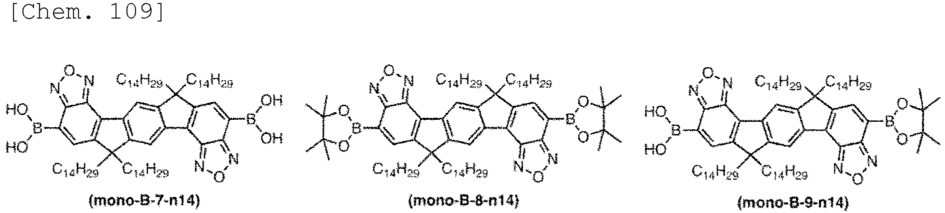

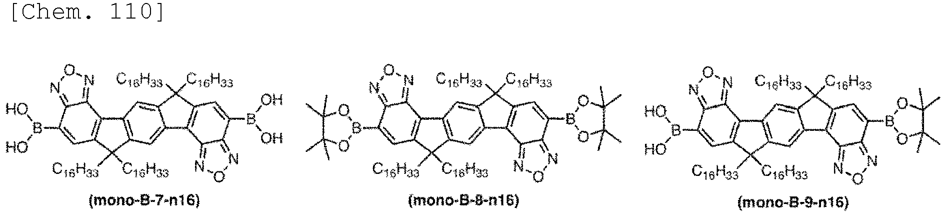

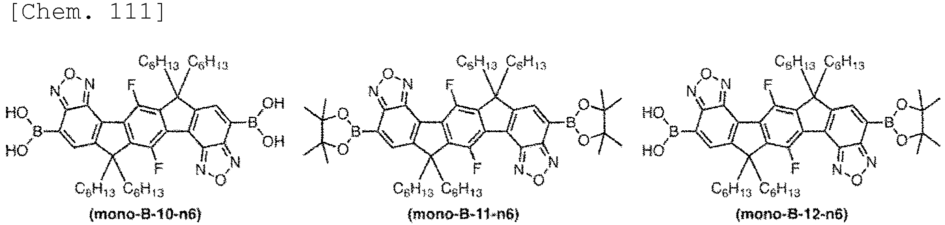

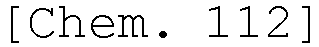

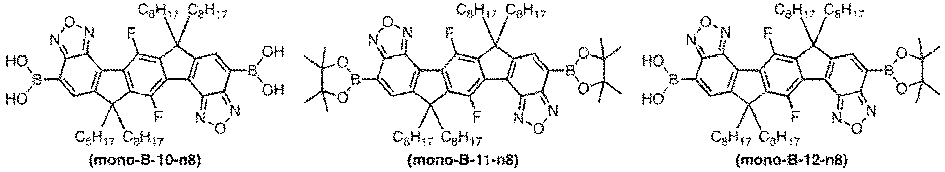

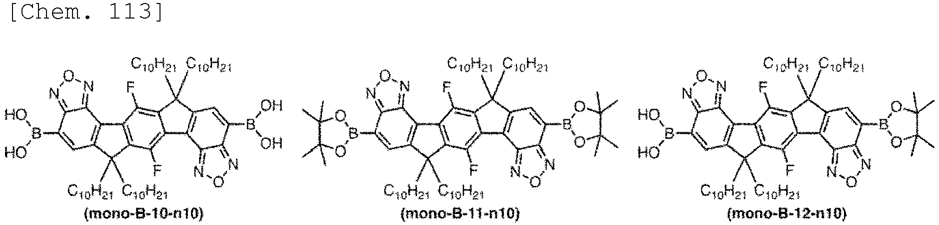

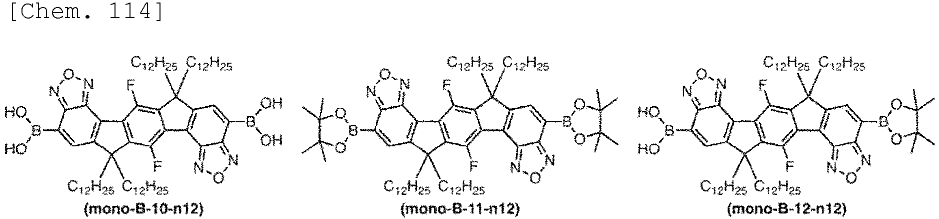

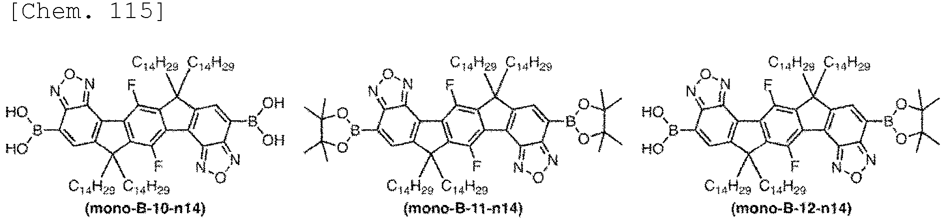

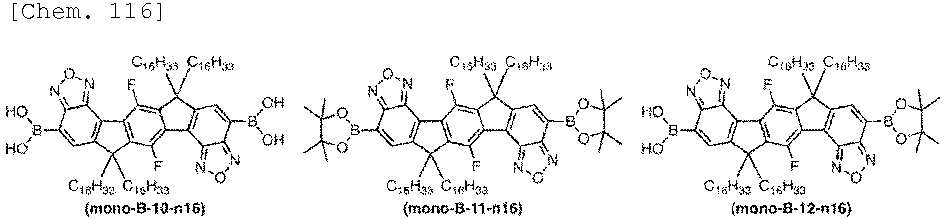

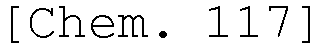

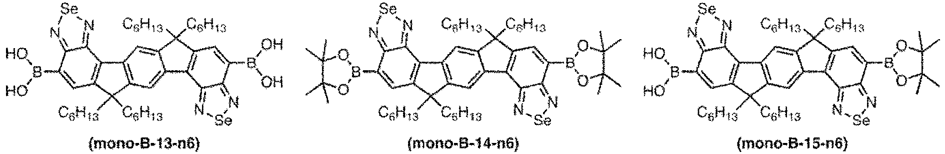

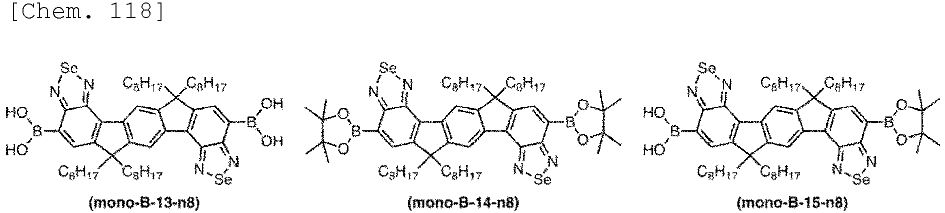

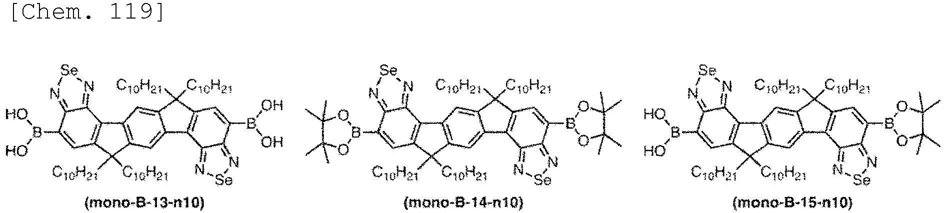

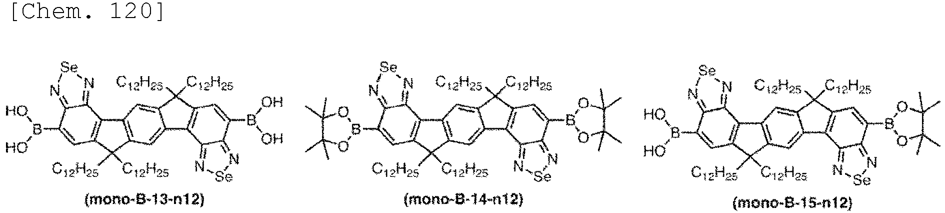

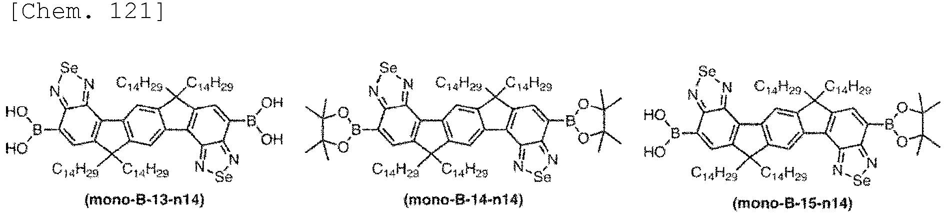

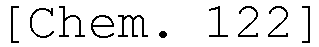

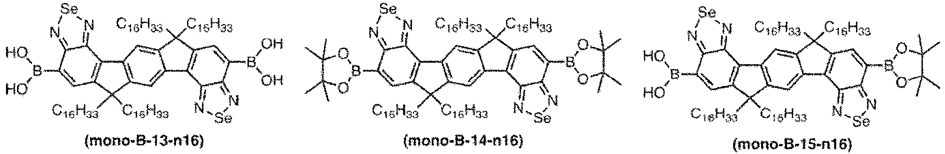

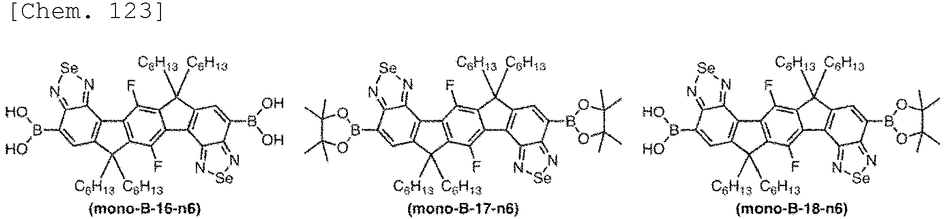

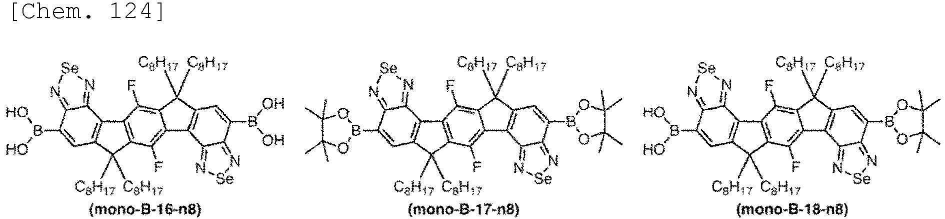

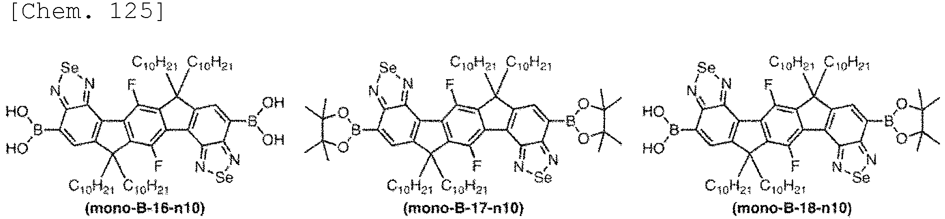

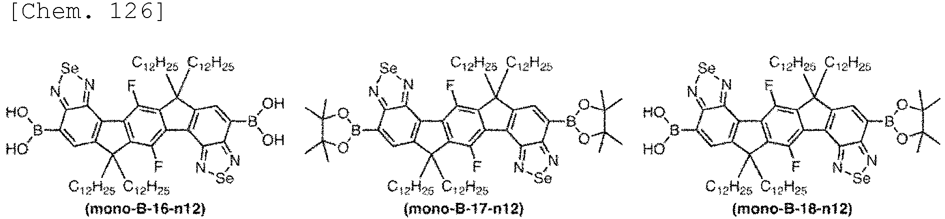

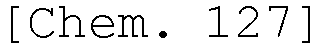

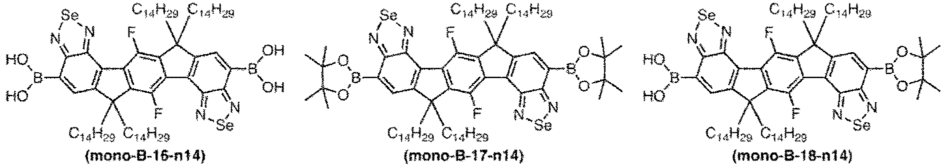

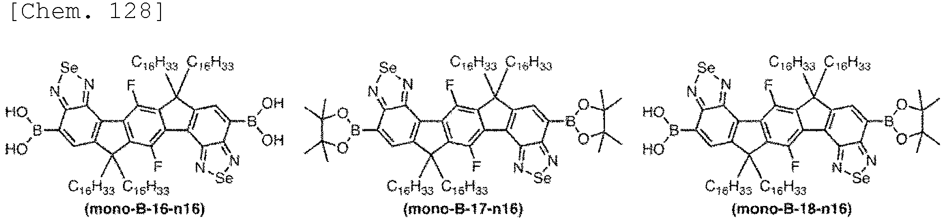

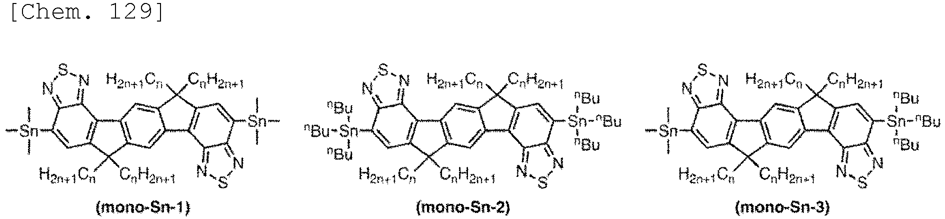

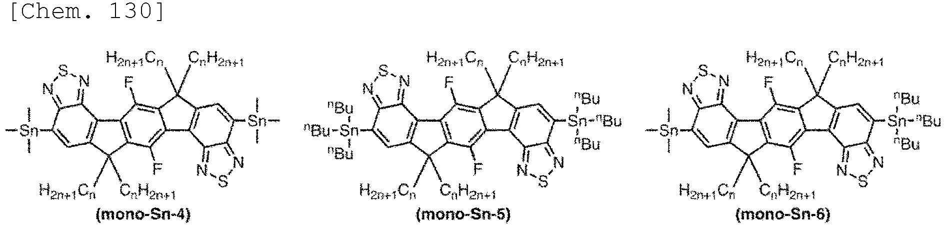

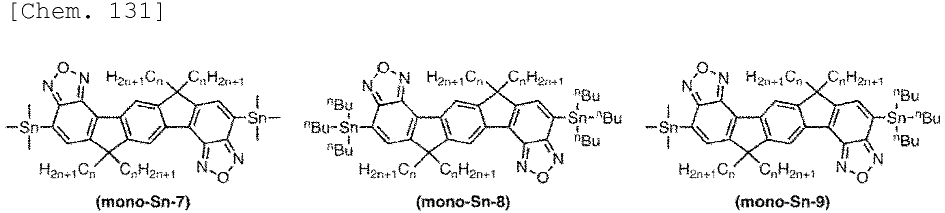

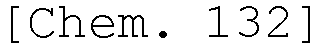

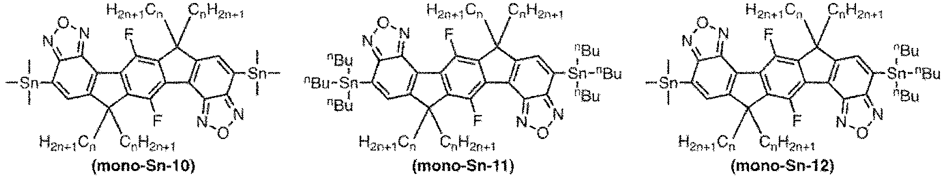

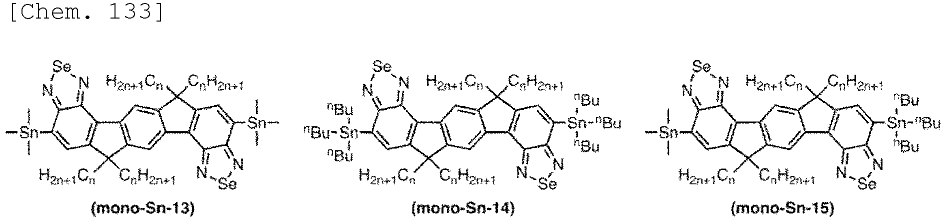

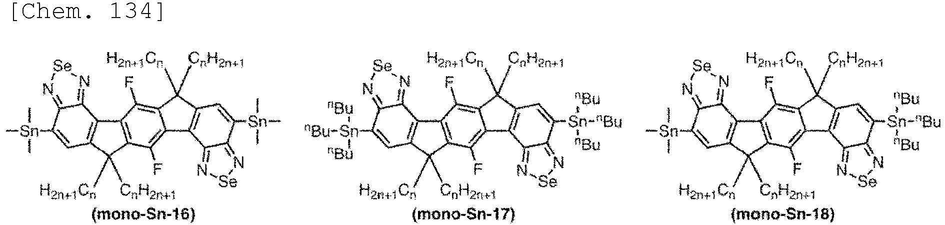

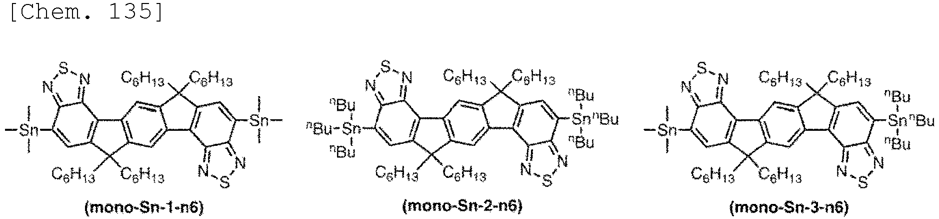

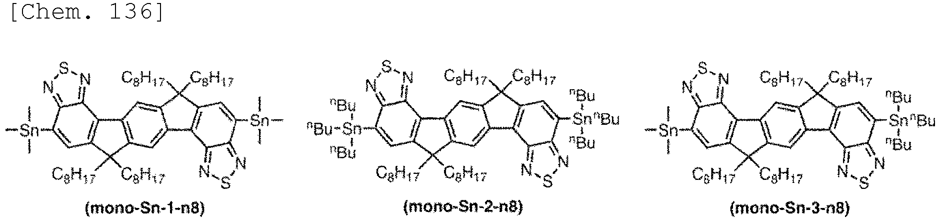

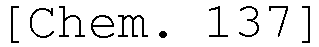

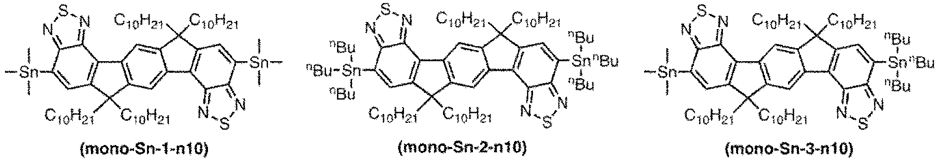

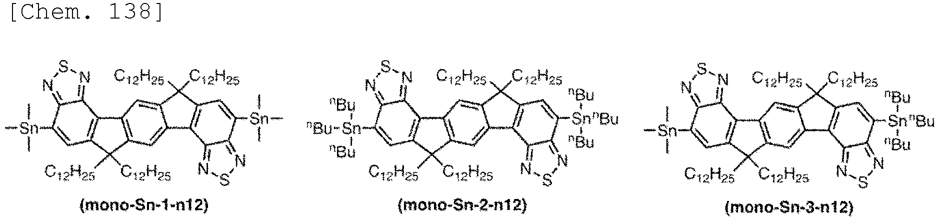

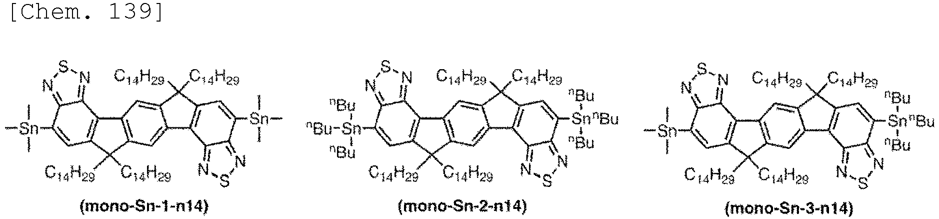

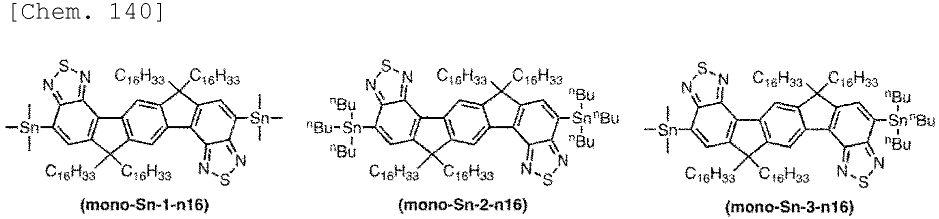

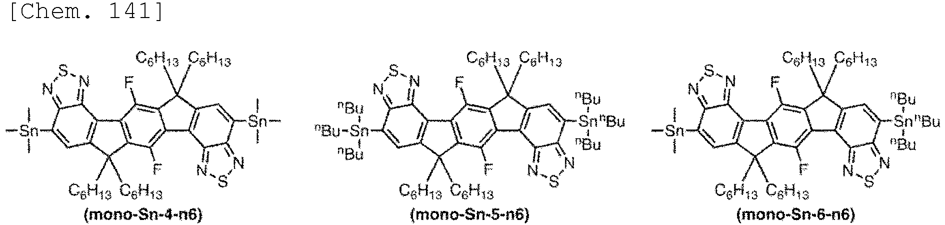

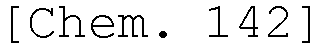

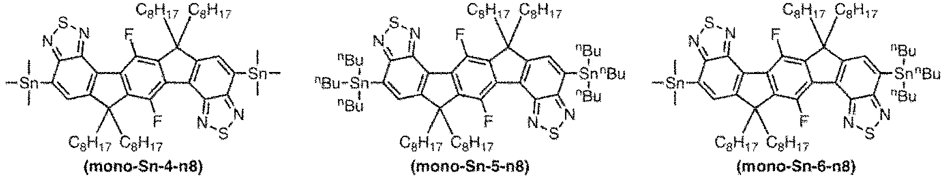

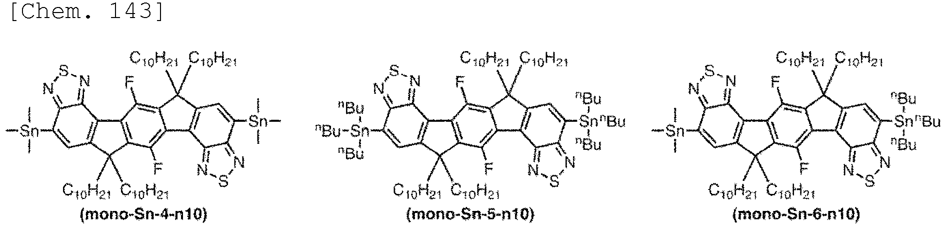

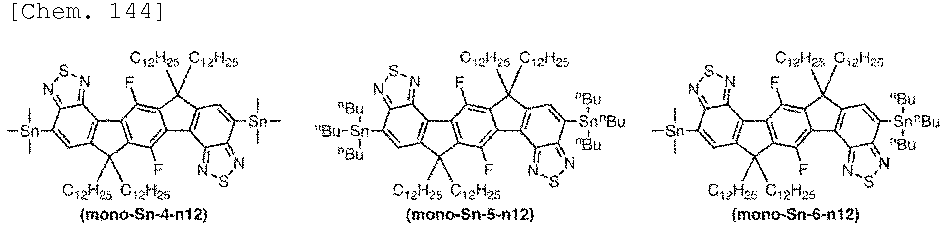

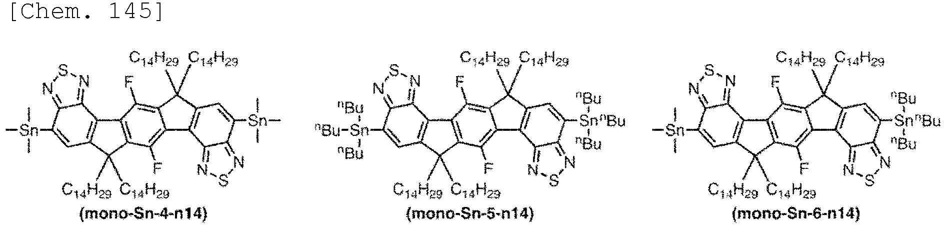

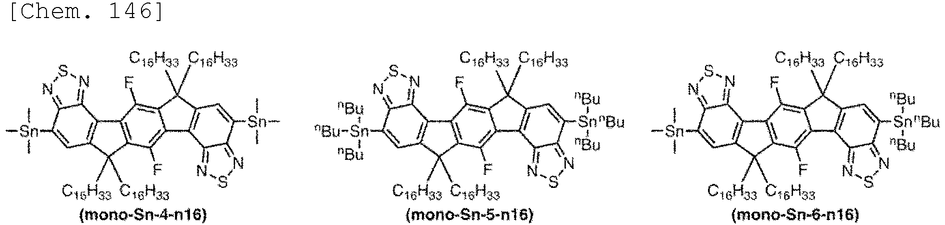

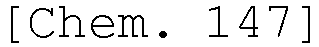

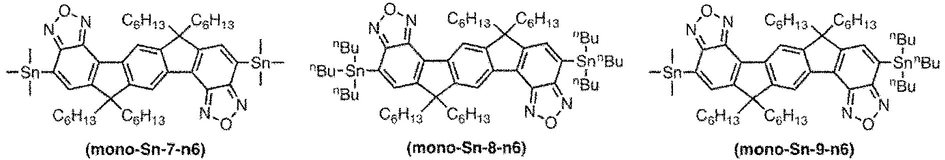

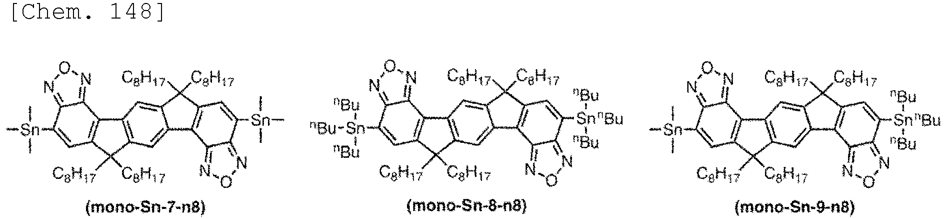

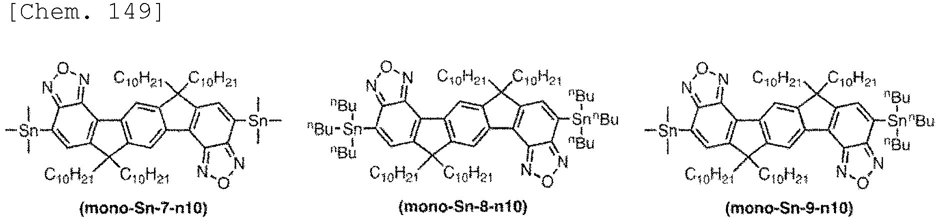

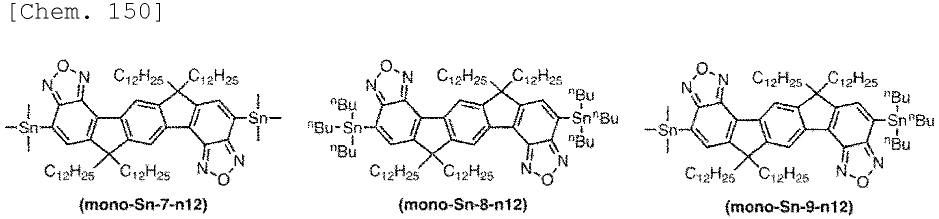

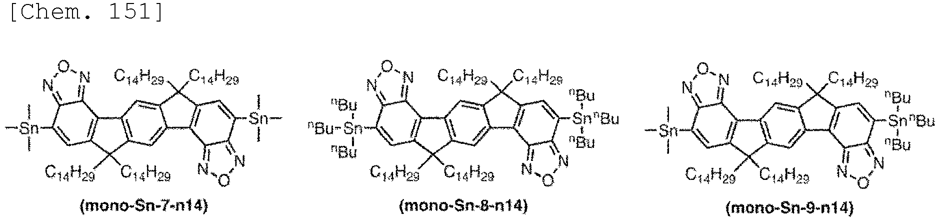

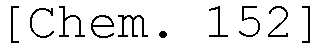

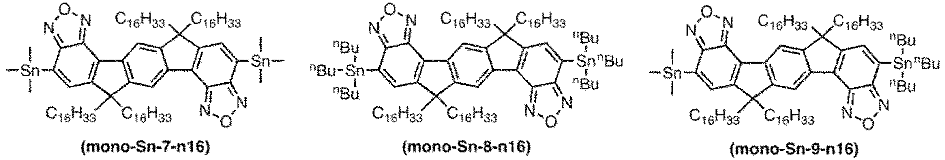

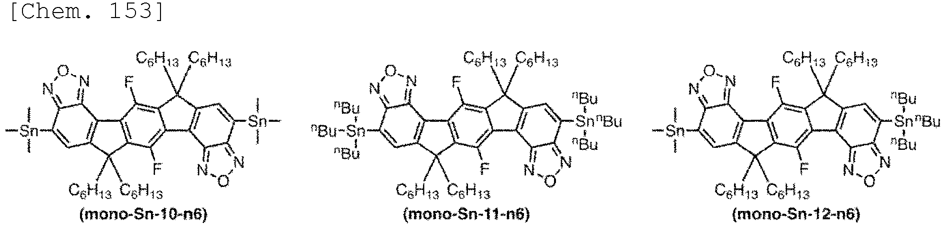

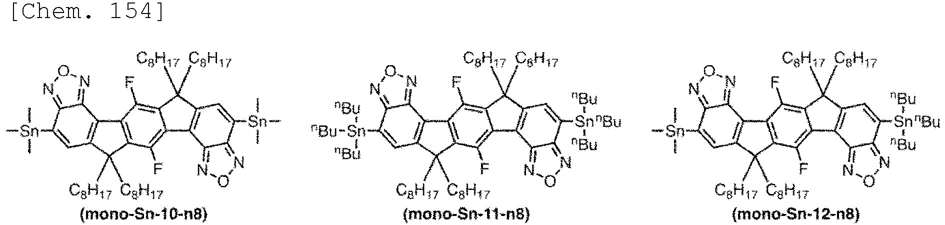

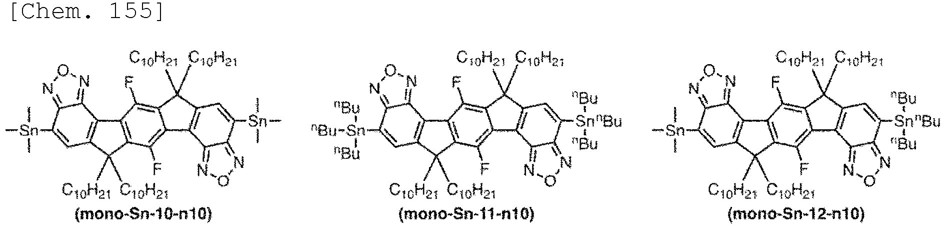

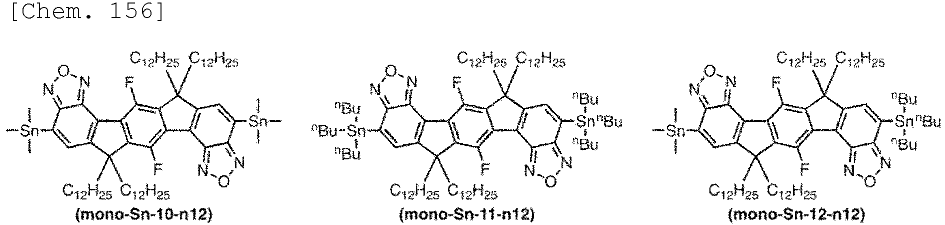

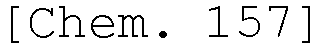

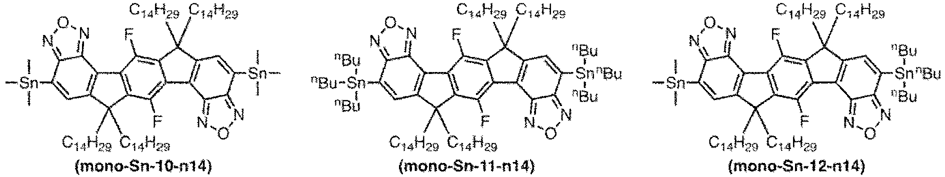

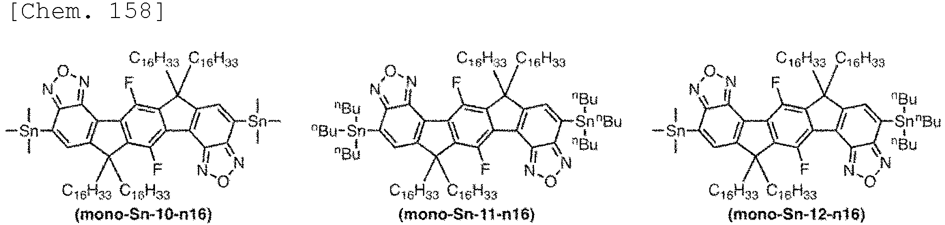

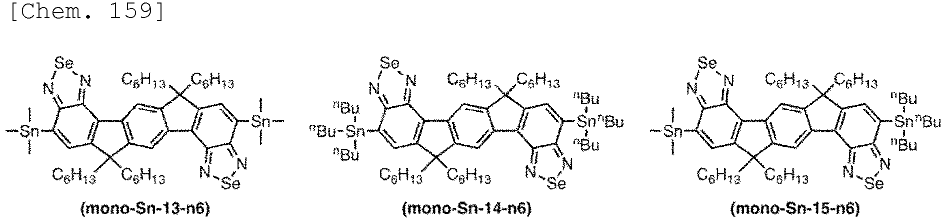

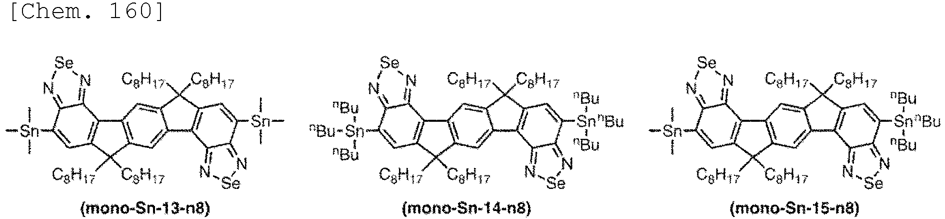

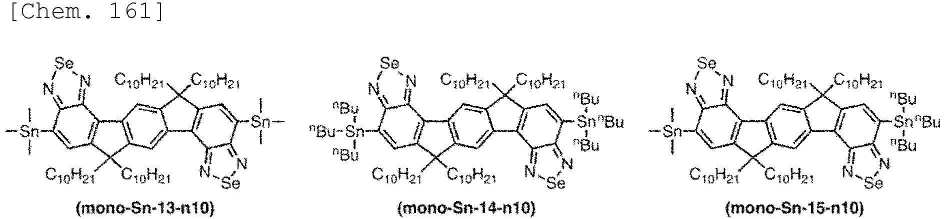

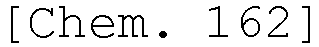

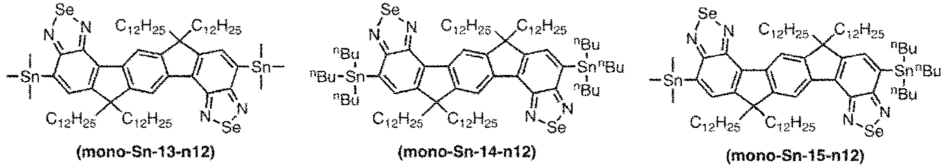

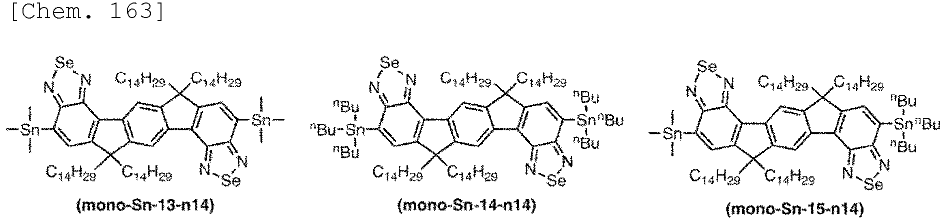

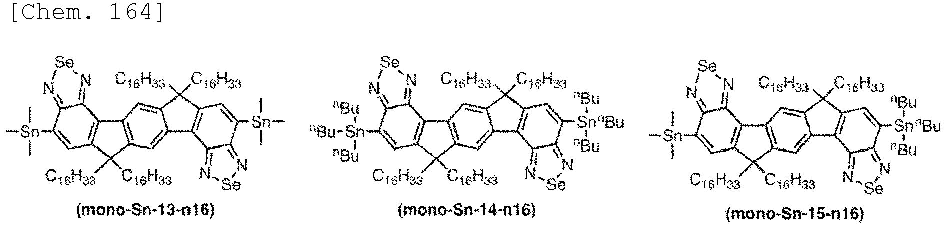

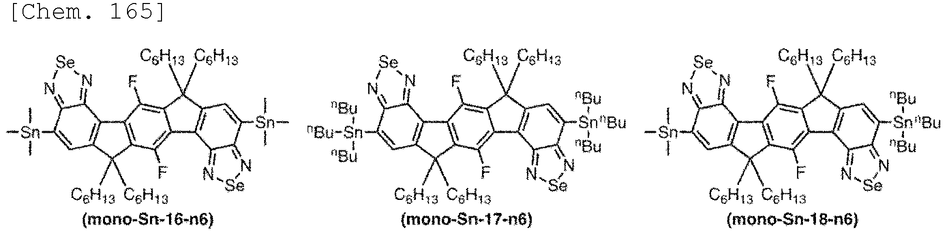

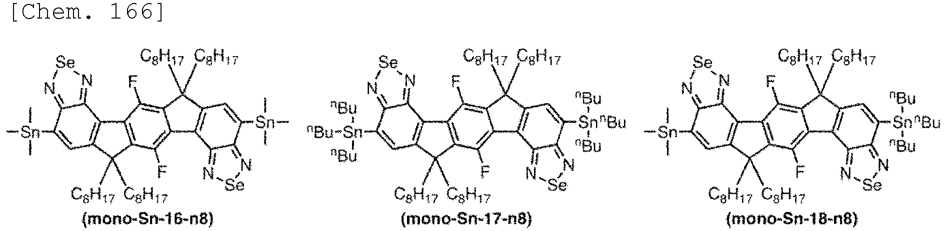

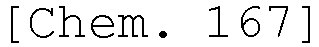

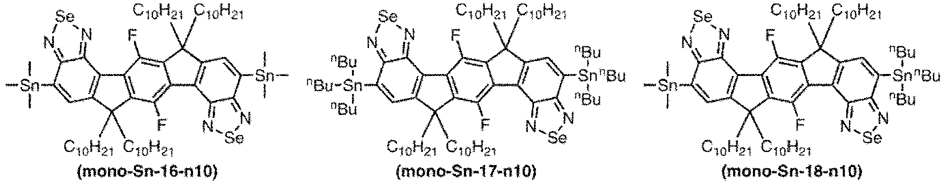

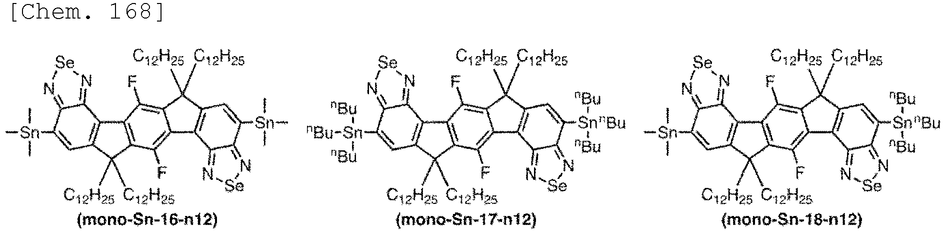

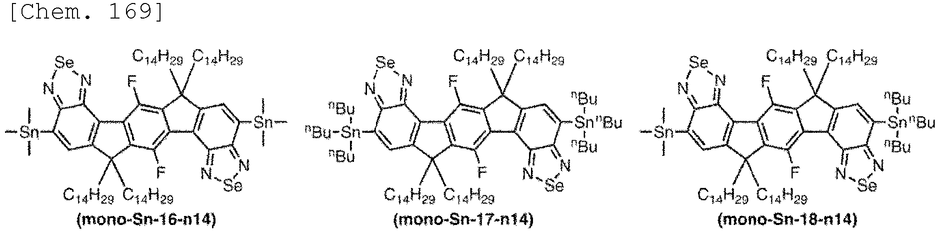

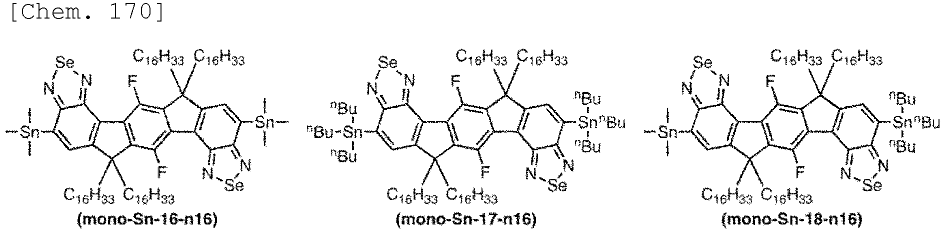

In these formulae, CₙH₂ₙ₊₁, CₓH₂ₓ₊₁ and C_{y}H_{2y+1} represent linear-chain alkyl groups.

The compound in this embodiment is preferably any compound represented by formula (mono-hal-1) to formula (mono-hal-6), formula (mono-hal-1-n6) to formula (mono-hal-3-n20) or formula (mono-hal-4-n6) to formula (mono-hal-6-n20) because this leads to higher film-forming properties and carrier mobility of conjugated polymers made using the compound. More preferably, the compound is any compound represented by formula (mono-hal-1-n6), formula (mono-hal-1-n7), formula (mono-hal-1-n8), formula (mono-hal-1-n9), formula (mono-hal-1-n10), formula (mono-hal-1-n11), formula (mono-hal-1-n12), formula (mono-hal-1-n13), formula (mono-hal-1-n14), formula (mono-hal-1-n15), formula (mono-hal-1-n16), formula (mono-hal-1-n17), formula (mono-hal-1-n18), formula (mono-hal-1-n19), formula (mono-hal-1-n20), formula (mono-hal-4-n6), formula (mono-hal-4-n7), formula (mono-hal-4-n8), formula (mono-hal-4-n9), formula (mono-hal-4-n10), formula (mono-hal-4-n11), formula (mono-hal-4-n12), formula (mono-hal-4-n13), formula (mono-hal-4-n14), formula (mono-hal-4-n15), formula (mono-hal-4-n16), formula (mono-hal-4-n17), formula (mono-hal-4-n18), formula (mono-hal-4-n19) or formula (mono-hal-4-n20), even more preferably any compound represented by formula (mono-hal-1-n6), formula (mono-hal-1-n7), formula (mono-hal-1-n8), formula (mono-hal-1-n9), formula (mono-hal-1-n10), formula (mono-hal-1-n11), formula (mono-hal-1-n12), formula (mono-hal-1-n13), formula (mono-hal-1-n14), formula (mono-hal-1-n15), formula (mono-hal-1-n16), formula (mono-hal-1-n17), formula (mono-hal-1-n18), formula (mono-hal-1-n19) or formula (mono-hal-1-n20), especially preferably any compound represented by formula (mono-hal-1-n8), formula (mono-hal-1-n9), formula (mono-hal-1-n10), formula (mono-hal-1-n11), formula (mono-hal-1-n12), formula (mono-hal-1-n14), formula (mono-hal-1-n15), formula (mono-hal-1-n16) or formula (mono-hal-1-n18).

### [Methods for Producing the Compound]

Methods for producing a compound in this embodiment will now be described. The methods for producing a compound in this embodiment are as indicated in production processes (a) and (b) presented below.

### <Production Process (a)>

Production process (a) is a method in which a compound represented by general formula (mono-H) is produced by generating an anionic species corresponding to a compound represented by general formula (int-1) using a base such as an organometallic reagent and allowing the anionic species to react with a halogenated alkyl.

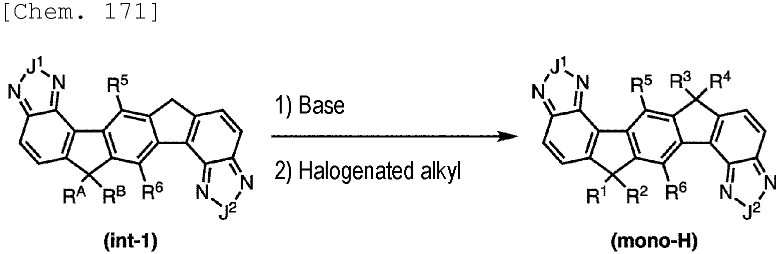

(In the formula, R¹, R², R³, R⁴, R⁵, R⁶, J¹ and J² have the same meanings as described above. R^{A} and R^{B} represent hydrogen atoms or C1 to C50 alkyl groups. R^{A} and R^{B} may be bound to each other to form a ring together with the carbon atom to which they are bound.)

C1 to C50 alkyl groups represented by R^{A} and R^{B} may be any of linear-chain, branched or cyclic. Examples include linear-chain alkyl groups, such as a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a heneicosyl group, a docosyl group, a tricosyl group, a tetracosyl group, a pentacosyl group, a hexacosyl group, a heptacosyl group, an octacosyl group, a nonacosyl group, a triacontyl group, a hentriacontyl group, a dotriacontyl group, a tritriacontyl group, a tetratriacontyl group, a pentatriacontyl group, a hexatriacontyl group, a tetracontyl group, a hentetracontyl group, a dotetracontyl group, a tritetracontyl group, a tetratetracontyl group and a pentacontyl group; branched alkyl groups, such as an isopropyl group, an isobutyl group, a sec-butyl group, a tert-butyl group, a 2-ethylhexyl group, a 3,7-dimethyloctyl group, a 2-hexyloctyl group, a 2-hexyldecyl group, a 2-octyldodecyl group, a 2-decyltetradecyl group, a 2-dodecyltetradecyl group, a 2-dodecylhexadecyl group, a 2-tetradecylhexadecyl group, a 3-decylpentadecyl group, a 3-dodecylheptadecyl group, a 3-tetradecylnonacosyl group, a 4-decylhexadecyl group, a 4-dodecyloctadecyl group and a 4-tetradecylicosyl group; and cyclic alkyl groups, such as a cyclopentyl group and a cyclohexyl group.

Examples of compounds represented by general formula (int-1) include the following compounds. The present invention, however, is not limited to these. In the formulae, the ns represent natural numbers of 1 to 50 that may be the same or different from one another. Examples of specific compounds included in them are also presented below.

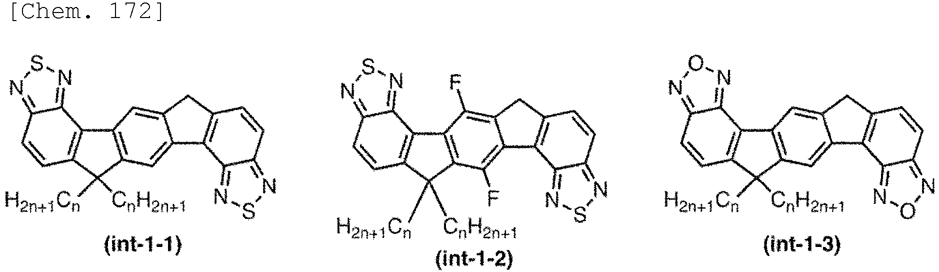

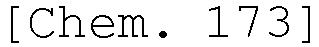

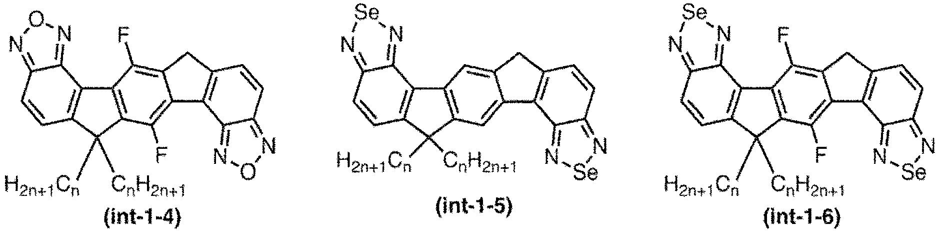

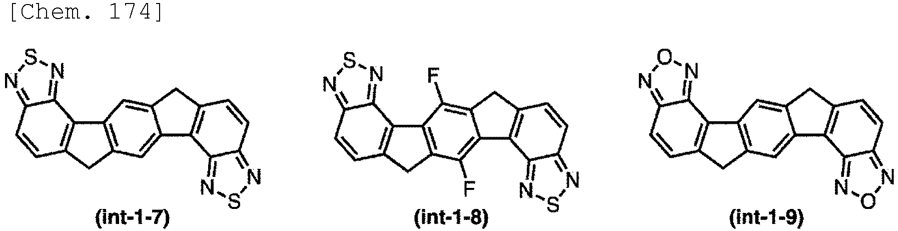

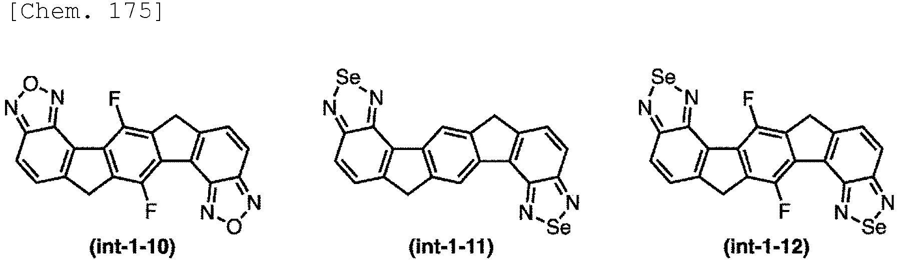

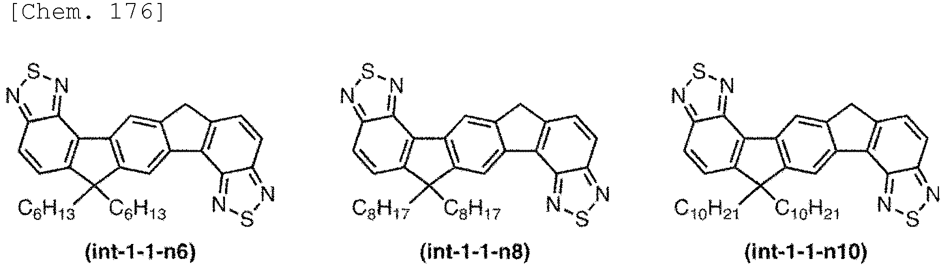

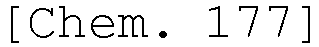

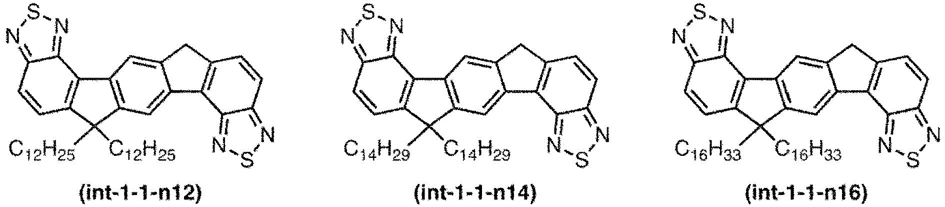

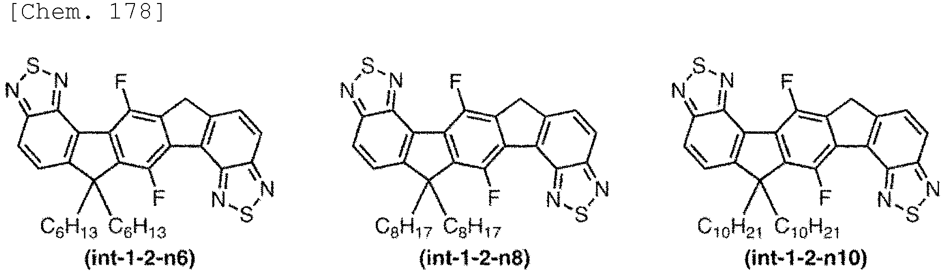

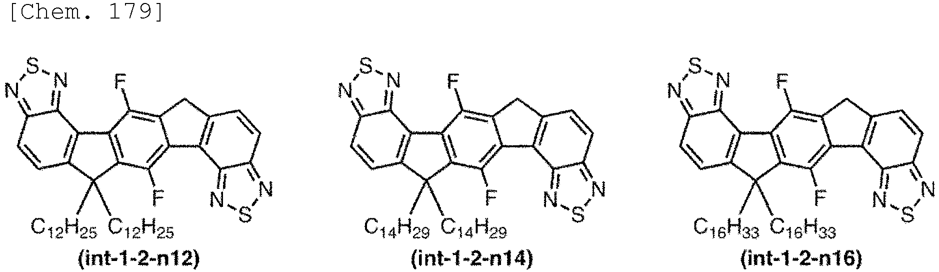

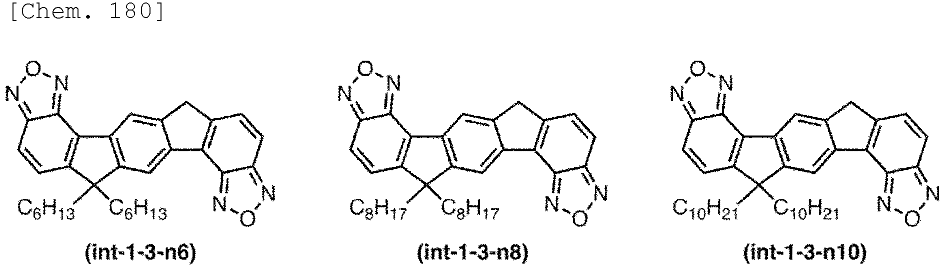

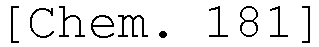

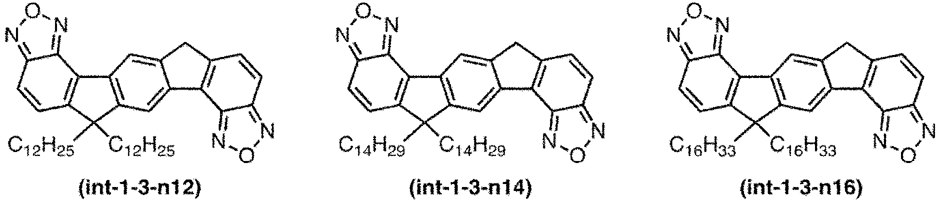

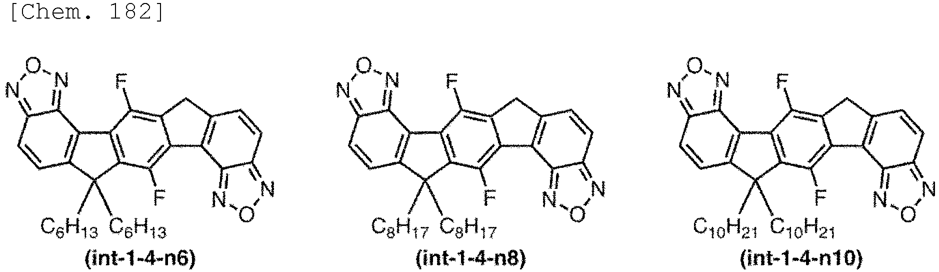

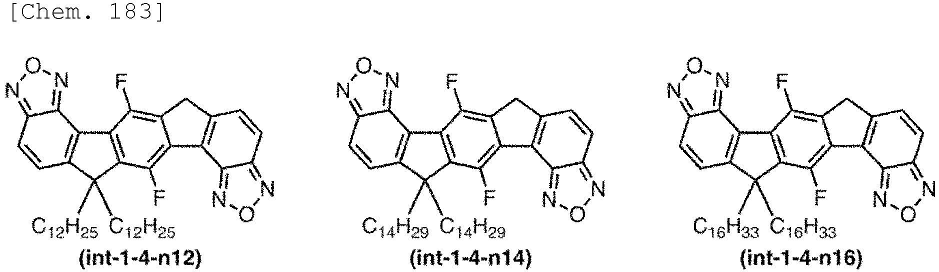

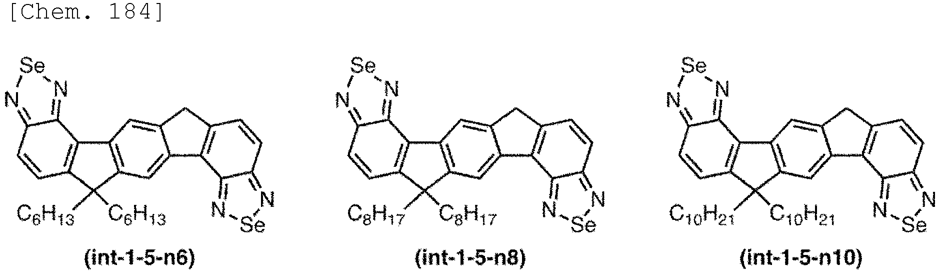

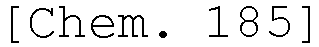

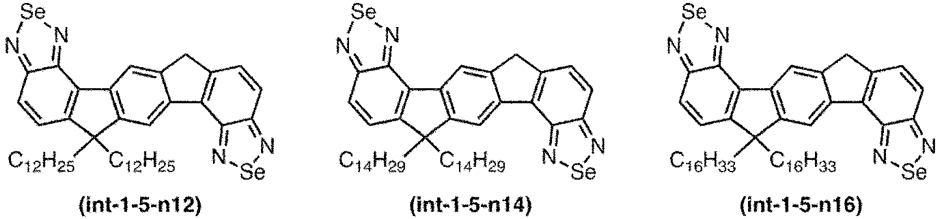

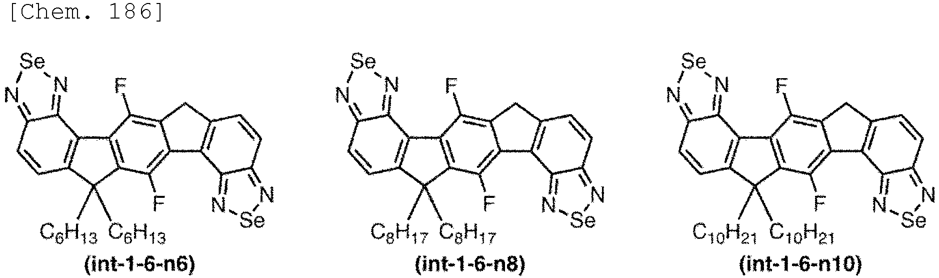

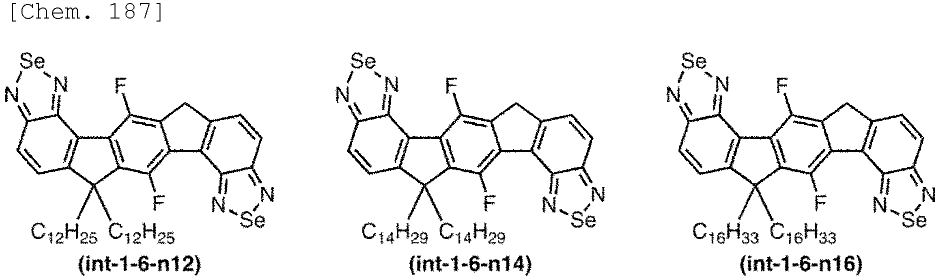

In these formulae, CₙH₂ₙ₊₁ represents a linear-chain alkyl group.

For the compound represented by general formula (int-1) used in production process (a), there is no limitation on the method for obtaining it. It can, however, be produced using a combination of known organic synthesis reactions as necessary. For example, the compound can be produced with reference to Synthesis Reference Examples 1 to 15 described herein. A commercially available compound may also be used.

The base is not particularly limited, provided that it allows the anionic species to be generated without decomposing the compound represented by general formula (int-1). Specific examples include metal hydrides, such as lithium hydride, sodium hydride and potassium hydride; metal alkoxides, such as lithium tert-butoxide, sodium tert-butoxide and potassium tert-butoxide; organic bases, such as 1,8-diazabicyclo[5.4.0]-7-undecene (DBU), 1,5-diazabicyclo[4.3.0]non-5-ene (DBN) and 1,4-diazabicyclo[2.2.2]octane (DABCO); metallic magnesium reagents (Grignard reagents), such as 1,1-dimethylpropylmagnesium chloride, sec-butylmagnesium chloride, tert butylmagnesium chloride, isopropylmagnesium chloride and isopropylmagnesium bromide; metal amides, such as lithium diisopropylamide (LDA), lithium dicyclohexylamide, lithium 2,2,6,6-tetramethylpiperidinyl and the 2,2,6,6-tetramethylpiperidinyl magnesium chloride lithium chloride complex; and silazides, such as lithium bis(trimethylsilyl)amide (also known as: lithium hexamethyldisilazide), sodium bis(trimethylsilyl)amide (also known as: sodium hexamethyldisilazide) and potassium bis(trimethylsilyl)amide (also known as: potassium hexamethyldisilazide). It is preferred to use sodium hydride, sodium tert-butoxide, potassium tert-butoxide, lithium diisopropylamide or the 2,2,6,6-tetramethylpiperidinyl magnesium chloride lithium chloride complex because it results in a good reaction yield. More preferably, the base is sodium hydride, sodium tert-butoxide, potassium tert-butoxide or lithium diisopropylamide.

There is no specific restriction on the amount of base used. Preferably, the amount is from 2.0 to 10.0 molar equivalents per molar equivalent of the compound represented by general formula (int-1) because this results in a good reaction yield. More preferably, the amount is from 2.0 to 6.0 molar equivalents.

As for the halogenated alkyl, examples include chloroalkanes, such as chloromethane, chloroethane, 1-chloropropane, 1-chlorobutane, 1-chloropentane, 1-chlorohexane, 1-chloroheptane, 1-chlorooctane, 1-chlorononane, 1-chlorodecane, 1-chloroundecane, 1-chlorododecane, 1-chlorotridecane, 1-chlorotetradecane, 1-chloropentadecane, 1-chlorohexadecane, 1-chloroheptadecane, 1-chlorooctadecane, 1-chlorononadecane and 1-chloroeicosane; bromoalkanes, such as bromomethane, bromoethane, 1-bromopropane, 1-bromobutane, 1-bromopentane, 1-bromohexane, 1-bromoheptane, 1-bromooctane, 1-bromononane, 1-bromodecane, 1-bromoundecane, 1-bromododecane, 1-bromotridecane, 1-bromotetradecane, 1-bromopentadecane, 1-bromohexadecane, 1-bromoheptadecane, 1-bromooctadecane, 1-bromononadecane and 1-bromoeicosane; and iodoalkanes, such as iodomethane, iodoethane, 1-iodopropane, 1-iodobutane, 1-iodopentane, 1-iodohexane, 1-iodoheptane, 1-iodooctane, 1-iodononane, 1-iododecane, 1-iodoundecane, 1-iodododecane, 1-iodotridecane, 1-iodotetradecane, 1-iodopentadecane, 1-iodohexadecane, 1-iodoheptadecane, 1-iodooctadecane, 1-iodononadecane and 1-iodoeicosane. The halogenated alkyl is preferably a bromoalkane or iodoalkane because this results in a good reaction yield. More preferably, the halogenated alkyl is an iodoalkane.

There is no specific restriction on the amount of halogenated alkyl used. Preferably, the amount is from 2.0 to 12.0 molar equivalents per molar equivalent of the compound represented by general formula (int-1) because this results in a good reaction yield. More preferably, the amount is from 2.0 to 8.0 molar equivalents.

Production process (a) can be conducted in a solvent. There is no specific restriction on the solvents that can be used, provided that the solvent does not inhibit the reactions. Examples include aliphatic hydrocarbon solvents, such as hexane, heptane, decane and tridecane; ether solvents, such as diisopropyl ether, dibutyl ether, cyclopentyl methyl ether (CPME), tetrahydrofuran (THF), 2-methyltetrahydrofuran, 1,4-dioxane and 1,2-dimethoxyethane; aromatic hydrocarbon solvents, such as benzene, toluene, xylene, mesitylene and tetralin; carbonate solvents, such as ethylene carbonate, propylene carbonate, dimethyl carbonate, diethyl carbonate, ethyl methyl carbonate and 4-fluoroethylene carbonate; ester solvents, such as ethyl acetate, butyl acetate, methyl propionate, ethyl propionate, methyl butyrate and γ-lactone; amide solvents, such as N,N-dimethylformamide (DMF), dimethylacetamide (DMAc) and N-methylpyrrolidone (NMP); urea solvents, such as N,N,N',N'-tetramethylurea (TMU) and N,N'-dimethylpropyleneurea (DMPU); sulfoxide solvents, such as dimethylsulfoxide (DMSO); alcohol solvents, such as methanol, ethanol, 2-propanol, butanol, octanol, benzyl alcohol, ethylene glycol, propylene glycol, diethylene glycol, triethylene glycol and 2,2,2-trifluoroethanol; halogen solvents, such as chloroform, dichloromethane, carbon tetrachloride, 1,2-dichloroethane, chlorobenzene and o-dichlorobenzene (DCB); fluorosolvents, such as bis(2,2,2-trifluoroethyl)=N,N-diisopropyl phosphoramidate (PF-37) and tris(2,2,2-trifluoroethyl)=phosphate (TFEP); nitromethane; and water, and these may be mixed in certain ratios before use. Of these, the solvent is preferably an aliphatic hydrocarbon solvent, an ether solvent, an aromatic hydrocarbon solvent, a solvent mixture of an aliphatic hydrocarbon solvent and an ether solvent or a solvent mixture of an aliphatic hydrocarbon solvent and an aromatic hydrocarbon solvent because this results in a good reaction yield. More preferably, the solvent is an ether solvent, even more preferably THF.

There is no specific restriction on the amount of solvent used. It is preferred that the amount fall within the range of 0.001 to 100 mL/mg in relation to the weight of the compound (compound represented by general formula (int-1)) used, it is preferred that the amount fall within the range of 0.001 to 100 mL/mg, it is more preferred that the amount fall within the range of 0.001 to 10 mL/mg, and it is even more preferred that the amount fall within the range of 0.005 to 1.0 mL/mg.

Production process (a) is preferably performed in an inert gas atmosphere, such as an argon gas or nitrogen gas, because this results in a good reaction yield.

Production process (a) can be conducted at a temperature selected as appropriate from -80°C to 100°C. Preferably, production process (a) is conducted at a temperature selected as appropriate from -50°C to 50°C because this results in a good reaction yield. More preferably, production process (a) is conducted at a temperature selected as appropriate from -30°C to 30°C.

The duration of reaction varies depending on the compound (compound represented by general formula (int-1)) used and the solvent and the reaction temperature. Preferably, however, the duration of reaction is from 0.1 to 100 hours, more preferably from 1 to 48 hours.

The compound represented by general formula (mono-H) is obtained by performing ordinary treatment after the end of production process (a). When necessary, the compound may be purified using general-purpose means that those skilled in the art use to purify organic compounds, such as washing, precipitation, filtration, dialysis, recrystallization, column chromatography, preparative HPLC and Soxhlet extraction, as needed.

Examples of compounds represented by general formula (mono-H) obtained through production process (a) include the following compound. The present invention, however, is not limited to these. In the formulae, the ns represent natural numbers of 1 to 50 that may be the same or different from one another. The xs represent natural numbers of 2 to 24 that may be the same or different from one another. The ys represent natural numbers of 1 to 24 that may be the same or different from one another. The zs represent natural numbers of 1 to 20 that may be the same or different from one another. Examples of specific compounds included in them are also presented below.

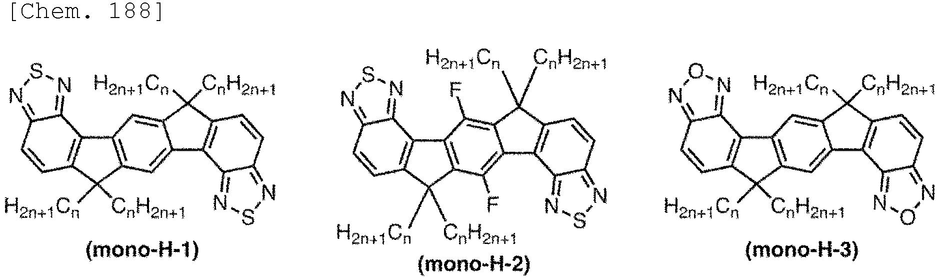

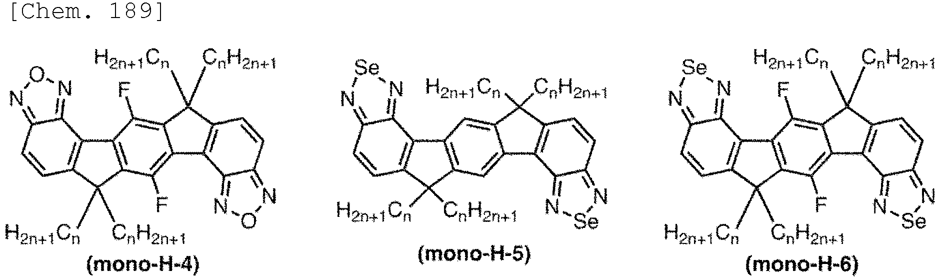

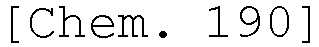

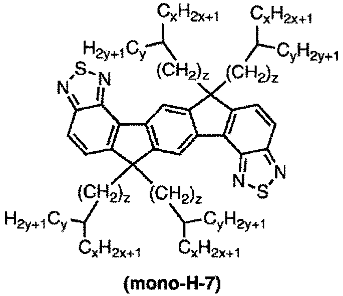

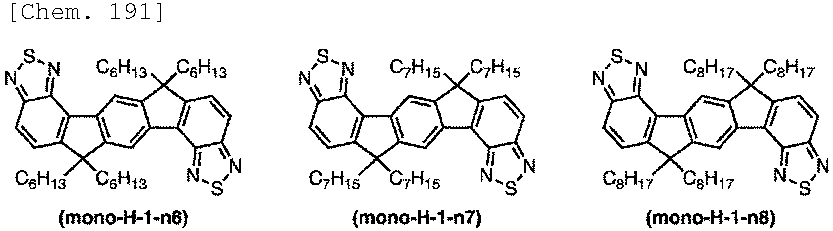

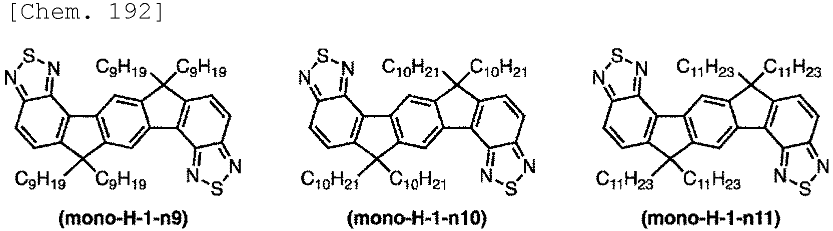

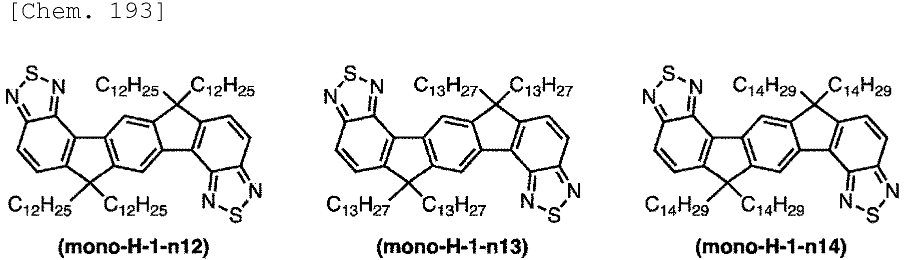

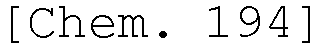

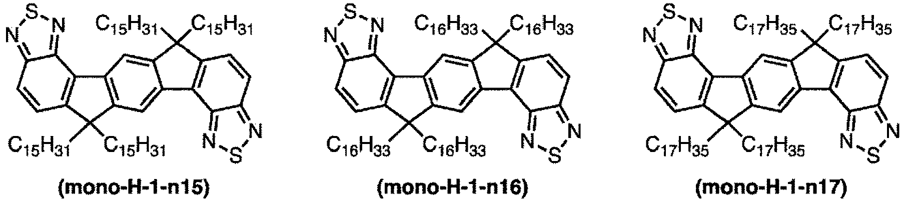

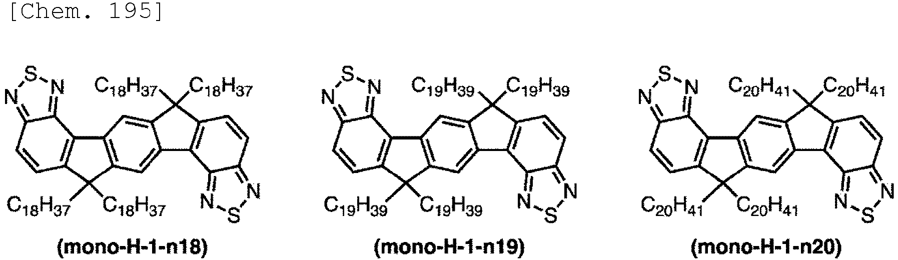

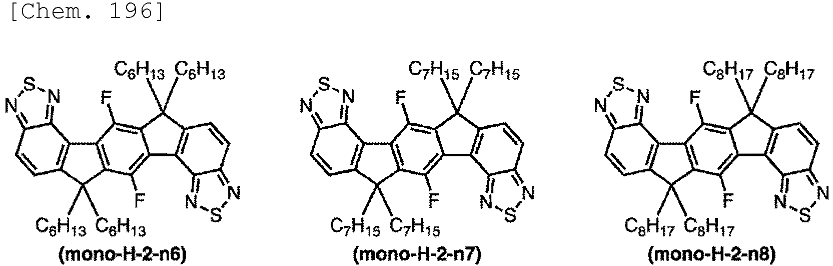

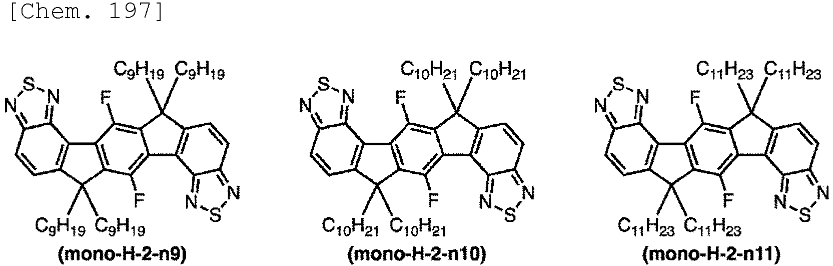

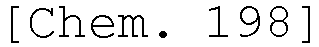

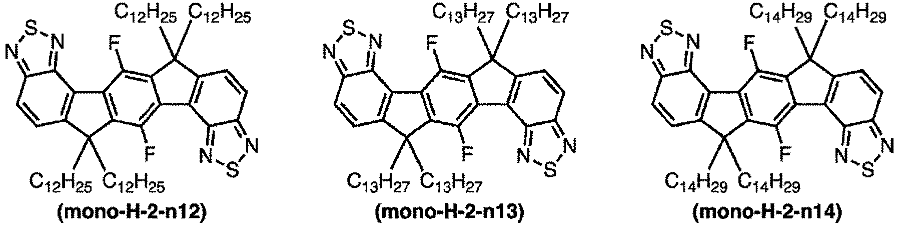

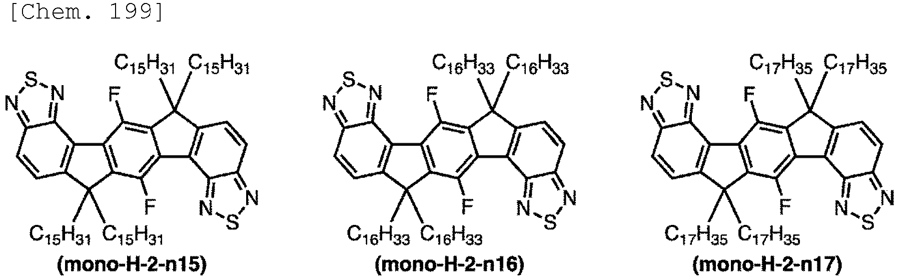

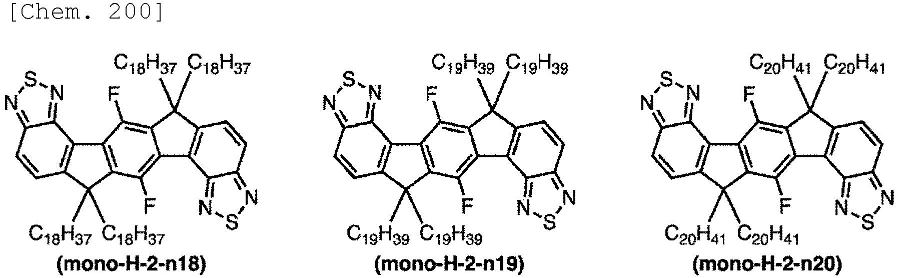

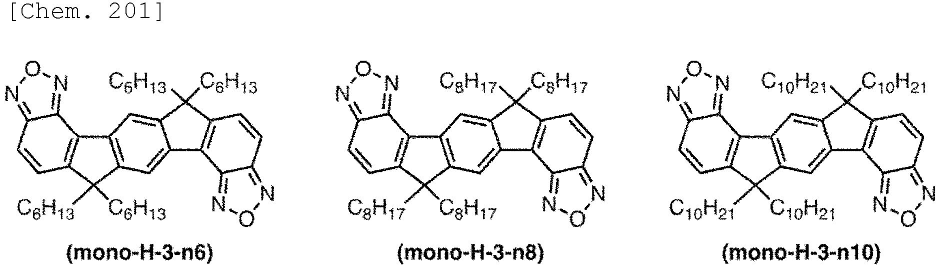

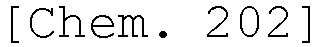

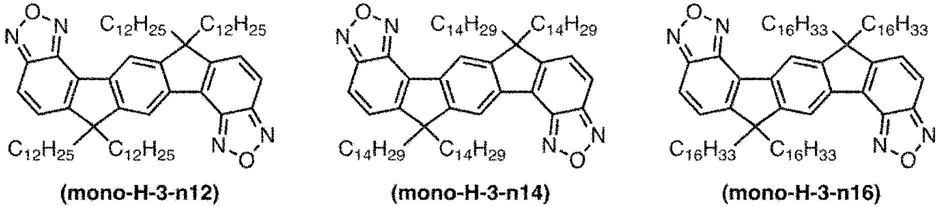

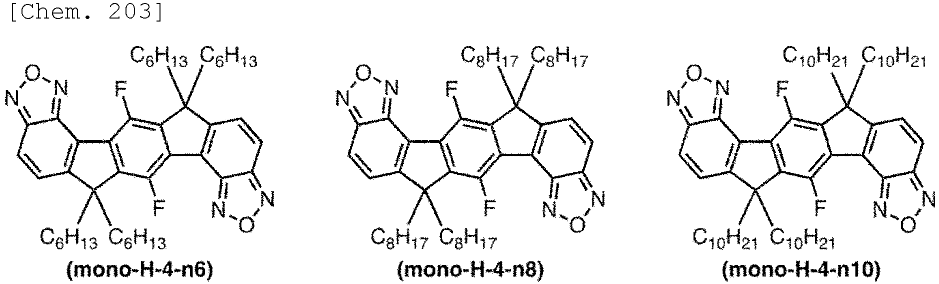

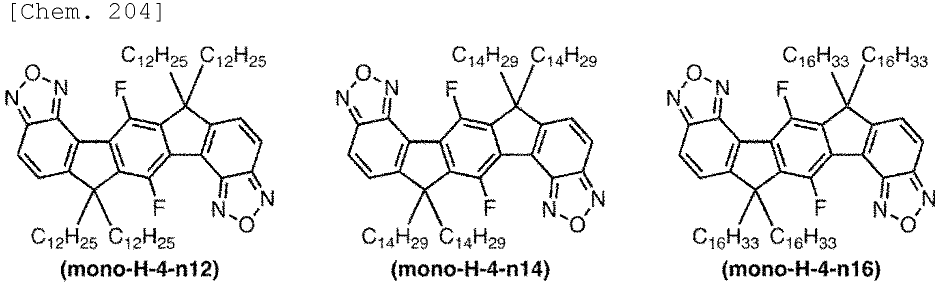

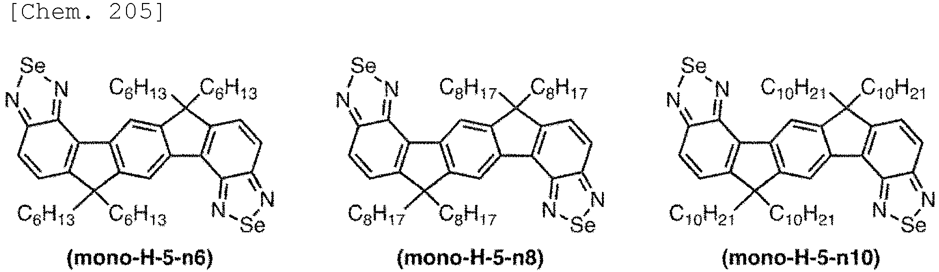

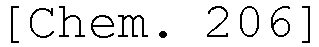

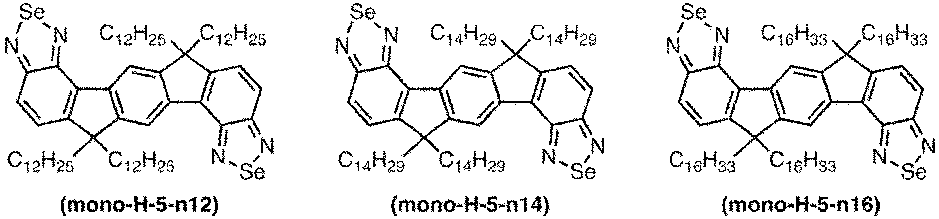

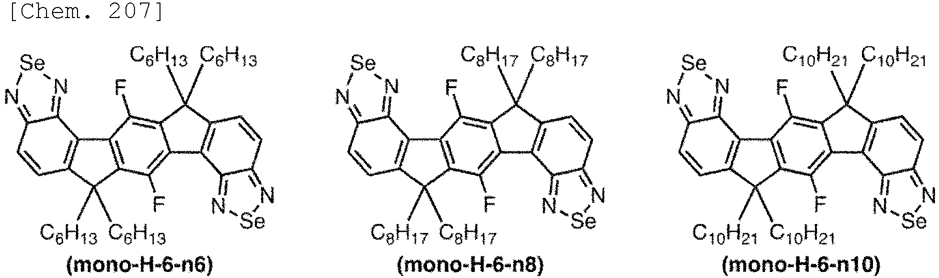

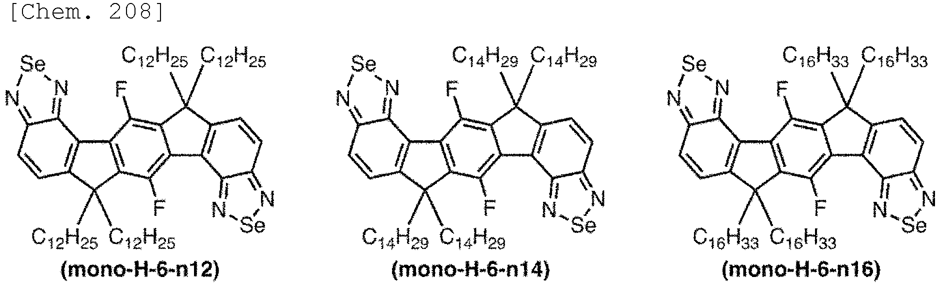

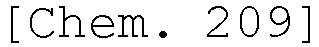

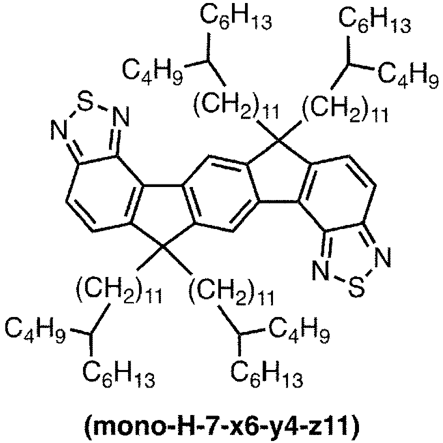

In these formulae, CₙH₂ₙ₊₁, CₓH₂ₓ₊₁ and C_{y}H_{2y+1} represent linear-chain alkyl groups.

### <Production Process (b)>

Production process (b) is a method in which a compound represented by general formula (mono-hal), which is a compound in this embodiment, is produced by allowing at least one halogenating agent to act on a compound represented by general formula (mono-H).

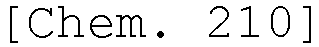

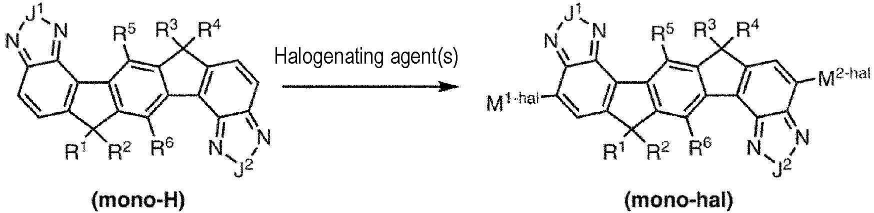

(In the formula, R¹, R², R³, R⁴, R⁵, R⁶, J¹, J², M^{1-hal} and M^{2-hal} have the same meanings as described above.)

For the halogenating agent, examples include iodizing agents, such as iodine hydroiodic acid, N-iodosuccinimide, trimethylsilyl iodide, N-iodophthalimide, tetramethylammonium dichloroiodate, benzyltrimethylammonium dichloroiodate, N-iodosaccharin, bis(pyridine)iodonium tetrafluoroborate, bis(2,4,6-trimethylpyridine)iodonium hexafluorophosphate, 1-chloro-2-iodoethane, pyridine iodine monochloride, 1,3-diiodo-5,5-dimethylhydantoin, N,N-dimethyl-N-(methylsulfanylmethylene)ammonium iodide and carbon tetraiodide; brominating agents, such as bromine, hydrobromic acid, N-bromosuccinimide, 1,2-dibromo-1,1,2,2-tetrachloroethane, trimethylsilyl bromide, N-bromophthalimide, tetrabutylammonium tribromide, N-bromosaccharin, bis(2,4,6-trimethylpyridine)bromonium hexafluorophosphate, pyridinium bromide perbromide, 4-dimethylaminopyridinium bromide perbromide, N-bromoacetamide, bromodimethylsulfonium bromide, 1-butyl-3-methylimidazolium tribromide, boron tribromide, phosphorus tribromide, trimethylphenylammonium tribromide, 1,3-dibromo-5,5-dimethylhydantoin, 2,4,4,6-tetrabromo-2,5-cyclohexadienone, dibromoisocyanuric acid, 1,8-diazabicyclo[5.4.0]-7-undecene hydrogen tribromide, benzyltrimethylammonium tribromide, bromotrichloromethane and carbon tetrabromide; and chlorinating agents, such as oxalyl chloride, methoxyacetyl chloride, methanesulfonyl chloride, N-chlorosuccinimide, N-chlorophthalimide, trimethylsilyl chloride, 1,3-dichloro-5,5-dimethylhydantoin, thionyl chloride, benzyltrimethylammonium tetrachloroiodate, phosphorus trichloride, phosphorus pentachloride, cyanuric chloride, N-chlorosaccharin, trichloromethanesulfonyl chloride and trichlorocyanuric acid, and these may be mixed in certain ratios. The halogenating agent is preferably a brominating agent because this results in a good reaction yield. More preferably, the halogenating agent is bromine or hydrogen bromide.

There is no specific restriction on the amount of halogenating agent used. Preferably, the amount is from 2.0 to 20 molar equivalents per molar equivalent of the compound represented by general formula (mono-H) because this results in a good reaction yield. More preferably, the amount is from 2.0 to 10 molar equivalents.

Production process (b) can be conducted in a solvent. There is no specific restriction on the solvents that can be used, provided that the solvent does not inhibit the reaction. Examples include the solvents listed by way of example for production process (a). Halogen solvents, acidic solvents and solvent mixtures of them are preferred because they result in a good reaction yield. Chloroform, dichloromethane, 1,2-dichloroethane, chlorobenzene, o-DCB, hydrochloric acid, hydrobromic acid, hydroiodic acid, sulfuric acid, nitric acid, acetic acid and solvent mixtures of them are more preferred, and a mixture of chloroform, hydrobromic acid and acetic acid solution is especially preferred.

There is no specific restriction on the amount of solvent used. It is preferred that the amount fall within the range of 0.001 to 100 mL/mg in relation to the weight of the compound (compound represented by general formula (mono-H)) used, it is more preferred that the amount fall within the range of 0.001 to 10 mL/mg, and it is even more preferred that the amount fall within the range of 0.005 to 1.0 mL/mg.

Production process (b) is preferably performed in an inert gas atmosphere, such as an argon gas or nitrogen gas, because this results in a good reaction yield.

Production process (b) can be conducted at a temperature selected as appropriate from 0°C to 180°C. Preferably, production process (b) is conducted at a temperature selected as appropriate from 20°C to 160°C because this results in a good reaction yield. More preferably, production process (b) is conducted at a temperature selected as appropriate from 20°C to 140°C.

The duration of reaction varies depending on the compound (compound represented by general formula (mono-H)) used and the solvent and the reaction temperature. Preferably, however, the duration of reaction is from 0.1 to 24 hours, more preferably from 1 to 12 hours.

The compound represented by general formula (mono-hal) is obtained by performing ordinary treatment after the end of production process (b). When necessary, the compound may be purified using general-purpose means that those skilled in the art use to purify organic compounds, such as washing, precipitation, filtration, dialysis, recrystallization, column chromatography, preparative HPLC and Soxhlet extraction, as needed.

Examples of compounds represented by general formula (mono-hal) obtained through production process (b) include the compounds represented by formula (mono-hal-1) to formula (mono-hal-18), formula (mono-hal-1-n6), formula (mono-hal-1-n8), formula (mono-hal-1-n10), formula (mono-hal-1-n12), formula (mono-hal-1-n14) and formula (mono-hal-1-n16) above. The present invention, however, is not limited to these.

### [Conjugated Polymer]

A conjugated polymer according to an embodiment of the present invention (Also referred to as "a conjugated polymer in this embodiment" herein.) comprises a structural unit represented by general formula (2) and a structural unit represented by general formula (3).

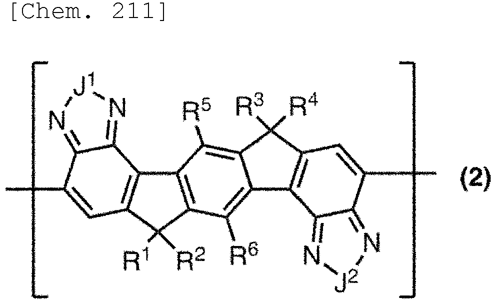

(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom.)

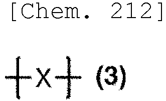

(In the formula, X represents a divalent heteroaromatic ring linking group optionally substituted with a C1 to C50 alkyl group or a C1 to C50 alkoxy group.)

C1 to C50 alkyl groups represented by R¹, R², R³, R⁴, R⁵ and R⁶ may be any of linear-chain, branched or cyclic. Examples include linear-chain alkyl groups, such as a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a heneicosyl group, a docosyl group, a tricosyl group, a tetracosyl group, a pentacosyl group, a hexacosyl group, a heptacosyl group, an octacosyl group, a nonacosyl group, a triacontyl group, a hentriacontyl group, a dotriacontyl group, a tritriacontyl group, a tetratriacontyl group, a pentatriacontyl group, a hexatriacontyl group, a tetracontyl group, a hentetracontyl group, a dotetracontyl group, a tritetracontyl group, a tetratetracontyl group and a pentacontyl group; branched alkyl groups, such as an isopropyl group, an isobutyl group, a sec-butyl group, a tert-butyl group, a 2-ethylhexyl group, a 3,7-dimethyloctyl group, a 2-hexyloctyl group, a 2-hexyldecyl group, a 2-octyldodecyl group, a 2-decyltetradecyl group, a 2-dodecyltetradecyl group, a 2-dodecylhexadecyl group, a 2-tetradecylhexadecyl group, a 3-decylpentadecyl group, a 3-dodecylheptadecyl group, a 3-tetradecylnonacosyl group, a 4-decylhexadecyl group, a 4-dodecyloctadecyl group and a 4-tetradecylicosyl group; and cyclic alkyl groups, such as a cyclopentyl group and a cyclohexyl group.

The alkyl groups represented by R¹, R², R³ and R⁴ are preferably C1 to C34 alkyl groups because this leads to higher solubility of the conjugated polymer in this embodiment. More preferably, the alkyl groups are C1 to C20 alkyl groups, even more preferably methyl groups, ethyl groups, propyl groups, butyl groups, pentyl groups, hexyl groups, heptyl groups, octyl groups, nonyl groups, decyl groups, undecyl groups, dodecyl groups, tridecyl groups, tetradecyl groups, pentadecyl groups, hexadecyl groups, heptadecyl groups, octadecyl groups, nonadecyl groups, icosyl groups, 2-ethylhexyl groups, 3,7-dimethyloctyl groups, 2-hexyloctyl groups, 2-hexyldecyl groups or 2-octyldodecyl groups, especially preferably decyl groups or hexadecyl groups.

R¹ and R² may be bound to each other to form a ring together with the carbon atom to which they are bound. Examples of such rings include a cyclopropan-1,1-diyl group, a cyclobutan-1,1-diyl group, a cyclopentan-1,1-diyl group, a cyclohexan-1,1-diyl group, a cycloheptan-1,1-diyl group, a cyclooctan-1,1-diyl group, an inden-1,1-diyl group and a fluoren-9,9-diyl group. Preferably, this ring is a cyclopentan-1,1-diyl group, a cyclohexan-1,1-diyl group, an inden-1,1-diyl group or a fluoren-9,9-diyl group because this leads to higher solubility of the conjugated polymer in this embodiment. More preferably, the ring is a cyclohexan-1,1-diyl group or a fluoren-9,9-diyl group.

R³ and R⁴ may be bound to each other to form a ring together with the carbon atom to which they are bound. Examples of such rings include a cyclopropan-1,1-diyl group, a cyclobutan-1,1-diyl group, a cyclopentan-1,1-diyl group, a cyclohexan-1,1-diyl group, a cycloheptan-1,1-diyl group, a cyclooctan-1,1-diyl group, an inden-1,1-diyl group and a fluoren-9,9-diyl group. Preferably, this ring is a cyclopentan-1,1-diyl group, a cyclohexan-1,1-diyl group, an inden-1,1-diyl group or a fluoren-9,9-diyl group because this leads to higher solubility of the conjugated polymer in this embodiment. More preferably, the ring is a cyclohexan-1,1-diyl group or a fluoren-9,9-diyl group.

R⁵ and R⁶ are preferably hydrogen atoms, fluorine atoms or C1 to C34 alkyl groups because this leads to higher carrier mobility of the conjugated polymer in this embodiment. More preferably, R⁵ and R⁶ are hydrogen atoms, fluorine atoms or C1 to C20 alkyl groups, even more preferably hydrogen atoms, fluorine atoms, methyl groups, ethyl groups, propyl groups, butyl groups, pentyl groups, hexyl groups, heptyl groups, octyl groups, nonyl groups, decyl groups, undecyl groups, dodecyl groups, tridecyl groups, tetradecyl groups, pentadecyl groups, hexadecyl groups, heptadecyl groups, octadecyl groups, nonadecyl groups, icosyl groups, 2-ethylhexyl groups, 3,7-dimethyloctyl groups, 2-hexyloctyl groups, 2-hexyldecyl groups or 2-octyldodecyl groups. Of these, it is especially preferred that R⁵ and R⁶ be hydrogen atoms because this increases carrier mobility.

The chalcogen atoms represented by J¹ and J² are preferably oxygen atoms, sulfur atoms or selenium atoms because this leads to higher solubility of the conjugated polymer in this embodiment. More preferably, the chalcogen atoms are oxygen atoms or sulfur atoms, even more preferably sulfur atoms.

Examples of structural units represented by general formula (2) include the structural units presented below because they lead to higher film-forming properties and carrier mobility of the conjugated polymer in this embodiment. The present invention, however, is not limited to these. In the formulae, the ns represent natural numbers of 1 to 50 that may be the same or different from one another. The xs represent natural numbers of 2 to 24 that may be the same or different from one another. The ys represent natural numbers of 1 to 24 that may be the same or different from one another. The zs represent natural numbers of 1 to 20 that may be the same or different from one another. Examples of specific structural units included in them are also presented below.

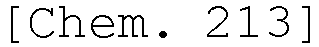

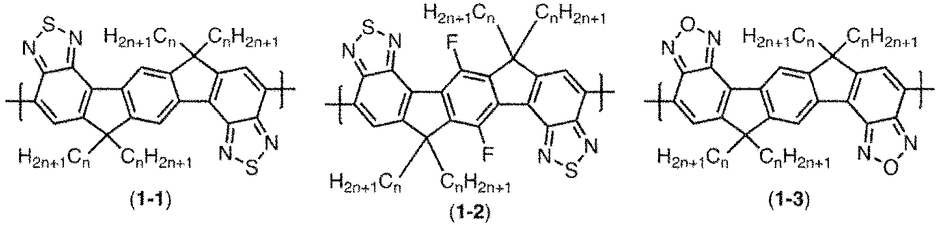

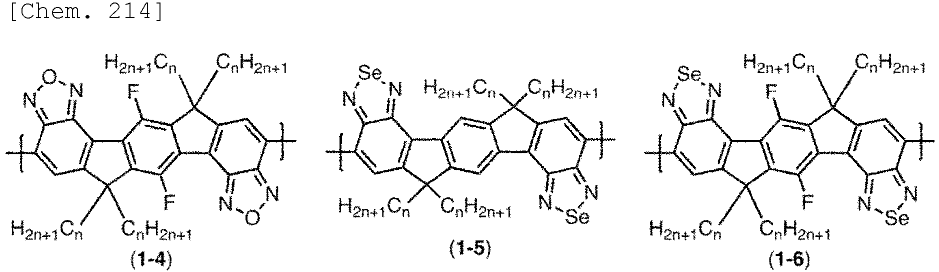

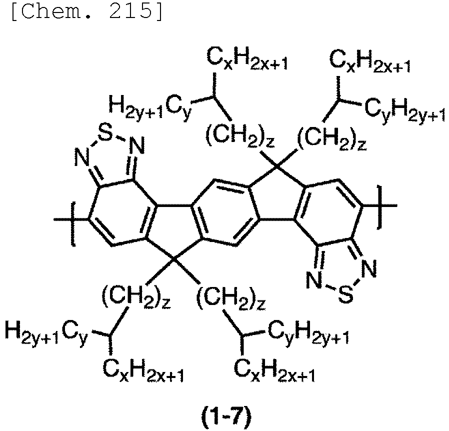

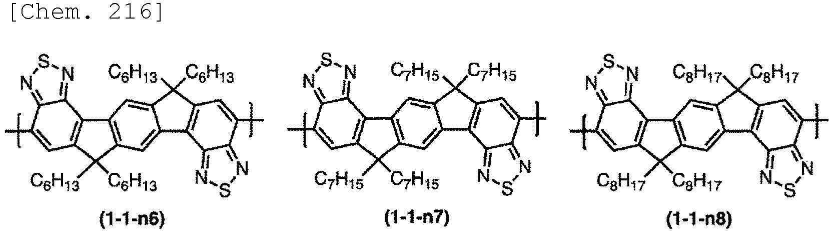

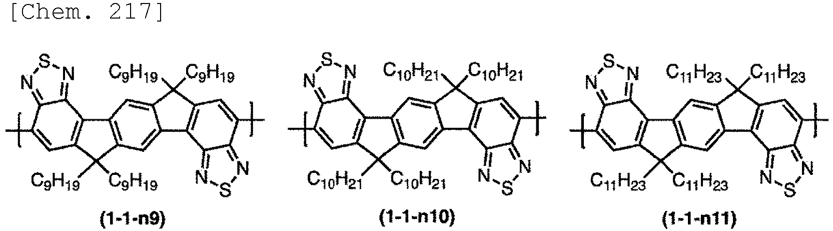

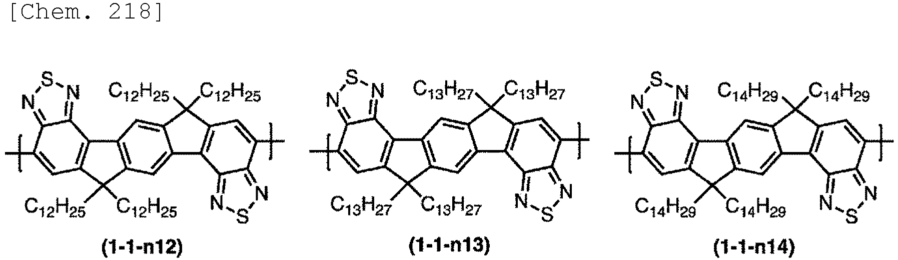

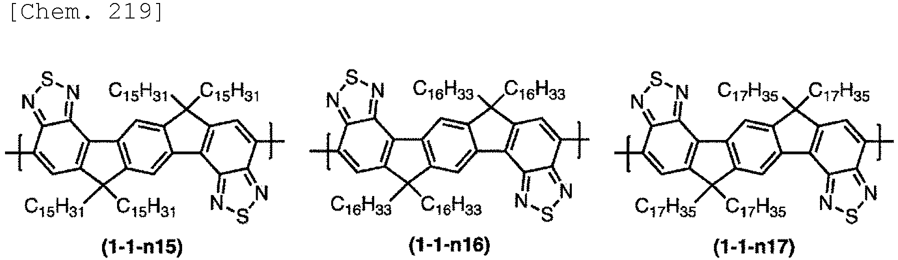

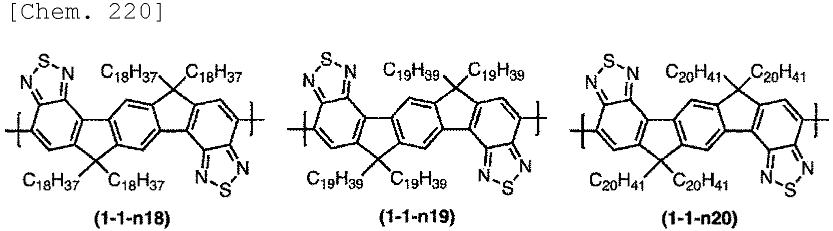

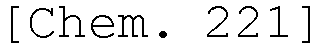

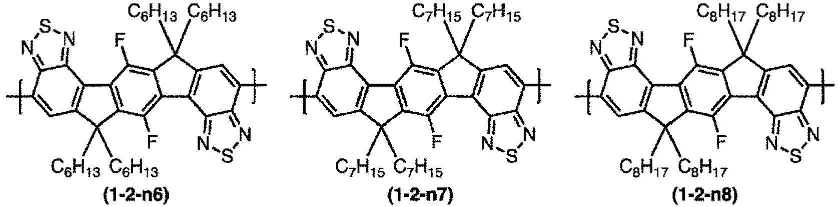

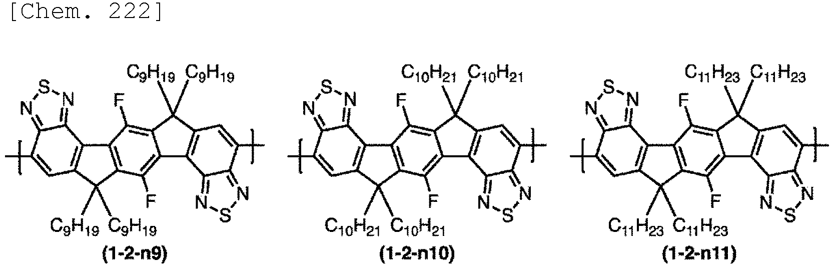

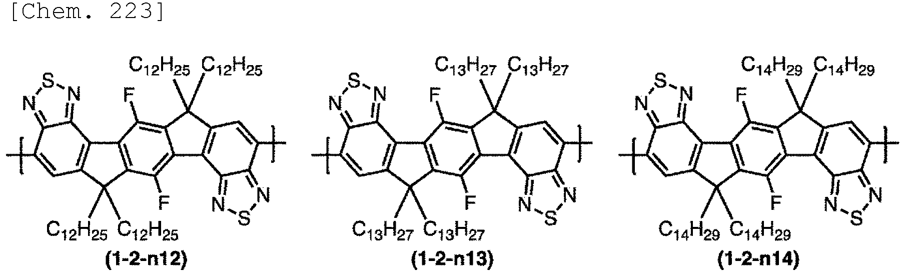

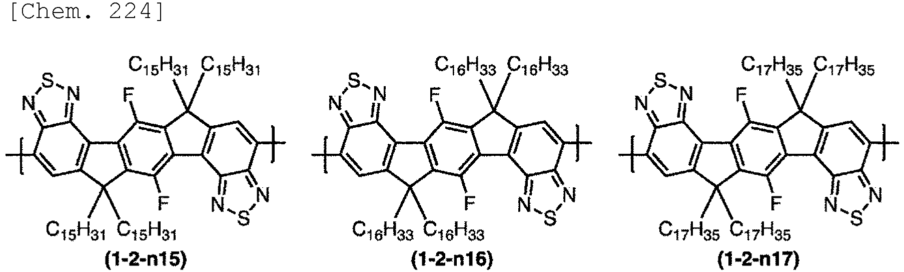

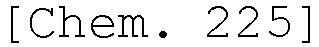

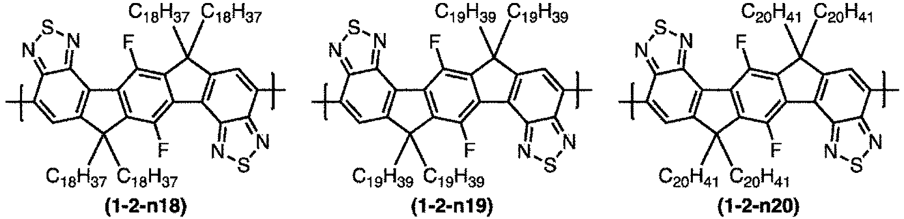

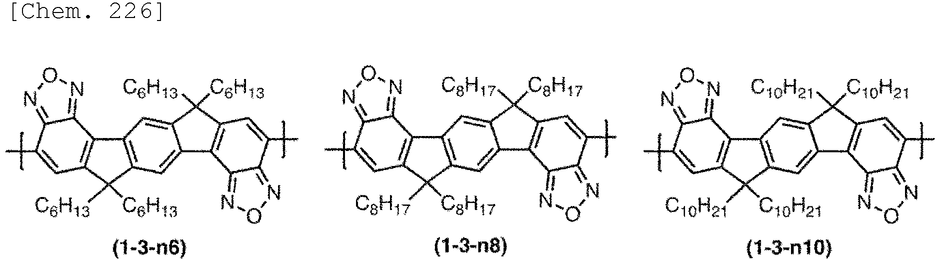

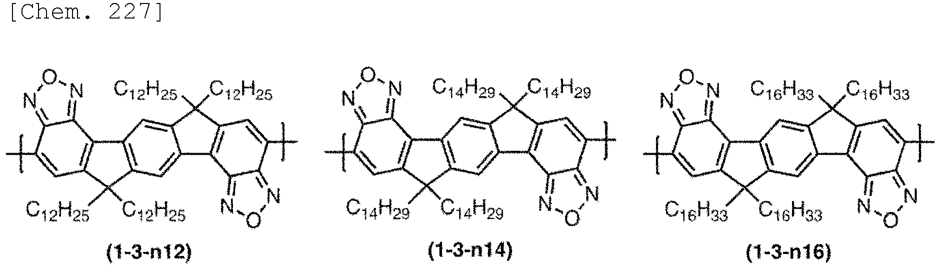

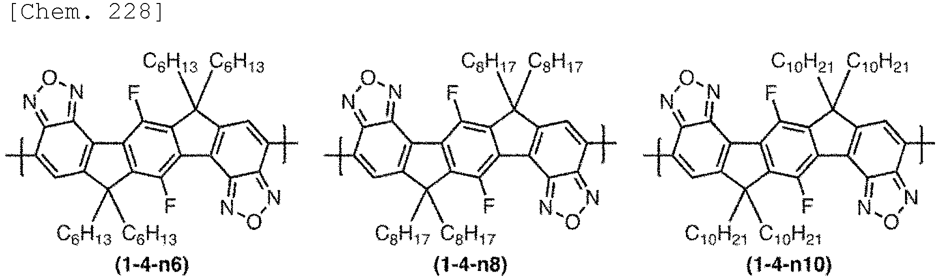

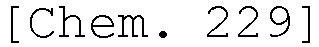

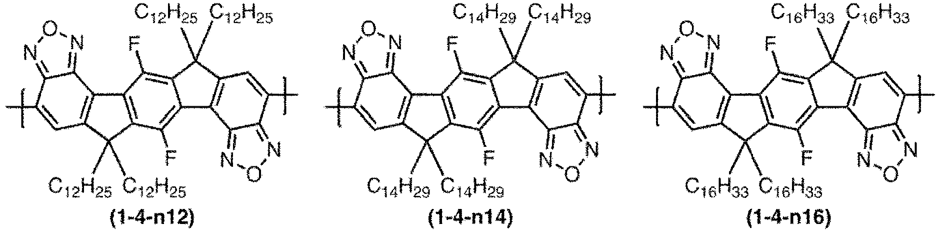

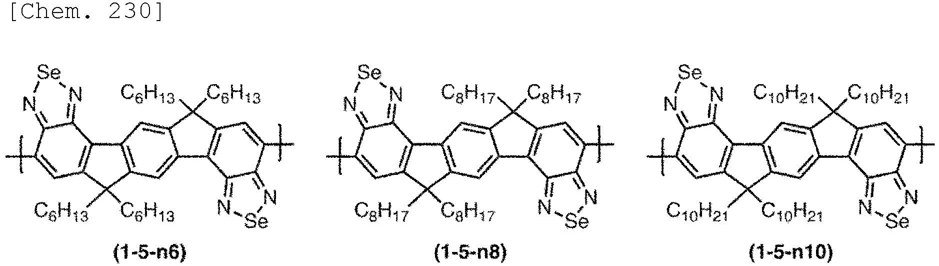

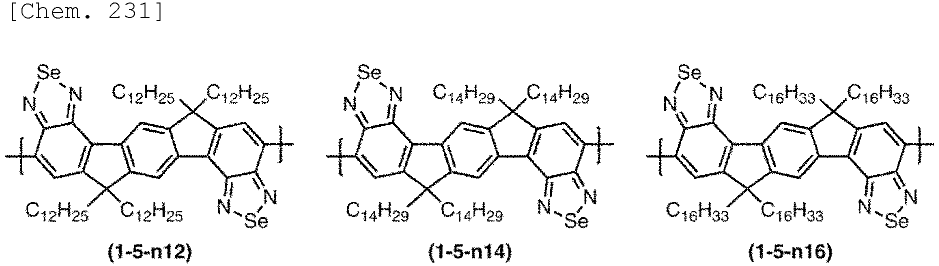

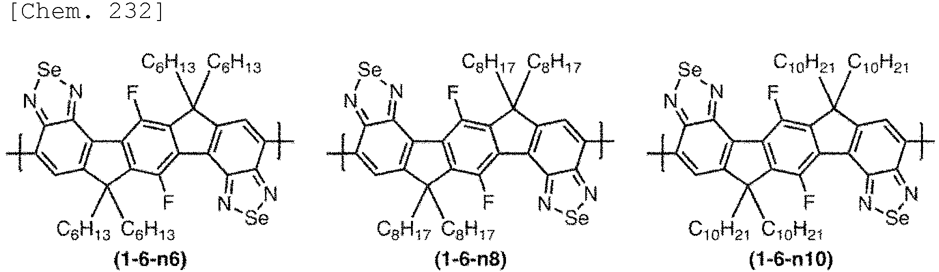

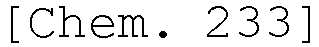

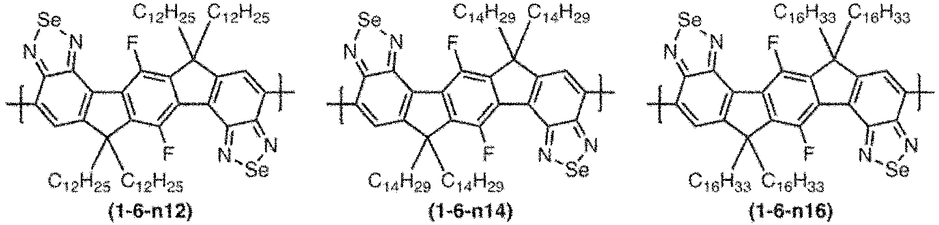

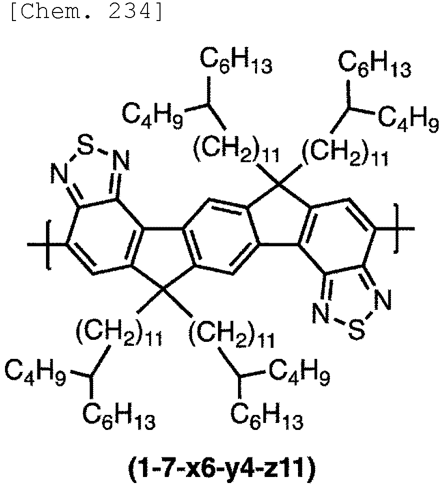

In these formulae, CₙH₂ₙ₊₁, CₓH₂ₓ₊₁ and C_{y}H_{2y+1} represent linear-chain alkyl groups.

The structural unit represented by general formula (2) is preferably any structural unit represented by formula (1-1), formula (1-2), formula (1-1-n6) to formula (1-1-n20) or formula (1-2-n6) to formula (1-2-n20) because this leads to higher film-forming properties and carrier mobility of the conjugated polymer in this embodiment. More preferably, the structural unit is any structural unit represented by formula (1-1) or formula (1-1-n6) to formula (1-1-n20), even more preferably any structural unit represented by formula (1-1-n10) to formula (1-1-n18).

For X, examples include divalent heteroaromatic ring linking groups optionally substituted with a C1 to C50 alkyl group or a C1 to C50 alkoxy group. A specific example is a divalent heteroaromatic ring linking group selected from the group consisting of the linking groups represented by general formula (4) to general formula (23) below.

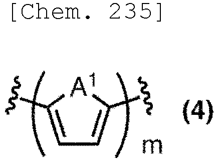

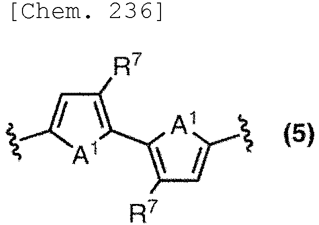

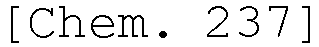

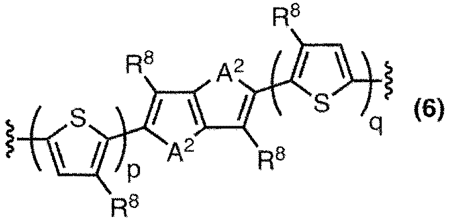

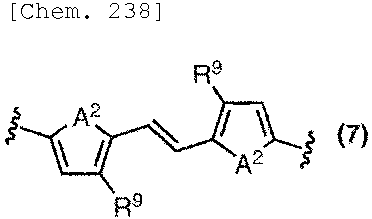

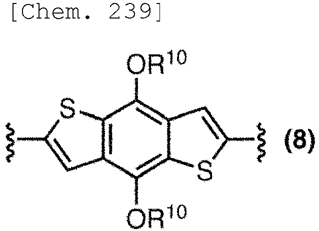

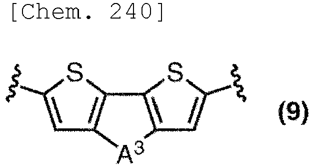

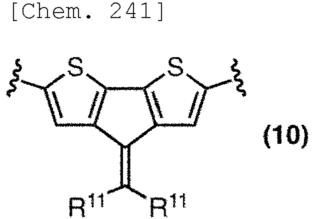

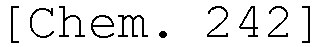

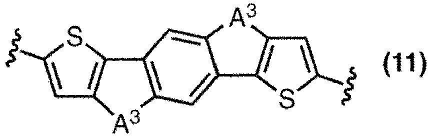

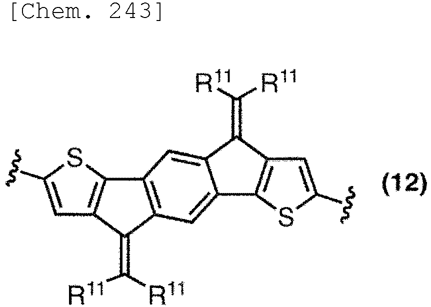

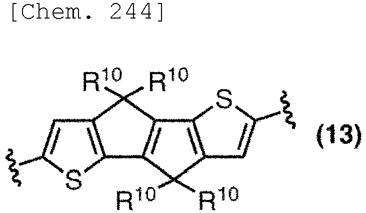

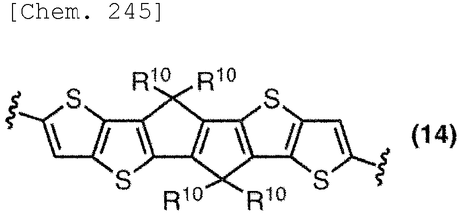

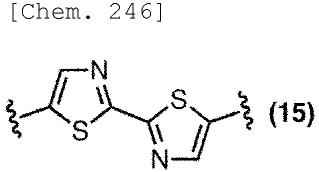

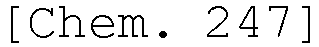

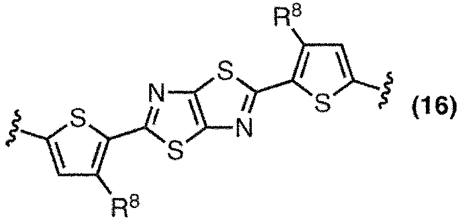

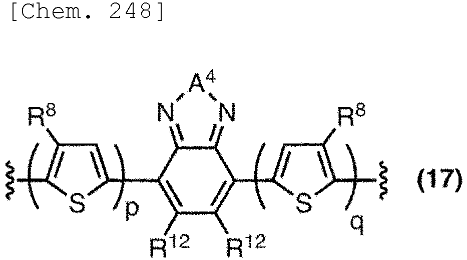

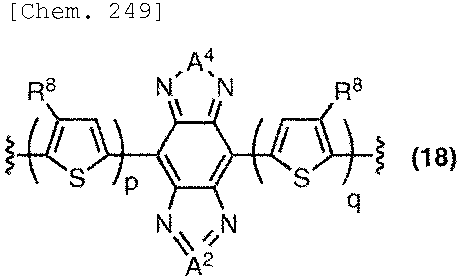

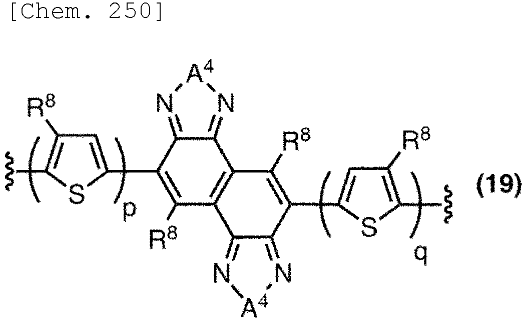

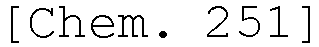

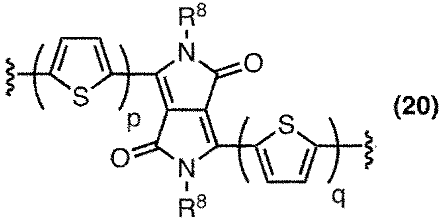

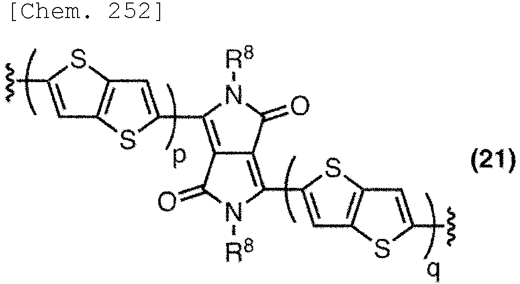

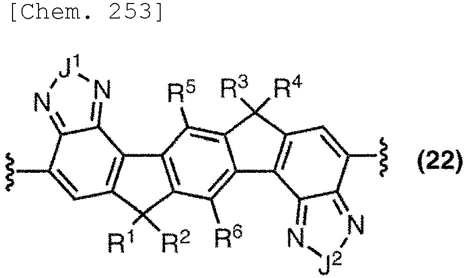

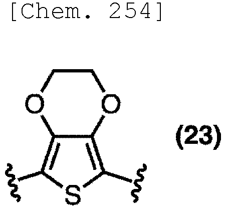

(In the formula, R¹, R², R³, R⁴, R⁵, R⁶, J¹ and J² have the same meanings as described above. A¹ and A² each independently represent a chalcogen atom. A³ represents a chalcogen atom, C(R¹⁰)₂, C (H)(R¹⁰), Si(R¹⁰)₂ or NR¹⁰, independently at each occurrence. A⁴ represents a chalcogen atom or NR¹⁰, independently at each occurrence. R⁷ represents a hydrogen atom, a fluorine atom or a C1 to C50 alkyl group. Multiple R⁷s may be the same or different from one another. R⁸ represents a hydrogen atom or a C1 to C50 alkyl group. Multiple R⁸s may be the same or different from one another. R⁹ represents a hydrogen atom, a fluorine atom or a C1 to C50 alkyl group. Multiple R⁹s may be the same or different from one another. R¹⁰ represents a C1 to C50 alkyl group. Multiple R¹⁰s may be the same or different from one another. R¹¹ represents a C1 to C50 alkyl group or a C1 to C50 thioalkyl group. R¹² represents a hydrogen atom, a fluorine atom, a C1 to C50 alkyl group or a C1 to C50 alkoxy group. m represents 1 to 3. p and q each independently represent 0 or 1.)

The structural unit represented by general formula (3) is preferably a structural unit represented by general formula (4) to general formula (14) or general formula (20) because this leads to higher film-forming properties and carrier mobility of the conjugated polymer in this embodiment. More preferably, the structural unit is a structural unit represented by general formula (5), general formula (7) to general formula (11) or general formula (20).

C1 to C50 alkyl groups represented by R⁷, R⁸, R⁹, R¹⁰, R¹¹ and R¹² may be any of linear-chain or branched. Examples include linear-chain alkyl groups, such as a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a heneicosyl group, a docosyl group, a tricosyl group, a tetracosyl group, a pentacosyl group, a hexacosyl group, a heptacosyl group, an octacosyl group, a nonacosyl group, a triacontyl group, a hentriacontyl group, a dotriacontyl group, a tritriacontyl group, a tetratriacontyl group, a pentatriacontyl group, a hexatriacontyl group, a tetracontyl group, a hentetracontyl group, a dotetracontyl group, a tritetracontyl group, a tetratetracontyl group and a pentacontyl group; and branched alkyl groups, such as an isopropyl group, an isobutyl group, a sec-butyl group, a tert-butyl group, a 2-ethylhexyl group, a 3,7-dimethyloctyl group, a 2-hexyloctyl group, a 2-hexyldecyl group, a 2-octyldodecyl group, a 2-decyltetradecyl group, a 2-dodecyltetradecyl group, a 2-dodecylhexadecyl group, a 2-tetradecylhexadecyl group, a 3-decylpentadecyl group, a 3-dodecylheptadecyl group, a 3-tetradecylnonacosyl group, a 4-decylhexadecyl group, a 4-dodecyloctadecyl group and a 4-tetradecylicosyl group.

R⁷ is preferably a hydrogen atom, a fluorine atom or a C1 to C34 alkyl group because this leads to higher carrier mobility of the conjugated polymer in this embodiment. More preferably, R⁷ is a hydrogen atom, a fluorine atom or a C1 to C20 alkyl group, even more preferably a hydrogen atom, a fluorine atom or a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a 2-ethylhexyl group, a 2-methylheptyl group, a 2-propylhexyl group, a 2-ethylheptyl group, a 2-methyloctyl group, a 2-butylhexyl group, a 2-ethyloctyl group, a 2-methylnonyl group, a 2-butylheptyl group, a 2-propyloctyl group, a 2-ethylnonyl group, a 2-pentylheptyl group, a 2-butyloctyl group, a 2-propylnonyl group, a 2-pentyloctyl group, a 2-butylnonyl group, a 2-methyldecyl group, a 2-hexyloctyl group, a 2-pentylnonyl group, a 2-hexylnonyl group, a 2-hexyldecyl group, a 2-heptylnonyl group or a 2-octyldodecyl group, especially preferably a hydrogen atom, a fluorine atom or a methyl group, an octyl group or a 2-octyldodecyl group, particularly preferably a hydrogen atom.

R⁸ is preferably a hydrogen atom or a C1 to C34 alkyl group because this leads to higher carrier mobility of the conjugated polymer in this embodiment. More preferably, R^{B} is a hydrogen atom or a C1 to C20 alkyl group, even more preferably a hydrogen atom, a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a 2-ethylhexyl group, a 2-methylheptyl group, a 2-propylhexyl group, a 2-ethylheptyl group, a 2-methyloctyl group, a 2-butylhexyl group, a 2-ethyloctyl group, a 2-methylnonyl group, a 2-butylheptyl group, a 2-propyloctyl group, a 2-ethylnonyl group, a 2-pentylheptyl group, a 2-butyloctyl group, a 2-propylnonyl group, a 2-pentyloctyl group, a 2-butylnonyl group, a 2-methyldecyl group, a 2-hexyloctyl group, a 2-pentylnonyl group, a 2-hexylnonyl group, a 2-hexyldecyl group, a 2-heptylnonyl group or a 2-octyldodecyl group, especially preferably a hydrogen atom, a methyl group, an octyl group or a 2-octyldodecyl group, particularly preferably a hydrogen atom.

R⁹ is preferably a hydrogen atom, a fluorine atom or a C1 to C34 alkyl group because this leads to higher carrier mobility of the conjugated polymer in this embodiment. More preferably, R⁹ is a hydrogen atom, a fluorine atom or a C1 to C20 alkyl group, even more preferably a hydrogen atom, a fluorine atom, a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a 2-ethylhexyl group, a 2-methylheptyl group, a 2-propylhexyl group, a 2-ethylheptyl group, a 2-methyloctyl group, a 2-butylhexyl group, a 2-ethyloctyl group, a 2-methylnonyl group, a 2-butylheptyl group, a 2-propyloctyl group, a 2-ethylnonyl group, a 2-pentylheptyl group, a 2-butyloctyl group, a 2-propylnonyl group, a 2-pentyloctyl group, a 2-butylnonyl group, a 2-methyldecyl group, a 2-hexyloctyl group, a 2-pentylnonyl group, a 2-hexylnonyl group, a 2-hexyldecyl group, a 2-heptylnonyl group or a 2-octyldodecyl group, especially preferably a hydrogen atom, a fluorine atom, a methyl group or a hexyl group, particularly preferably a hydrogen atom.

R¹⁰ is preferably a C1 to C34 alkyl group because this leads to higher carrier mobility of the conjugated polymer in this embodiment. More preferably, R¹⁰ is a C1 to C20 alkyl group, even more preferably a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a 2-ethylhexyl group, a 2-methylheptyl group, a 2-propylhexyl group, a 2-ethylheptyl group, a 2-methyloctyl group, a 2-butylhexyl group, a 2-ethyloctyl group, a 2-methylnonyl group, a 2-butylheptyl group, a 2-propyloctyl group, a 2-ethylnonyl group, a 2-pentylheptyl group, a 2-butyloctyl group, a 2-propylnonyl group, a 2-pentyloctyl group, a 2-butylnonyl group, a 2-methyldecyl group, a 2-hexyloctyl group, a 2-pentylnonyl group, a 2-hexylnonyl group, a 2-hexyldecyl group, a 2-heptylnonyl group or a 2-octyldodecyl group, especially preferably a methyl group or an octyl group.

An alkyl group represented by R¹¹ is preferably a C1 to C34 alkyl group because this leads to higher carrier mobility of the conjugated polymer in this embodiment. More preferably, the alkyl group is a C1 to C20 alkyl group, even more preferably a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a 2-ethylhexyl group, a 2-methylheptyl group, a 2-propylhexyl group, a 2-ethylheptyl group, a 2-methyloctyl group, a 2-butylhexyl group, a 2-ethyloctyl group, a 2-methylnonyl group, a 2-butylheptyl group, a 2-propyloctyl group, a 2-ethylnonyl group, a 2-pentylheptyl group, a 2-butyloctyl group, a 2-propylnonyl group, a 2-pentyloctyl group, a 2-butylnonyl group, a 2-methyldecyl group, a 2-hexyloctyl group, a 2-pentylnonyl group, a 2-hexylnonyl group, a 2-hexyldecyl group, a 2-heptylnonyl group or a 2-octyldodecyl group, especially preferably a methyl group, a propyl group or a hexyl group.

Examples of C1 to C50 thioalkyl groups represented by R¹¹ include a methylthio group, an ethylthio group, a propylthio group, a butylthio group, a pentylthio group, a hexylthio group, a heptylthio group, an octylthio group, a nonylthio group, a decylthio group, an undecylthio group, a dodecylthio group, a tridecylthio group, a tetradecylthio group, a pentadecylthio group, a hexadecylthio group, a heptadecylthio group, an octadecylthio group, a nonadecylthio group, an icosylthio group, a 2-ethylhexylthio group, a 2-methylheptylthio group, a 2-propylhexylthio group, a 2-ethylheptylthio group, a 2-methyloctylthio group, a 2-butylhexylthio group, a 2-ethyloctylthio group, a 2-methylnonylthio group, a 2-butylheptylthio group, a 2-propyloctylthio group, a 2-ethylnonylthio group, a 2-pentylheptylthio group, a 2-butyloctylthio group, a 2-propylnonylthio group, a 2-pentyloctylthio group, a 2-butylnonylthio group, a 2-methyldecylthio group, a 2-hexyloctylthio group, a 2-pentylnonylthio group, a 2-hexylnonylthio group, a 2-hexyldecylthio group, a 2-heptylnonylthio group and a 2-octyldodecylthio group. Preferably, the thioalkyl group is a C1 to C34 thioalkyl group because this leads to higher carrier mobility of the conjugated polymer in this embodiment. More preferably, the thioalkyl group is a C1 to C20 thioalkyl group, even more preferably a methylthio group, an ethylthio group, a propylthio group, a butylthio group, a pentylthio group, a hexylthio group, a heptylthio group, an octylthio group, a nonylthio group, a decylthio group, an undecylthio group, a dodecylthio group, a tridecylthio group, a tetradecylthio group, a pentadecylthio group, a hexadecylthio group, a heptadecylthio group, an octadecylthio group, a nonadecylthio group, an icosylthio group, a 2-ethylhexylthio group, a 2-methylheptylthio group, a 2-propylhexylthio group, a 2-ethylheptylthio group, a 2-methyloctylthio group, a 2-butylhexylthio group, a 2-ethyloctylthio group, a 2-methylnonylthio group, a 2-butylheptylthio group, a 2-propyloctylthio group, a 2-ethylnonylthio group, a 2-pentylheptylthio group, a 2-butyloctylthio group, a 2-propylnonylthio group, a 2-pentyloctylthio group, a 2-butylnonylthio group, a 2-methyldecylthio group, a 2-hexyloctylthio group, a 2-pentylnonylthio group, a 2-hexylnonylthio group, a 2-hexyldecylthio group, a 2-heptylnonylthio group or a 2-octyldodecylthio group, especially preferably a methylthio group or a propylthio group.

R¹¹ is preferably a C1 to C34 alkyl group or a C1 to C34 thioalkyl group because this leads to higher carrier mobility of the conjugated polymer in this embodiment. More preferably, R¹¹ is a C1 to C20 alkyl group or a C1 to C20 thioalkyl group, even more preferably a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a methylthio group, an ethylthio group, a propylthio group, a butylthio group, a pentylthio group, a hexylthio group, a heptylthio group, an octylthio group, a nonylthio group, a decylthio group, an undecylthio group, a dodecylthio group, a tridecylthio group, a tetradecylthio group, a pentadecylthio group, a hexadecylthio group, a heptadecylthio group, an octadecylthio group, a nonadecylthio group or an icosylthio group, especially preferably a methyl group, a propyl group, a hexyl group, a methylthio group or a propylthio group.

A C1 to C50 alkyl group represented by R¹² is preferably a C1 to C34 alkyl group because this leads to higher carrier mobility of the conjugated polymer in this embodiment. More preferably, the alkyl group is a C1 to C20 alkyl group, even more preferably a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, an isopropyl group, an isobutyl group, a sec-butyl group, a tert-butyl group, a 2-ethylhexyl group, a 3,7-dimethyloctyl group, a 2-hexyloctyl group, a 2-hexyldecyl group or a 2-octyldodecyl group, especially preferably a methyl group.

A C1 to C50 alkoxy group represented by R¹² may be any of linear-chain or branched. Examples include linear-chain alkoxy groups, such as a methoxy group, an ethoxy group, a propoxy group, a butoxy group, a pentyloxy group, a hexyloxy group, a heptyloxy group, an octyloxy group, a nonyloxy group, a decyloxy group, an undecyloxy group, a dodecyloxy group, a tridecyloxy group, a tetradecyloxy group, a pentadecyloxy group, a hexadecyloxy group, a heptadecyloxy group, an octadecyloxy group, a nonadecyloxy group, an icosyloxy group, a heneicosyloxy group, a docosyloxy group, a tricosyloxy group, a tetracosyloxy group, a pentacosyloxy group, a hexacosyloxy group, a heptacosyloxy group, an octacosyloxy group, a nonacosyloxy group, a triacontyloxy group, a hentriacontyloxy group, a dotriacontyloxy group, a tritriacontyloxy group, a tetratriacontyloxy group, a pentatriacontyloxy group, a hexatriacontyloxy group, a tetracontyloxy group, a hentetracontyloxy group, a dotetracontyl group, a tritetracontyloxy group, a tetratetracontyloxy group and a pentacontyloxy group; and branched alkoxy groups, such as an isopropyloxy group, a 1-(2-methylpropyl)oxy group, a 2-butyloxy group, a tert-butoxy group, a 2-ethylhexyloxy group, a 3,7-dimethyloctyloxy group, a 2-decyltetradecyloxy group, a 2-dodecyltetradecyloxy group, a 2-dodecylhexadecyloxy group and a 2-tetradecylhexadecyloxy group. The C1 to C50 alkoxy group is preferably a C1 to C34 alkoxy group because this leads to higher carrier mobility of the conjugated polymer in this embodiment. More preferably, the alkoxy group is a C1 to C20 alkoxy group, even more preferably a methoxy group, an ethoxy group, a propoxy group, a butoxy group, a pentyloxy group, a hexyloxy group, a heptyloxy group, an octyloxy group, a nonyloxy group, a decyloxy group, an undecyloxy group, a dodecyloxy group, a tridecyloxy group, a tetradecyloxy group, a pentadecyloxy group, a hexadecyloxy group, a heptadecyloxy group, an octadecyloxy group, a nonadecyloxy group, an icosyloxy group, an isopropyloxy group, a 1-(2-methylpropyl)oxy group, a 2-butyloxy group, a tert-butoxy group, a 2-ethylhexyloxy group or a 3,7-dimethyloctyloxy group, especially preferably a methoxy group.

R¹² is preferably a hydrogen atom, a fluorine atom, a C1 to C34 alkyl group or a C1 to C34 alkoxy group because this leads to higher carrier mobility of the conjugated polymer in this embodiment. More preferably, R¹² is a hydrogen atom, a fluorine atom, a C1 to C20 alkyl group or a C1 to C20 alkoxy group, even more preferably a hydrogen atom, a fluorine atom, a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a 2-ethylhexyl group, a 2-methylheptyl group, a 2-propylhexyl group, a 2-ethylheptyl group, a 2-methyloctyl group, a 2-butylhexyl group, a 2-ethyloctyl group, a 2-methylnonyl group, a 2-butylheptyl group, a 2-propyloctyl group, a 2-ethylnonyl group, a 2-pentylheptyl group, a 2-butyloctyl group, a 2-propylnonyl group, a 2-pentyloctyl group, a 2-butylnonyl group, a 2-methyldecyl group, a 2-hexyloctyl group, a 2-pentylnonyl group, a 2-hexylnonyl group, a 2-hexyldecyl group, a 2-heptylnonyl group, a 2-octyldodecyl group, a methoxy group, an ethoxy group, a propoxy group, a butoxy group, a pentyloxy group, a hexyloxy group, a heptyloxy group, an octyloxy group, a nonyloxy group, a decyloxy group, an undecyloxy group, a dodecyloxy group, a tridecyloxy group, a tetradecyloxy group, a pentadecyloxy group, a hexadecyloxy group, a heptadecyloxy group, an octadecyloxy group, a nonadecyloxy group, an icosyloxy group, an isopropyloxy group, a 1-(2-methylpropyl)oxy group, a 2-butyloxy group, a tert-butoxy group, a 2-ethylhexyloxy group or a 3,7-dimethyloctyloxy group, especially preferably a hydrogen atom, a fluorine atom, a methyl group or a methoxy group.

Examples of chalcogen atoms represented by A¹, A², A³ and A⁴ include oxygen atoms, sulfur atoms and selenium atoms.

The chalcogen atom represented by A¹ is preferably an oxygen atom, a sulfur atom or a selenium atom because this leads to higher carrier mobility of the conjugated polymer in this embodiment. More preferably, the chalcogen atom is an oxygen atom or a sulfur atom, even more preferably a sulfur atom.

The chalcogen atom represented by A² is preferably an oxygen atom, a sulfur atom or a selenium atom because this leads to higher carrier mobility of the conjugated polymer in this embodiment. More preferably, the chalcogen atom is an oxygen atom or a sulfur atom, even more preferably a sulfur atom.

A³ is preferably a sulfur atom, a selenium atom, C(R¹⁰)₂, C(H)(R¹⁰), Si(R¹⁰)₂ or NR¹⁰ because this leads to higher carrier mobility of the conjugated polymer in this embodiment. More preferably, A³ is C(R¹⁰)₂, C(H)(R¹⁰), Si (R¹⁰)₂ or NR¹⁰, even more preferably C (R¹⁰)₂ or C (H) (R¹⁰).

A⁴ is preferably an oxygen atom, a sulfur atom, a selenium atom or NR¹⁰ because this leads to higher carrier mobility of the conjugated polymer in this embodiment. More preferably, A⁴ is an oxygen atom or a sulfur atom, even more preferably a sulfur atom.

m represents 1 to 3, and preferably is 2 because this leads to higher film-forming properties and carrier mobility of the conjugated polymer in this embodiment.

p and q represent 0 or 1, and preferably are 1 because this leads to higher film-forming properties and carrier mobility of the conjugated polymer in this embodiment.

More specific examples of structural units represented by general formula (3) include the structural units presented below because they lead to higher solubility of the conjugated polymer in this embodiment. The present invention, however, is not limited to these. In the formulae, the ks represent natural numbers of 1 to 50 that may be the same or different from one another. The xs represent natural numbers of 2 to 24 that may be the same or different from one another. The ys represent natural numbers of 1 to 24 that may be the same or different from one another. Examples of specific structural units included in them are also presented below.

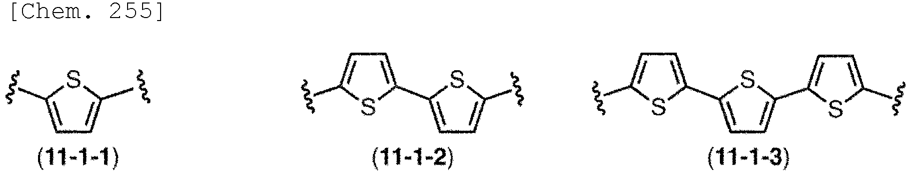

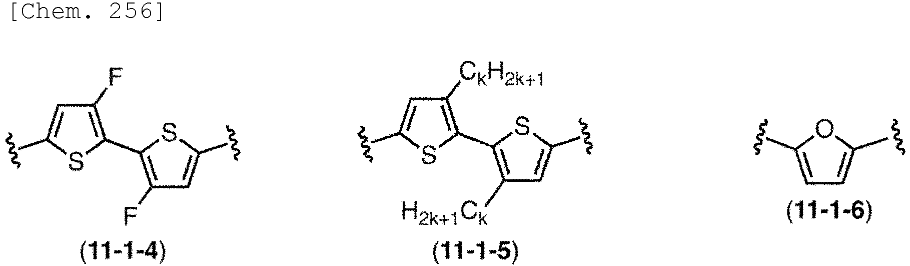

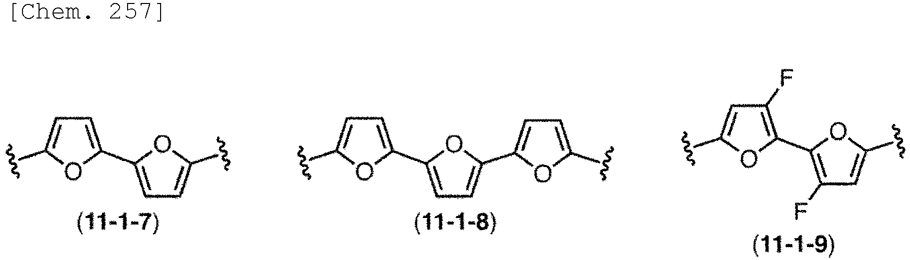

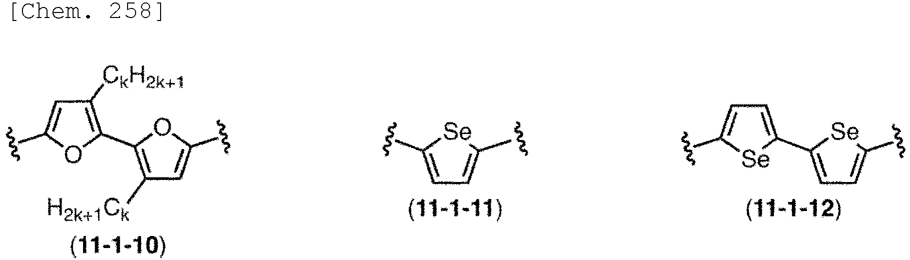

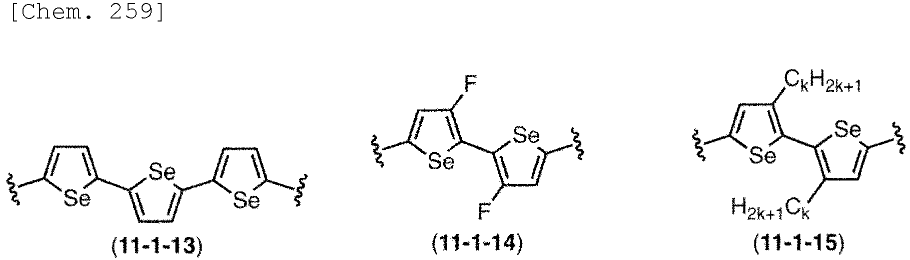

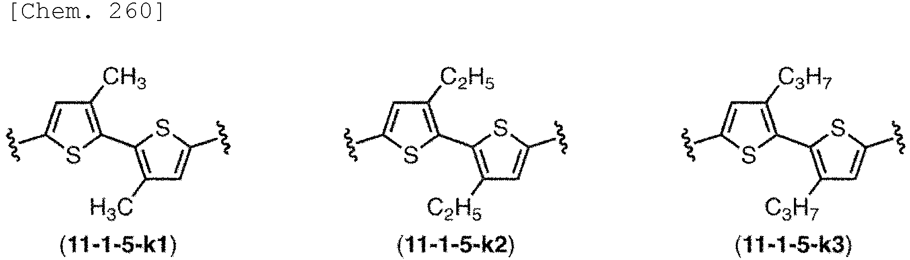

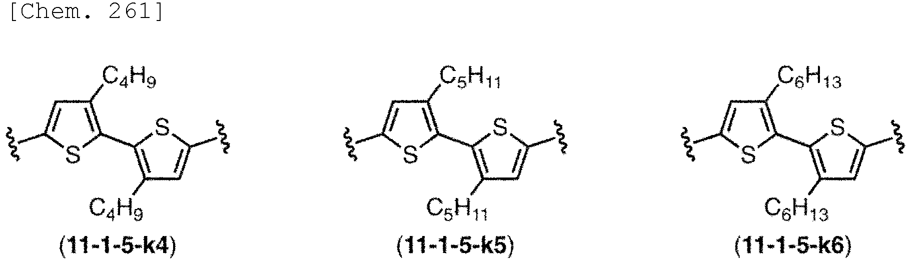

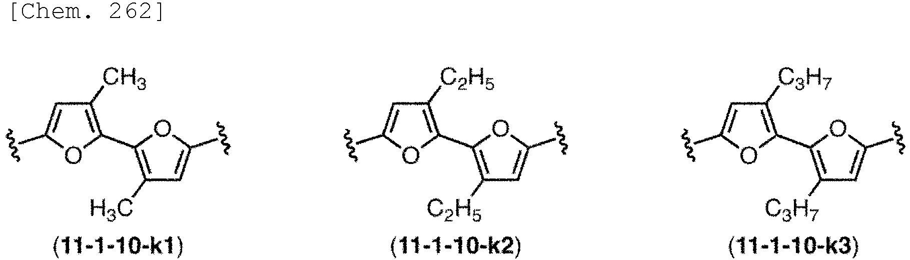

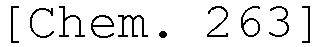

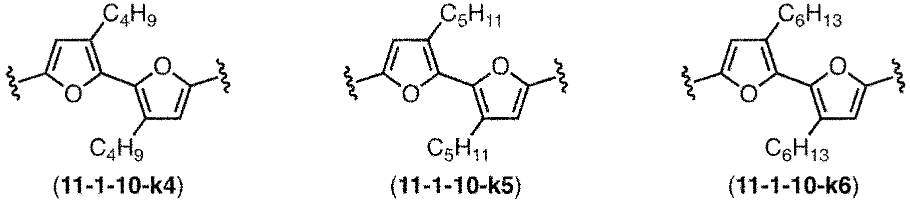

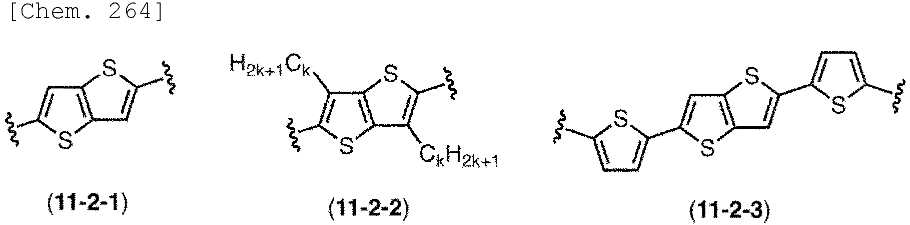

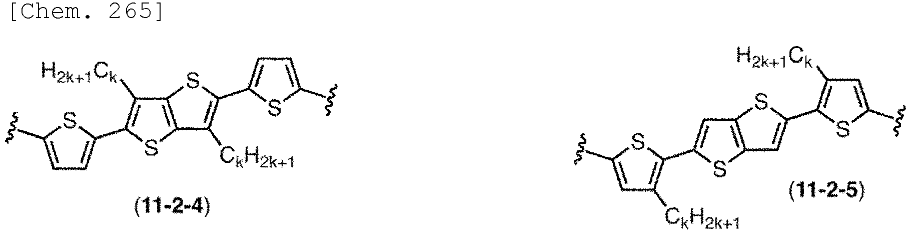

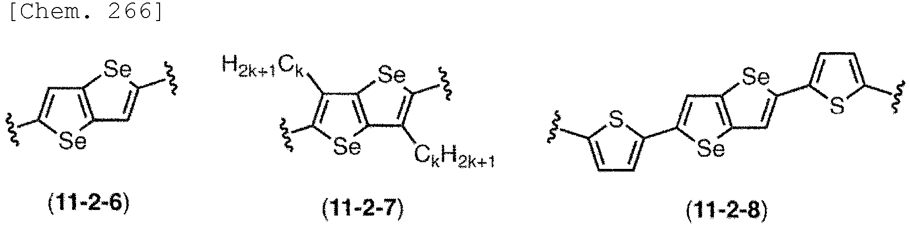

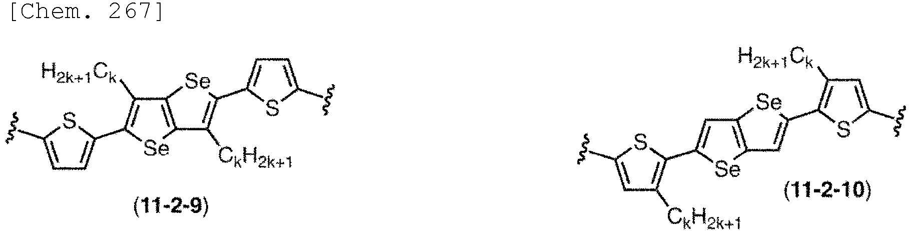

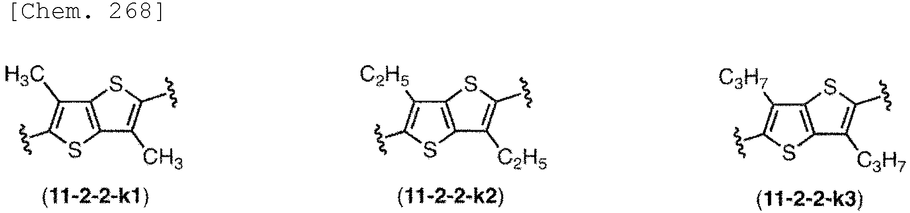

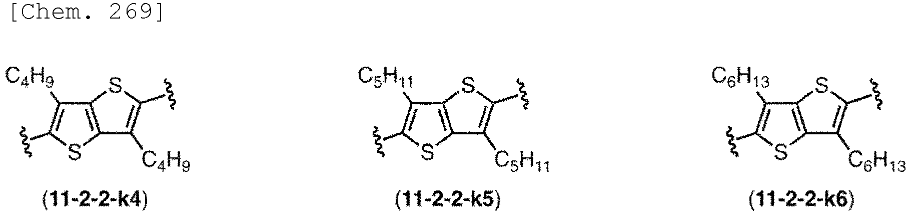

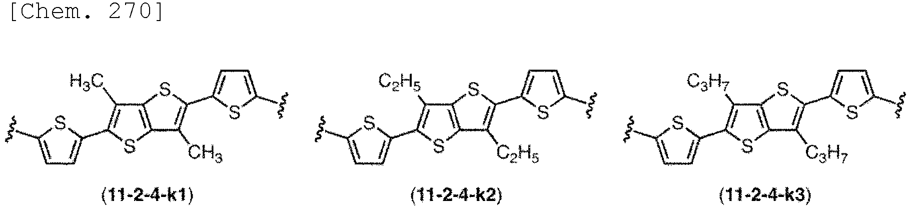

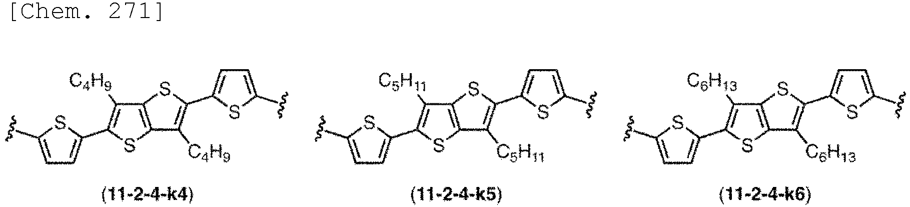

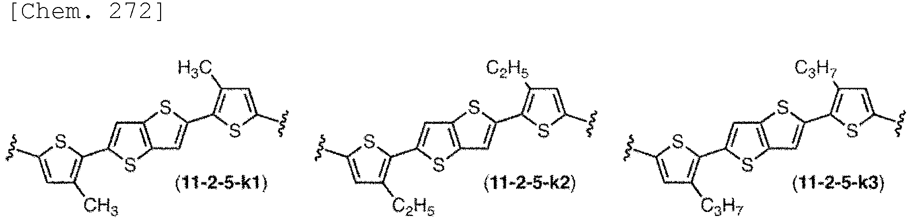

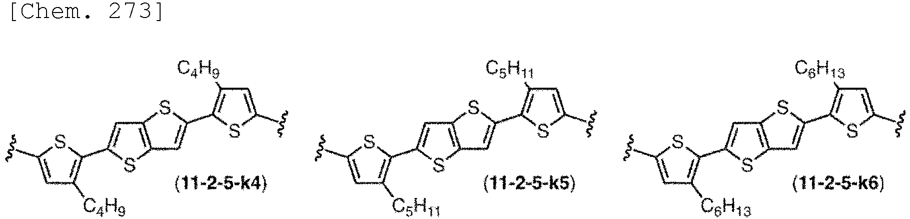

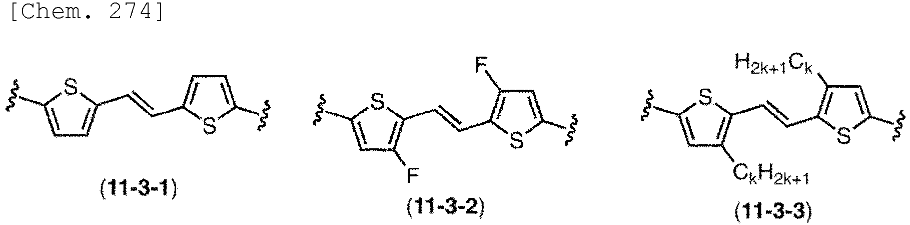

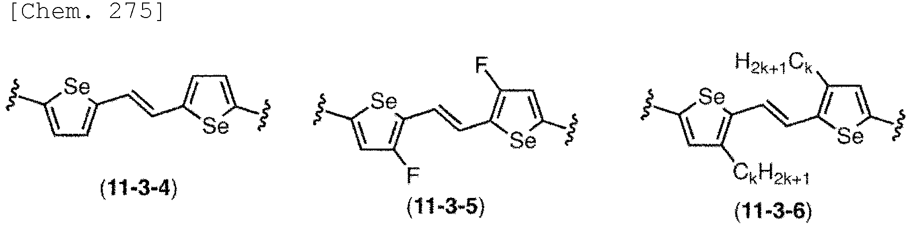

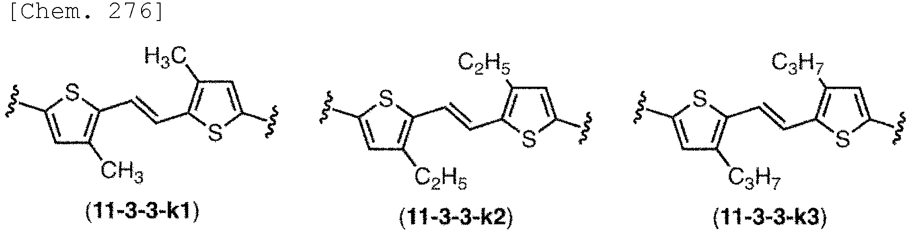

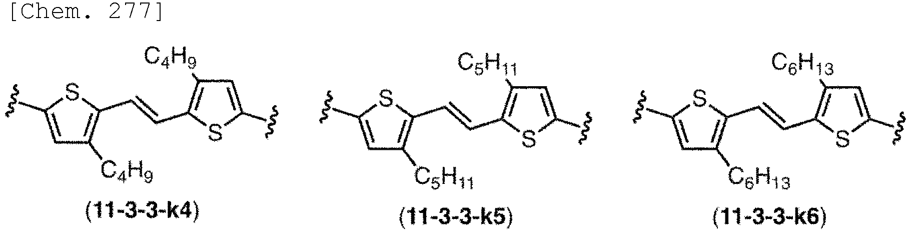

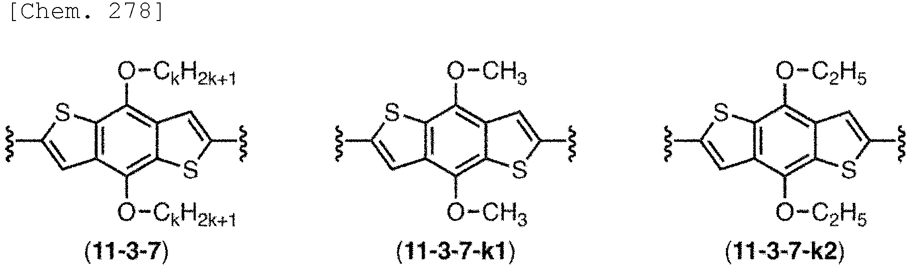

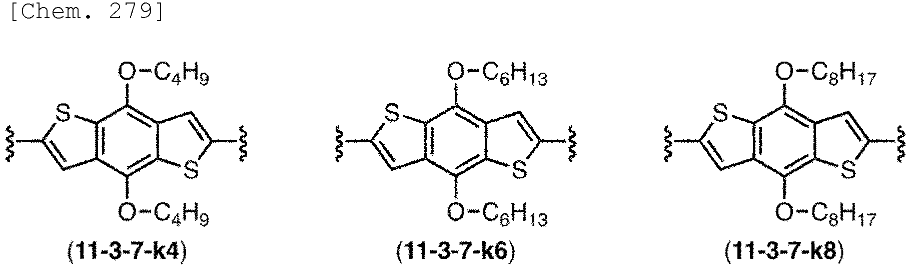

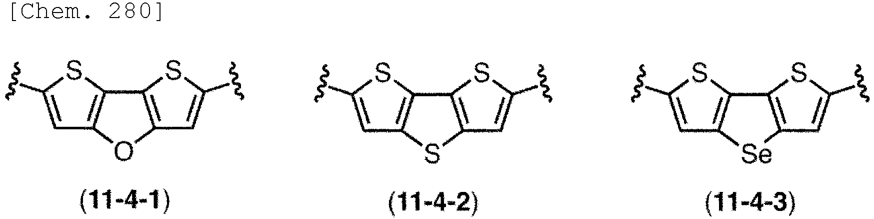

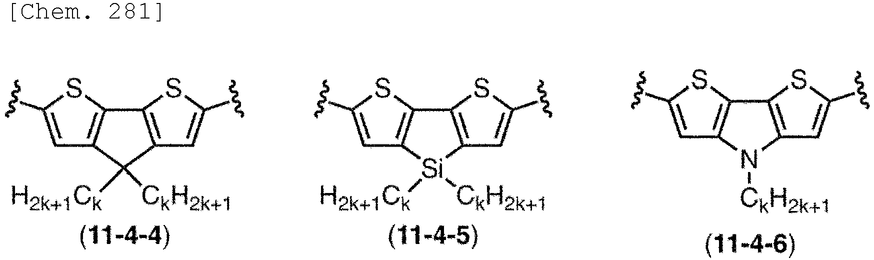

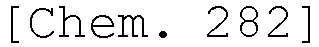

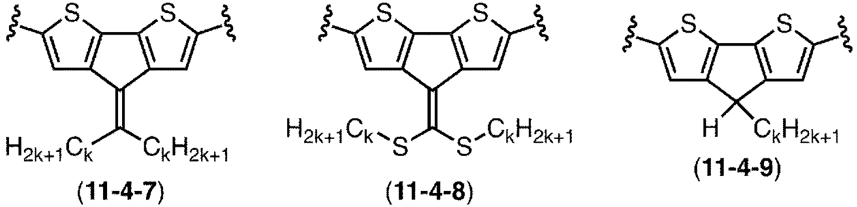

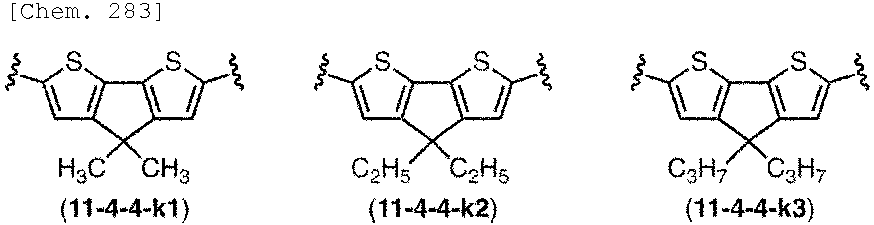

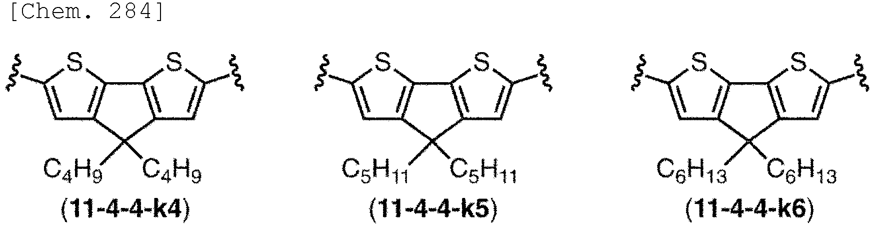

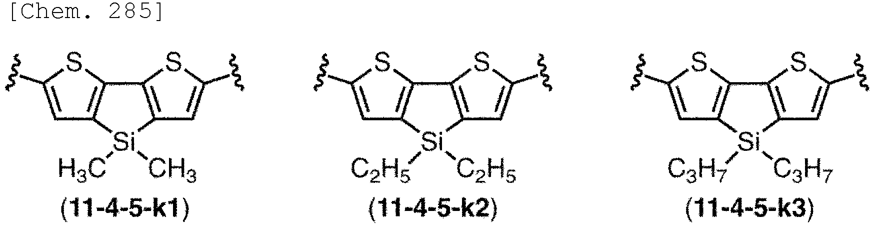

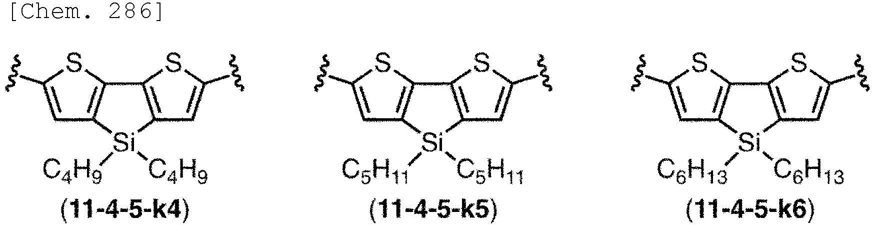

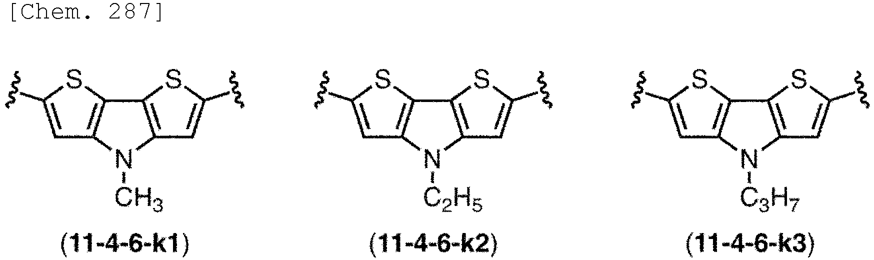

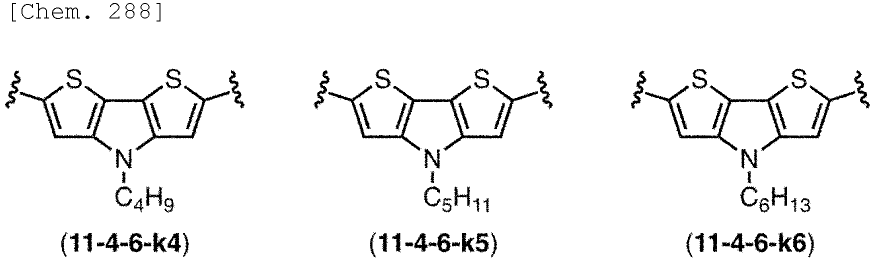

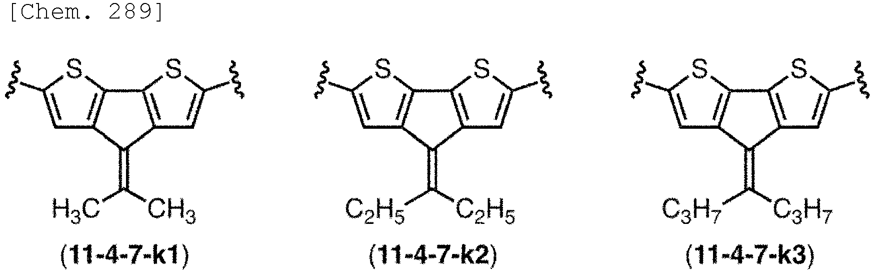

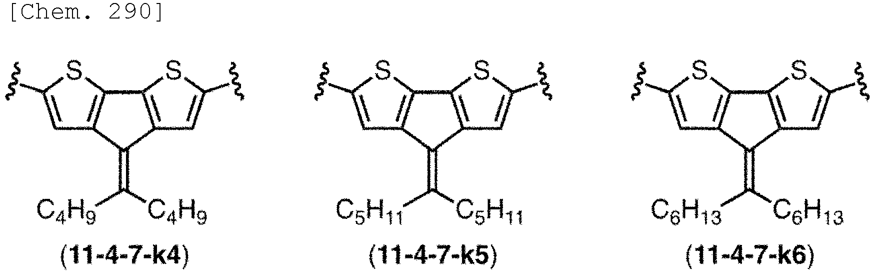

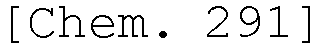

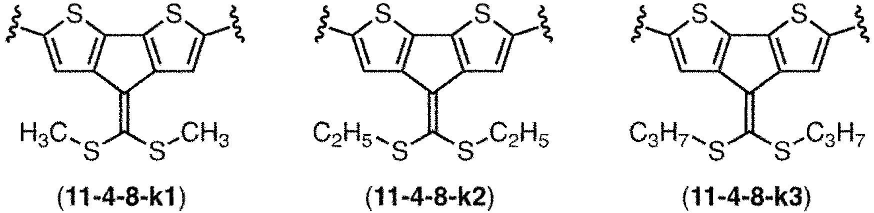

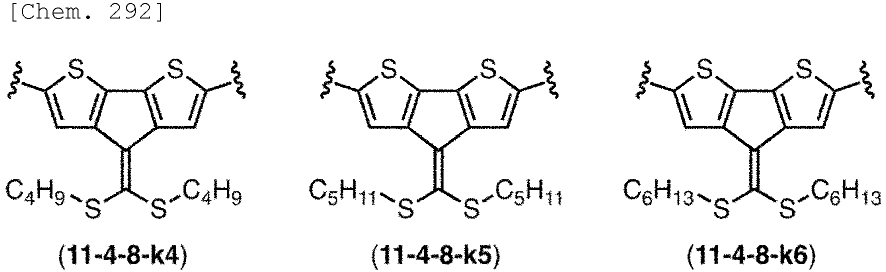

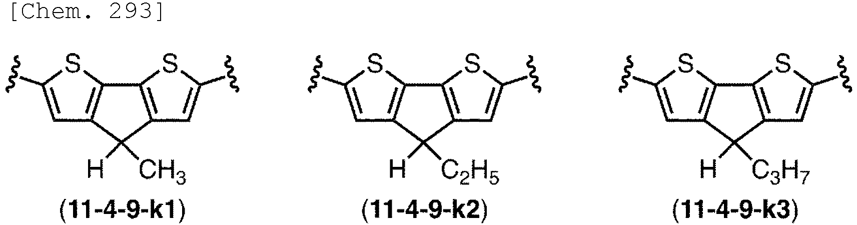

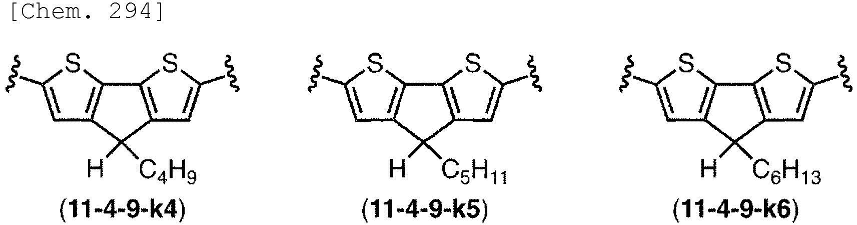

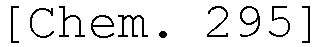

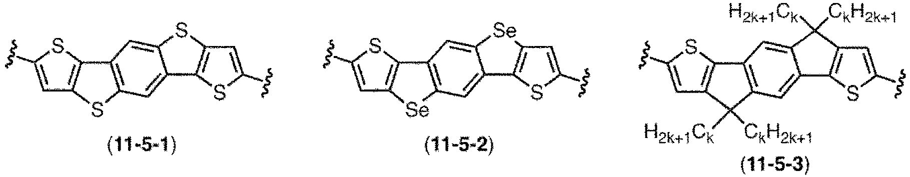

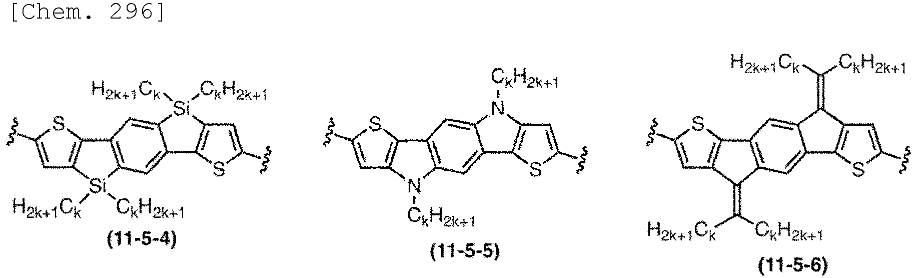

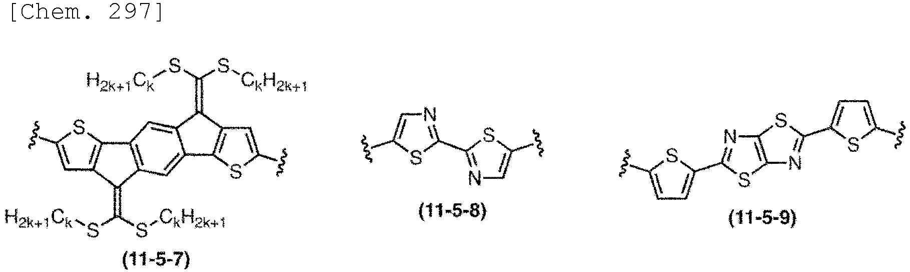

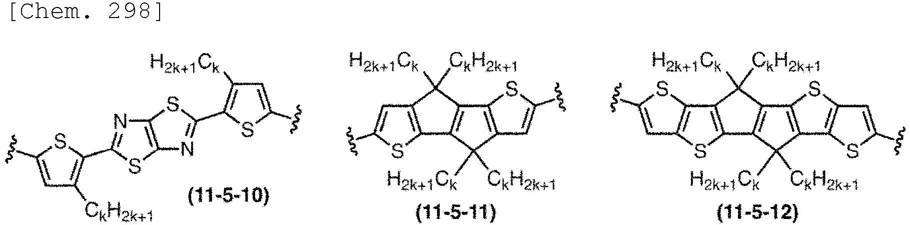

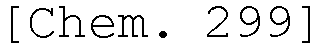

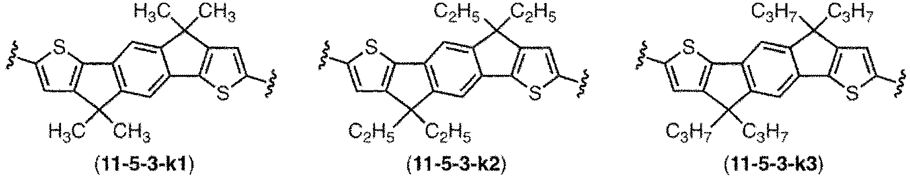

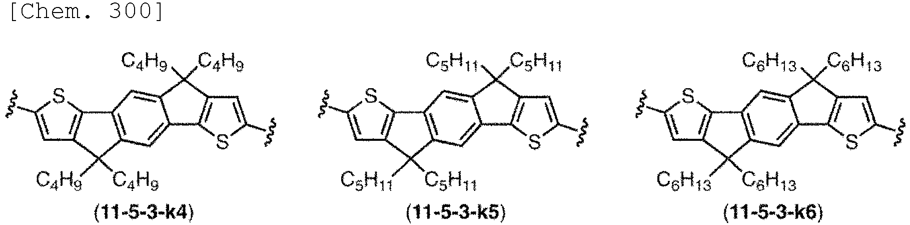

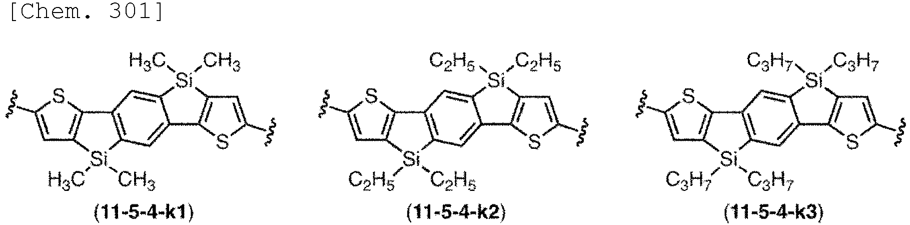

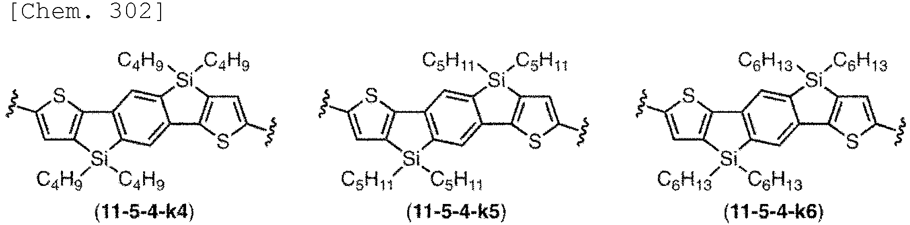

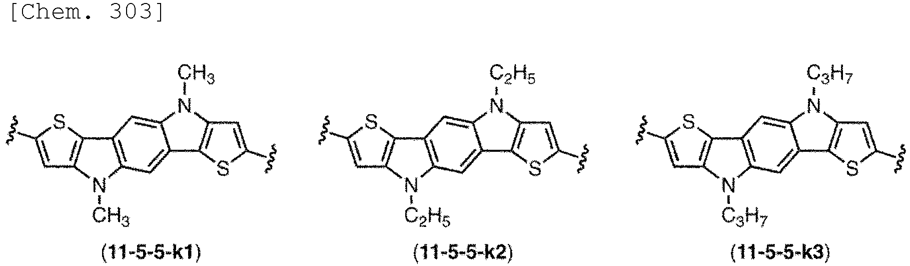

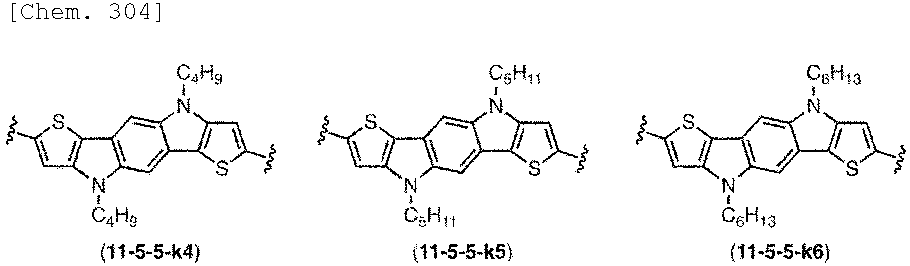

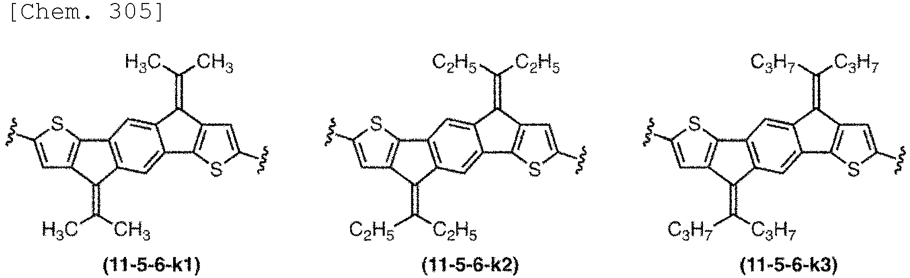

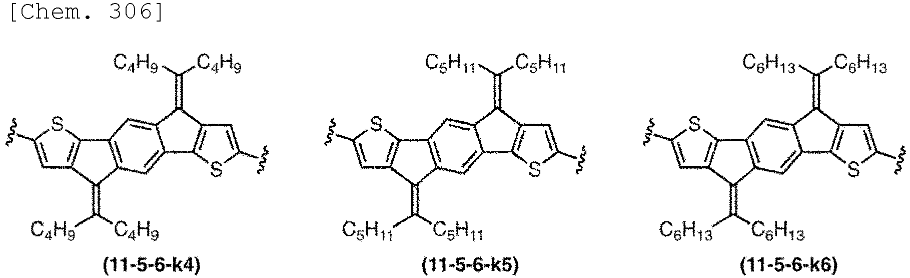

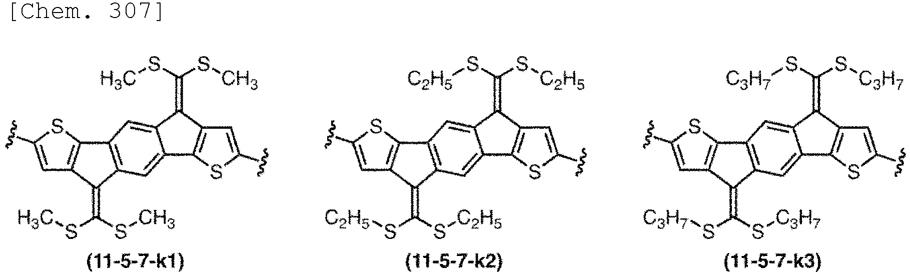

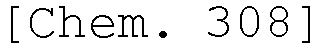

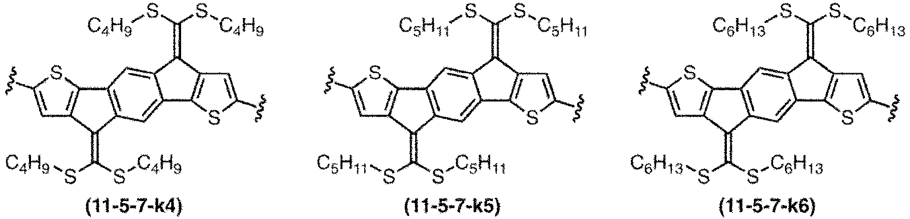

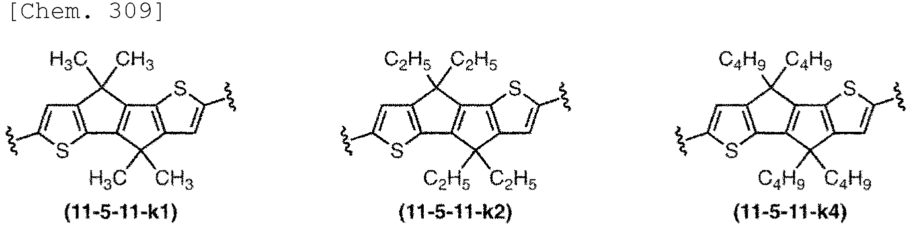

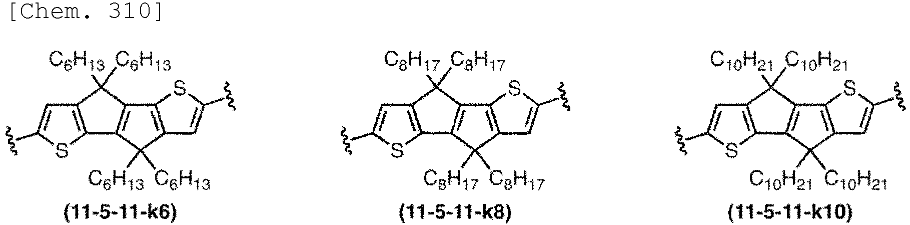

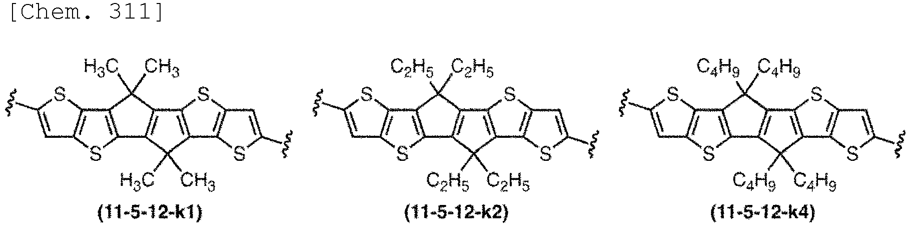

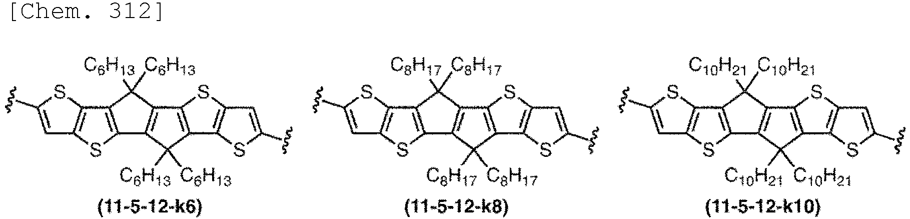

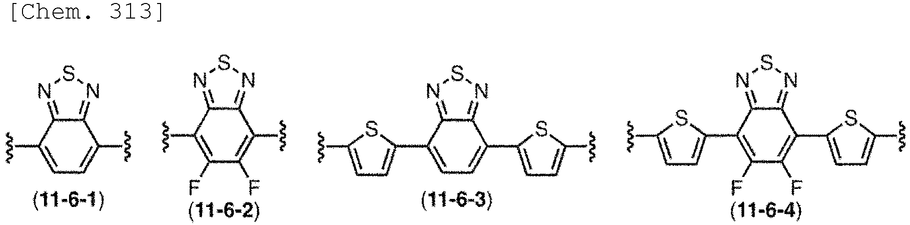

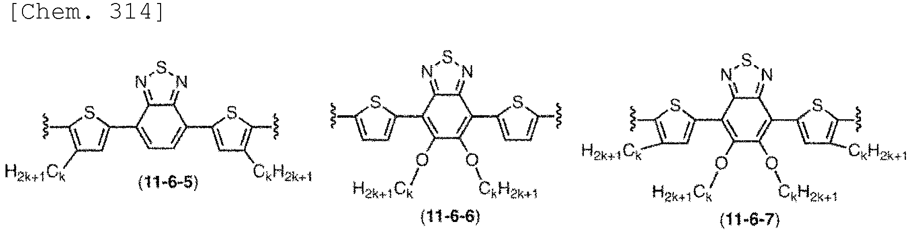

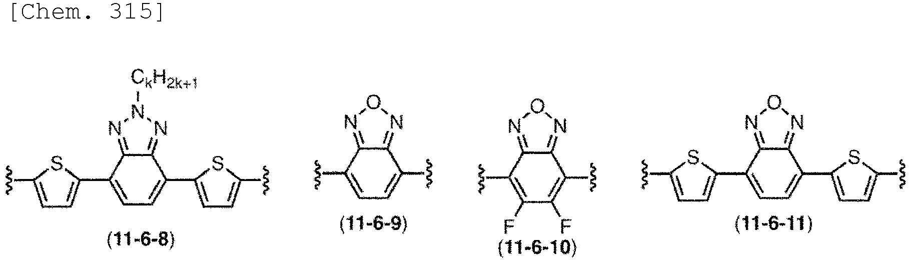

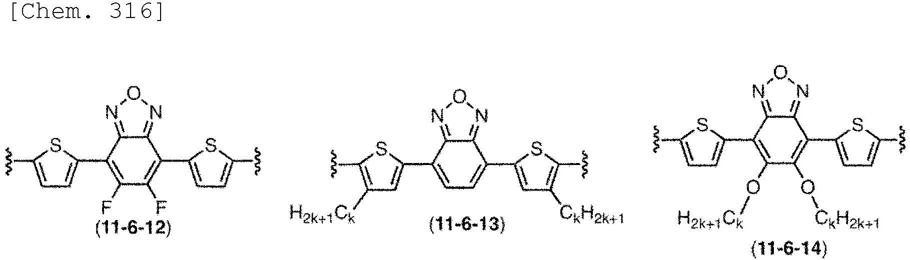

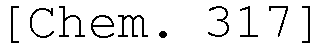

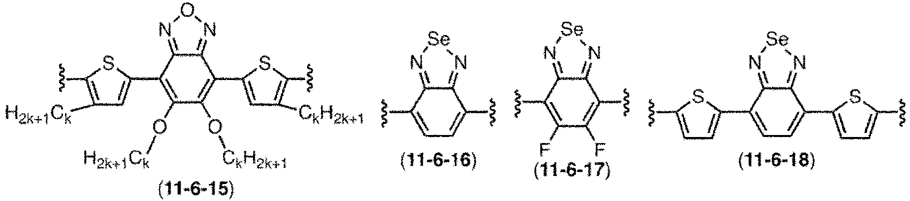

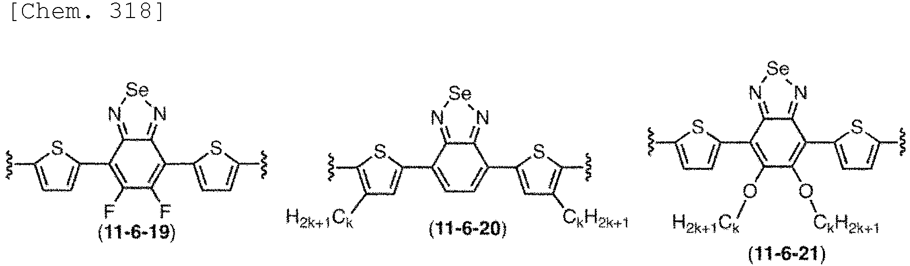

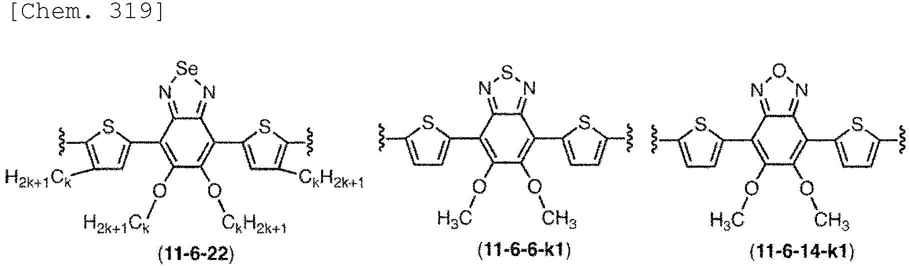

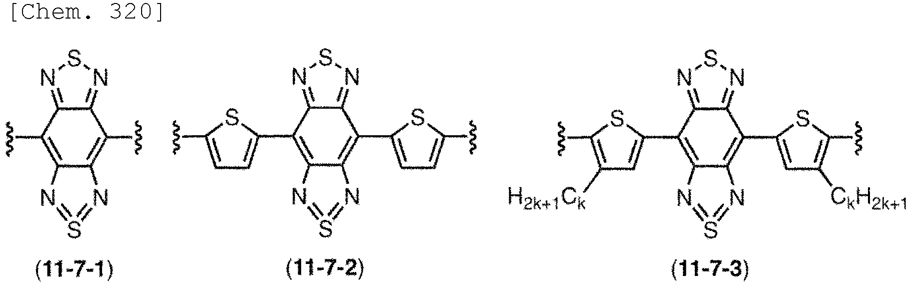

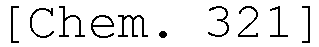

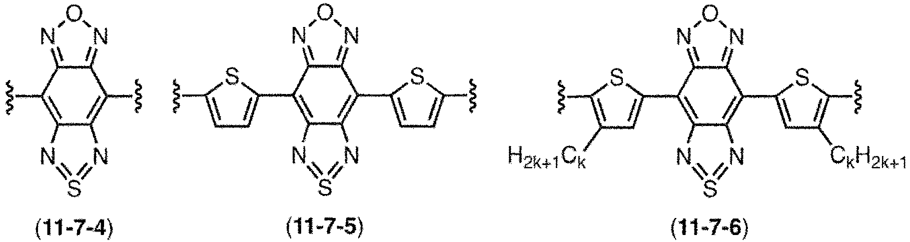

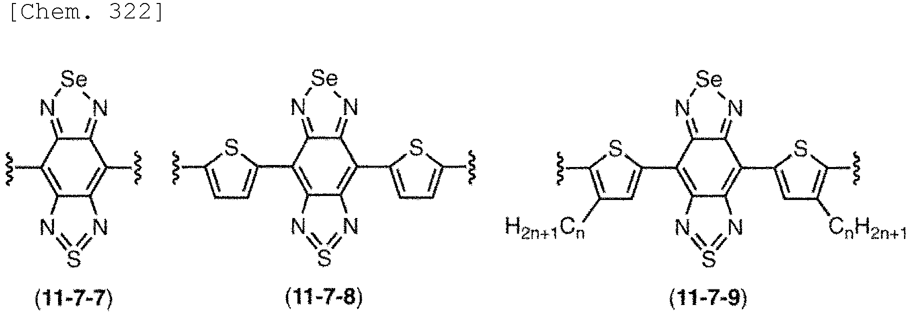

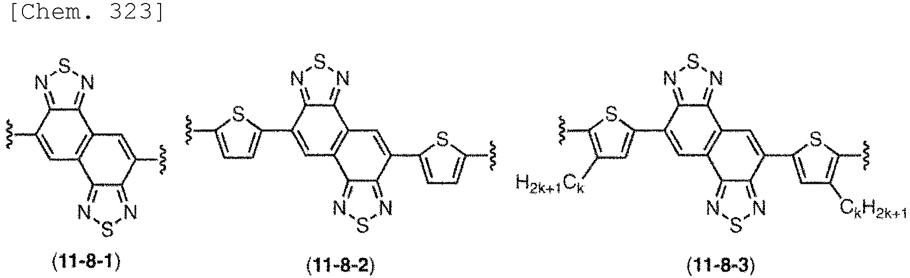

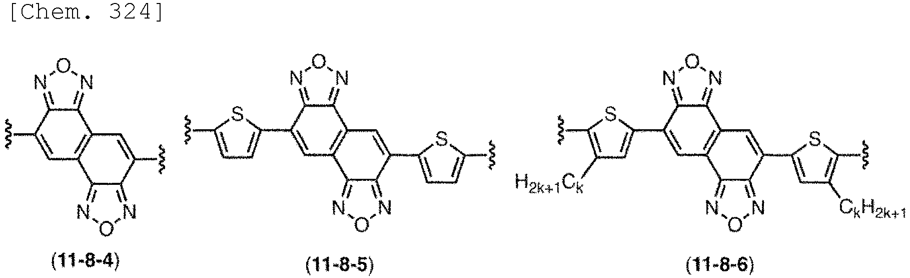

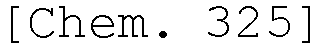

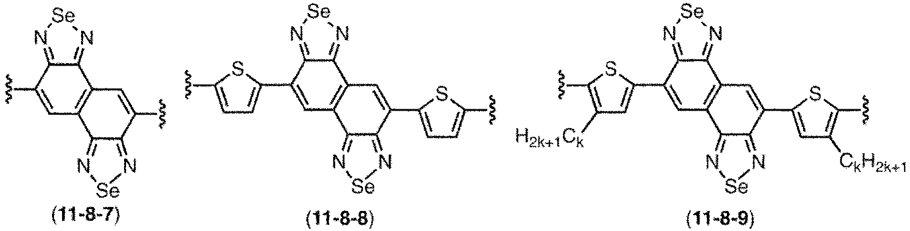

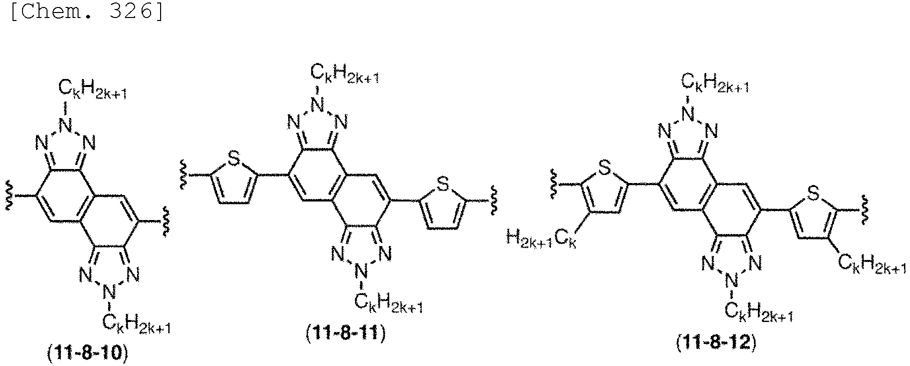

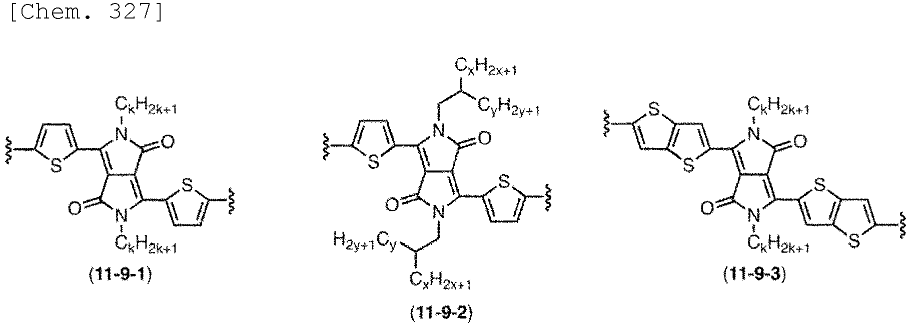

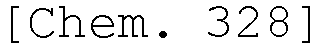

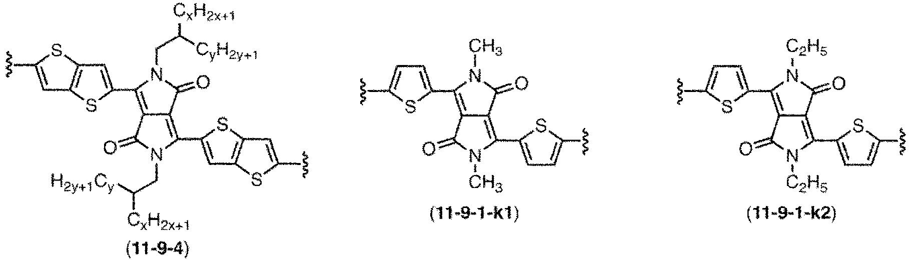

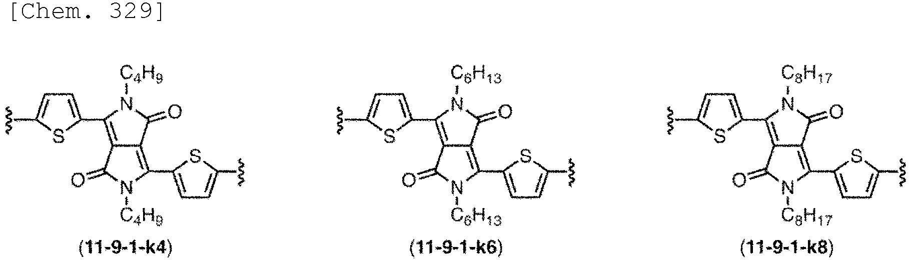

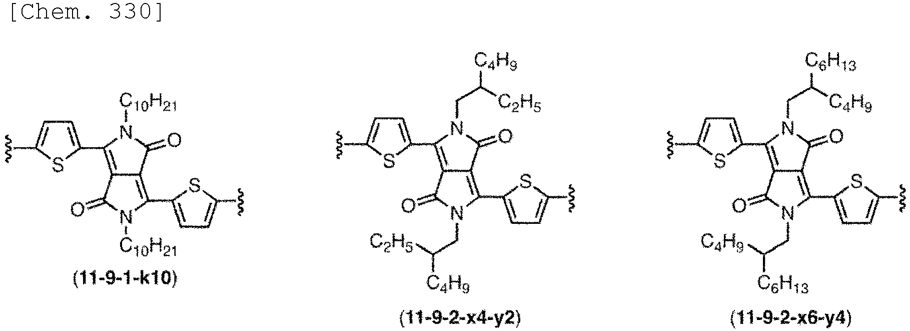

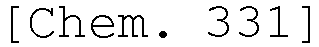

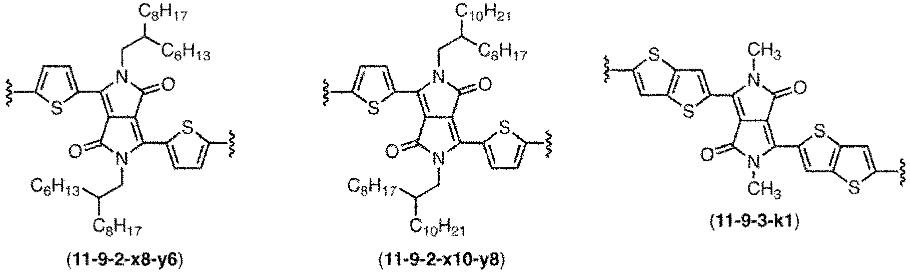

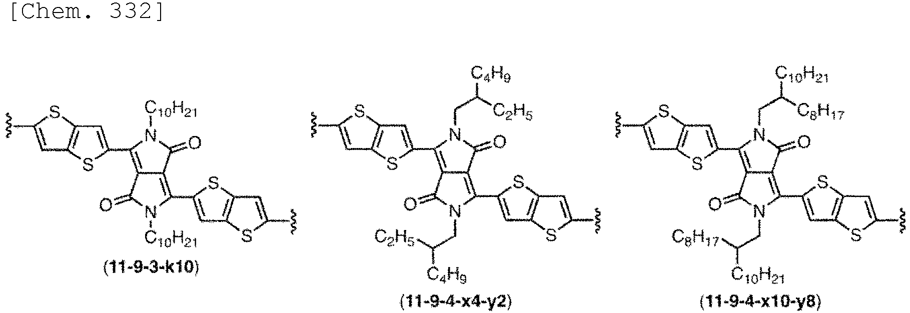

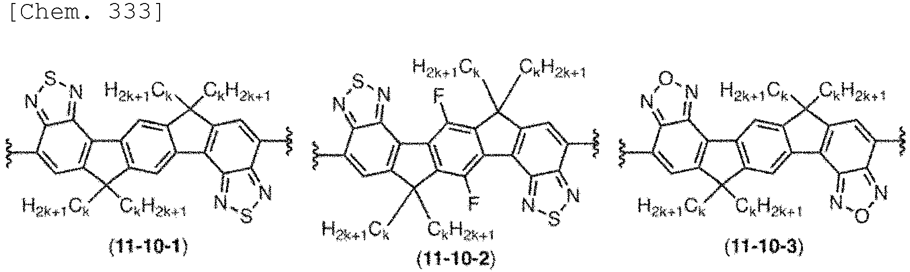

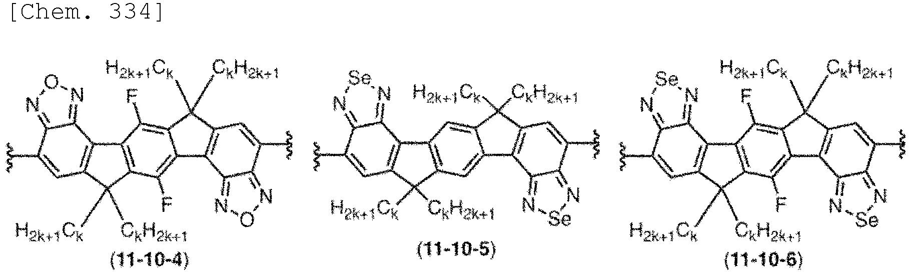

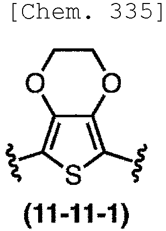

In these formulae, C_{R}H₂ₖ₊₁, CₓH₂ₓ₊₁ and C_{y}H_{2y+1} represent linear-chain alkyl groups.

The structural unit represented by general formula (3) is preferably any structural unit represented by formula (11-1-1) to formula (11-1-5), formula (11-2-1) to formula (11-2-5), formula (11-3-1) to formula (11-3-3), formula (11-3-7), formula (11-4-1) to formula (11-4-9), formula (11-5-1) to formula (11-5-7), formula (11-5-11), formula (11-5-12), formula (11-6-1) to formula (11-6-6), formula (11-7-1), formula (11-7-2), formula (11-8-1), formula (11-8-2), formula (11-9-1) to formula (11-9-4), formula (11-1-5-k1) to formula (11-1-5-k6), formula (11-2-4-k1) to formula (11-2-4-k6), formula (11-2-5-k1) to formula (11-2-5-k6), formula (11-3-3-k1) to formula (11-3-3-k6), formula (11-3-7-k1) to formula (11-3-7-k8), formula (11-4-4-k1) to formula (11-4-4-k6), formula (11-4-5-k1) to formula (11-4-5-k6), formula (11-4-6-k1) to formula (11-4-6-k6), formula (11-4-7-k1) to formula (11-4-7-k6), formula (11-4-8-k1) to formula (11-4-8-k6), formula (11-4-9-k1) to formula (11-4-9-k6), formula (11-5-3-k1) to formula (11-5-3-k6), formula (11-5-4-k1) to formula (11-5-4-k6), formula (11-5-5-k1) to formula (11-5-5-k6), formula (11-5-6-k1) to formula (11-5-6-k6), formula (11-5-7-k1) to formula (11-5-7-k6), formula (11-5-11-k1) to formula (11-5-11-k10), formula (11-5-12-k1) to formula (11-5-12-k10), formula (11-6-6-k1), formula (11-9-1-k1) to formula (11-9-1-k10), formula (11-9-2-x4-y2) to formula (11-9-2-x10-y8), formula (11-9-3-k1), formula (11-9-3-k10), formula (11-9-4-x4-y2) or formula (11-9-4-x10-y8) because this leads to higher film-forming properties and carrier mobility of the conjugated polymer in this embodiment. More preferably, the structural unit is any structural unit represented by formula (11-1-2), formula (11-1-4), formula (11-2-1), formula (11-2-3), formula (11-2-5), formula (11-3-1), formula (11-3-2), formula (11-3-7), formula (11-4-4), formula (11-4-5), formula (11-4-7), formula (11-4-8), formula (11-4-9), formula (11-5-3), formula (11-5-4), formula (11-5-6), formula (11-5-7), formula (11-5-11), formula (11-5-12), formula (11-6-3), formula (11-6-4), formula (11-7-2), formula (11-8-2), formula (11-9-1), (11-9-2), formula (11-2-5-k1) to formula (11-2-5-k6), formula (11-3-3-k1) to formula (11-3-3-k6), formula (11-3-7-k1) to formula (11-3-7-k8), formula (11-4-4-k1) to formula (11-4-4-k6), formula (11-4-5-k1) to formula (11-4-5-k6), formula (11-4-7-k1) to formula (11-4-7-k6), formula (11-4-8-k1) to formula (11-4-8-k6), formula (11-4-9-k1) to formula (11-4-9-k6), formula (11-5-3-k1) to formula (11-5-3-k6), formula (11-5-4-k1) to formula (11-5-4-k6), formula (11-5-6-k1) to formula (11-5-6-k6), formula (11-5-7-k1) to formula (11-5-7-k6), formula (11-5-11-k1) to formula (11-5-11-k10), formula (11-5-12-k1) to formula (11-5-12-k10), formula (11-9-1-k1) to formula (11-9-1-k10) or formula (11-9-2-x4-y2) to formula (11-9-2-x10-y8), even more preferably any structural unit represented by formula (11-1-2), formula (11-2-1), formula (11-3-1), formula (11-3-7), formula (11-4-4), formula (11-4-7), formula (11-4-8), formula (11-4-9), formula (11-5-3), formula (11-9-1), formula (11-9-2), formula (11-3-3-k1) to formula (11-3-3-k6), formula (11-3-7-k1) to formula (11-3-7-k8), formula (11-4-4-k1) to formula (11-4-4-k6), formula (11-4-7-k1) to formula (11-4-7-k6), formula (11-4-8-k1) to formula (11-4-8-k6), formula (11-4-9-k1) to formula (11-4-9-k6), formula (11-5-3-k1) to formula (11-5-3-k6), formula (11-9-1-k1) to formula (11-9-1-k10) or formula (11-9-2-x4-y2) to formula (11-9-2-x10-y8), especially preferably any structural unit represented by formula (11-1-2), formula (11-2-1), formula (11-3-1), formula (11-3-3-k4), formula (11-3-3-k6), formula (11-3-7-k8), formula (11-4-4-k1), formula (11-4-7-k1), formula (11-4-8-k1), formula (11-4-8-k3), formula (11-4-9-k1), formula (11-5-3-k1), formula (11-9-1-k1), formula (11-9-1-k8) or formula (11-9-2-x10-y8).

For the conjugated polymer in this embodiment, there is no specific restriction on the order of structural units, provided that it includes a structural unit represented by general formula (2) and a structural unit represented by general formula (3). Examples include copolymerization forms such as alternating, random, block or gradient. The conjugated polymer in this embodiment is preferably one having a structure in which a structural unit represented by general formula (2) and a structural unit represented by general formula (3) are alternately repeated because this leads to higher film-forming properties and mobility of the conjugated polymer. This structure can be represented by general formula (24).

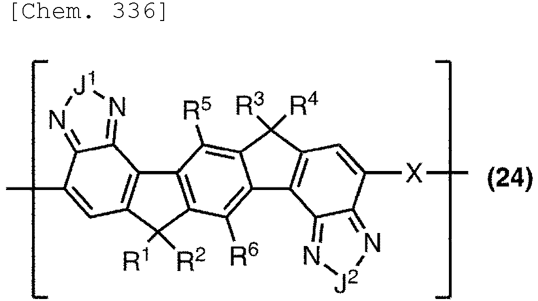

(In the formula, R¹, R², R³, R⁴, R⁵, R⁶, J¹, J² and X have the same meanings as described above.)

Examples of structural units represented by general formula (24) include the structural units presented below. The present invention, however, is not limited to these. In the formulae, the ns represent natural numbers of 1 to 50 that may be the same or different from one another. The ks represent natural numbers of 1 to 50 that may be the same or different from one another. The xs represent natural numbers of 2 to 24 that may be the same or different from one another. The ys represent natural numbers of 1 to 24 that may be the same or different from one another. The zs represent natural numbers of 1 to 20 that may be the same or different from one another. Examples of specific structural units included in them are also presented below.

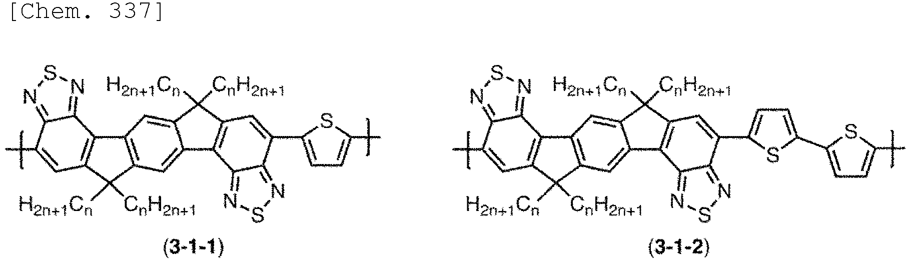

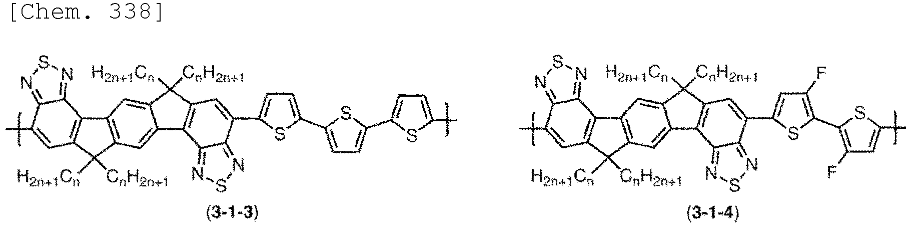

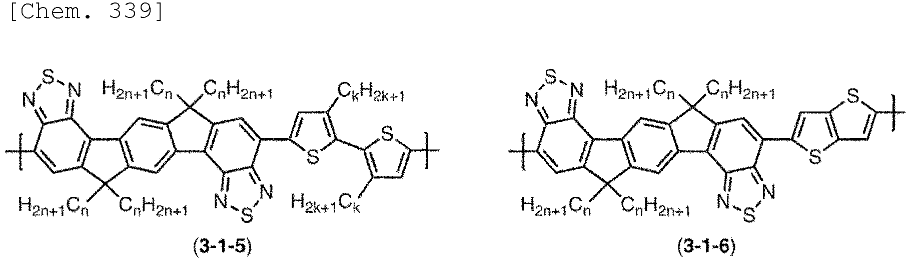

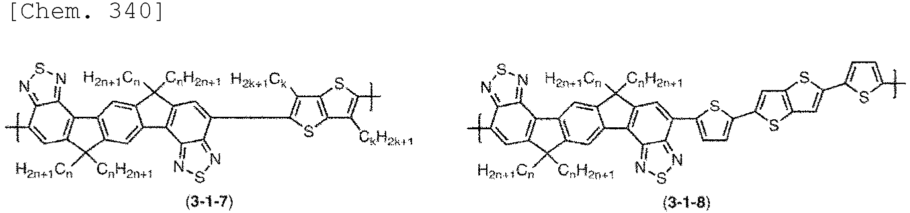

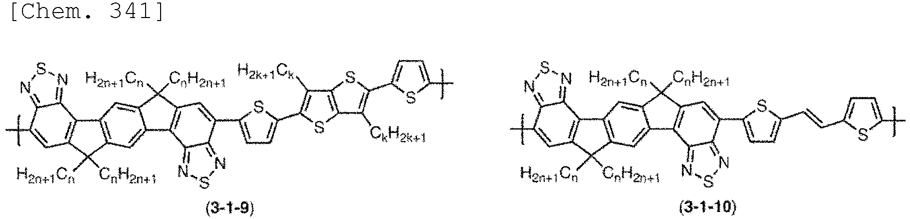

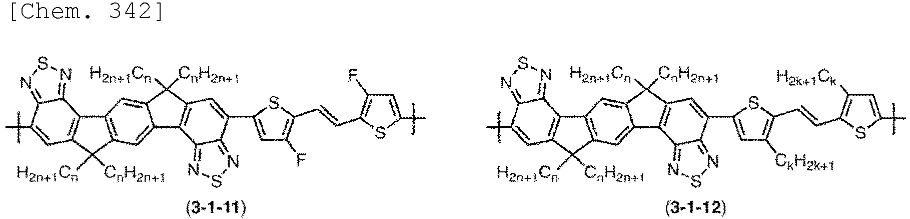

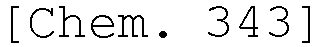

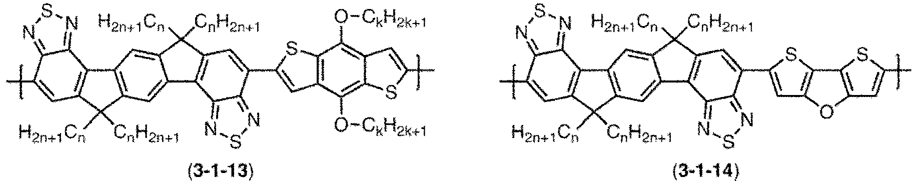

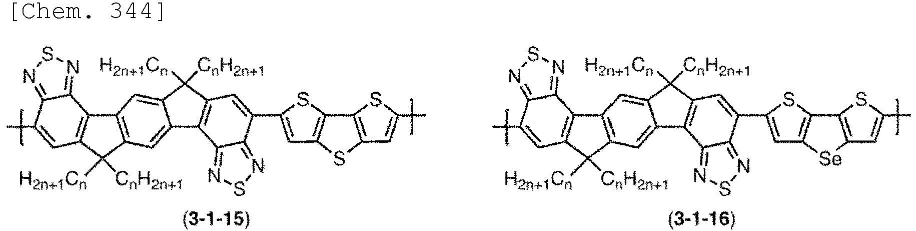

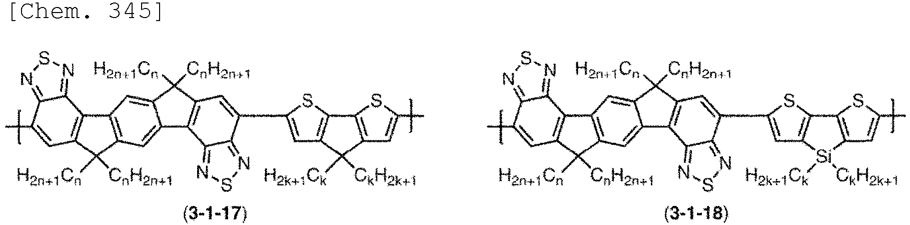

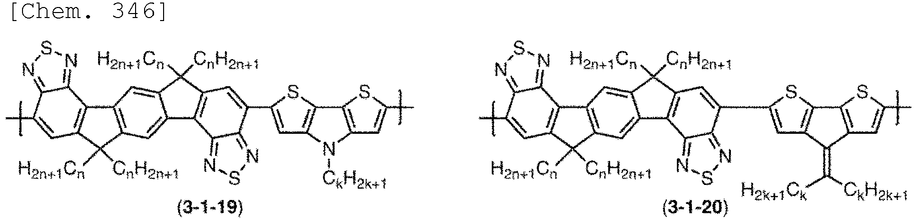

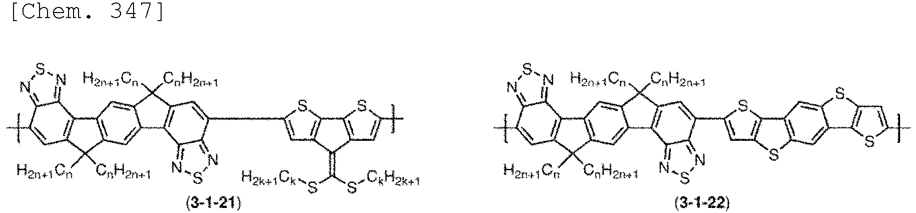

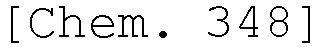

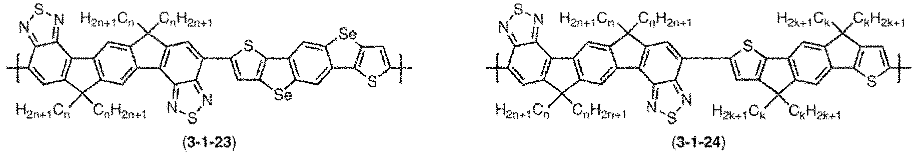

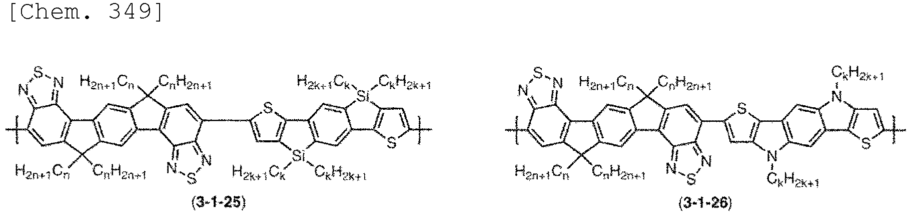

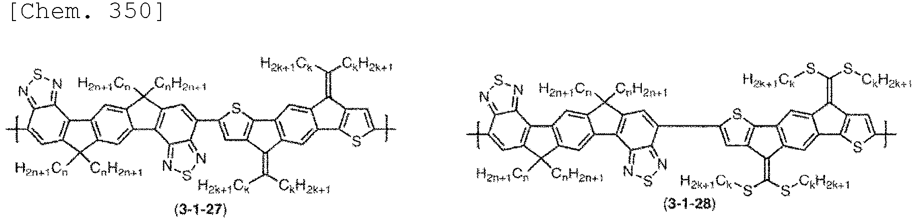

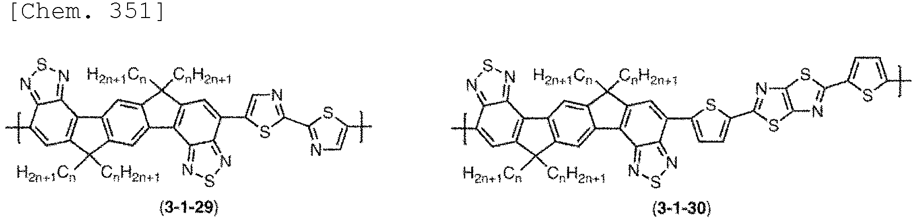

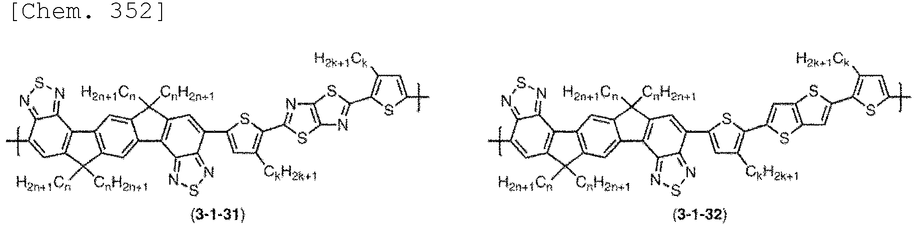

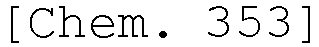

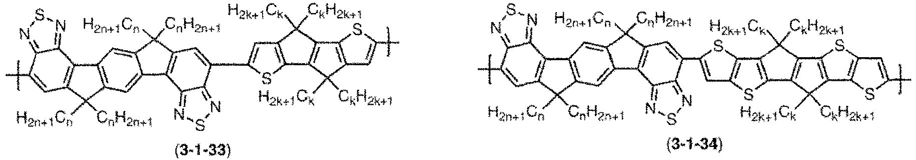

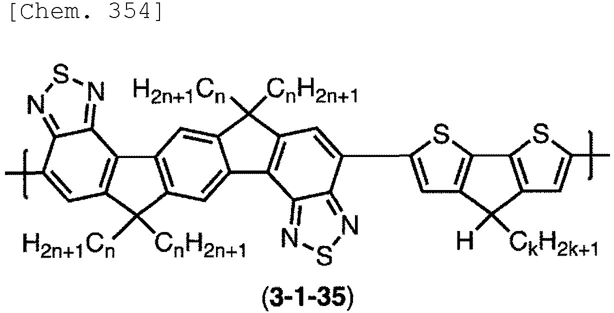

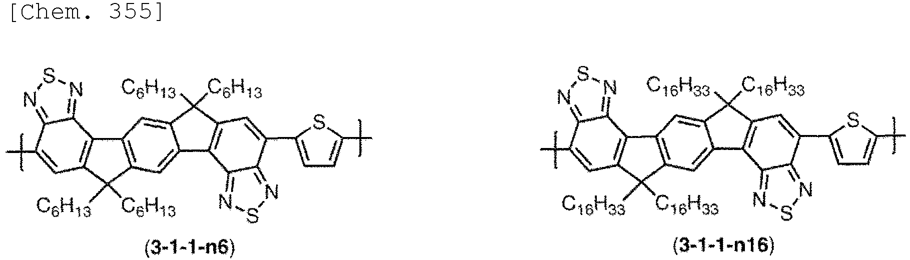

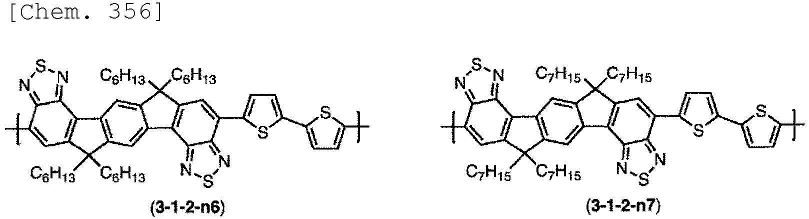

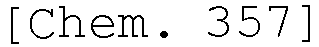

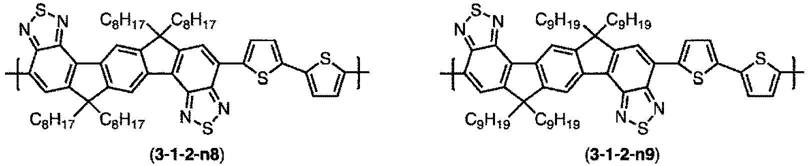

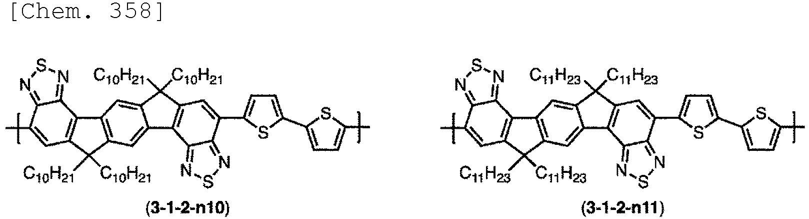

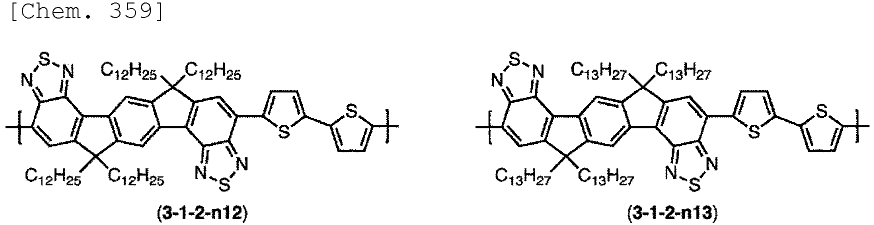

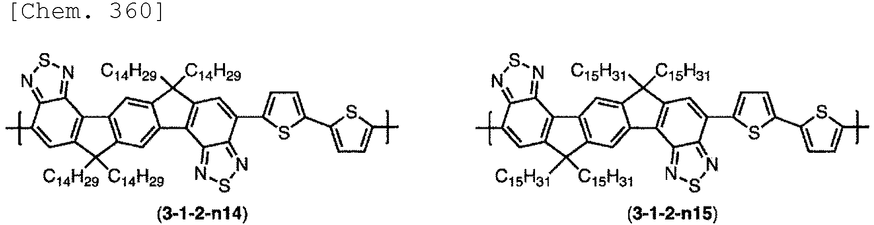

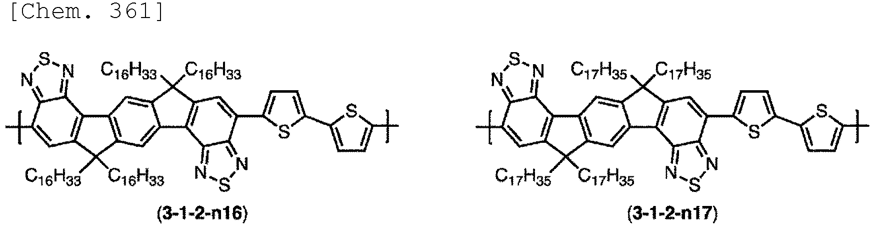

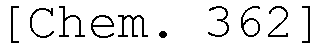

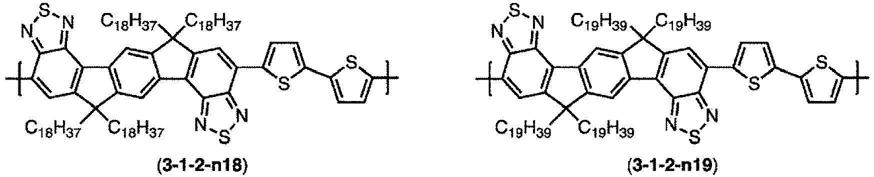

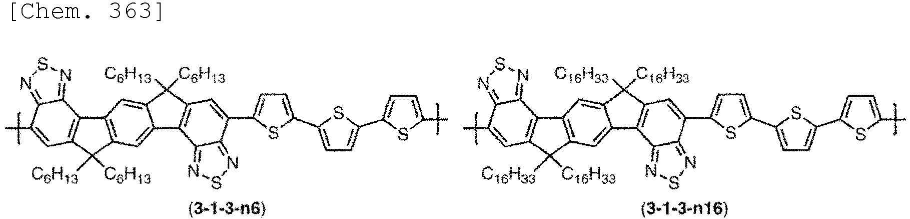

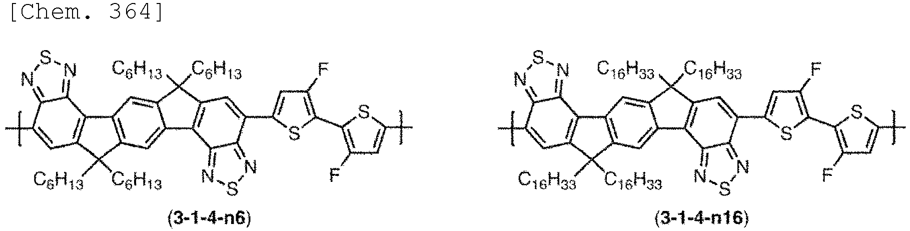

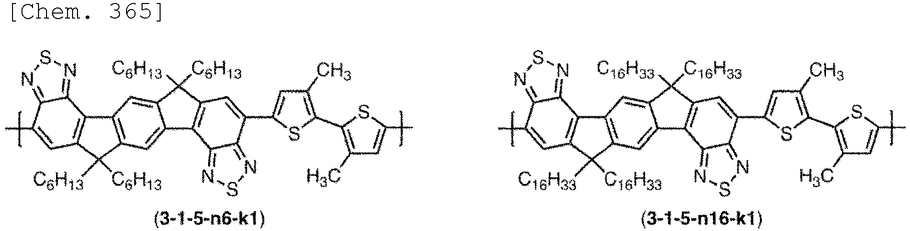

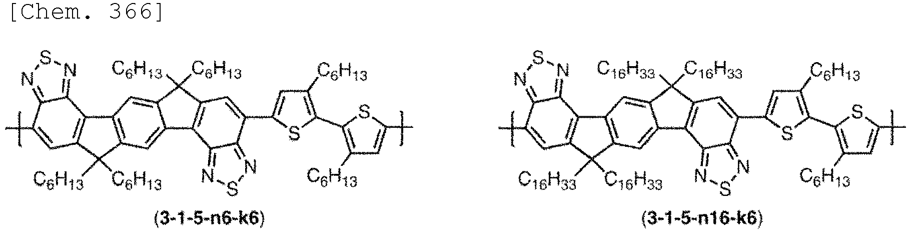

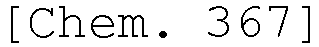

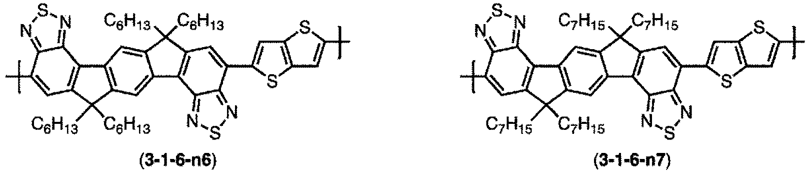

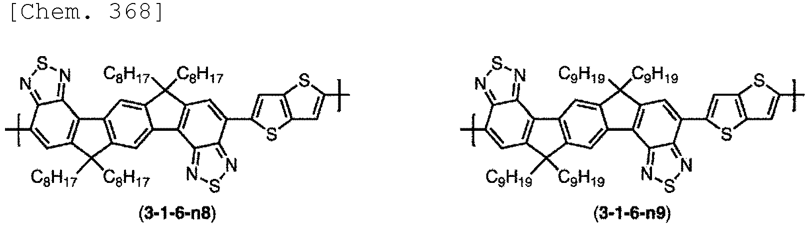

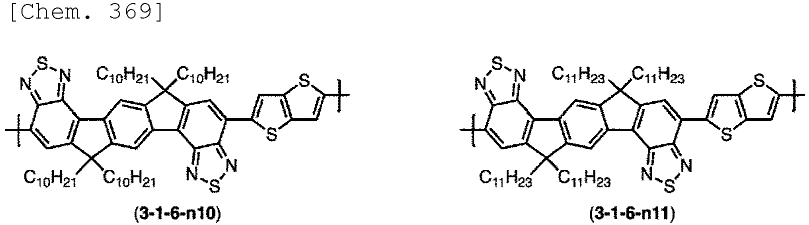

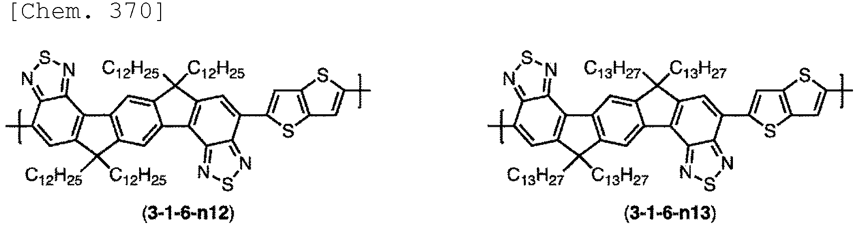

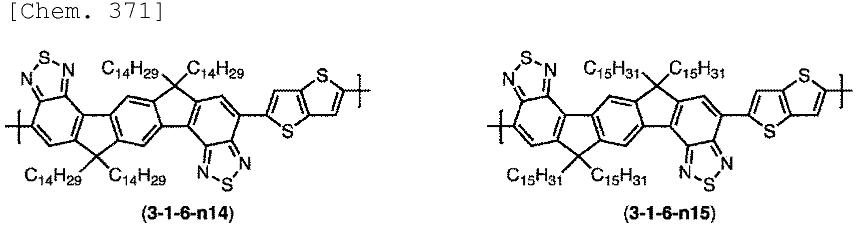

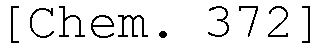

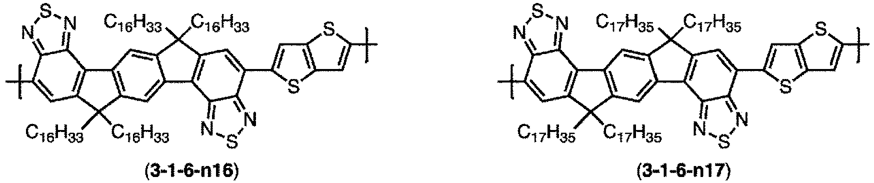

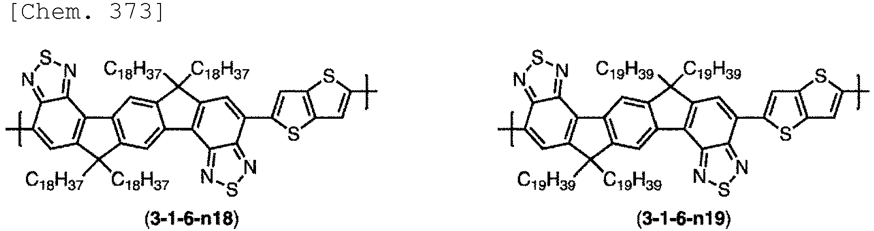

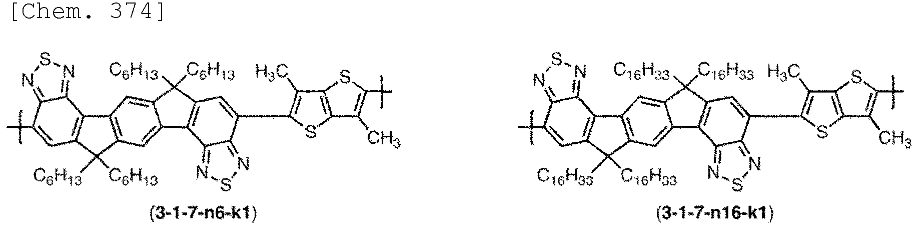

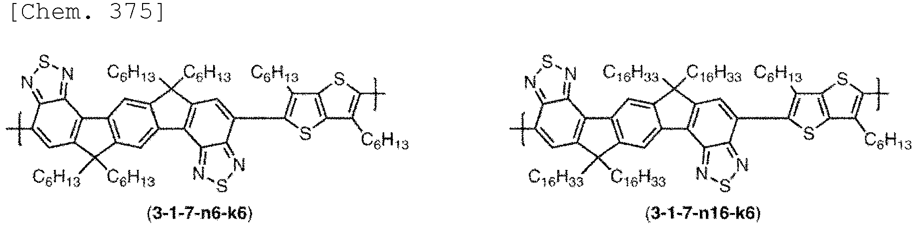

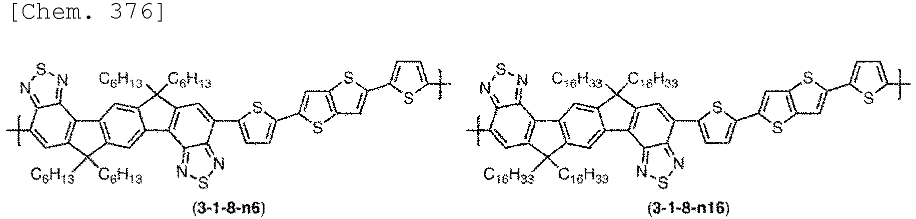

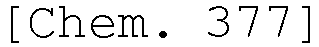

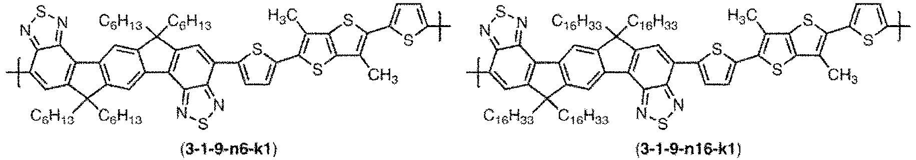

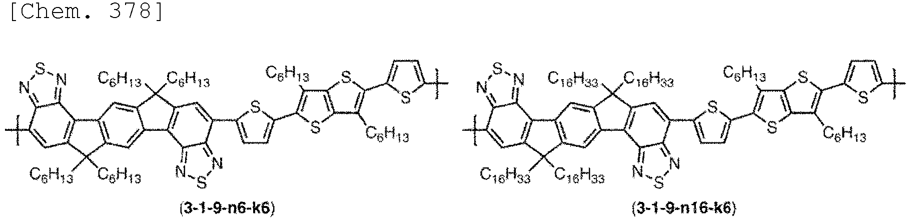

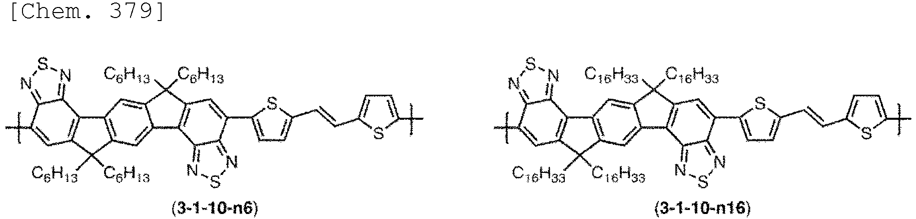

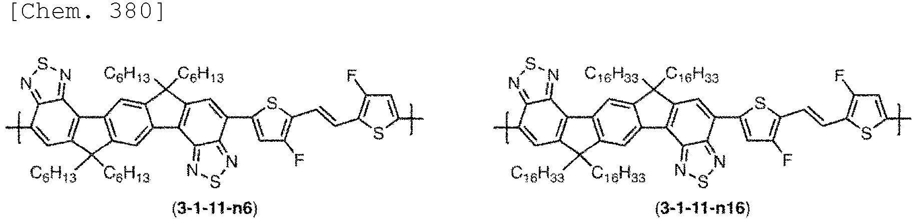

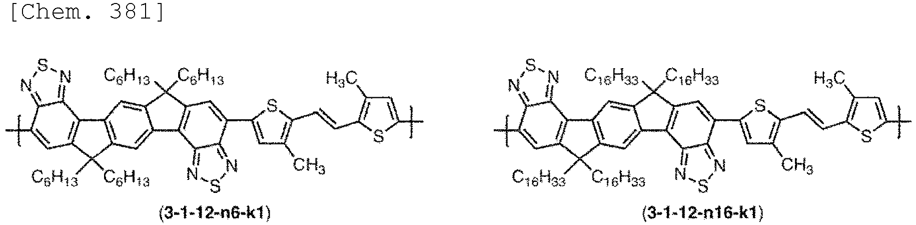

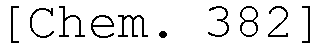

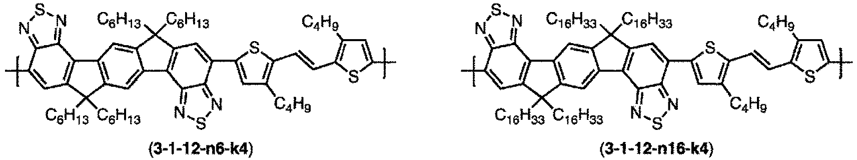

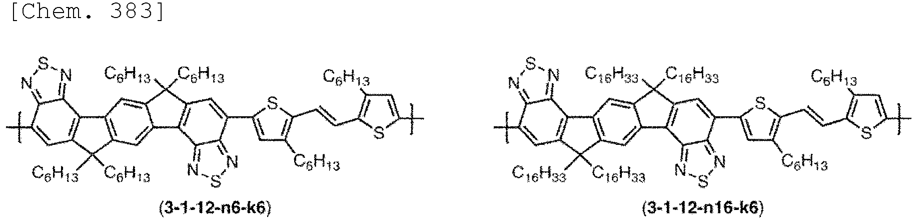

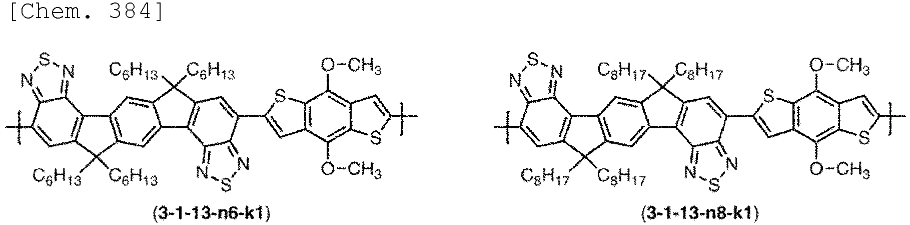

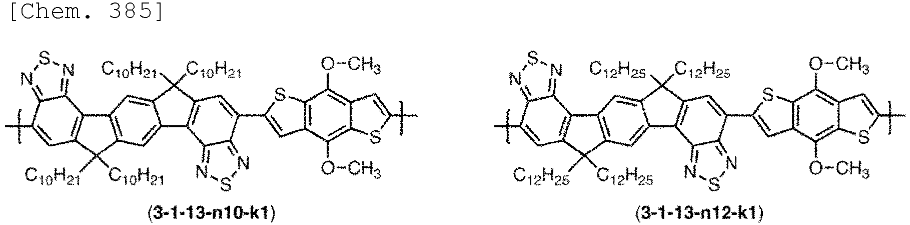

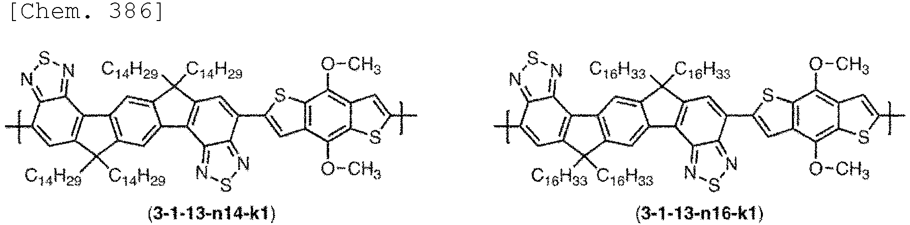

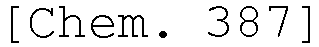

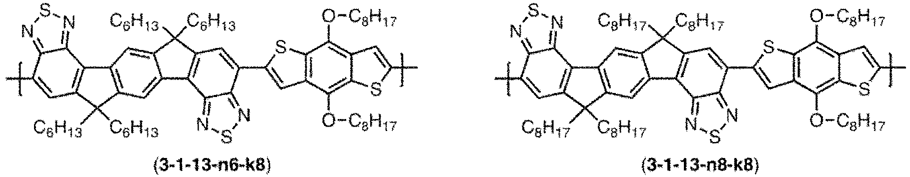

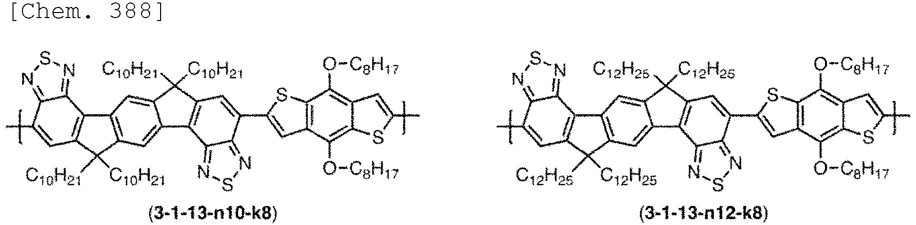

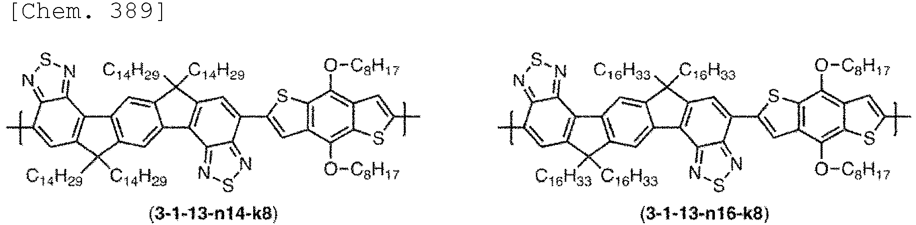

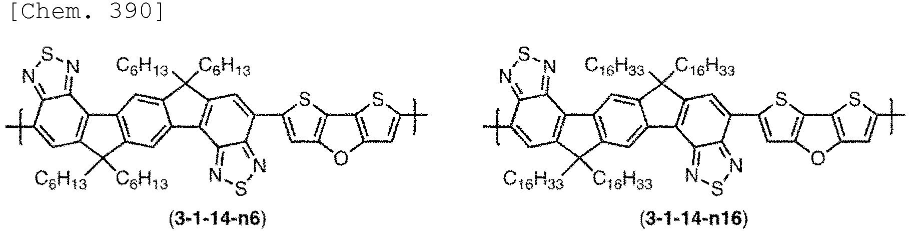

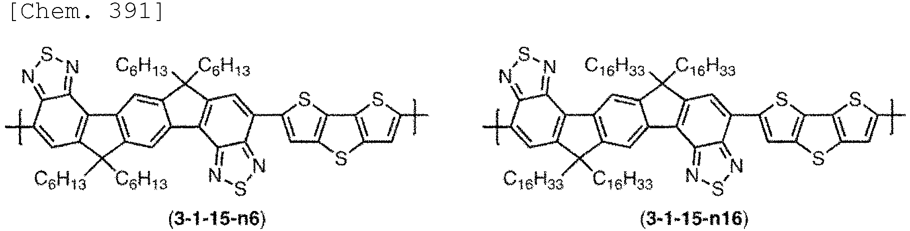

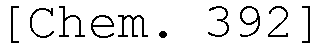

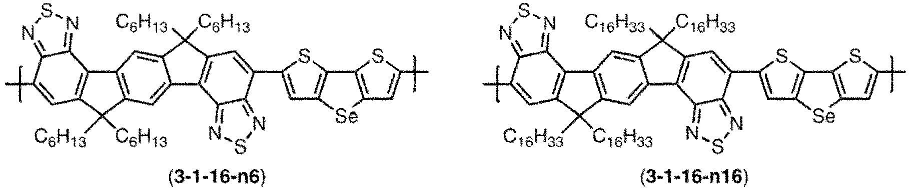

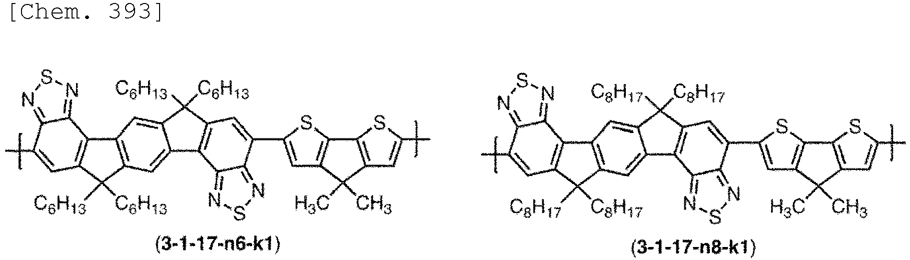

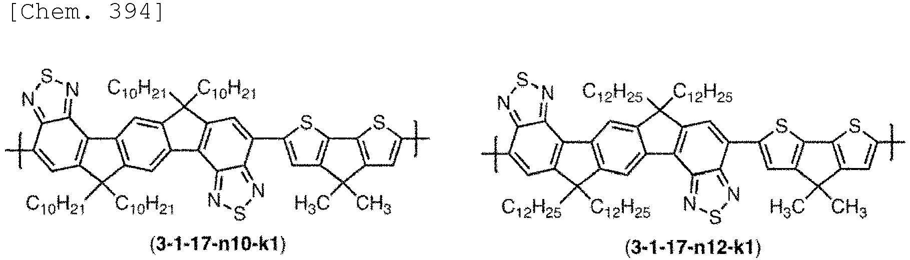

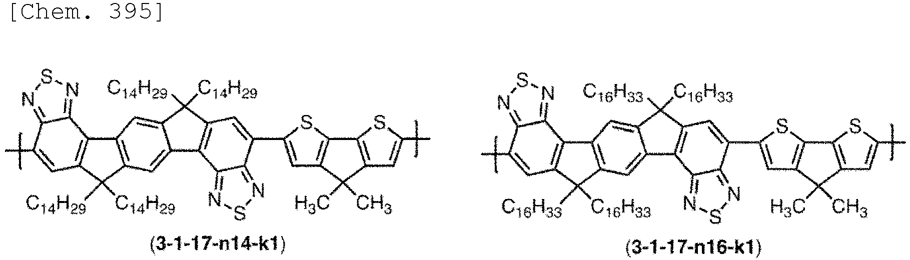

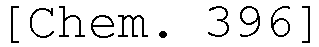

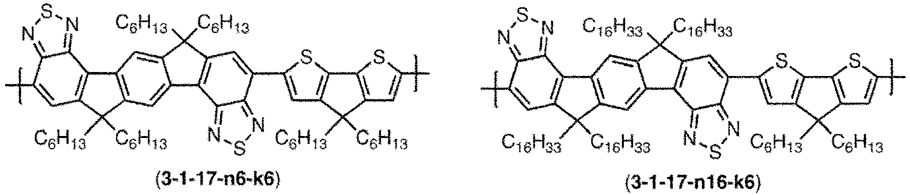

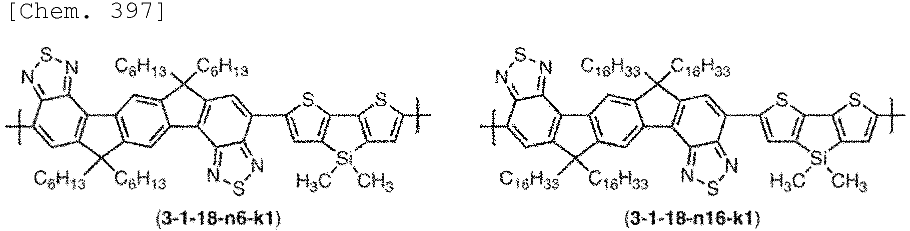

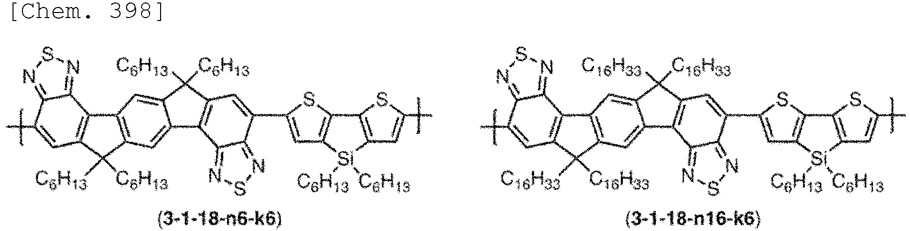

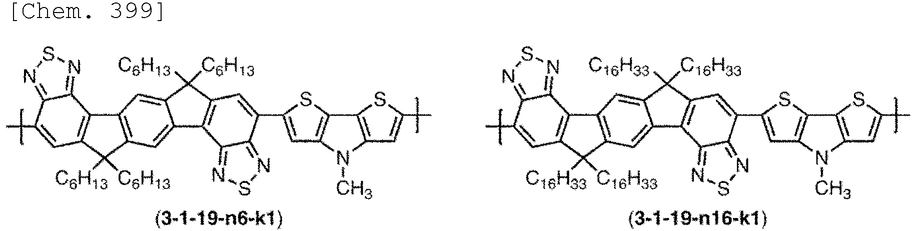

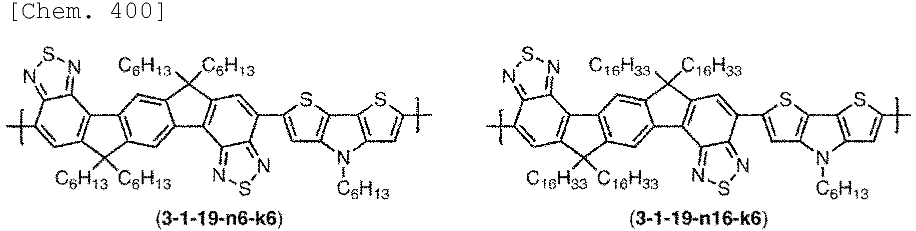

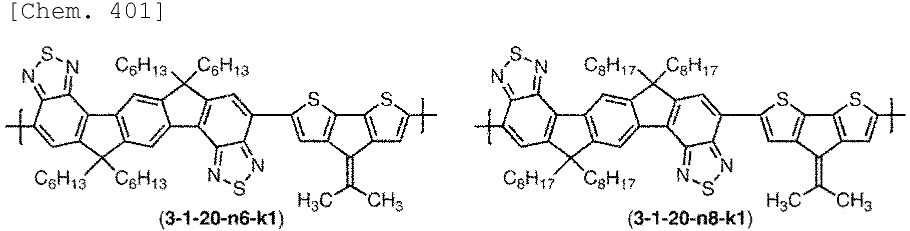

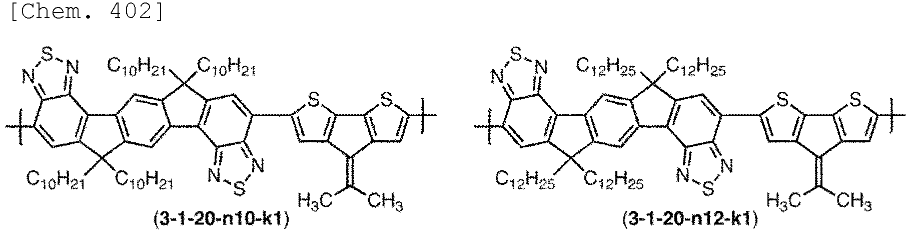

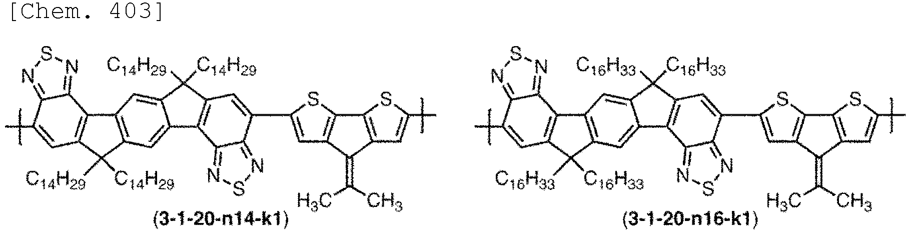

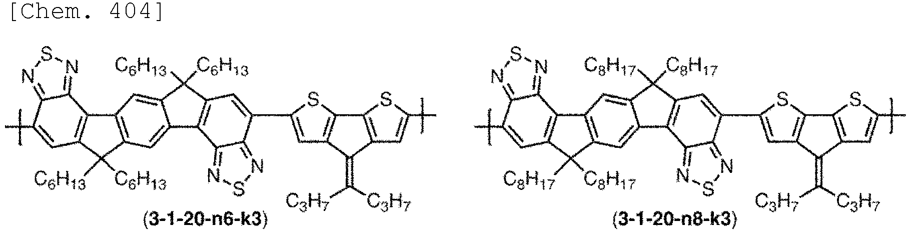

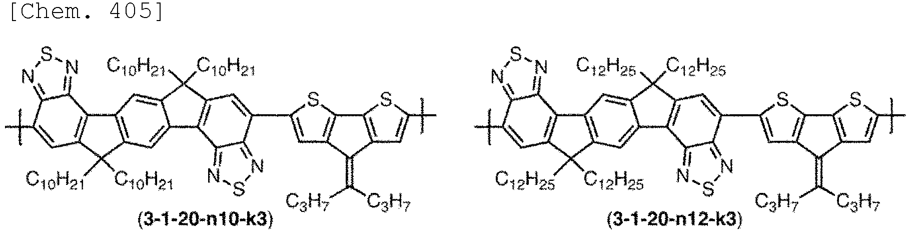

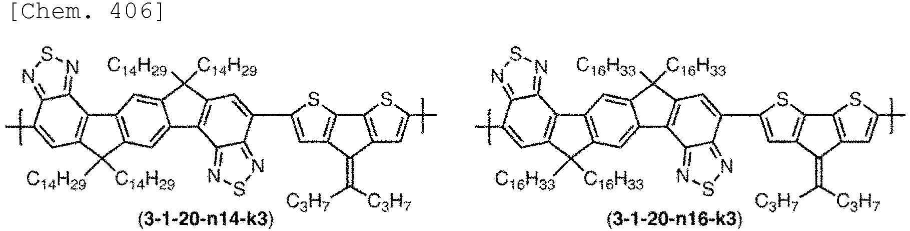

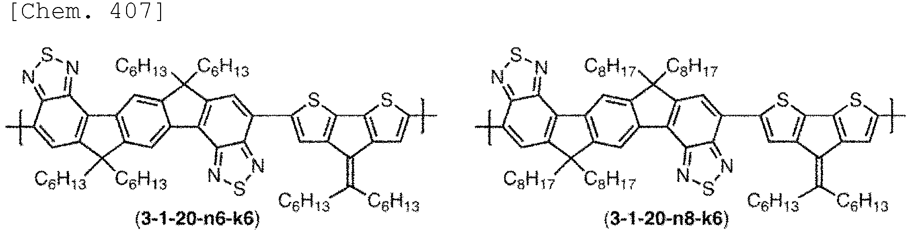

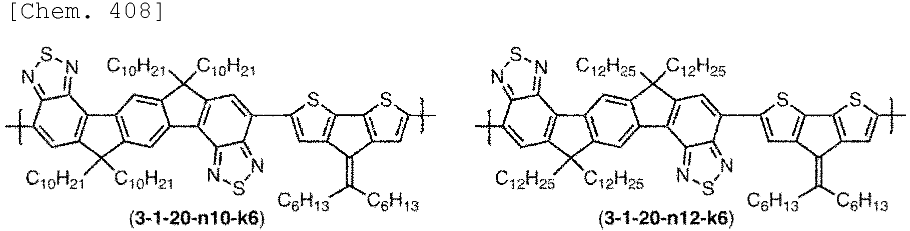

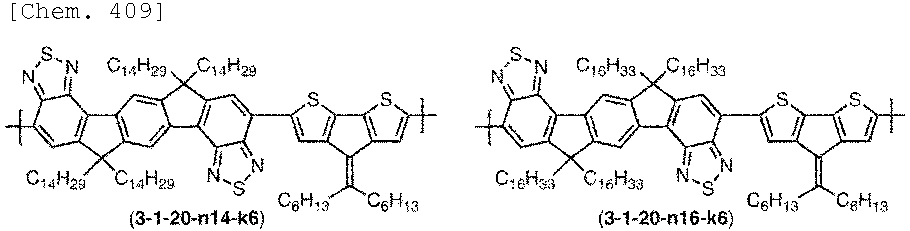

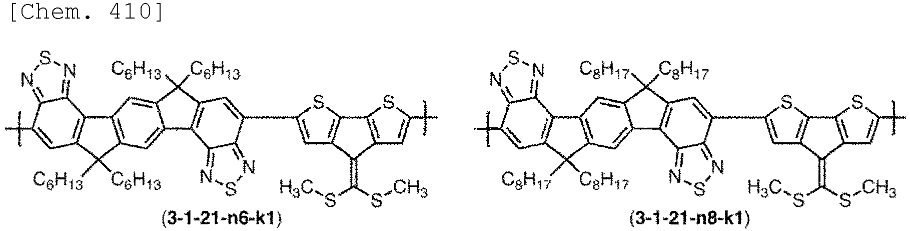

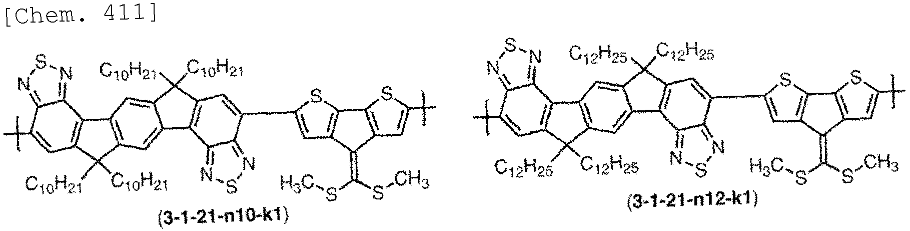

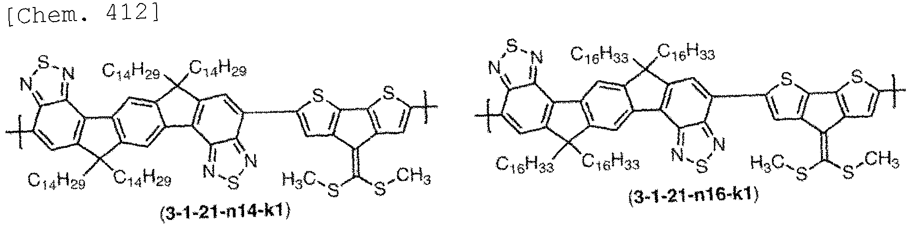

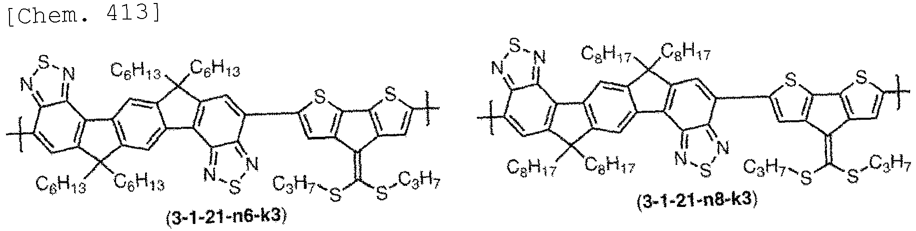

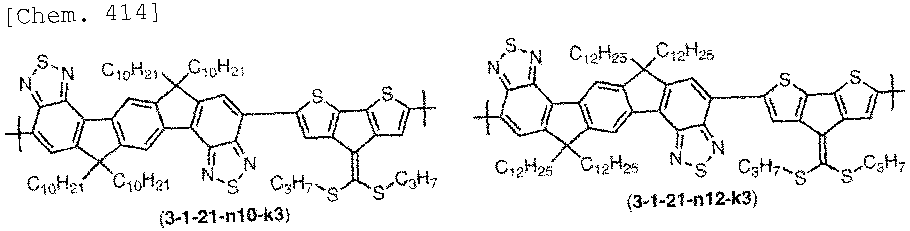

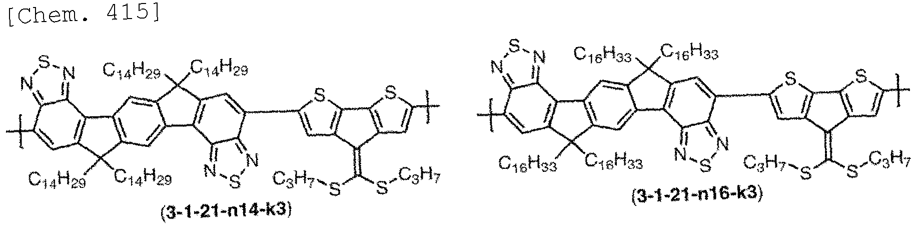

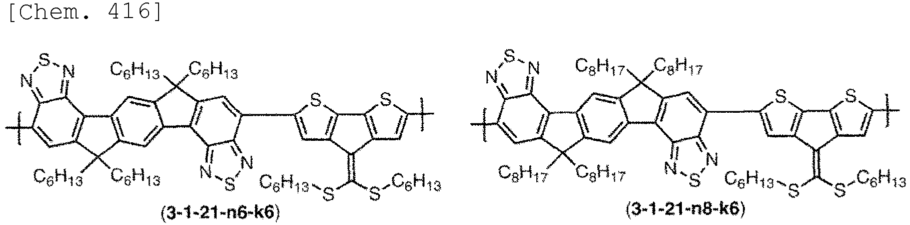

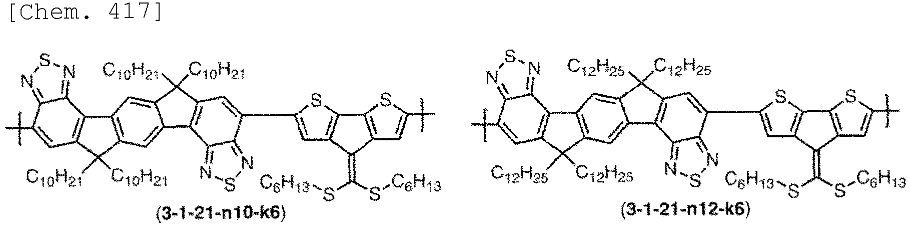

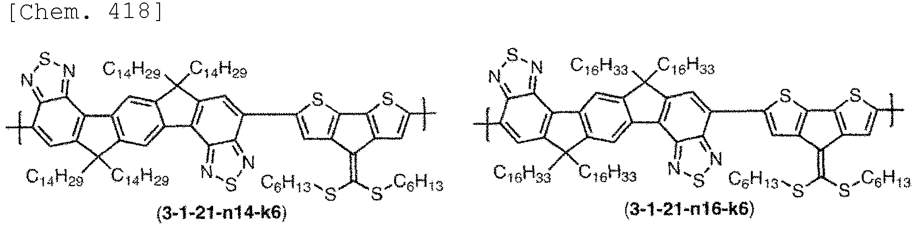

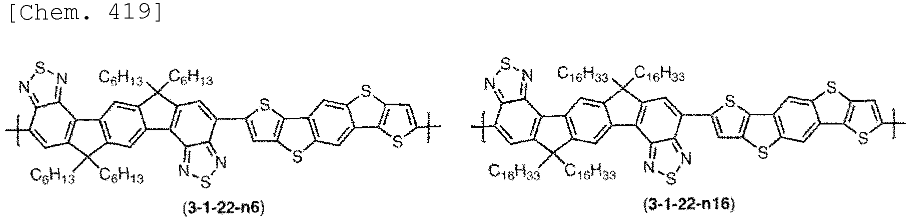

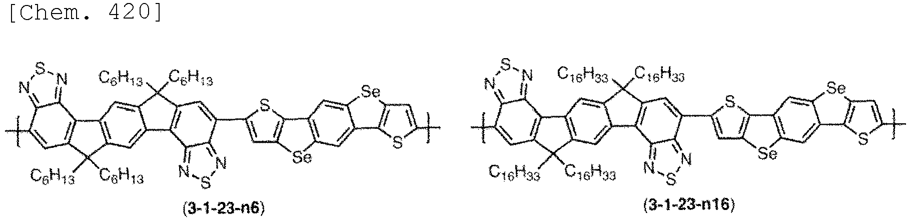

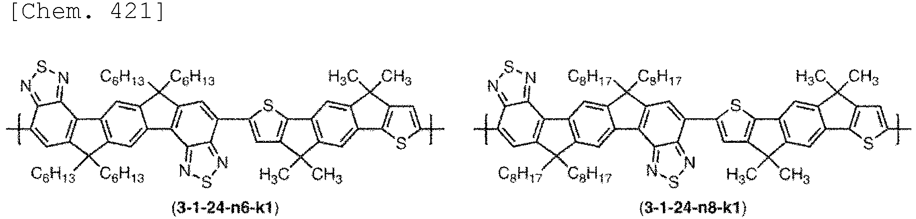

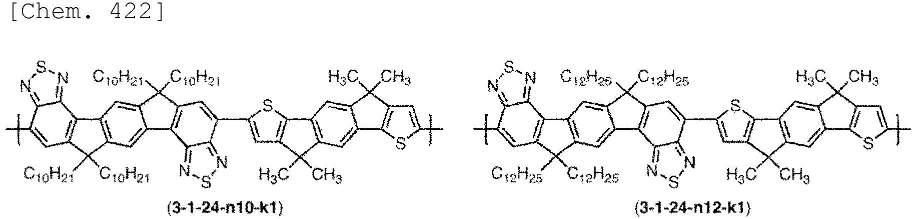

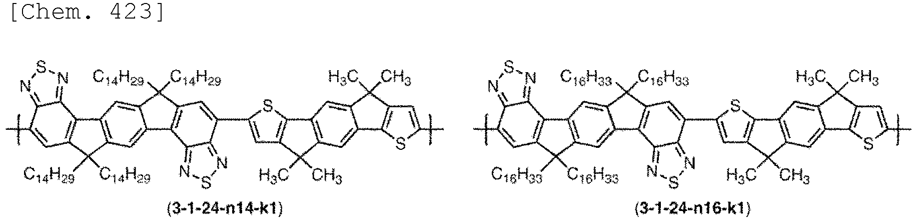

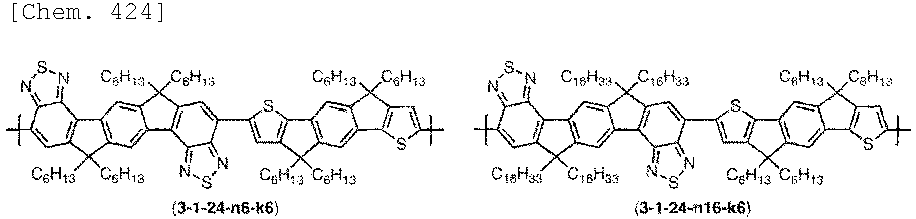

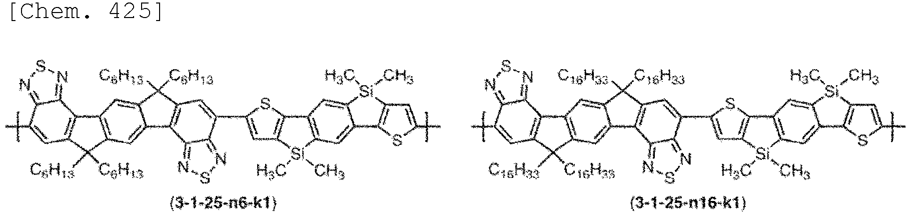

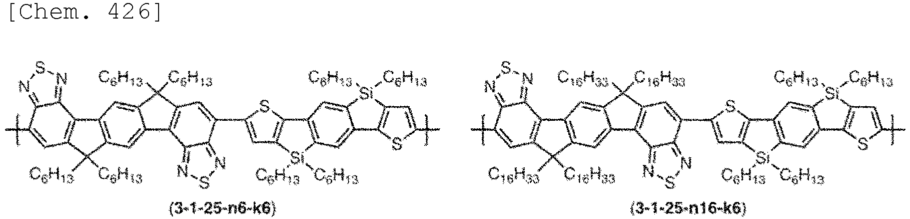

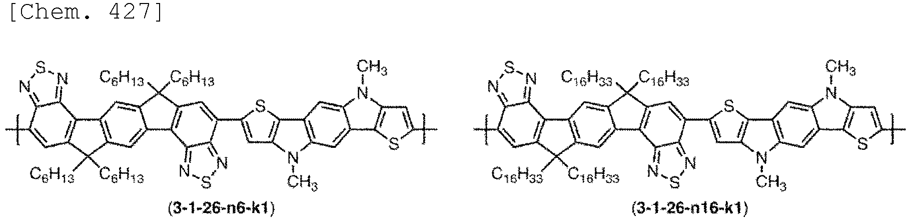

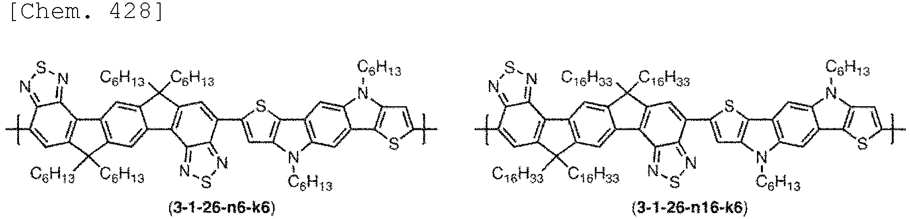

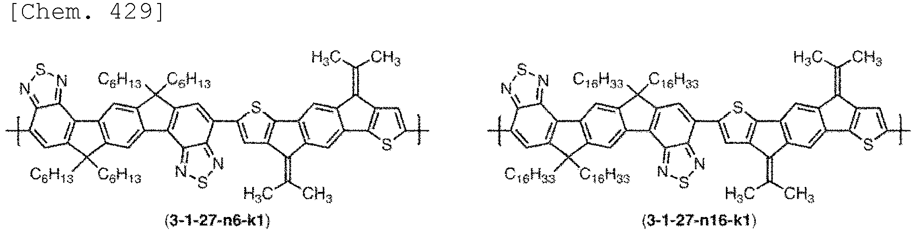

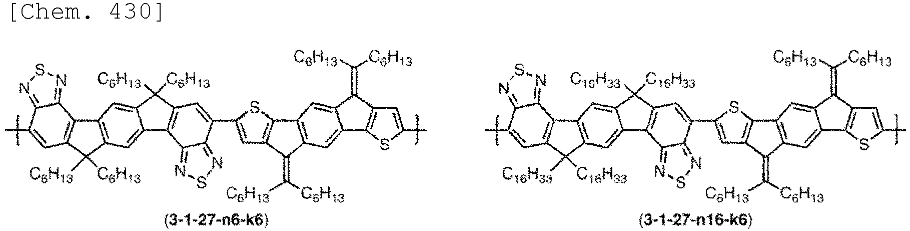

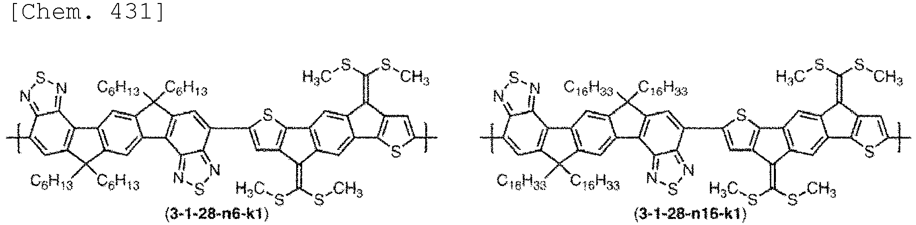

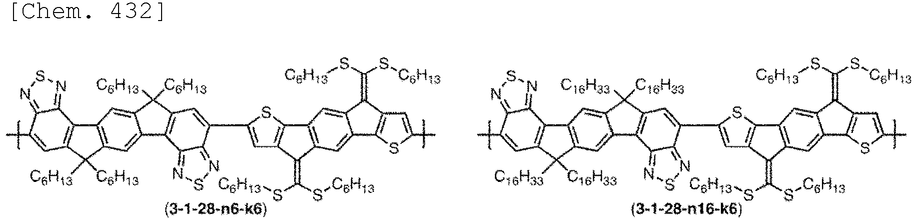

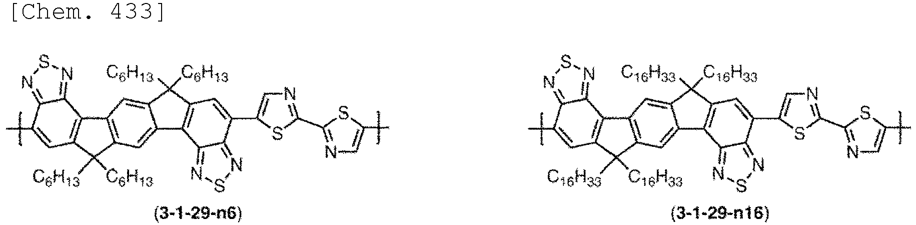

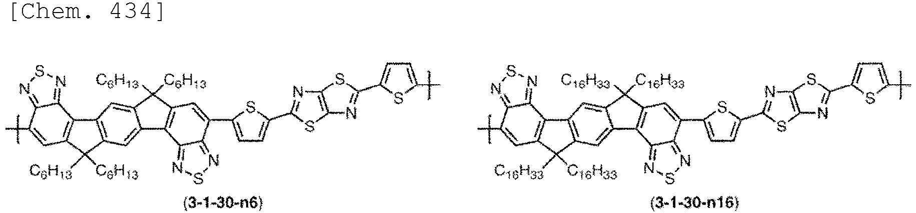

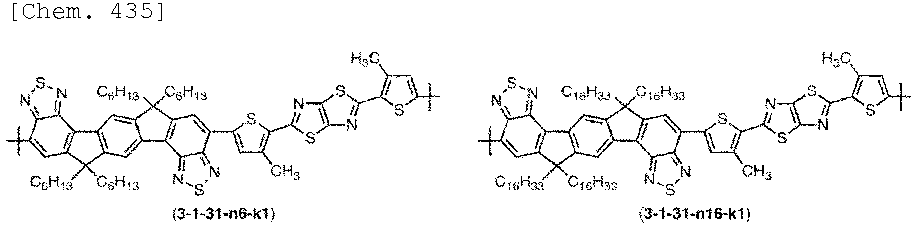

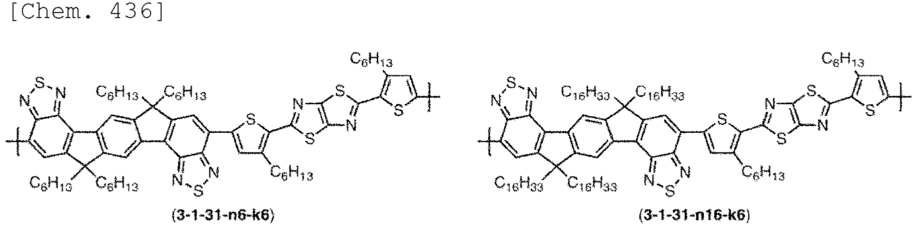

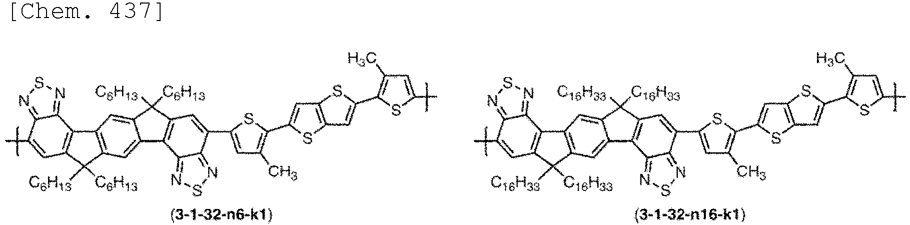

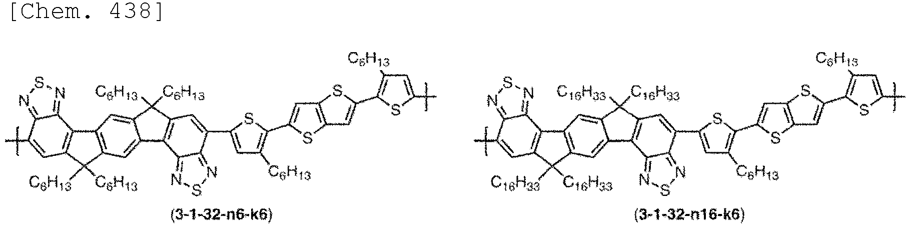

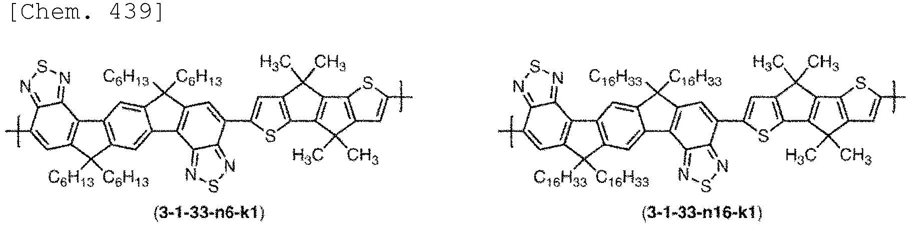

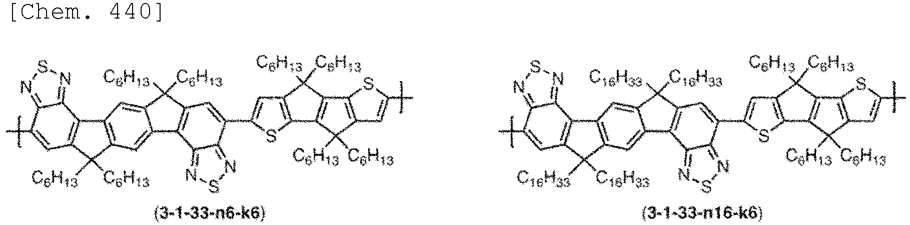

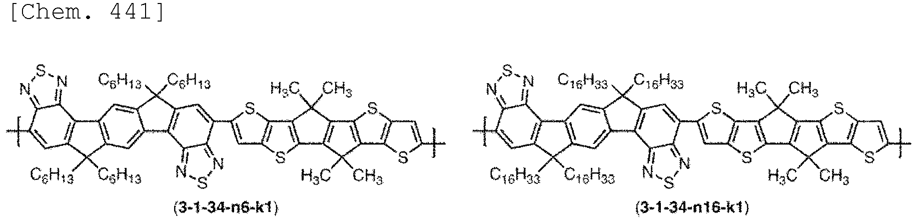

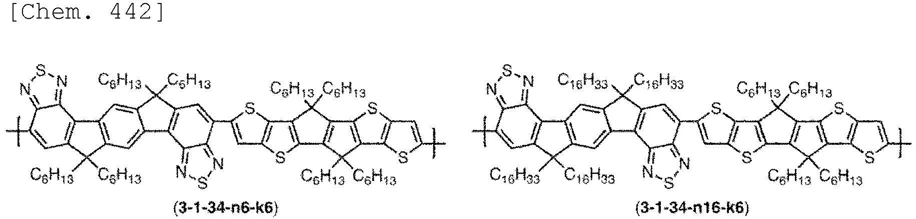

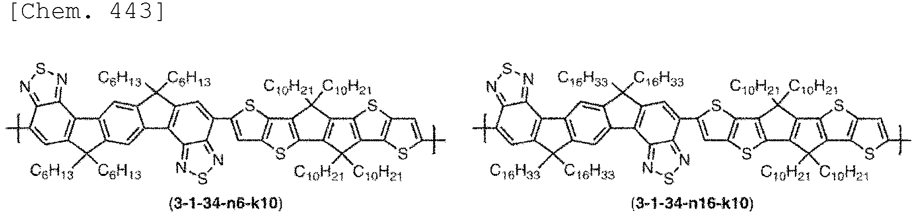

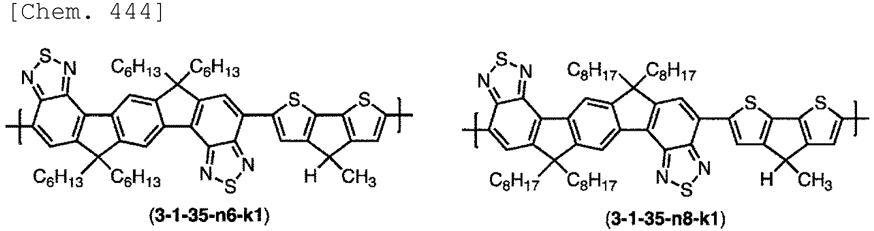

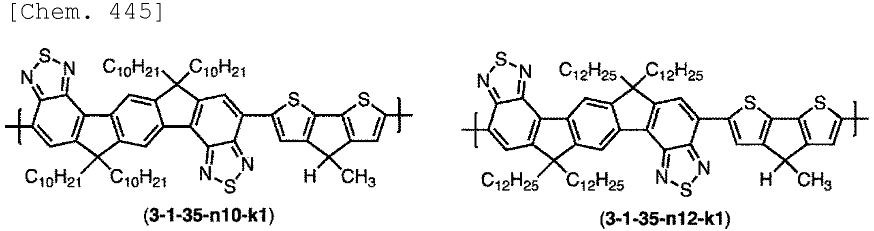

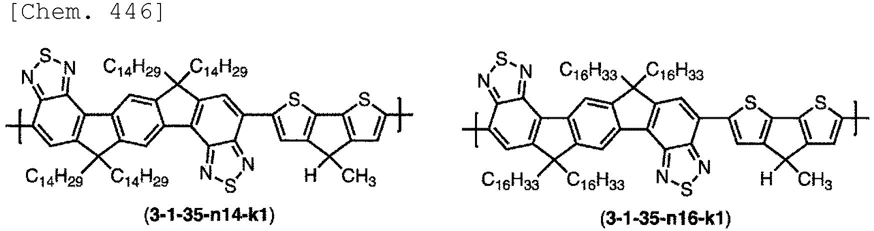

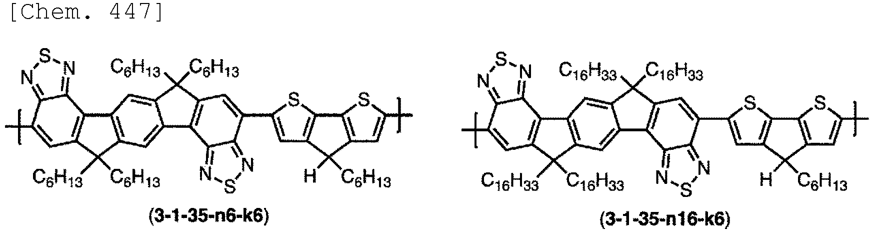

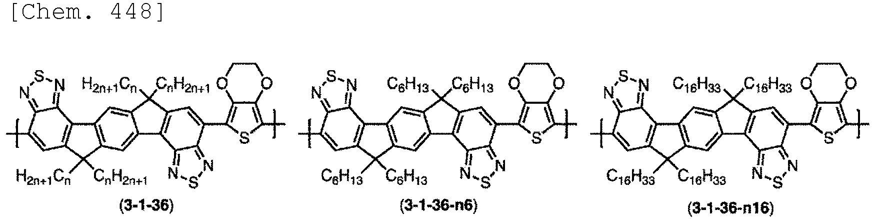

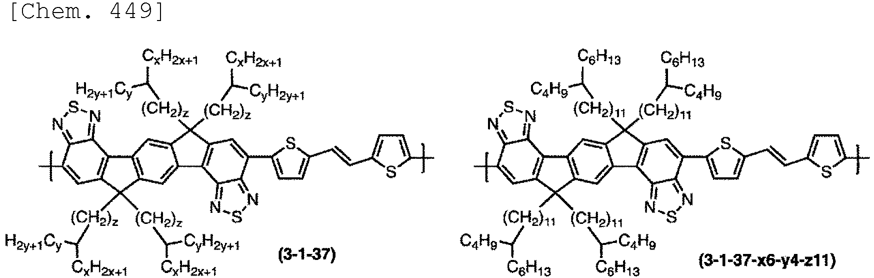

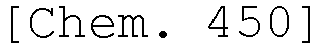

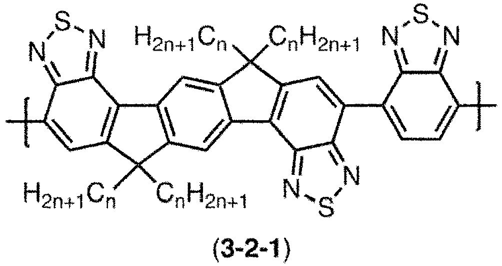

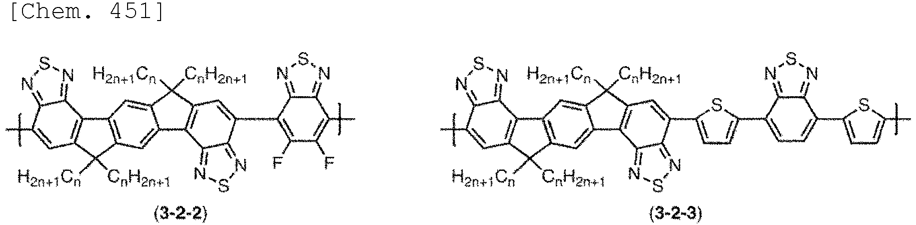

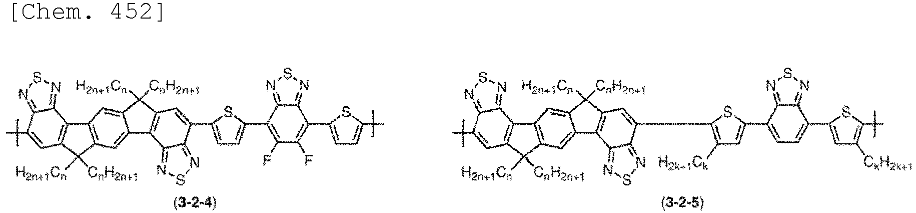

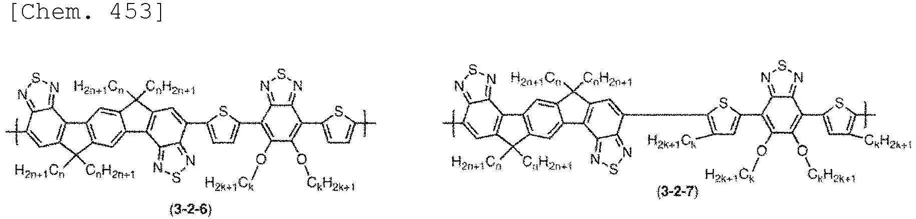

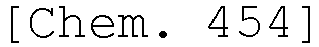

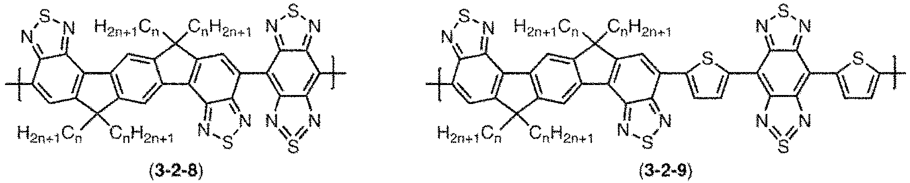

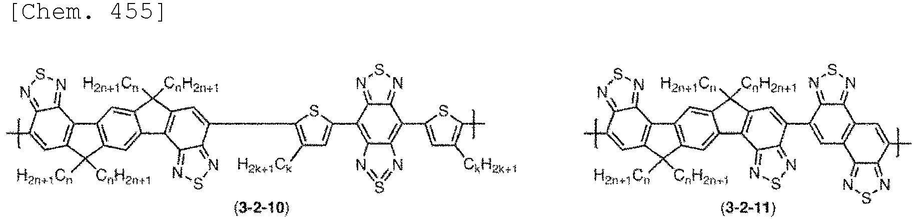

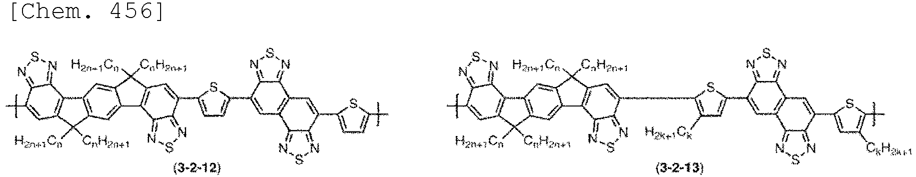

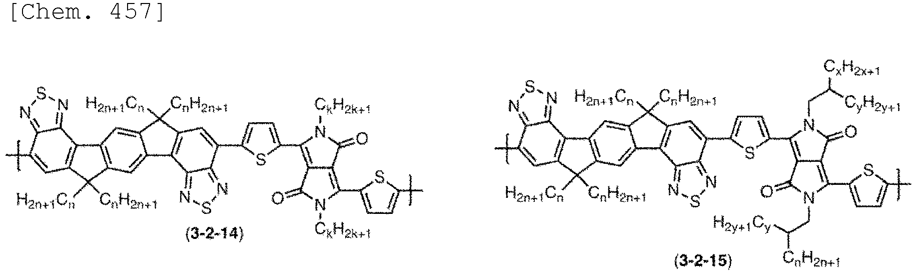

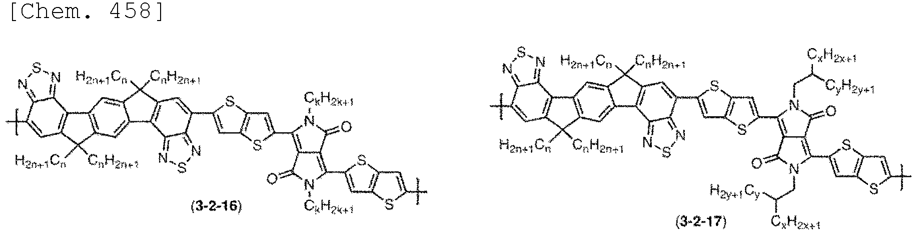

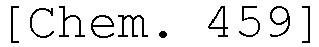

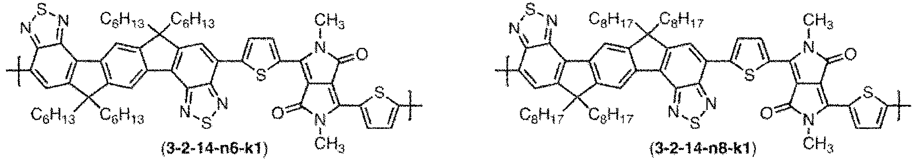

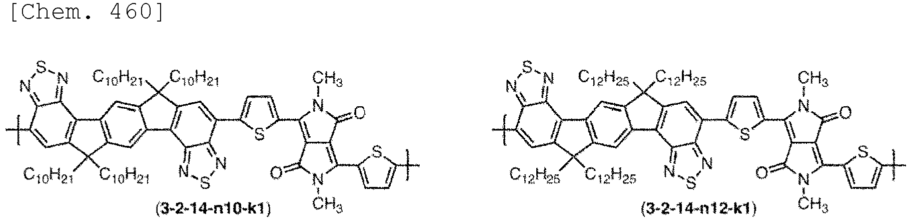

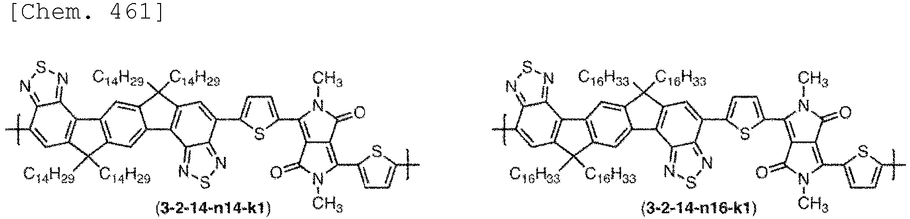

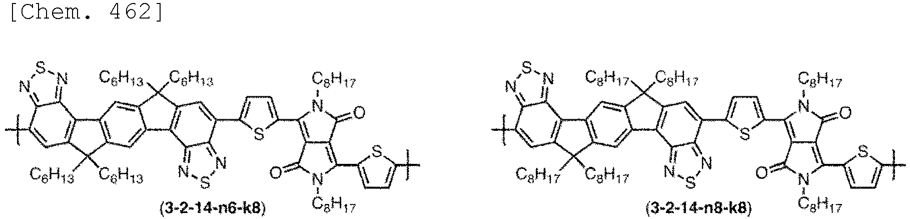

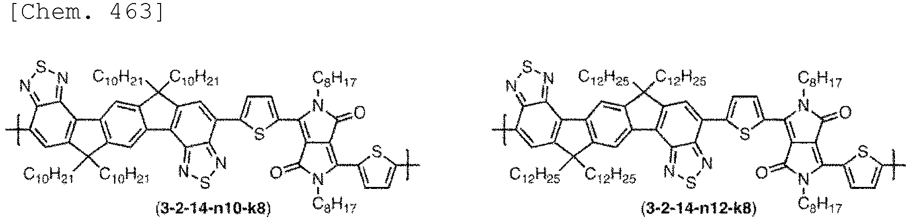

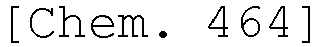

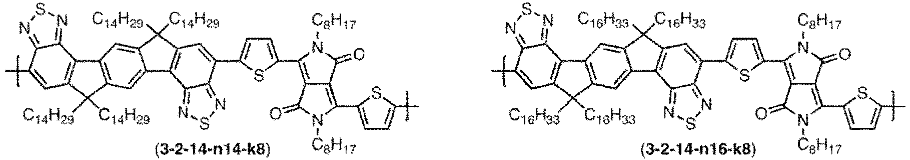

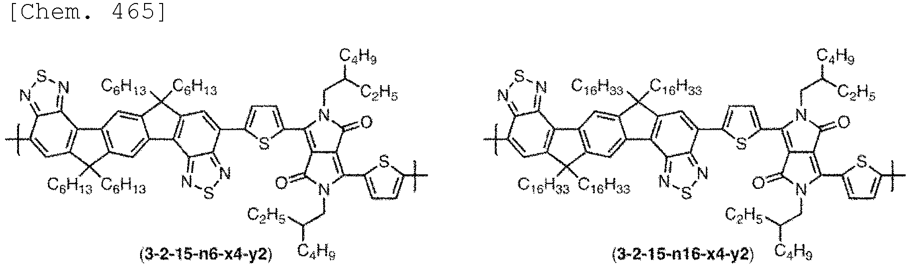

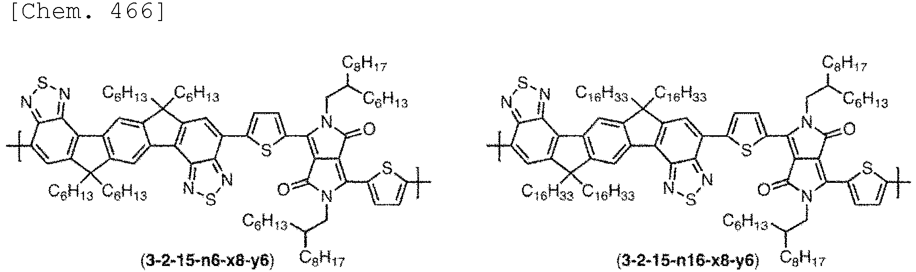

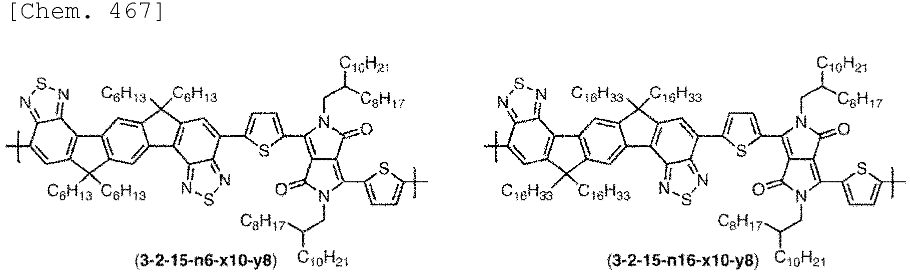

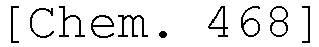

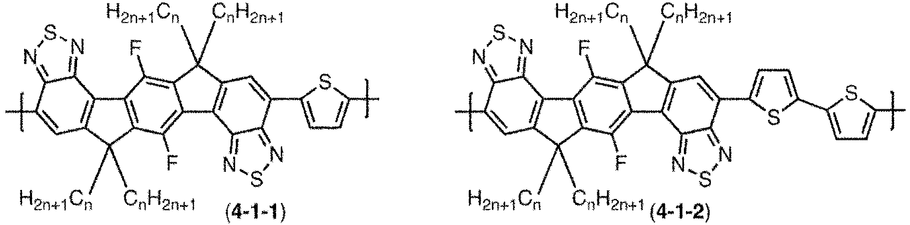

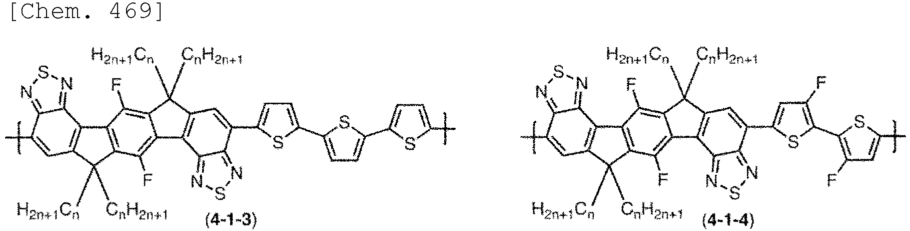

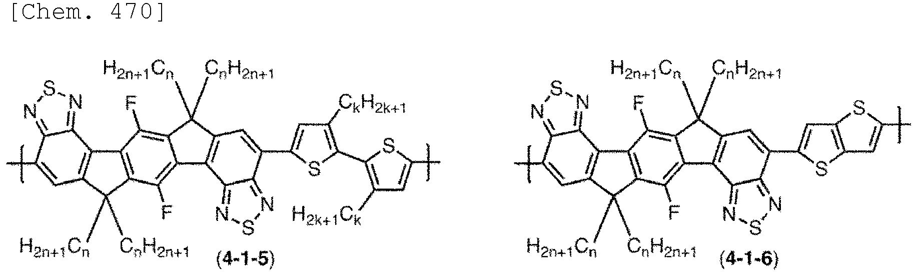

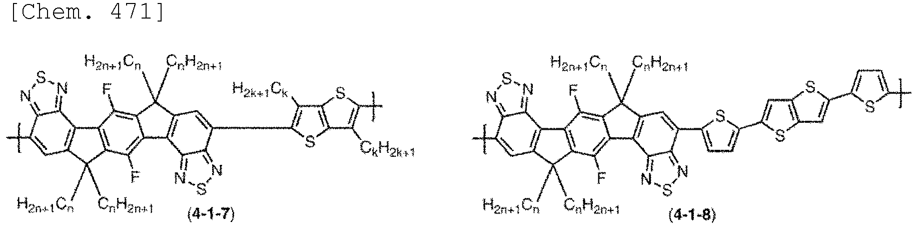

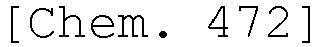

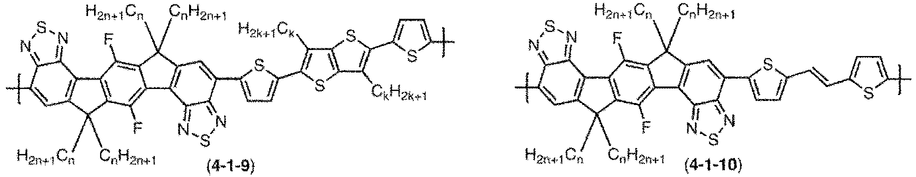

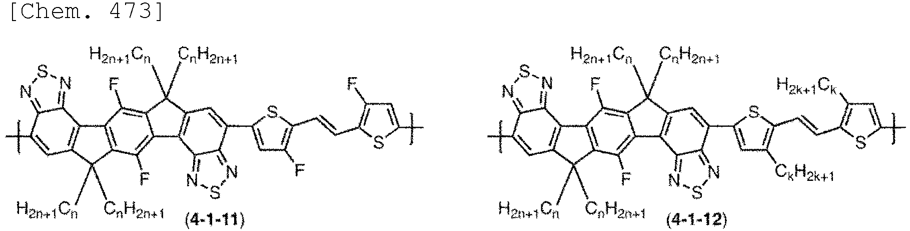

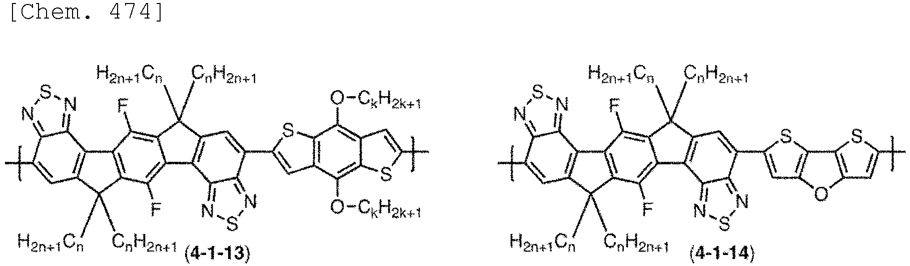

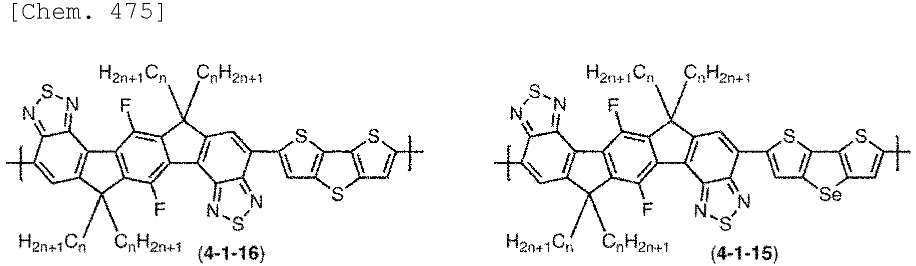

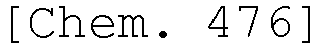

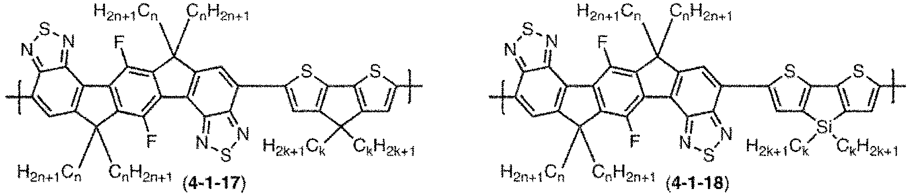

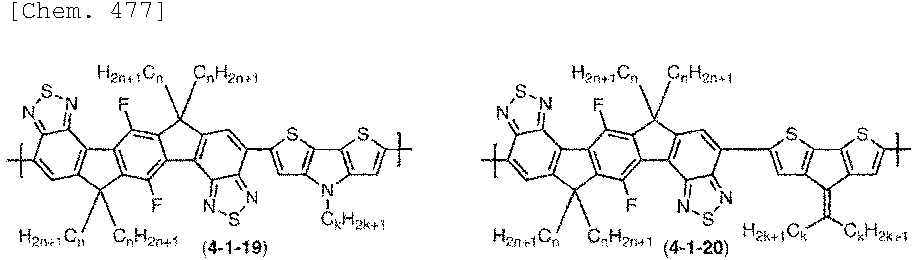

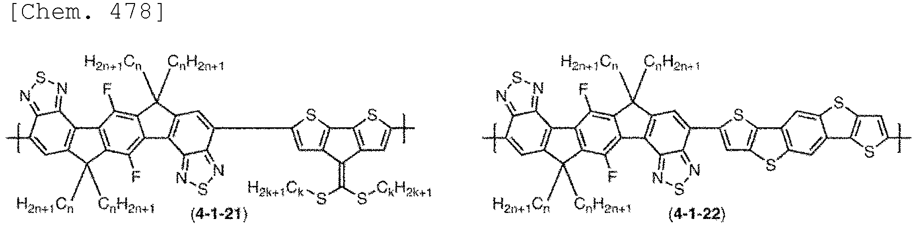

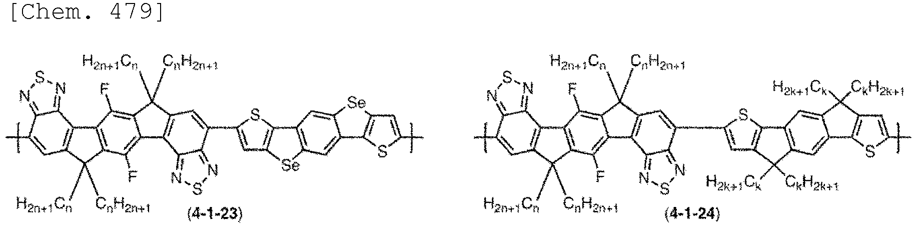

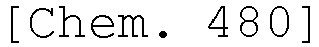

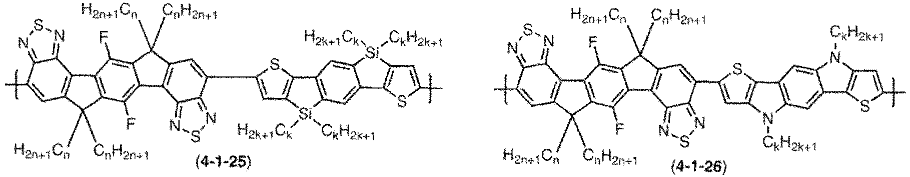

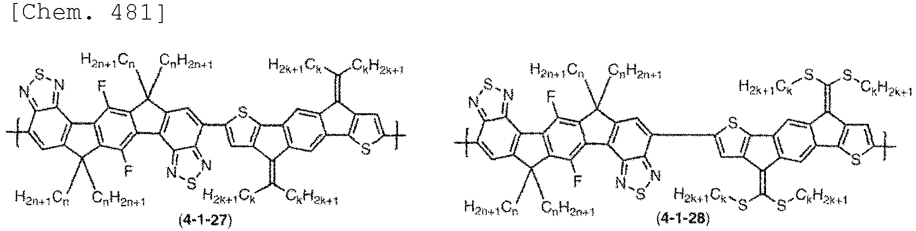

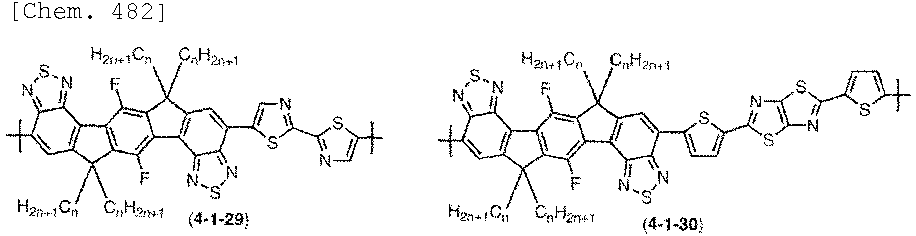

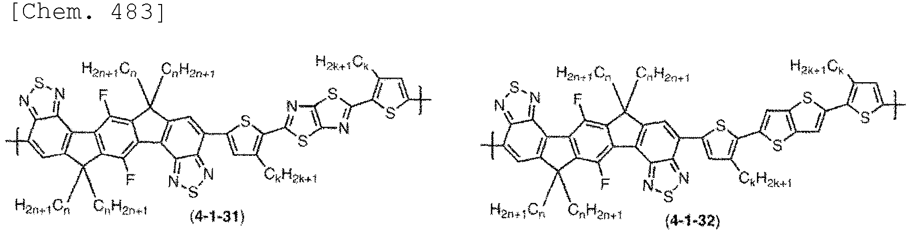

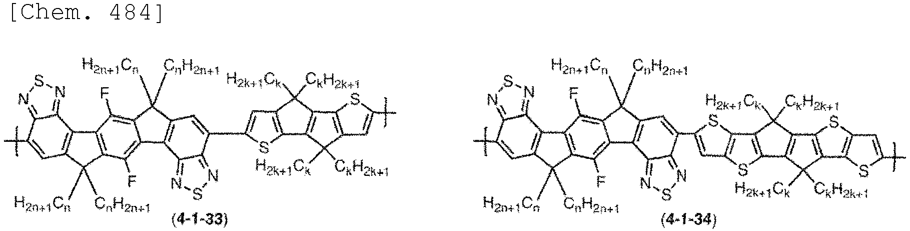

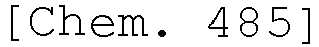

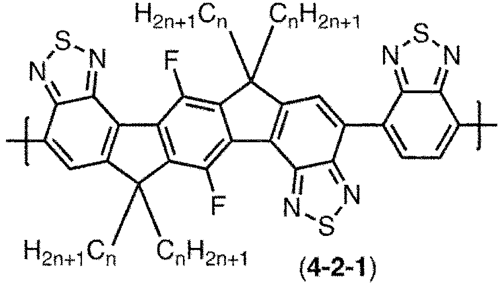

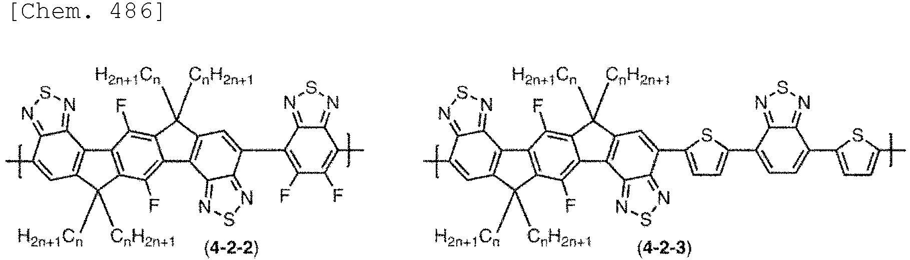

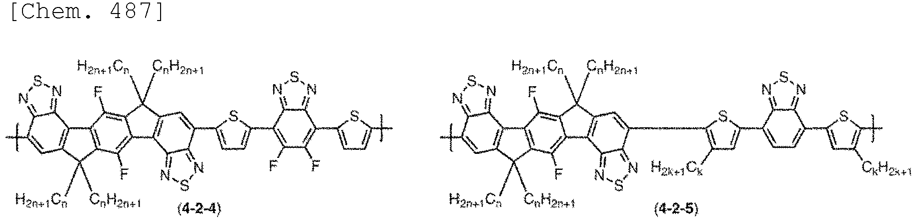

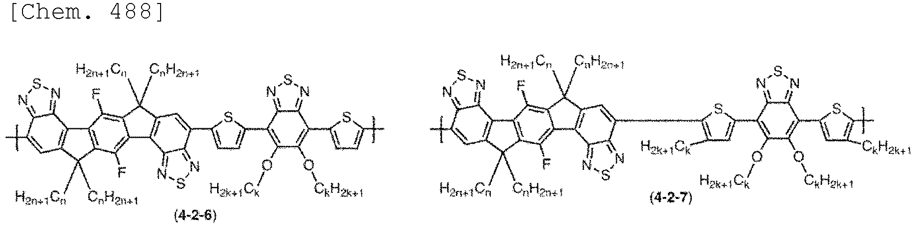

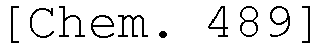

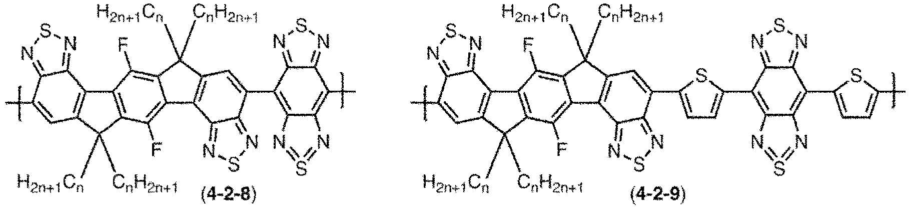

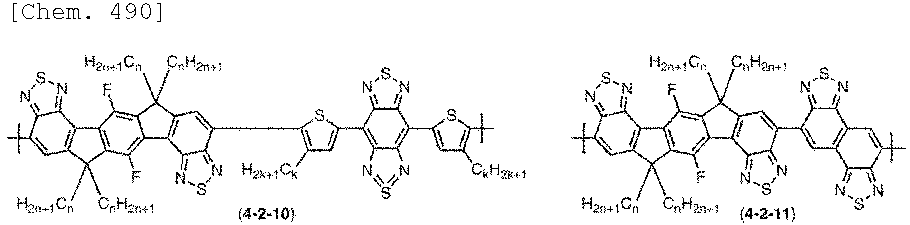

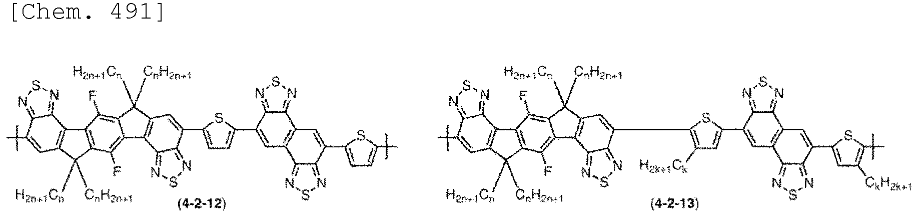

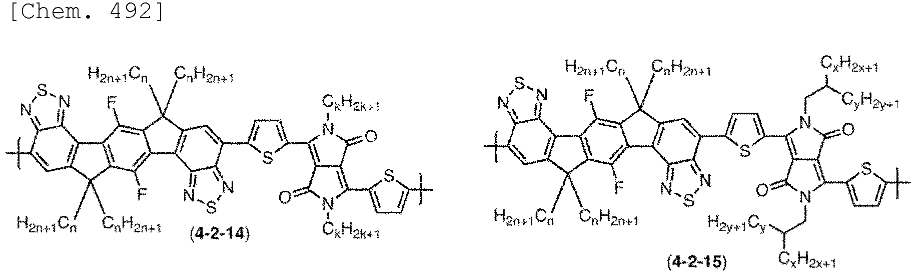

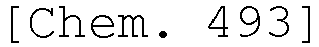

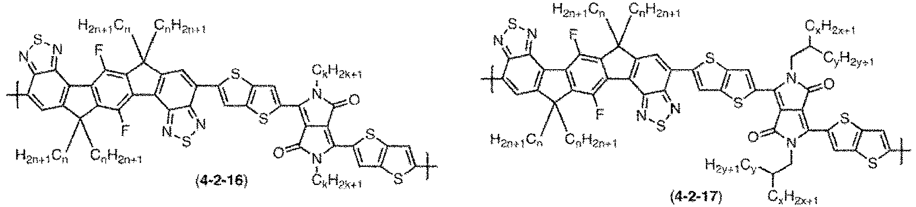

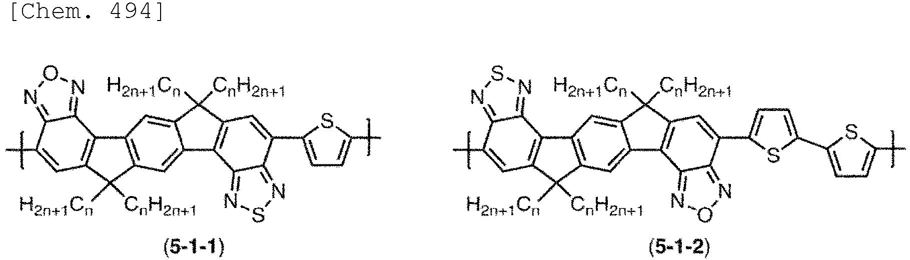

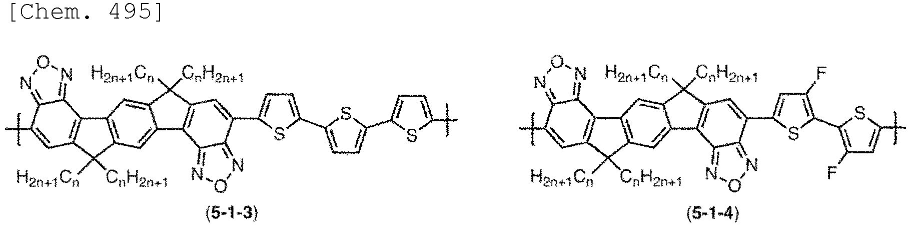

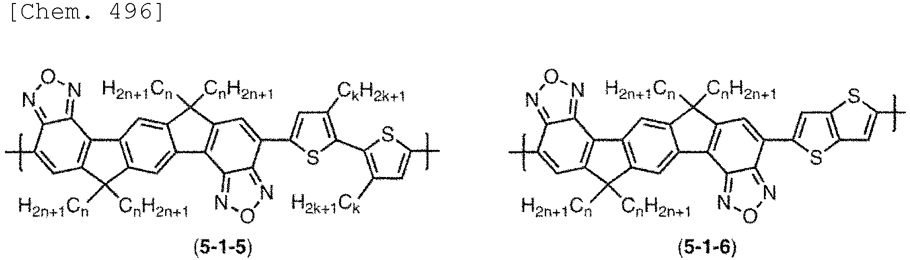

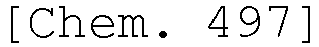

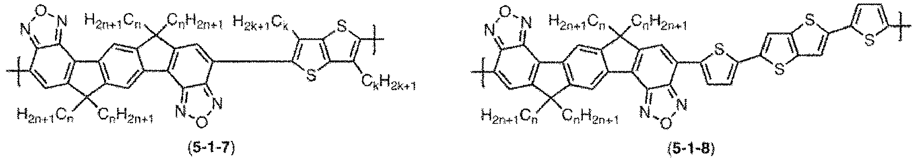

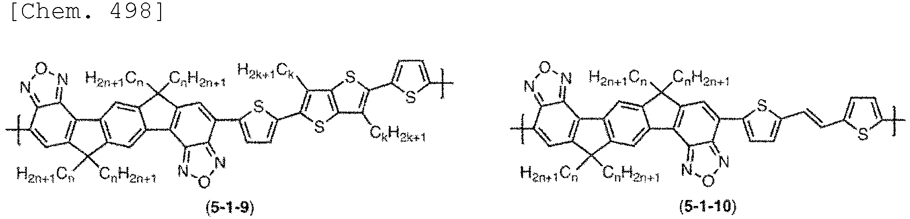

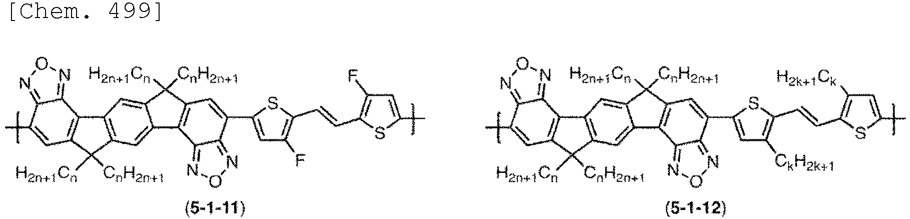

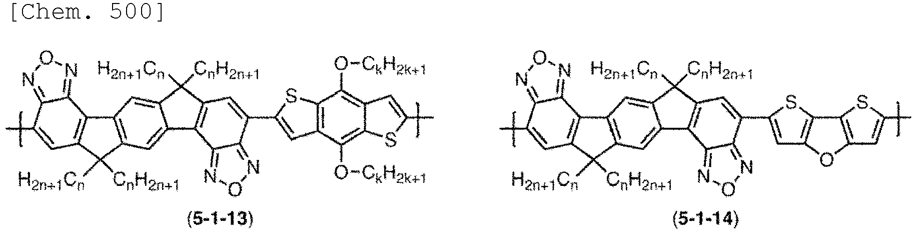

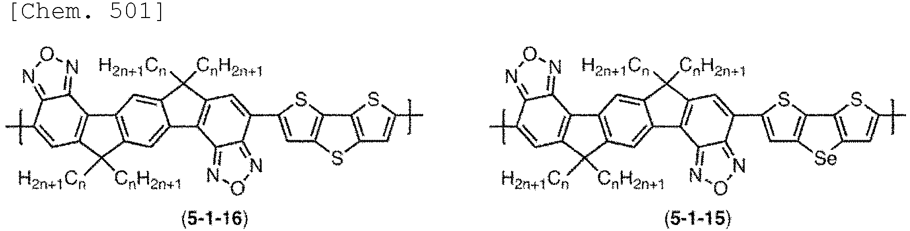

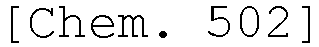

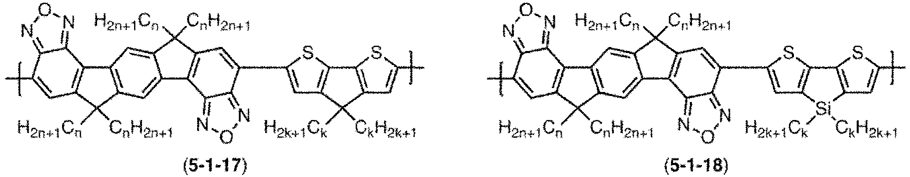

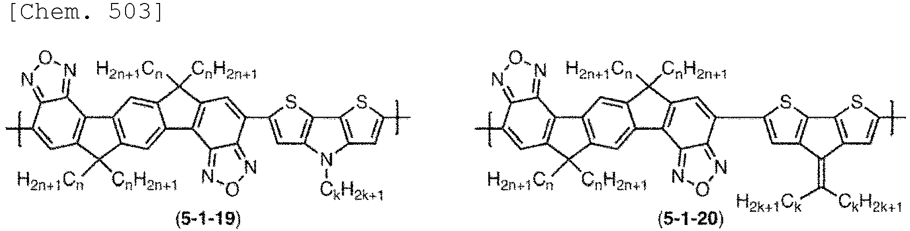

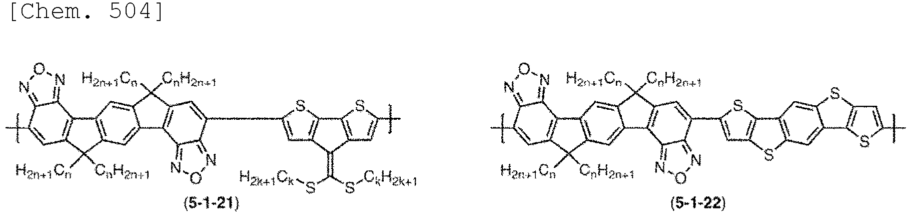

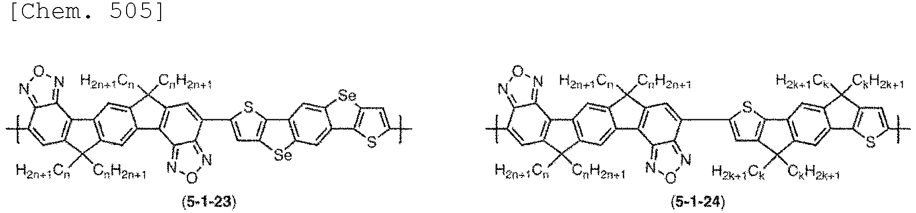

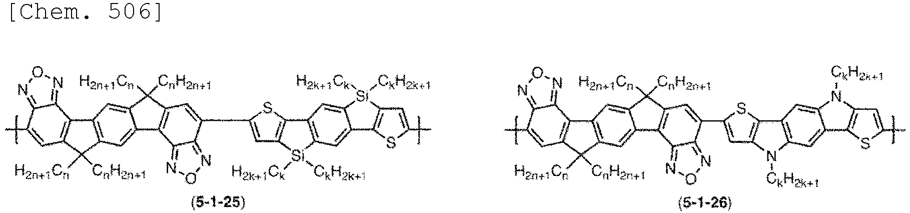

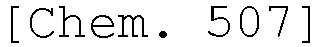

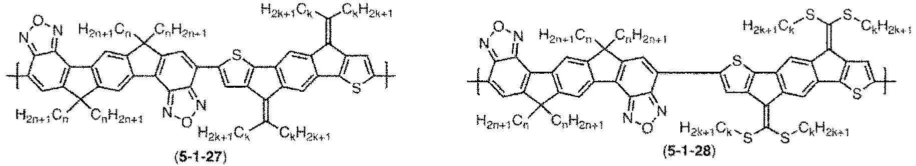

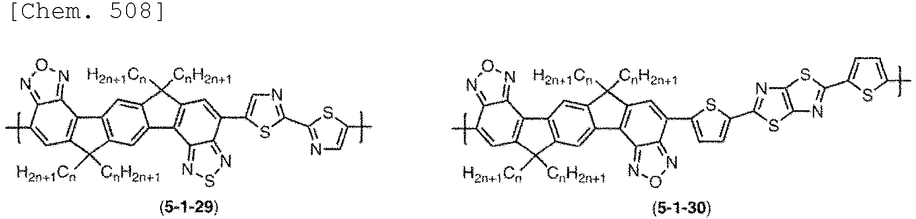

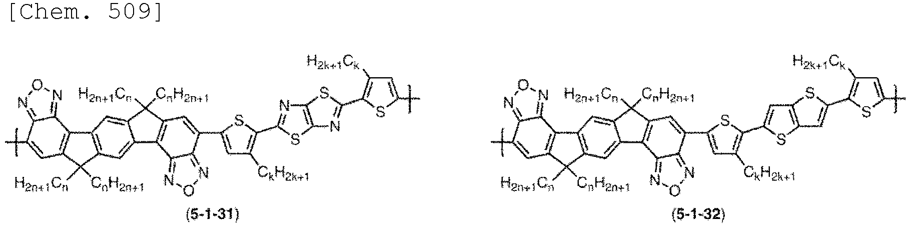

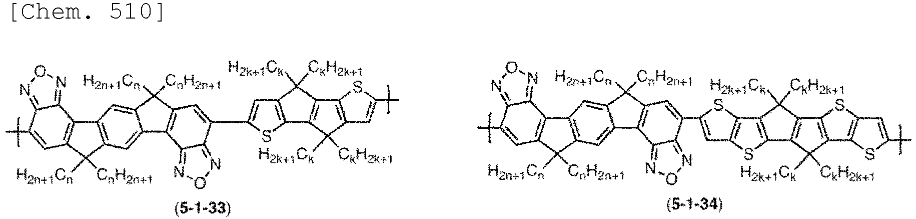

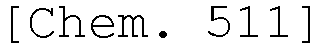

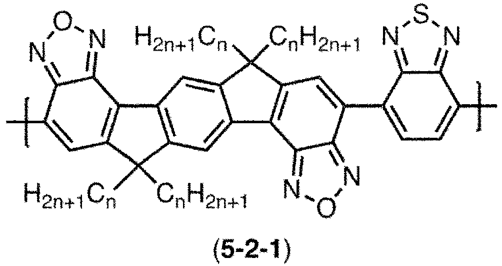

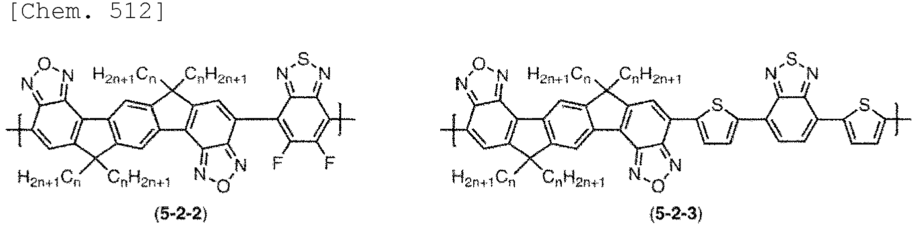

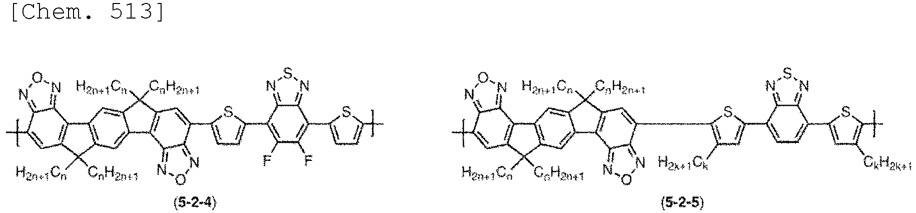

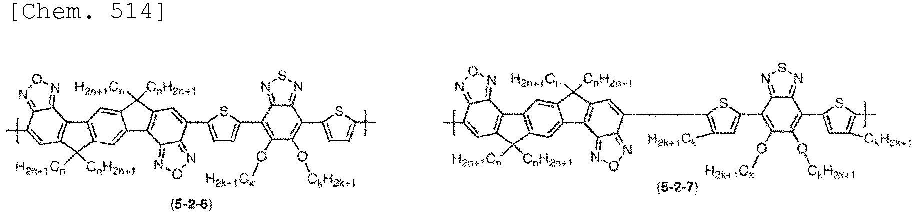

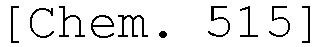

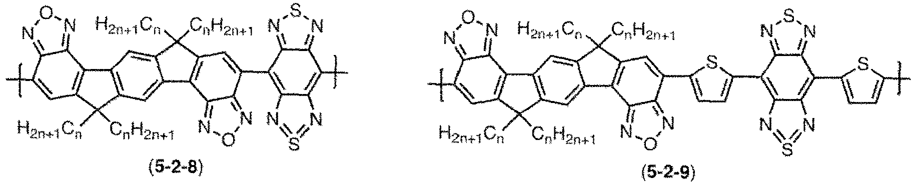

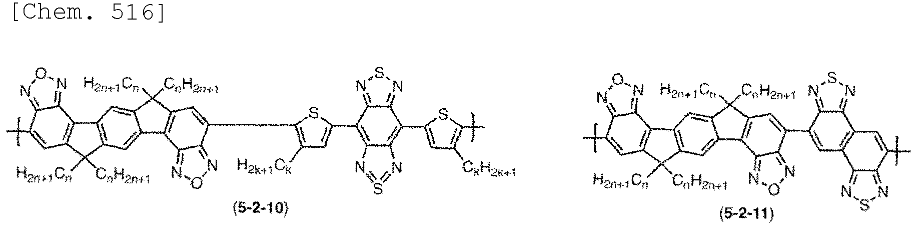

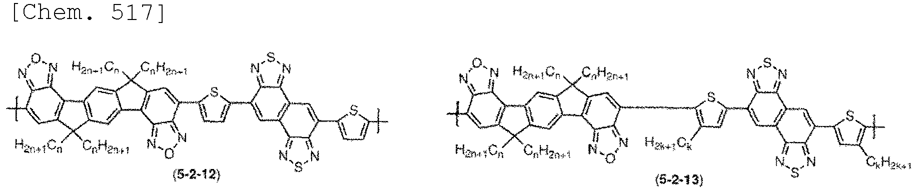

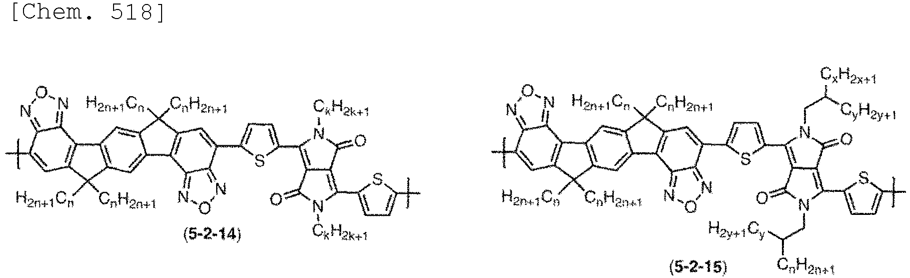

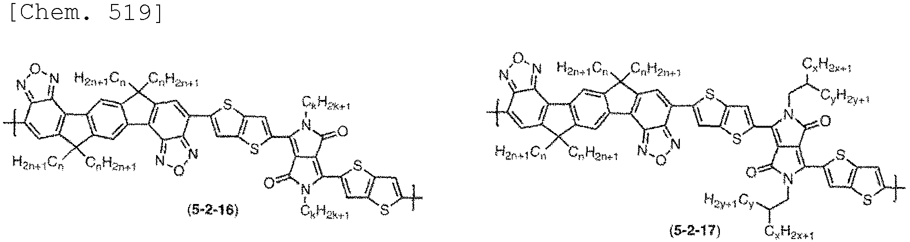

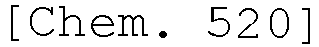

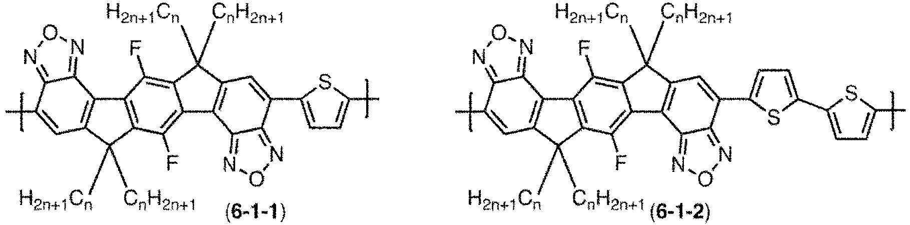

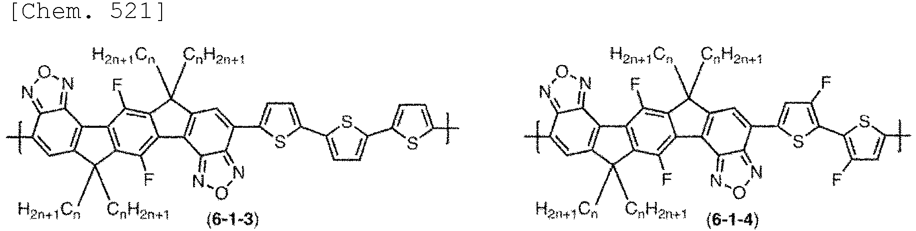

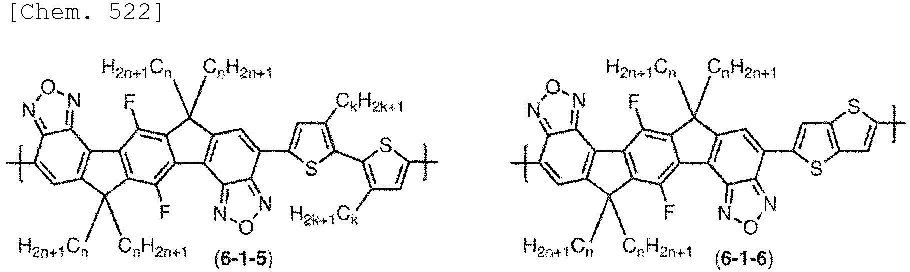

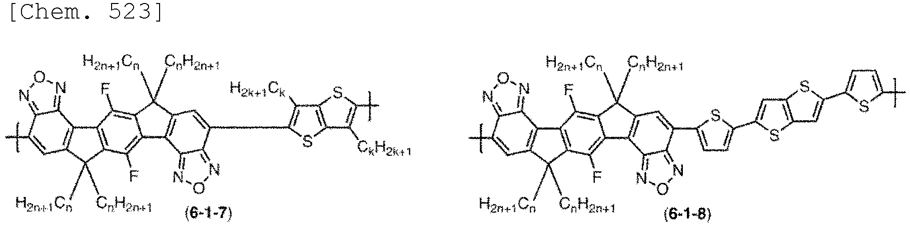

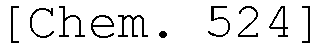

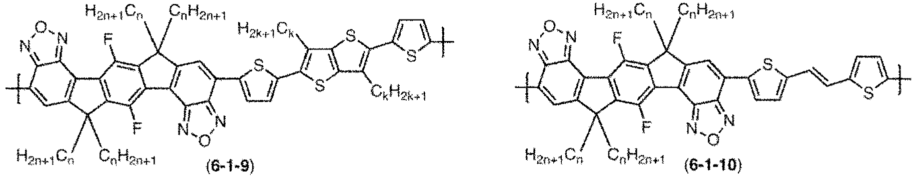

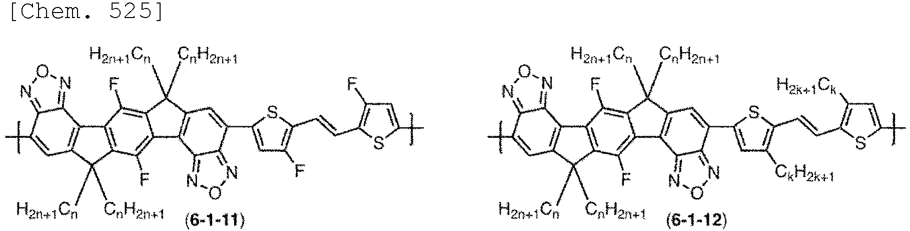

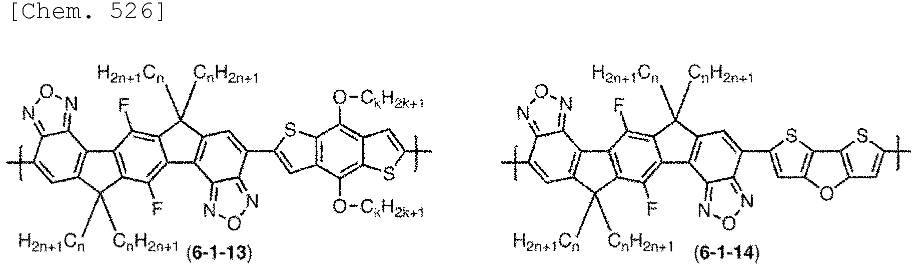

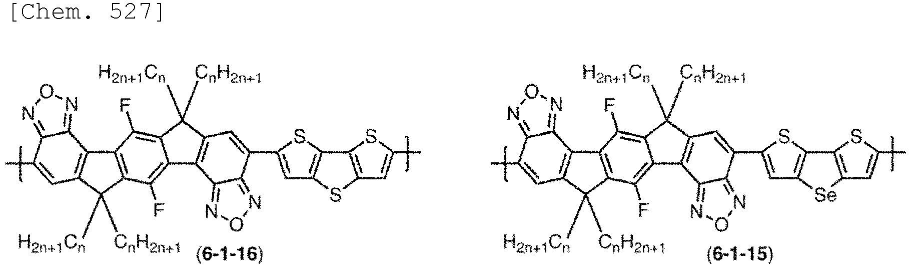

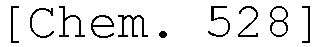

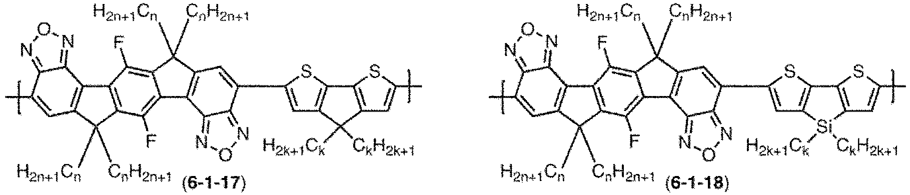

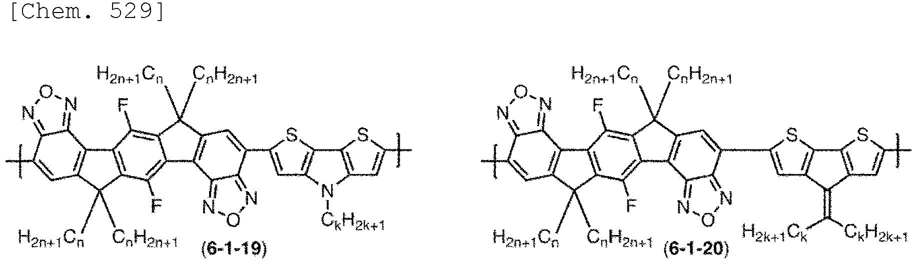

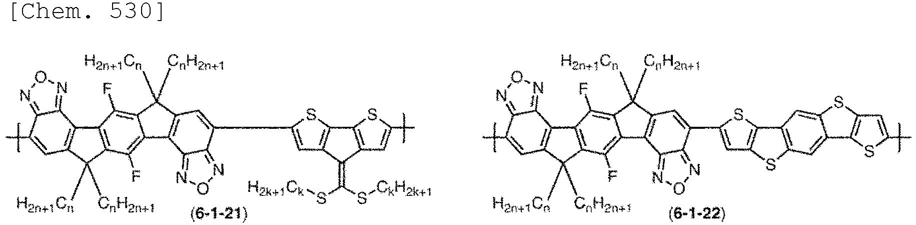

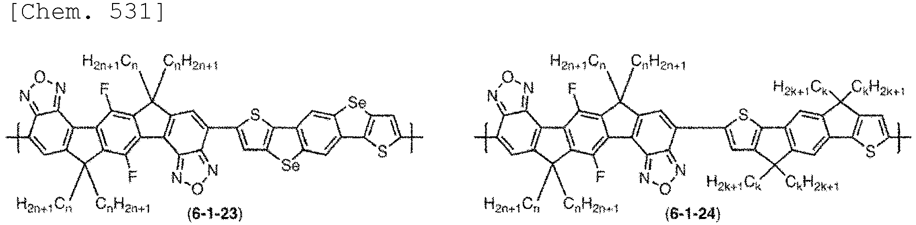

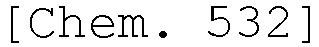

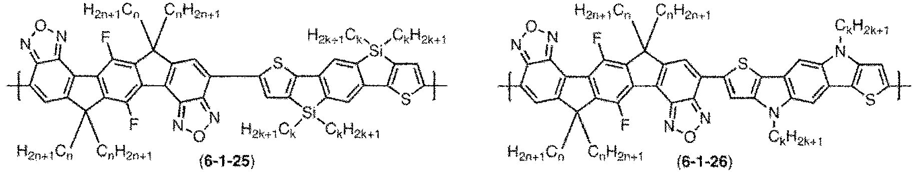

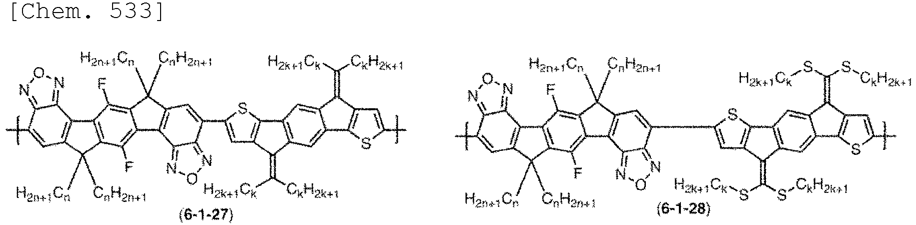

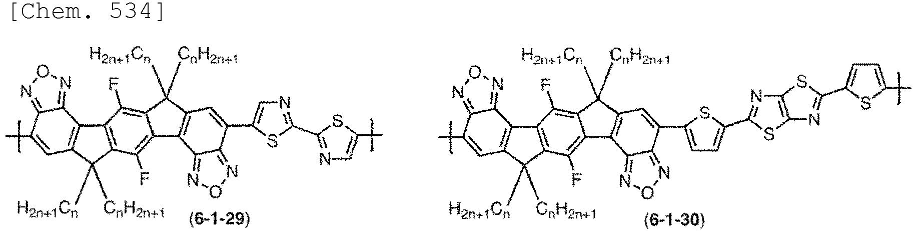

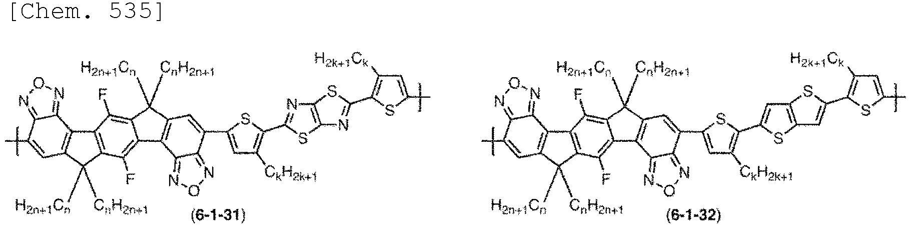

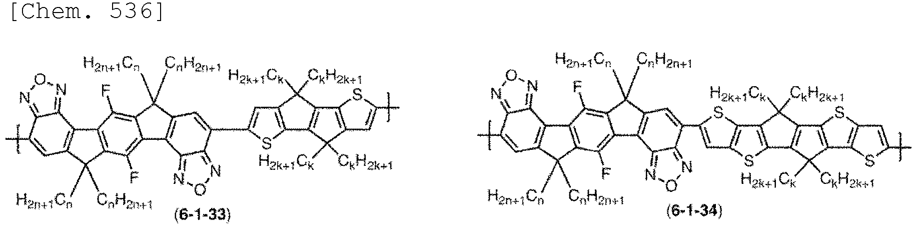

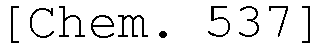

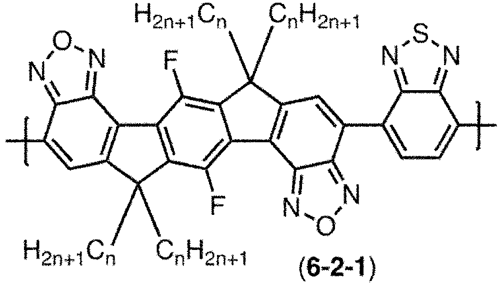

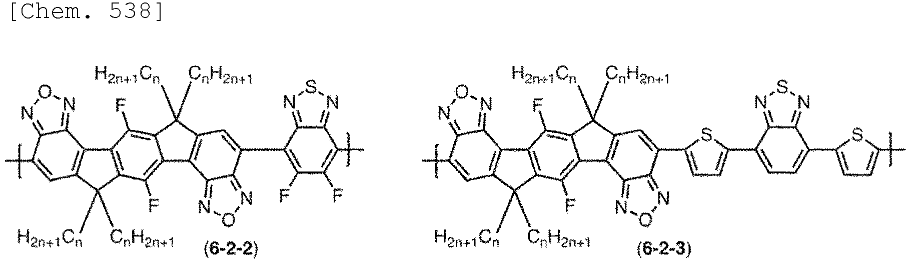

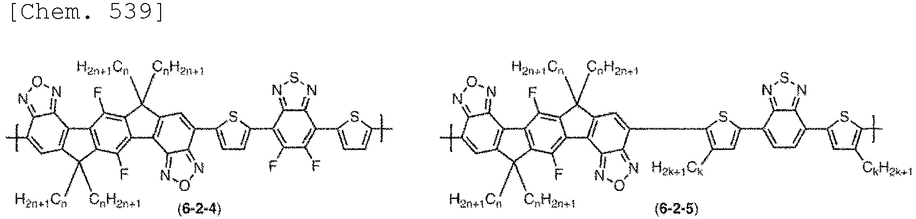

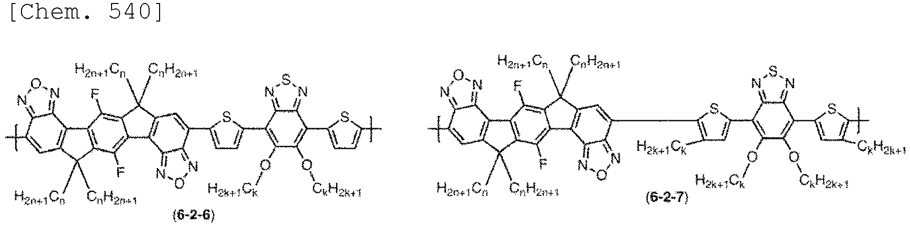

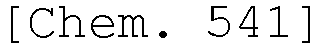

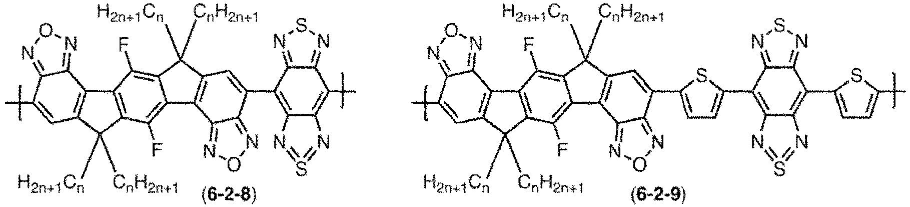

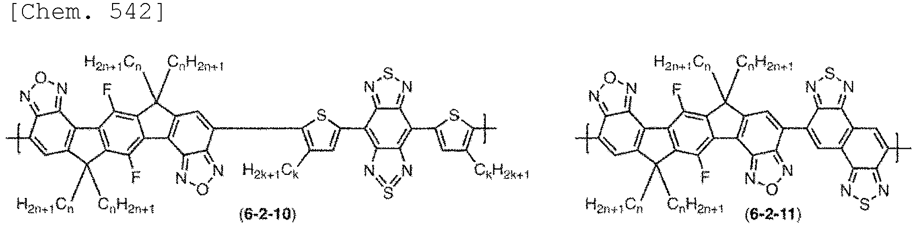

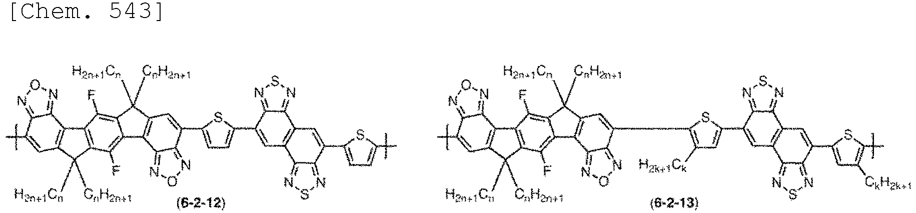

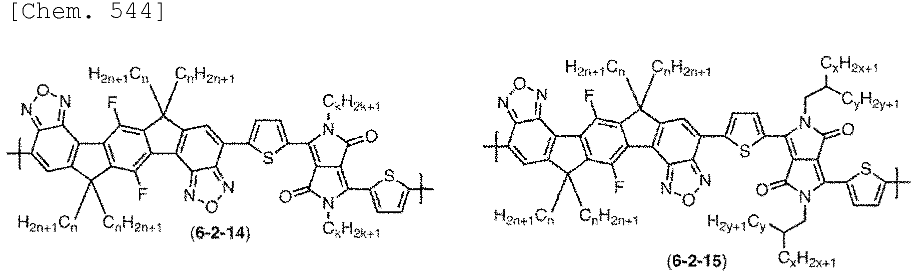

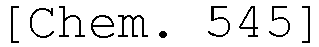

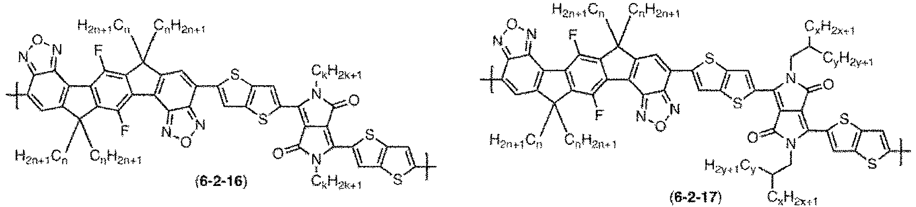

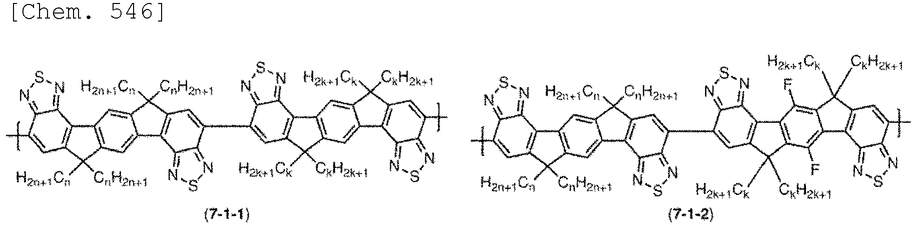

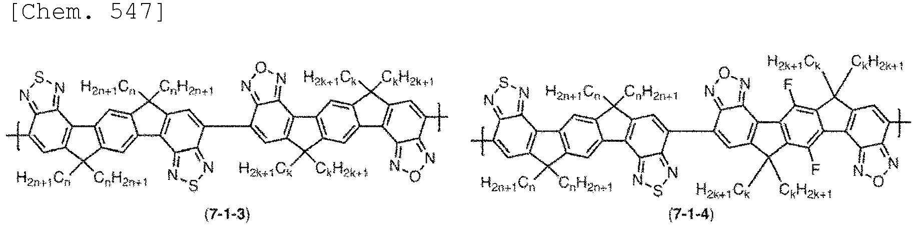

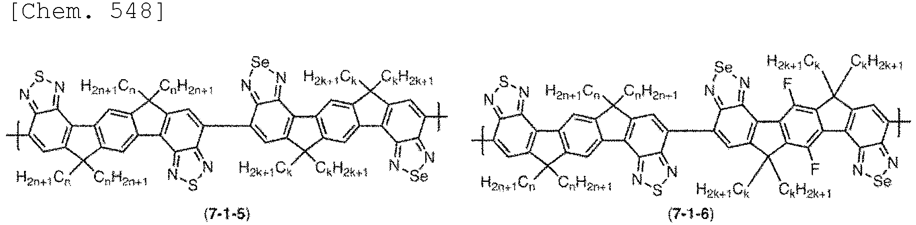

In these formulae, CₙH₂ₙ₊₁, CₖH₂ₖ₊₁, CₓH₂ₓ₊₁ and C_{y}H_{2y+1} represent linear-chain alkyl groups.

The structural unit represented by general formula (24) is preferably any structural unit represented by formula (3-1-2), formula (3-1-4), formula (3-1-6), formula (3-1-8), formula (3-1-10), formula (3-1-11), formula (3-1-13), formula (3-1-17), formula (3-1-18), formula (3-1-20), formula (3-1-21), formula (3-1-24), formula (3-1-25), formula (3-1-27), formula (3-1-28), formula (3-1-32), formula (3-1-33), formula (3-1-34), formula (3-1-35), formula (3-1-2-n6) to formula (3-1-2-n19), formula (3-1-4-n6), formula (3-1-4-n16), formula (3-1-6-n6) to formula (3-1-6-n19), formula (3-1-8-n6), formula (3-1-8-n16), formula (3-1-10-n6), formula (3-1-10-n16), formula (3-1-11-n6), formula (3-1-11-n16), formula (3-1-12-n6-k1), formula (3-1-12-n16-k1), formula (3-1-12-n16-k4), formula (3-1-12-n16-k4), formula (3-1-12-n1-k6), formula (3-1-12-n16-k6), formula (3-1-13-n6-k1) to formula (3-1-13-n16-k1), formula (3-1-13-n6-k8) to formula (3-1-13-n16-k8), formula (3-1-17-n6-k1) to formula (3-1-17-n16-k1), formula (3-1-17-n6-k6) to formula (3-1-17-n16-k6), formula (3-1-18-n6-k1), formula (3-1-18-n16-k1), formula (3-1-18-n6-k6), formula (3-1-18-n16-k6), formula (3-1-20-n6-k1) to formula (3-1-20-n16-k1), formula (3-1-20-n6-k3) to formula (3-1-20-n16-k3), formula (3-1-20-n6-k6) to formula (3-1-20-n16-k6), formula (3-1-21-n6-k1) to formula (3-1-21-n16-k1), formula (3-1-21-n6-k3) to formula (3-1-21-n16-k3), formula (3-1-21-n6-k6) to formula (3-1-21-n16-k6), formula (3-1-24-n6-k1) to formula (3-1-24-n16-k1), formula (3-1-24-n6-k6) to formula (3-1-24-n16-k6), formula (3-1-25-n6-k1), formula (3-1-25-n16-k1), formula (3-1-25-n6-k6), formula (3-1-25-n16-k6), formula (3-1-27-n6-k1), formula (3-1-27-n16-k1), formula (3-1-27-n6-k6), formula (3-1-27-n16-k6), formula (3-1-28-n6-k1), formula (3-1-28-n16-k1), formula (3-1-28-n6-k6), formula (3-1-28-n16-k6), formula (3-1-32-n6-k1), formula (3-1-32-n16-k1), formula (3-1-32-n6-k6), formula (3-1-32-n16-k6), formula (3-1-33-n6-k1), formula (3-1-33-n16-k1), formula (3-1-33-n6-k6), formula (3-1-33-n16-k6), formula (3-1-34-n6-k1), formula (3-1-34-n16-k1), formula (3-1-34-n6-k6), formula (3-1-34-n16-k6), formula (3-1-34-n6-k10), formula (3-1-34-n16-k10), formula (3-2-3), formula (3-2-4), formula (3-2-9), formula (3-2-12), formula (3-2-14), formula (3-2-15), formula (3-2-14-n6-k1) to formula (3-2-14-n16-k1), formula (3-2-14-n6-k8) to formula (3-2-14-n16-k8), formula (3-2-15-n6-x4-y2), formula (3-2-15-n16-x4-y2), formula (3-2-15-n6-x8-y6), formula (3-2-15-n16-x8-y6), formula (3-2-15-n6-x10-y8), formula (3-2-15-n16-x10-y8), formula (4-1-2), formula (4-1-4), formula (4-1-6), formula (4-1-8), formula (4-1-10), formula (4-1-11), formula (4-1-13), formula (4-1-17), formula (4-1-18), formula (4-1-20), formula (4-1-21), formula (4-1-24), formula (4-1-25), formula (4-1-27), formula (4-1-28), formula (4-1-34), formula (4-2-3), formula (4-2-4), formula (4-2-9), formula (4-2-12), formula (4-2-14) or formula (4-2-15) because this leads to higher film-forming properties and carrier mobility of the conjugated polymer in this embodiment. More preferably, the structural unit is any structural unit represented by formula (3-1-2), formula (3-1-6), formula (3-1-10), formula (3-1-13), formula (3-1-17), formula (3-1-20), formula (3-1-21), formula (3-1-24), formula (3-1-25), formula (3-1-2-n6) to formula (3-1-2-n19), formula (3-1-6-n6) to formula (3-1-6-n19), formula (3-1-10-n6), formula (3-1-10-n16), formula (3-1-12-n6-k1), formula (3-1-12-n16-k1), formula (3-1-12-n16-k4), formula (3-1-12-n16-k4), formula (3-1-12-n1-k6), formula (3-1-12-n16-k6), formula (3-1-13-n6-k1) to formula (3-1-13-n16-k1), formula (3-1-13-n6-k8) to formula (3-1-13-n16-k8), formula (3-1-17-n6-k1) to formula (3-1-17-n16-k1), formula (3-1-17-n6-k6) to formula (3-1-17-n16-k6), formula (3-1-20-n6-k1) to formula (3-1-20-n16-k1), formula (3-1-20-n6-k3) to formula (3-1-20-n16-k3), formula (3-1-20-n6-k6) to formula (3-1-20-n16-k6), formula (3-1-21-n6-k1) to formula (3-1-21-n16-k1), formula (3-1-21-n6-k3) to formula (3-1-21-n16-k3), formula (3-1-21-n6-k6) to formula (3-1-21-n16-k6), formula (3-1-24-n6-k1) to formula (3-1-24-n16-k1), formula (3-1-24-n6-k6) to formula (3-1-24-n16-k6), formula (3-1-35-n6-k1), formula (3-1-35-n8-k1), formula (3-1-35-n10-k1), formula (3-1-35-n12-k1), formula (3-1-35-n14-k1), formula (3-1-35-n16-k1), formula (3-2-14), formula (3-2-15), formula (3-2-14-n6-k1) to formula (3-2-14-n16-k1), formula (3-2-14-n6-k8) to formula (3-2-14-n16-k8), formula (3-2-15-n6-x4-y2), formula (3-2-15-n16-x4-y2), formula (3-2-15-n6-x8-y6), formula (3-2-15-n16-x8-y6), formula (3-2-15-n6-x10-y8) or formula (3-2-15-n16-x10-y8), even more preferably any structural unit represented by formula (3-1-2-n6) to formula (3-1-2-n19), formula (3-1-6-n6) to formula (3-1-6-n19), formula (3-1-10-n6), formula (3-1-10-n16), formula (3-1-12-n6-k1), formula (3-1-12-n16-k1), formula (3-1-12-n1-k4), formula (3-1-12-n16-k4), formula (3-1-12-n16-k6), formula (3-1-12-n16-k6), formula (3-1-13-n6-k8) to formula (3-1-13-n16-k8), formula (3-1-17-n6-k1) to formula (3-1-17-n16-k1), formula (3-1-20-n6-k1) to formula (3-1-20-n16-k1), formula (3-1-21-n6-k1) to formula (3-1-21-n16-k1), formula (3-1-21-n6-k3) to formula (3-1-21-n16-k3), formula (3-1-24-n6-k1) to formula (3-1-24-n16-k1), formula (3-1-35-n6-k1), formula (3-1-35-n8-k1), formula (3-1-35-n10-k1), formula (3-1-35-n12-k1), formula (3-1-35-n14-k1), formula (3-1-35-n16-k1), formula (3-2-14-n6-k1) to formula (3-2-14-n16-k1), formula (3-2-14-n6-k8) to formula (3-2-14-n16-k8), formula (3-2-15-n6-x10-y8) or formula (3-2-15-n16-x10-y8), especially preferably any structural unit represented by formula (3-1-2-n9) to formula (3-1-2-n12), formula (3-1-2-n16), formula (3-1-6-n10), formula (3-1-6-n16), formula (3-1-6-n18), formula (3-1-10-n16), formula (3-1-13-n16-k4), formula (3-1-13-n16-k6), formula (3-1-13-n10-k8), formula (3-1-13-n16-k8), formula (3-1-17-n10-k1), formula (3-1-17-n16-k1), formula (3-1-20-n16-k1), formula (3-1-21-n16-k1), formula (3-1-21-n16-k3), formula (3-1-24-n16-k1), formula (3-1-35-n16-k1), formula (3-2-14-n6-k1), formula (3-2-14-n6-k8) or formula (3-2-15-n16-x10-y8).

For the conjugated polymer in this embodiment, the conjugated polymer in this embodiment may include an additional structural unit that is neither a structural unit represented by general formula (2) nor a structural unit represented by general formula (3), provided that it does not impair the advantages of the present invention. It is preferred that the total percentage of the structural unit represented by general formula (2) and the structural unit represented by general formula (3) in the conjugated polymer in this embodiment be 90% by mass or more, and it is more preferred that this percentage be 95% by mass or more.

There is no specific restriction on the terminal structure of the conjugated polymer in this embodiment, but examples include a hydrogen atom, a boron-containing group, such as a dihydroxyboryl group or a dialkoxyboryl group, a tin-containing group, such as a trimethylstannyl group or a tributylstannyl group, a halogen atom, such as a chlorine atom, a bromine atom or an iodine atom, and an aromatic group, such as a phenyl group or a thienyl group. The structures at both ends may be the same or different from each other.

The weight-average molecular weight (Mw) of the conjugated polymer in this embodiment is preferably from 3000 to 10000000, more preferably from 3000 to 1000000, even more preferably from 3000 to 500000.

The molecular weight distribution (PDI) of the conjugated polymer in this embodiment is preferably from 1.05 to 20.0, more preferably from 1.2 to 10.0, even more preferably from 1.2 to 9.0, especially preferably from 1.2 to 8.0.

There is no specific restriction on the molar ratio between the structural unit represented by general formula (2) and the structural unit represented by general formula (3) (the structural unit represented by general formula (2):the structural unit represented by general formula (3)) in the conjugated polymer in this embodiment. It is, however, preferred that the molar ratio fall within the range of 10:1 to 1:10, it is more preferred that the molar ratio fall within the range of 5:1 to 1:5, it is even more preferred that the molar ratio fall within the range of **2:1** to 1:2, it is especially preferred that the molar ratio fall within the range of 1.2:1 to 1:1.2, particularly preferably 1:1.

### [Methods for Producing the Conjugated Polymer]

Methods for producing a conjugated polymer in this embodiment (Hereinafter referred to as "production methods in this embodiment.") will now be described. The methods for producing a conjugated polymer in this embodiment are as indicated in production processes (A) to (E) presented below.

### <Production Process (A)>

Production process (A) is a method in which a conjugated polymer in this embodiment is produced by coupling a compound (monomer) represented by general formula (mono-hal) with a compound (monomer) represented by general formula (mono-X-B) in the presence of a transition metal catalyst.

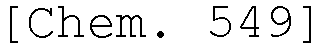

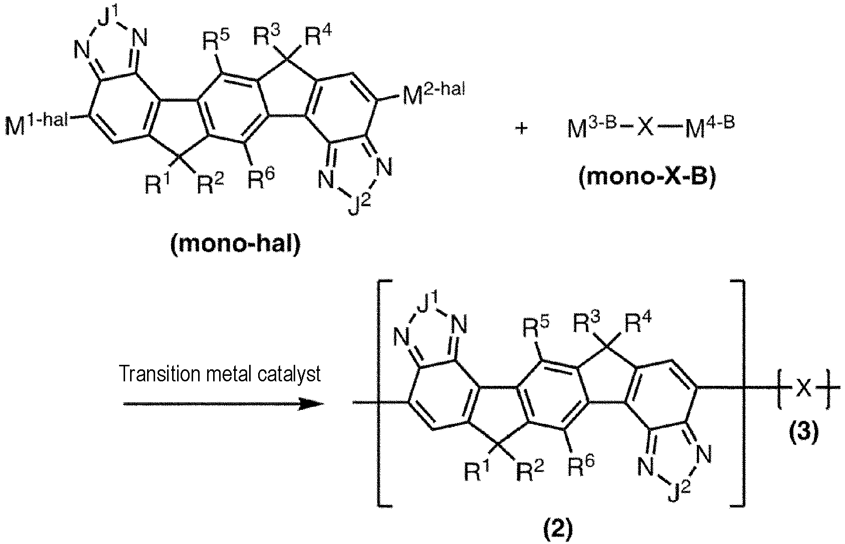

(In the formula, R¹, R², R³, R⁴, R⁵, R⁶, J¹, J² and X have the same meanings as described above. M^{1-hal} and M^{2-hal} each independently represent a halogen atom. M^{3-B} and M^{4-B} each independently represent a boron-containing group.)

The halogen atoms represented by M^{1-hal} and M^{2-hal} are preferably chlorine atoms, bromine atoms or iodine atoms because this leads to good production efficiency for the conjugated polymer in this embodiment. More preferably, the halogen atoms are bromine atoms or iodine atoms, even more preferably bromine atoms.

Examples of boron-containing groups represented by M^{3-B} and M^{4-B} include dihydroxyboryl groups and dialkoxyboryl groups. Preferably, the boron-containing groups are dihydroxyboryl groups or dialkoxyboryl groups because this leads to good production efficiency for the conjugated polymer in this embodiment. More preferably, the boron-containing groups are any groups of the groups represented by formula (12-1) to formula (12-6) below, even more preferably groups represented by formula (12-1), formula (12-3), formula (12-5) or formula (12-6), still more preferably formula (12-1) or formula (12-3), especially preferably formula (12-3).

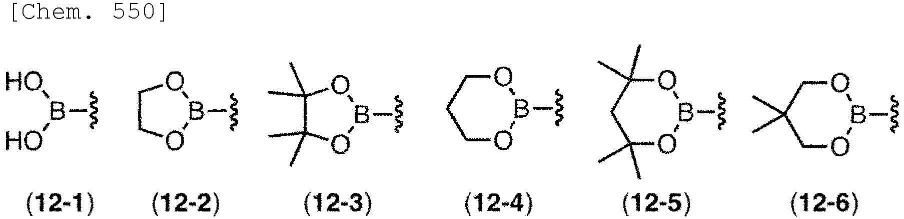

Production process (A) must be performed in the presence of a transition metal catalyst, and examples of transition metal catalysts that can be used include palladium catalysts, nickel catalysts and platinum catalysts. These transition metal catalysts can be "metals," "supported metals," "metal salts, such as chlorides, bromides, iodides, nitrates, sulfates, carbonates, oxalates, acetates and oxides of metals" or "complex compounds, such as olefin complexes, phosphine complexes, amide complexes, amine complexes, carbene complexes and acetylacetonato complexes." It is preferred to use a palladium catalyst or nickel catalyst because it results in a good reaction yield, and it is more preferred to use a palladium catalyst. It is also possible to use these metals, supported metals, metal salts and complex compounds in combination with a compound such as a tertiary phosphorus compound or carbene compound.

The palladium catalyst is not particularly limited, but examples include types of palladium metal, such as palladium black and palladium sponge. Types of supported palladium metal, such as palladium/alumina, palladium/carbon, palladium/silica and palladium/Y zeolite, are also examples of palladium catalysts. Examples, furthermore, include metal salts, such as palladium chloride, palladium bromide, palladium iodide, palladium acetate, palladium trifluoroacetate and palladium nitrate, and palladium catalysts such as the π-allyl palladium chloride dimer, palladium acetylacetonato, dichlorobis(acetonitrile)palladium, dichlorobis(benzonitrile)palladium, bis(dibenzylideneacetone)palladium, tris(dibenzylideneacetone)dipalladium, dichlorodiammine palladium, dichlorobis(triphenylphosphine)palladium, dichlorobis(tricyclohexylphosphine)palladium, tetrakis(triphenylphosphine)palladium, dichloro[1,2-bis(diphenylphosphino)ethane]palladium, dichloro[1,3-bis(diphenylphosphino)propane]palladium, dichloro[1,4-bis(diphenylphosphino)butane]palladium and dichloro[1,1'-bis(diphenylphosphino)ferrocene]palladium, bis(tri-tert-butylphosphine)palladium, bis(tricyclohexylphosphine)palladium, [1,3-bis(2,6-diisopropylphenyl)imidazol-2-ylidene](3-chloropyridyl)palladium(II) dichloride (Pd-PEPPSI-IPent), [1,3-bis(2,6-di-3-pentylphenyl)imidazol-2-ylidene](3-chloropyridyl)palladium(II) dichloride (Pd-PEPPSI-IPr) and [1,3-bis(2,6-diisopropylphenyl)imidazolidin-2-ylidene](3-chloropyridyl)palladium(II) dichloride (Pd-PEPPSI-SIPr).
Of these palladium catalysts, it is preferred to use palladium acetate, palladium acetylacetonato, bis(dibenzylideneacetone)palladium, tris(dibenzylideneacetone)dipalladium, tetrakis(triphenylphosphine)palladium (Pd(PPh₃)₄) or bis(tri-tert-butylphosphine) palladium because it results in a good reaction yield.

The nickel catalyst is not particularly limited, but specific examples include nickel catalysts such as nickel(II) chloride, bis(triphenylphosphine)nickel(II) dichloride, bis(2,4-pentanedionato)nickel(II) hydrate, bis(1,5-cyclooctadiene)nickel(0), dichloro(1,1'-bis(diphenylphosphino)ethane)nickel, dichloro(1,1'-bis(diphenylphosphino)propane)nickel and [1,3-bis(2,6-diisopropylphenyl)imidazol-2-ylidene]triphenylphosphinenickel(II) dichloride.

These palladium catalysts or nickel catalysts may be used alone, but may alternatively be used in combination with a tertiary phosphorus compound or carbene compound. Examples of tertiary phosphorus compounds that can be used include triphenylphosphine, trimethylphosphine, triethylphosphine, tributylphosphine, tri(tert-butyl)phosphine, tri-tert-butylphosphonium tetrafluoroborate, tricyclohexylphosphine, tri(o-tolyl)phosphine, tris(2-methoxyphenyl)phosphine, trioctylphosphine, 9,9-dimethyl-4,5-bis(diphenylphosphino)xanthene, 2-(di-tert-butylphosphino) biphenyl, 2-(dicyclohexylphosphino)biphenyl, 1,2-bis(diphenylphosphino)ethane, 1,3-bis(diphenylphosphino)propane, 1,4-bis(diphenylphosphino)butane, 1,1'-bis(diphenylphosphino)ferrocene, tert-butyldiphenylphosphine, 2-(diphenylphosphino)-2'-(N,N-dimethylamino)biphenyl, bis(diphenylphosphino)methane, 1,4-bis(diphenylphosphino)butane, tri(2-furyl)phosphine, tris(2,5-xylyl)phosphine, (R)-(+)-2,2'-bis(diphenylphosphino)-1,1'-binaphthyl, (S)-(-)-2,2'-bis(diphenylphosphino)-1,1'-binaphthyl and (±)-2,2'-bis(diphenylphosphino)-1,1'-binaphthyl. Examples of carbene compounds that can be used include 1,3-bis(2,6-diisopropylphenyl)imidazol-2-ylidene, 1,3-bis(2,6-diisopropylphenyl)imidazolidin-2-ylidene, 1,3-di-tert-butylimidazol-2-ylidene and 1,3-dimesitylimidazol-2-ylidene.

It is preferred to use triphenylphosphine, tri(tert-butyl)phosphine, tri-tert-butylphosphonium tetrafluoroborate, tricyclohexylphosphine or tri(o-tolyl)phosphine as a tertiary phosphorus compound because it results in a good reaction yield.

There is no specific restriction on the amount of transition metal catalyst used, but preferably, the amount is from 0.001 to 50 mole percent in relation to the compound represented by general formula (mono-hal) because this results in a good reaction yield. More preferably, the amount is from 0.1 to 20 mole percent.

The molar ratio between the tertiary phosphorus compound and the transition metal catalyst (the tertiary phosphorus compound:the transition metal catalyst) preferably falls within the range of 1:10 to 10:1. More preferably, it falls within the range of 1:5 to 5:1 because this results in a good reaction yield.

In production process (A), it is also possible to use a catalytic promoter. The catalytic promoter is not particularly limited, but specific examples include monovalent or divalent copper salts, such as copper fluoride, copper chloride, copper bromide, copper iodide and copper oxide.

Production process (A) can be conducted in a solvent. There is no specific restriction on the solvents that can be used, provided that the solvent does not inhibit the reaction. Examples include aliphatic hydrocarbon solvents, such as hexane, heptane, decane and tridecane; ether solvents, such as diisopropyl ether, dibutyl ether, cyclopentyl methyl ether (CPME), tetrahydrofuran (THF), 2-methyltetrahydrofuran, 1,4-dioxane and 1,2-dimethoxyethane; aromatic hydrocarbon solvents, such as benzene, toluene, xylene, mesitylene and tetralin; carbonate solvents, such as ethylene carbonate, propylene carbonate, dimethyl carbonate, diethyl carbonate, ethyl methyl carbonate and 4-fluoroethylene carbonate; ester solvents, such as ethyl acetate, butyl acetate, methyl propionate, ethyl propionate, methyl butyrate and γ-lactone; amide solvents, such as N,N-dimethylformamide (DMF), dimethylacetamide (DMAc) and N-methylpyrrolidone (NMP); urea solvents, such as N,N,N',N'-tetramethylurea (TMU) and N,N'-dimethylpropyleneurea (DMPU); sulfoxide solvents, such as dimethylsulfoxide (DMSO); alcohol solvents, such as methanol, ethanol, 2-propanol, butanol, octanol, benzyl alcohol, ethylene glycol, propylene glycol, diethylene glycol, triethylene glycol and 2,2,2-trifluoroethanol; halogen solvents, such as chloroform, dichloromethane, carbon tetrachloride, 1,2-dichloroethane, chlorobenzene and o-dichlorobenzene (DCB); fluorosolvents, such as bis(2,2,2-trifluoroethyl)=N,N-diisopropylphosphoramidate (PF-37) and tris(2,2,2-trifluoroethyl)=phosphate (TFEP); nitromethane; and water, and these may be mixed in certain ratios before use. Of these, the solvent is preferably an aliphatic hydrocarbon solvent, an aromatic hydrocarbon solvent, a halogen solvent, an ether solvent, an amide solvent, a sulfoxide solvent, a fluorosolvent, a solvent mixture of an aromatic hydrocarbon solvent and water, a solvent mixture of a halogen solvent and water, a solvent mixture of an ether solvent and water, a solvent mixture of an aromatic hydrocarbon solvent and a sulfoxide solvent, a solvent mixture of a halogen solvent and a sulfoxide solvent, an ether solvent and a sulfoxide solvent, a solvent mixture of an aromatic hydrocarbon solvent and a fluorosolvent, a solvent mixture of a halogen solvent and a fluorosolvent or a solvent mixture of an ether solvent and a fluorosolvent because this results in a good reaction yield. More preferably, the solvent is an aliphatic hydrocarbon solvent, an aromatic hydrocarbon solvent, a halogen solvent, an ether solvent, a solvent mixture of an aromatic hydrocarbon solvent and water, a solvent mixture of a halogen solvent and water, a solvent mixture of an ether solvent and water, a solvent mixture of an aromatic hydrocarbon solvent and a sulfoxide solvent, a solvent mixture of a halogen solvent and a sulfoxide solvent, a solvent mixture of an ether solvent and a sulfoxide solvent, a solvent mixture of an aromatic hydrocarbon solvent and a fluorosolvent, a solvent mixture of a halogen solvent and a fluorosolvent or a solvent mixture of an ether solvent and a fluorosolvent, even more preferably tetralin, toluene, chlorobenzene, o-DCB, CPME, THF, a solvent mixture of toluene and water or a solvent mixture of tetralin and water.

There is no specific restriction on the amount of solvent used, but preferably, the amount falls within the range of 0.001 to 100 mL/mg in relation to the weight of the compound represented by general formula (mono-hal).

Production process (A) can also be conducted with the addition of a phase transfer catalyst. Examples of phase transfer catalysts that can be used include ammonium salts, such as ethyltrimethylammonium iodide, didodecyldimethylammonium chloride, dimethyldioctadecylammonium iodide, dimethyldioctylammonium bromide, didecyldimethylammonium bromide, dimethyldimyristylammonium bromide, dihexadecyldimethylammonium bromide, diallyldimethylammonium chloride, dimethyldioctadecylammonium chloride, didodecyldimethylammonium bromide, 4-dimethylamino-1-neopentylpyridinium chloride, dodecyltrimethylammonium bromide, decyltrimethylammonium bromide, 1,1-dimethyl-4-phenylpiperazinium iodide, decamethonium iodide, decamethonium bromide, decyltrimethylammonium chloride, decyltrimethylammonium chloride, ethylhexadecyldimethylammonium bromide, (3-chloro-2-hydroxypropyl)trimethylammonium chloride, carbachol, choline chloride, chlorocholine chloride, bis(2-hydroxyethyl) dimethylammonium chloride, benzyldodecyldimethylammonium bromide, benzyltrimethylammonium bromide, benzyldodecyldimethylammonium chloride dihydrate, benzyltrimethylammonium dichloroiodate, benzyltributylammonium chloride, benzyltributylammonium bromide, bromocholine bromide, benzyltrimethylammonium chloride, benzyltriethylammonium iodide, benzyltriethylammonium hydroxide, benzyltriethylammonium chloride, benzyltriethylammonium bromide, benzyldimethylphenylammonium chloride, benzalkonium chloride, benzoylthiocholine iodide, benzyldimethylhexadecylammonium chloride hydrate, benzoylcholine iodide, benzoylcholine chloride, benzoylcholine bromide, Zephiran chloride hydrate, tetrabutylammonium p-toluenesulfonate, tetrabutylammonium hydrate, tetrahexylammonium hydrogen sulfate, tetraethylammonium nitrate, tributylammonium chloride, trimethylpropylammonium bromide, trimethylnonylammonium bromide, tetrabutylammonium acetate, tetrabutylammonium tetrafluoroborate, trimethyl[2-[(trimethylsilyl)methyl]benzyl]ammonium iodide, triethylammonium tetrafluoroborate, tris(2-hydroxyethyl)methylammonium hydroxide, tetrapropylammonium chloride, tetraethylammonium trifluoromethanesulfonate, tetra-n-octylammonium bromide, tetraheptylammonium bromide, tetrahexylammonium bromide, tetrabutylammonium triflate, tetrabutylammonium tetraphenylborate, tetrapentylammonium chloride, tetrapentylammonium bromide, tetramethylammonium acetate, tetraheptylammonium iodide, methyltri-n-octylammonium chloride, tetramethylammonium hexafluorophosphate, tetrabutylammonium bifluoride, tetrabutylammonium tribromide, tetrabutylammonium hexafluorophosphate, tetrabutylammonium thiocyanate, tetrabutylammonium triiodide, tetramethylammonium sulfate, tetra-n-octylammonium iodide, tetra(decyl)ammonium bromide, tetramethylammonium acetate, tetrahexylammonium iodide, tetraethylammonium tetrafluoroborate, tetraethylammonium p-toluenesulfonate, trimethyltetradecylammonium chloride, tetrabutylammonium tetrafluoroborate, tetradecyltrimethylammonium bromide, tetramethylammonium tetrafluoroborate, tetrabutylammonium perchlorate, tetrabutylammonium hydrogen sulfate, trioctylmethylammonium chloride (Aliquat 336), tetrapropylammonium bromide, tetrapropylammonium iodide, tetrabutylammonium chloride, tetrabutylammonium iodide, tetraethylammonium bromide, tetraethylammonium chloride, tetraethylammonium iodide, tetramethylammonium bromide, tetramethylammonium chloride, tetramethylammonium iodide, (ferrocenylmethyl)trimethylammonium bromide, (ferrocenylmethyl)dodecyl, hexadecyltrimethylammonium bromide, hexadecyltrimethylammonium chloride, hexyltrimethylammonium bromide, triethylammonium chloride, methyltri-n-octylammonium hydrogen sulfate, trimethyl-n-octylammonium bromide, trimethyl-n-octylammonium chloride, trimethylphenylammonium bromide, trimethylphenylammonium chloride, trimethylphenylammonium tribromide, octadecyltrimethylammonium bromide and tetrabutylammonium bromide, and phosphonium salts, such as tetrabutylphosphonium tetraphenylborate, tetrabutylphosphonium hexafluorophosphate, tetrabutylphosphonium tetrafluoroborate, tetraethylphosphonium tetrafluoroborate, tetraethylphosphonium hexafluorophosphate, tetra-n-octylphosphonium bromide, tetrabutylphosphonium chloride, tetraethylphosphonium bromide, tetraphenylphosphonium chloride, tetrabutylphosphonium bromide, tetrakis(hydroxymethyl)phosphonium sulfate, tetraphenylphosphonium bromide, tetrakis(hydroxymethyl)phosphonium chloride, tributyl-n-octylphosphonium bromide, hexadecyltributylphosphonium bromide, tributyldodecylphosphonium bromide, (2-carboxyethyl)triphenylphosphonium bromide and tributyl(cyanomethyl)phosphonium chloride, and these may be mixed in certain ratios. Of these, the phase transfer catalyst is preferably an ammonium salt because this results in a good reaction yield. More preferably, the phase transfer catalyst is Aliquat 336.

Production process (A) can be conducted in the presence of a base. Examples of bases that can be used include metal alkoxides, such as sodium butoxide and potassium butoxide; metal alkyls, such as butyl lithium; metal amides, such as lithium hexamethyldisilazide, lithium diisopropylamide, 2,2,6,6-tetramethylpiperidinyl lithium and the 2,2,6,6-tetramethylpiperidinyl magnesium chloride lithium chloride complex; inorganic bases, such as lithium hydroxide, sodium hydroxide, potassium hydroxide, cesium hydroxide, sodium carbonate, potassium carbonate, cesium carbonate and tripotassium phosphate; and organic bases, such as triethylamine, diisopropylethylamine, pyridine, lutidine, diazabicycloundecene (DBU), 1,5,7-triazabicyclo[4.4.0]dec-5-ene (TBD), 7-methyl-1,5,7-triazabicyclo[4.4.0]dec-5-ene (MTBD) and 1,4-diazabicyclo[2,2,2]octane (DABCO), and these may be mixed in certain ratios. Of these, the base is preferably an inorganic base because this results in a good reaction yield. More preferably, the base is sodium carbonate, potassium carbonate or tripotassium phosphate.

There is no specific restriction on the amount of base used, but preferably, the amount is from 0.1 to 10 molar equivalents in relation to the compound represented by general formula (mono-hal) because this results in a good reaction yield. More preferably, the amount is from 1 to 5 molar equivalents.

Production process (A) can be conducted at a temperature selected as appropriate from 0°C to 240°C. Preferably, production process (A) is conducted at a temperature selected as appropriate from 70°C to 220°C because this results in a good reaction yield. More preferably, production process (A) is conducted at a temperature selected as appropriate from 80°C to 200°C.

Production process (A) can also be conducted using a microwave reactor.

Production process (A) is preferably performed in an inert gas atmosphere, such as an argon gas or nitrogen gas, or under reduced pressure.

The duration of reaction varies depending on the compounds (the compounds represented by general formula (mono-hal) or general formula (mono-X-B)) used and the solvent and the reaction temperature. Usually, however, it is preferred that the duration of reaction be from 0.1 to 100 hours, more preferably from 1 to 78 hours.

The conjugated polymer in this embodiment is obtained by performing ordinary treatment after the end of production process (A). When necessary, the polymer may be purified using general-purpose means that those skilled in the art use to purify polymeric compounds, such as washing, precipitation, filtration, dialysis, column chromatography, preparative HPLC and Soxhlet extraction, as needed.

It is also possible to produce a conjugated polymer in this embodiment with a functional group, such as a thienyl group or phenyl group, introduced at its end by adding an organic boron compound or organic tin compound during the reaction or after the reaction for the purpose of improving the carrier mobility or solubility of the resulting conjugated polymer. The functional group may be introduced using a combination of known methods; for example, it can be introduced according to methods disclosed in non-patent literature (e.g., Macromolecules, vol. 48, pp. 6994-7006, 2015).

For the compound represented by general formula (mono-X-B) used in production process (A), there is no limitation on the method for obtaining it. For example, however, it can be produced with reference to methods described in non-patent literature (e.g., Journal of the American Chemical Society, vol. 134, pp. 3498-3507, 2012 and Nature Chem vol. 11, pp. 271-277, 2019). A commercially available compound may also be used.

### <Production Process (B)>

Production process (B) is a method in which a conjugated polymer in this embodiment is produced by coupling a compound (monomer) represented by general formula (mono-hal) with a compound (monomer) represented by general formula (mono-X-Sn) in the presence of a transition metal catalyst.

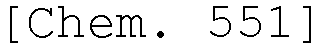

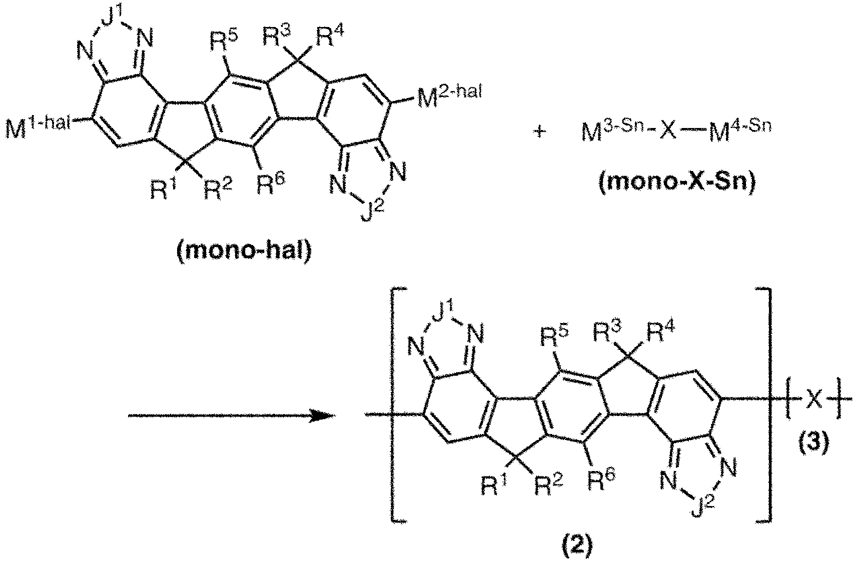

(In the formula, R¹, R², R³, R⁴, R⁵, R⁶, J¹, J², X, M^{1-hal} and M^{2-hal} have the same meanings as described above. M^{3-Sn} and M^{4-Sn} each independently represent a tin-containing group.)

The halogen atoms represented by M^{1-hal} and M^{2-hal} are preferably chlorine atoms, bromine atoms or iodine atoms because this leads to good production efficiency for the conjugated polymer in this embodiment. More preferably, the halogen atoms are bromine atoms or iodine atoms, even more preferably bromine atoms.

Examples of tin-containing groups represented by M^{3-Sn} and M^{4-Sn} include trialkylstannyl groups, dialkylarylstannyl groups, alkyldiarylstannyl groups and triarylstannyl groups. Preferably, the tin-containing groups are trialkylstannyl groups or triarylstannyl groups because this leads to good production efficiency for the conjugated polymer in this embodiment. More preferably, the tin-containing groups are any groups represented by formula (13-1) to formula (13-5), even more preferably groups represented by formula (13-1). It should be noted that Me, Et, Pr, Bu and Ph herein represent a methyl group, an ethyl group, a propyl group, a butyl group and a phenyl group, respectively.

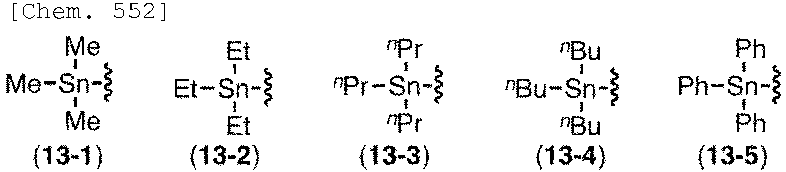

Production process (B) must be performed in the presence of a transition metal catalyst, and examples of transition metal catalysts that can be used include palladium catalysts, nickel catalysts and platinum catalysts. These transition metal catalysts can be "metals," "supported metals," "metal salts, such as chlorides, bromides, iodides, nitrates, sulfates, carbonates, oxalates, acetates or oxides of metals" or "complex compounds, such as olefin complexes, phosphine complexes, amide complexes, amine complexes, carbene complexes or acetylacetonate complexes." It is preferred to use a palladium catalyst or nickel catalyst because it results in a good reaction yield, and it is more preferred to use a palladium catalyst. It is also possible to use these metals, supported metals, metal salts and complex compounds in combination with a compound such as a tertiary phosphorus compound or carbene compound.

The palladium catalyst is not particularly limited, but examples include the palladium catalysts listed by way of example in production process (A).

Of these palladium catalysts, it is preferred to use palladium acetate, palladium acetylacetonato, bis(dibenzylideneacetone)palladium, tris(dibenzylideneacetone)dipalladium, tetrakis(triphenylphosphine)palladium (Pd(PPh₃)₄) or bis(tri-tert-butylphosphine)palladium as a palladium catalyst because it results in a good reaction yield.

The nickel catalyst is not particularly limited, but examples include the nickel catalysts listed by way of example in production process (A).

These palladium catalysts or nickel catalysts may be used alone, but may alternatively be used in combination with a tertiary phosphorus compound or carbene compound. Examples of tertiary phosphorus compounds or carbene compounds that can be used include the tertiary phosphorus compounds or carbene compounds listed by way of example in production process (A).

It is preferred to use triphenylphosphine, tri(tert-butyl)phosphine or tricyclohexylphosphine or tri(o-tolyl)phosphine as a tertiary phosphorus compound because it results in a good reaction yield.

The molar ratio between the tertiary phosphorus compound and the transition metal catalyst (the tertiary phosphorus compound:the transition metal catalyst) preferably falls within the range of 1:10 to 10:1. More preferably, it falls within the range of 1:5 to 5:1 because this results in a good reaction yield.

There is no specific restriction on the amount of transition metal catalyst used, but preferably, the amount is from 0.001 to 50 mole percent in relation to the compound represented by general formula (mono-hal) because this results in a good reaction yield. More preferably, the amount is from 0.1 to 20 mole percent.

In production process (B), it is also possible to use a catalytic promoter. The catalytic promoter is not particularly limited, but specific examples include monovalent or divalent copper salts, such as copper fluoride, copper chloride, copper bromide, copper iodide and copper oxide.

Production process (B) can be conducted in a solvent. There is no specific restriction on the solvents that can be used, provided that the solvent does not inhibit the reaction. Examples include the solvents listed by way of example in production process (A). Preferably, the solvent is an aliphatic hydrocarbon solvent, an aromatic hydrocarbon solvent, a halogen solvent, an ether solvent, an amide solvent, a sulfoxide solvent, a fluorosolvent, a solvent mixture of an aromatic hydrocarbon solvent and water, a solvent mixture of a halogen solvent and water, a solvent mixture of an ether solvent and water, a solvent mixture of an aromatic hydrocarbon solvent and a sulfoxide solvent, a solvent mixture of a halogen solvent and a sulfoxide solvent, a solvent mixture of an ether solvent and a sulfoxide solvent, a solvent mixture of an aromatic hydrocarbon solvent and a fluorosolvent, a solvent mixture of a halogen solvent and a fluorosolvent or a solvent mixture of an ether solvent and a fluorosolvent because this results in a good reaction yield. More preferably, the solvent is an aliphatic hydrocarbon solvent, an aromatic hydrocarbon solvent, a halogen solvent, an ether solvent, a solvent mixture of an aromatic hydrocarbon solvent and a fluorosolvent, a solvent mixture of a halogen solvent and a fluorosolvent or a solvent mixture of an ether solvent and a fluorosolvent, even more preferably tetralin, toluene, chlorobenzene, o-DCB or THF.

There is no specific restriction on the amount of solvent used, but preferably, the amount falls within the range of 0.001 to 100 mL/mg in relation to the weight of the compound represented by general formula (mono-hal).

Production process (B) can be conducted at a temperature selected as appropriate from 0°C to 240°C. Preferably, production process (B) is conducted at a temperature selected as appropriate from 70°C to 220°C because this results in a good reaction yield. More preferably, production process (B) is conducted at a temperature selected as appropriate from 100°C to 200°C.

Production process (B) can also be conducted using a microwave reactor.

Production process (B) is preferably performed in an inert gas atmosphere, such as an argon gas or nitrogen gas, or under reduced pressure.

The duration of reaction varies depending on the compounds (the compounds represented by general formula (mono-hal) or general formula (mono-X-Sn)) used and the solvent and the reaction temperature. Preferably, however, the duration of reaction is from 0.1 to 100 hours, more preferably from 1 to 90 hours.

The conjugated polymer in this embodiment is obtained by performing ordinary treatment after the end of production process (B). When necessary, the polymer may be purified using general-purpose means that those skilled in the art use to purify polymeric compounds, such as washing, precipitation, filtration, dialysis, column chromatography, preparative HPLC and Soxhlet extraction, as needed.

It is also possible to produce a conjugated polymer in this embodiment with a functional group, such as a thienyl group or phenyl group, introduced at its end by adding an organic boron compound or organic tin compound during the reaction or after the reaction for the purpose of improving the carrier mobility or solubility of the resulting conjugated polymer. The functional group may be introduced using a combination of known methods; for example, it can be introduced according to methods disclosed in non-patent literature (e.g., Macromolecules, vol. 48, pp. 6994-7006, 2015).

For the compound represented by general formula (mono-X-Sn) used in production process (B), there is no limitation on the method for obtaining it. For example, however, it can be produced with reference to methods described in non-patent literature (e.g., Journal of the

American Chemical Society, vol. 134, pp. 3498-3507, 2012 and Nature Chem vol. 11, pp. 271-277, 2019). A commercially available compound may also be used.

### <Production Process (C)>

Production process (C) is a method in which a conjugated polymer in this embodiment is produced by coupling a compound (monomer) represented by general formula (mono-B) with a compound (monomer) represented by general formula (mono-X-hal) in the presence of a transition metal catalyst.

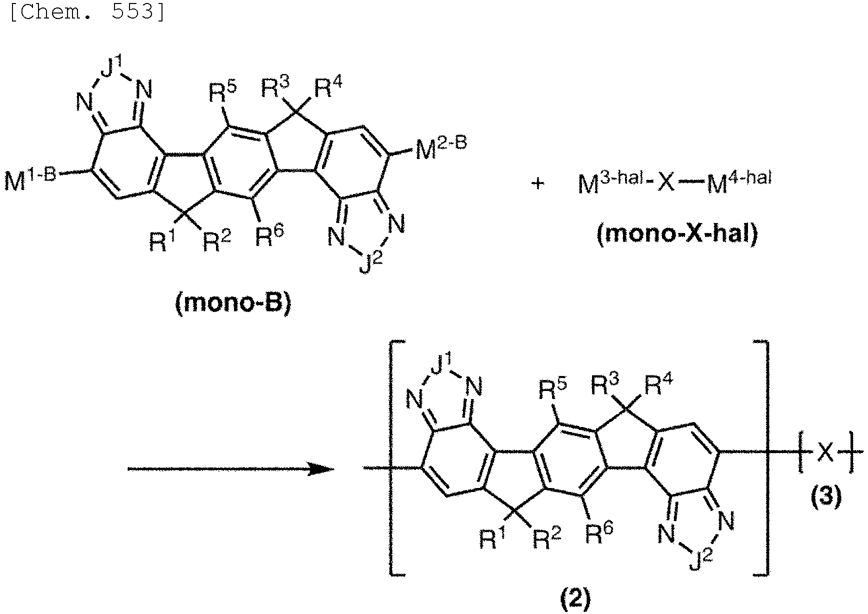

(In the formula, R¹, R², R³, R⁴, R⁵, R⁶, J¹, J², J³, J⁴ and X¹ have the same meanings as described above. M^{1-B} and M^{2-B} each independently represent a boron-containing group. M^{3-hal} and M^{4-hal} each independently represent a halogen atom.)

Examples of boron-containing groups represented by M^{1-B} and M^{2-B} include dihydroboryl groups and dialkoxyboryl groups. Preferably, the boron-containing groups are dihydroboryl groups or dialkoxyboryl groups because this leads to good production efficiency for the conjugated polymer in this embodiment. More preferably, the boron-containing groups are any groups of the groups represented by formula (12-1) to formula (12-6) above, even more preferably groups represented by formula (12-1), formula (12-3), formula (12-5) or formula (12-6), still more preferably formula (12-1) or formula (12-3), especially preferably formula (12-3).

An example of halogen atoms represented by M^{3-hal} and M^{4-hal} is chlorine atoms, bromine atoms or iodine atoms.

Production process (C) must be performed in the presence of a transition metal catalyst, and examples of transition metal catalysts that can be used include palladium catalysts, nickel catalysts and platinum catalysts. These transition metal catalysts can be "metals," "supported metals," "metal salts, such as chlorides, bromides, iodides, nitrates, sulfates, carbonates, oxalates, acetates or oxides of metals" or "complex compounds, such as olefin complexes, phosphine complexes, amide complexes, amine complexes, carbene complexes or acetylacetonate complexes." It is also possible to use these metals, supported metals, metal salts and complex compounds in combination with a compound such as a tertiary phosphorus compound or carbene compound.

The palladium catalysts are not particularly limited, but examples include the palladium catalysts listed by way of example in production process (A).

The nickel catalysts are not particularly limited, but examples include the nickel catalysts listed by way of example in production process (A).

These palladium catalysts or nickel catalysts may be used alone, but may alternatively be used in combination with a tertiary phosphorus compound or carbene compound. Examples of tertiary phosphorus compounds or carbene compounds that can be used include the tertiary phosphorus compounds or carbene compounds listed by way of example in production process (A).

In production process (C), it is also possible to use a catalytic promoter. The catalytic promoter is not particularly limited, but specific examples include monovalent or divalent copper salts, such as copper fluoride, copper chloride, copper bromide, copper iodide and copper oxide.

Production process (C) can be conducted in a solvent. There is no specific restriction on the solvents that can be used, provided that the solvent does not inhibit the reaction. Examples include the solvents listed by way of example in production process (A).

Production process (C) can also be conducted with the addition of a phase transfer catalyst. Examples of phase transfer catalysts that can be used include the phase transfer catalysts listed by way of example in production process (A).

Production process (C) can be conducted in the presence of a base. Examples of bases that can be used include the bases listed by way of example in production process (A).

Production process (C) can be conducted at a temperature selected as appropriate from 0°C to 240°C.

Production process (C) can also be conducted using a microwave reactor.

Production process (C) can be performed in an inert gas atmosphere, such as an argon gas or nitrogen gas, or under reduced pressure.

The conjugated polymer in this embodiment is obtained by performing ordinary treatment after the end of production process (C). When necessary, the polymer may be purified using general-purpose means that those skilled in the art use to purify polymeric compounds, such as washing, precipitation, filtration, dialysis, column chromatography, preparative HPLC and Soxhlet extraction, as needed.

It is also possible to produce a conjugated polymer in this embodiment with a functional group, such as a thienyl group or phenyl group, introduced at its end by adding an organic boron compound or organic tin compound during the reaction or after the reaction for the purpose of improving the carrier mobility or solubility of the resulting conjugated polymer. The functional group may be introduced using a combination of known methods; for example, it can be introduced according to methods disclosed in non-patent literature (e.g., Macromolecules, vol. 48, pp. 6994-7006, 2015).

For the compound represented by general formula (mono-X-hal) used in production process (C), there is no limitation on the method for obtaining it. For example, however, it can be produced with reference to methods described in non-patent literature (e.g., Journal of the American Chemical Society, vol. 134, pp. 3498-3507, 2012 and Nature Chem vol. 11, pp. 271-277, 2019). A commercially available compound may also be used.

### <Production Process (D)>

Production process (D) is a method in which a conjugated polymer in this embodiment is produced by performing a coupling reaction of a compound (monomer) represented by general formula (mono-Sn) with a compound (monomer) represented by general formula (mono-X-hal) in the presence of a transition metal catalyst.

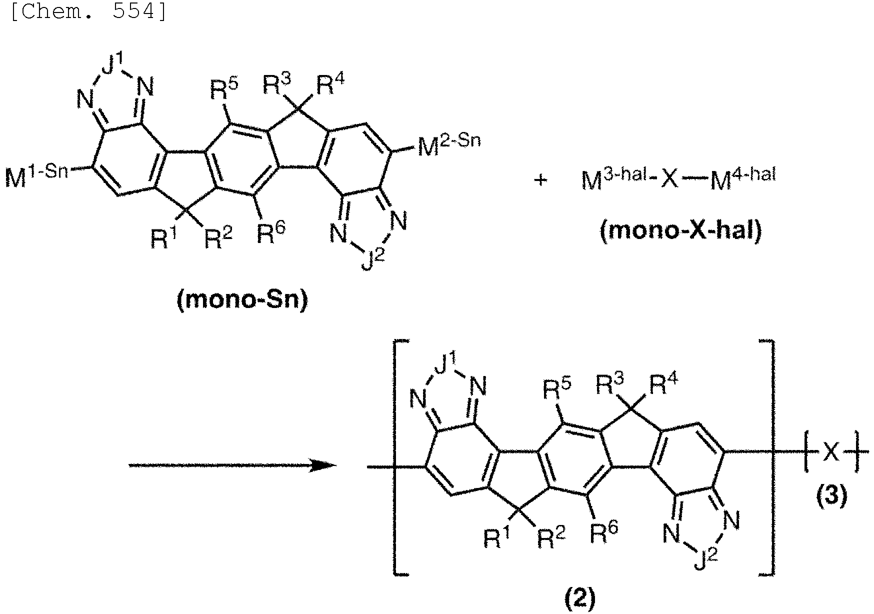

(In the formula, R¹, R², R³, R⁴, R⁵, R⁶, J¹, J², J³, J⁴, X¹, M^{3-hal} and M^{4-hal} have the same meanings as described above. M^{1-Sn} and M^{2-Sn} each independently represent a tin-containing group.)

Examples of tin-containing groups represented by M^{1-Sn} and M^{2-Sn} include trialkylstannyl groups, dialkylarylstannyl groups, alkyldiarylstannyl groups and triarylstannyl groups. Preferably, the tin-containing groups are trialkylstannyl groups or triarylstannyl groups because this leads to good production efficiency for the conjugated polymer in this embodiment. More preferably, the tin-containing groups are any groups represented by formula (13-1) to formula (13-5) above, even more preferably groups represented by formula (13-1).

Production process (D) must be performed in the presence of a transition metal catalyst, and examples of transition metal catalysts that can be used include palladium catalysts, nickel catalysts and platinum catalysts. These transition metal catalysts can be "metals," "supported metals," "metal salts, such as chlorides, bromides, iodides, nitrates, sulfates, carbonates, oxalates, acetates or oxides of metals" or "complex compounds, such as olefin complexes, phosphine complexes, amide complexes, amine complexes, carbene complexes or acetylacetonate complexes." It is also possible to use these metals, supported metals, metal salts and complex compounds in combination with a compound such as a tertiary phosphorus compound or carbene compound.

The palladium catalysts are not particularly limited, but examples include the palladium catalysts listed by way of example in production process (A).

The nickel catalysts are not particularly limited, but examples include the nickel catalysts listed by way of example in production process (A).

These palladium catalysts or nickel catalysts may be used alone, but may alternatively be used in combination with a tertiary phosphorus compound or carbene compound. Examples of tertiary phosphorus compounds or carbene compounds that can be used include the tertiary phosphorus compounds or carbene compounds listed by way of example in production process (A).

In production process (D), it is also possible to use a catalytic promoter. The catalytic promoter is not particularly limited, but specific examples include monovalent or divalent copper salts, such as copper fluoride, copper chloride, copper bromide, copper iodide and copper oxide.

Production process (D) can be conducted in a solvent. There is no specific restriction on the solvents that can be used, provided that the solvent does not inhibit the reaction. Examples include the solvents listed by way of example in production process (A).

Production process (D) can be conducted at a temperature selected as appropriate from 0°C to 240°C.

Production process (D) can also be conducted using a microwave reactor.

Production process (D) can be performed in an inert gas atmosphere, such as an argon gas or nitrogen gas, or under reduced pressure.

The conjugated polymer in this embodiment is obtained by performing ordinary treatment after the end of production process (D). When necessary, the polymer may be purified using general-purpose means that those skilled in the art use to purify polymeric compounds, such as washing, precipitation, filtration, dialysis, column chromatography, preparative HPLC and Soxhlet extraction, as needed.

It is also possible to produce a conjugated polymer in this embodiment with a functional group, such as a thienyl group or phenyl group, introduced at its end by adding an organic boron compound or organic tin compound during the reaction or after the reaction for the purpose of improving mobility or solubility. The functional group may be introduced using a combination of known methods; for example, it can be introduced according to methods disclosed in non-patent literature (e.g., Macromolecules, vol. 48, pp. 6994-7006, 2015).

For the compound represented by general formula (mono-X-hal) used in production process (D), there is no limitation on the method for obtaining it. For example, however, it can be produced with reference to methods described in non-patent literature (e.g., Journal of the American Chemical Society, vol. 134, pp. 3498-3507, 2012 and Nature Chem vol. 11, pp. 271-277, 2019). A commercially available compound may also be used.

### <Production Process (E)>

Production process (E) is a method in which a conjugated polymer in this embodiment is produced by performing a coupling reaction of a compound (monomer) represented by general formula (mono-hal) with a compound (monomer) represented by general formula (mono-X-H) in the presence of a transition metal catalyst.

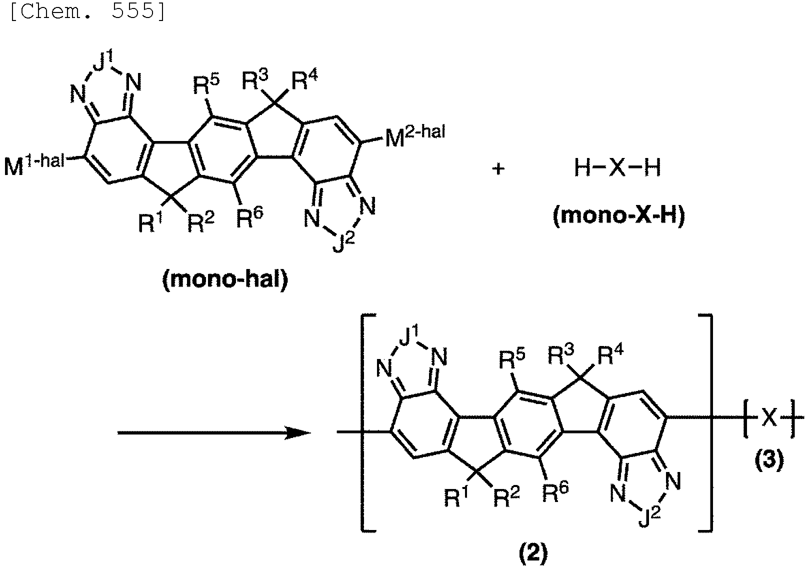

(In the formula, R¹, R², R³, R⁴, R⁵, R⁶, J¹, J², X, M^{1-Hal} and M^{2-hal} have the same meanings as described above.)

The halogen atoms represented by M^{1-hal} and M^{2-hal} are preferably chlorine atoms, bromine atoms or iodine atoms because this leads to good production efficiency for the conjugated polymer in this embodiment. More preferably, the halogen atoms are bromine atoms or iodine atoms, even more preferably bromine atoms.

Production process (E) must be performed in the presence of a transition metal catalyst, and examples of transition metal catalysts that can be used include palladium catalysts, nickel catalysts and platinum catalysts. These transition metal catalysts can be "metals," "supported metals," "metal salts, such as chlorides, bromides, iodides, nitrates, sulfates, carbonates, oxalates, acetates or oxides of metals" or "complex compounds, such as olefin complexes, phosphine complexes, amide complexes, amine complexes, carbene complexes or acetylacetonate complexes." It is preferred to use a palladium catalyst or nickel catalyst because it results in a good reaction yield, and it is more preferred to use a palladium catalyst. It is also possible to use these metals, supported metals, metal salts and complex compounds in combination with a compound such as a tertiary phosphorus compound or carbene compound.

The palladium catalyst or nickel catalyst is not particularly limited, but examples include the palladium catalysts or nickel catalysts listed by way of example in production process (A).

Of these palladium catalysts or nickel catalysts, palladium catalysts are preferred because they result in a good reaction yield. It is particularly preferred to use palladium acetate, palladium acetylacetonato, bis(dibenzylideneacetone)palladium or tris(dibenzylideneacetone)dipalladium.

These palladium catalysts may be used alone, but may alternatively be used in combination with a tertiary phosphorus compound. Examples of tertiary phosphorus compounds that can be used include the tertiary phosphorus compounds listed by way of example in production process (A).

It is preferred to use tris(2-methoxyphenyl)phosphine as a tertiary phosphorus compound because it results in a good reaction yield.

The molar ratio between the tertiary phosphorus compound and the transition metal catalyst (the tertiary phosphorus compound:the transition metal catalyst) preferably falls within the range of 1:10 to 10:1. More preferably, it falls within the range of 1:5 to 5:1 because this results in a good reaction yield.

There is no specific restriction on the amount of transition metal catalyst used, but preferably, the amount is from 0.001 to 50 mole percent in relation to the compound represented by general formula (mono-hal) because this results in a good reaction yield. More preferably, the amount is from 0.1 to 20 mole percent.

In production process (E), it is also possible to use a catalytic promoter. The catalytic promoter is not particularly limited, but specific examples include monovalent or divalent copper salts, such as copper fluoride, copper chloride, copper bromide, copper iodide and copper oxide.

Production process (E) can be conducted in a solvent. There is no specific restriction on the solvents that can be used, provided that the solvent does not inhibit the reaction. Examples include the solvents listed by way of example in production process (A). Preferably, the solvent is an aliphatic hydrocarbon solvent, an aromatic hydrocarbon solvent, a halogen solvent, an ether solvent, an amide solvent, a sulfoxide solvent, a fluorosolvent, a solvent mixture of an aromatic hydrocarbon solvent and water, a solvent mixture of a halogen solvent and water, a solvent mixture of an ether solvent and water, a solvent mixture of an aromatic hydrocarbon solvent and a sulfoxide solvent, a solvent mixture of a halogen solvent and a sulfoxide solvent, a solvent mixture of an ether solvent and a sulfoxide solvent, a solvent mixture of an aromatic hydrocarbon solvent and a fluorosolvent, a solvent mixture of a halogen solvent and a fluorosolvent or a solvent mixture of an ether solvent and a fluorosolvent because this results in a good reaction yield. More preferably, the solvent is an aliphatic hydrocarbon solvent, an aromatic hydrocarbon solvent, a halogen solvent, an ether solvent, a solvent mixture of an aromatic hydrocarbon solvent and a fluorosolvent, a solvent mixture of a halogen solvent and a fluorosolvent or a solvent mixture of an ether solvent and a fluorosolvent, even more preferably CPME, THF, toluene, xylene or mesitylene.

There is no specific restriction on the amount of solvent used, but preferably, the amount falls within the range of 0.001 to 100 mL/mg in relation to the weight of the compound represented by general formula (mono-hal).

Production process (E) can be conducted in the presence of a base. Examples of bases that can be used include the bases listed by way of example in production process (A). Preferably, the base is an inorganic base because this results in a good reaction yield. More preferably, the base is sodium carbonate, potassium carbonate or cesium carbonate.

There is no specific restriction on the amount of base used, but preferably, the amount is from 0.1 to 10 molar equivalents in relation to the compound represented by general formula (mono-hal) because this results in a good reaction yield. More preferably, the amount is from 1 to 5 molar equivalents.

Production process (E) can be conducted in the presence of an organic acid. Examples of organic acids that can be used include carboxylic acids, such as formic acid, acetic acid, propionic acid, butyric acid, pivalic acid, valeric acid, caproic acid, enanthic acid, caprylic acid, pelargonic acid, neodecanoic acid, capric acid, benzoic acid and 1-adamantanecarboxylic acid; and sulfonic acids, such as methanesulfonic acid, trifluoromethanesulfonic acid, benzenesulfonic acid, p-toluenesulfonic acid and 10-camphorsulfonic acid (CSA), and these may be mixed in certain ratios. Of these, carboxylic acids are preferred because they result in a good reaction yield, and acetic acid, pivalic acid, neodecanoic acid and 1-adamantanecarboxylic acid are more preferred.

There is no specific restriction on the amount of organic acid used, but preferably, the amount is from 0.01 to 10 molar equivalents in relation to the compound represented by general formula (mono-hal) because this results in a good reaction yield. More preferably, the amount is from 0.1 to 5 molar equivalents.

In production process (E), additives can be used. Examples of additives include amines, such as N,N,N',N'-tetramethylmethylenediamine (TMMDA), N,N,N',N'-tetramethylethylenediamine (TMEDA), N,N,N',N'-tetramethyltrimethylenediamine (TMPDA), N,N,N',N'-tetramethyltetramethylenediamine, N,N,N',N'-tetramethyl-1,6-diaminohexane (TMHDA) and N,N,N',N'-tetraethylethylenediamine (TEEDA), and these may be mixed in certain ratios. Of these, TMEDA is preferred because it results in a good reaction yield.

There is no specific restriction on the amount of additives used, but preferably, the amount is from 0.01 to 1 molar equivalent in relation to the compound represented by general formula (mono-hal) because this results in a good reaction yield. More preferably, the amount is from 0.05 to 0.5 molar equivalents.

Production process (E) can be conducted at a temperature selected as appropriate from 0°C to 240°C. Preferably, production process (E) is conducted at a temperature selected as appropriate from 70°C to 220°C because this results in a good reaction yield. More preferably, production process (E) is conducted at a temperature selected as appropriate from 80°C to 200°C.

Production process (E) can also be conducted using a microwave reactor.

Production process (E) is preferably performed in an inert gas atmosphere, such as an argon gas or nitrogen gas, or under reduced pressure.

The duration of reaction varies depending on the compounds (the compounds represented by general formula (mono-hal) or general formula (mono-X-H)) used and the solvent and the reaction temperature. Preferably, however, the duration of reaction is from 0.1 to 100 hours, more preferably from 1 to 90 hours.

The conjugated polymer in this embodiment is obtained by performing ordinary treatment after the end of production process (E). When necessary, the polymer may be purified using general-purpose means that those skilled in the art use to purify polymeric compounds, such as washing, precipitation, filtration, dialysis, column chromatography, preparative HPLC and Soxhlet extraction, as needed.

For the compound represented by general formula (mono-X-H) used in production process (E), there is no limitation on the method for obtaining it. For example, however, it can be produced with reference to methods described in non-patent literature (e.g., Journal of the American Chemical Society, vol. 126, pp. 16433-16439, 2004 and Polymer Journal, vol. 52, pp. 313-321, 2020). A commercially available compound may also be used.

### [Film Formation Composition]

A film formation composition containing a conjugated polymer in this embodiment (Hereinafter referred to as "a film formation composition in this embodiment) will now be described.

A film formation composition in this embodiment is a film formation composition containing a conjugated polymer in this embodiment and a solvent.

The solvent is not particularly restricted, provided that the conjugated polymer in this embodiment can be dissolved or dispersed in it. Examples, however, include ether solvents, such as diisopropyl ether, dibutyl ether, CPME, THF, 2-methyltetrahydrofuran, 1,4-dioxane and 1,2-dimethoxyethane; aromatic hydrocarbon solvents, such as benzene, toluene, xylene, mesitylene and tetralin; carbonate solvents, such as ethylene carbonate, propylene carbonate, dimethyl carbonate, diethyl carbonate, ethyl methyl carbonate and 4-fluoroethylene carbonate; ester solvents, such as ethyl acetate, butyl acetate, methyl propionate, ethyl propionate, methyl butyrate and γ-lactone; amide solvents, such as DMF, DMAc and NMP; urea solvents, such as TMU and DMPU; sulfoxide solvents, such as DMSO; alcohol solvents, such as methanol, ethanol, 2-propanol, butanol, octanol, benzyl alcohol, ethylene glycol, propylene glycol, diethylene glycol, triethylene glycol and 2,2,2-trifluoroethanol; halogen solvents, such as chloroform, dichloromethane, carbon tetrachloride, 1,2-dichloroethane, chlorobenzene and o-DCB; nitromethane; and water, and these may be mixed in certain ratios before use. Of these, aromatic hydrocarbons and halogen solvents are preferred because they have a high boiling point and volatilize mildly. Toluene, xylene, mesitylene, cyclohexylbenzene, tetralin, 3,4-dimethylanisole, chlorobenzene and o-DCB are more preferred.

There is no specific restriction on the amount of solvent used. It is more preferred to add the solvent in such a manner that it will reach a concentration of 0.01 to 30 percent by weight with the concentration of the conjugated polymer in this embodiment being from 0.001 to 95 percent by weight.

The film formation composition in this embodiment is obtained by dissolving or dispersing the conjugated polymer in this embodiment in the solvent. The method for dissolving or dispersing the conjugated polymer in this embodiment in the solvent can be a method well known to those skilled in the art, such as stirring, shaking or a ball mill. During this, heating may be performed.

In the film formation composition in this embodiment, a binder for improving film-forming properties may be contained. Examples of such binders include polymers, such as polystyrene, poly-α-methylstyrene, polyvinyl naphthalene, poly(ethylene-co-norbornene), polymethyl methacrylate, polytriarylamine and poly(9,9-dioctylfluorene-co-dimethyltriphenylamine). There is no specific restriction on the concentration of the binder. Preferably, however, the concentration is from 0.1 to 10.0 percent by weight because this results in good ease of application.

### [Organic Thin Film]

An organic thin film containing a conjugated polymer in this embodiment (Hereinafter referred to as "an organic thin film in this embodiment.) will now be described.

An organic thin film in this embodiment is formed using a film formation composition in this embodiment. There is no specific restriction on the method for forming the film using a film formation composition in this embodiment, and examples include simple coating methods, such as spin coating, drop casting, dip coating and cast coating; and printing methods, such as a dispenser, inkjet, slit coating, blade coating, flexography, screen printing, gravure printing and offset printing. In particular, spin coating, drop casting and inkjet are preferred because the film can be formed efficiently with them.

There is no specific restriction on the thickness of the organic thin film in this embodiment, but preferably, the thickness is from 1 nm to 1000 nm because this leads to high carrier mobility. More preferably, the thickness is from 10 nm to 500 nm.

### [Organic Semiconductor Element]

Examples of organic semiconductor elements containing a conjugated polymer in this embodiment include an organic thin-film transistor element, an organic thermoelectric transducer element, an organic photoelectric converter element and an organic imaging element. An organic thin-film transistor element and an organic photoelectric converter element are preferred, and an organic thin-film transistor element is more preferred.

A method for fabricating an organic thin-film transistor element containing a conjugated polymer in this embodiment (hereinafter referred to as "an organic thin-film transistor element in this embodiment), an organic thin-film transistor element containing the conjugated polymer in its active layer in particular, will be described.

An organic thin-film transistor element according to the present invention is obtained by forming an insulating layer and an organic thin film according to the present invention as an active layer on a substrate and attaching a source electrode, a drain electrode and a gate electrode to it.

In Fig. 1, the structure of elements included in organic thin-film transistor elements in this embodiment is illustrated. Here, 1001 is a bottom gate-top contact transistor element, 1002 is a bottom gate-bottom contact transistor element, 1003 is a top gate-top contact transistor element, and 1004 is a top gate-top contact transistor element. 1 indicates an active layer (organic semiconductor layer), 2 indicates a substrate, 3 indicates a gate electrode, 4 indicates a gate insulating layer, 5 indicates a source electrode, and 6 indicates a drain electrode.

Examples of substrates include plastic substrates, such as polyethylene terephthalate, polyethylene naphthalate, polymethyl methacrylate, polymethyl acrylate, polyethylene, polypropylene, polystyrene, cyclic polyolefin, polyimide, polycarbonate, polyvinyl phenol, polyvinyl alcohol, poly(diisopropyl fumarate), poly(diethyl fumarate), poly(diisopropyl maleate), polyether sulfone, polyphenylene sulfide and cellulose triacetate; inorganic substrates, such as glass, quartz, aluminum oxide, silicon, highly doped silicon, silicon oxide, tantalum dioxide, tantalum pentoxide and indium tin oxide; and metal substrates, such as gold, copper, chromium, titanium and aluminum. Of these, glass, silicon and highly doped silicon are preferred because they result in good transistor performance, and glass is more preferred.

Examples of gate electrodes include inorganic electrodes, such as aluminum, gold, silver, copper, highly doped silicon, tin oxides, indium oxide, indium tin oxide, chromium, titanium, tantalum, chromium, graphene and carbon nanotubes, and organic electrodes, such as doped conductive polymers (PEDOT-PSS). Of these, inorganic electrodes are preferred because they have good conductivity, and gold is more preferred.

Examples of insulating layers include inorganic insulating layers, such as silicon oxide, silicon nitride, aluminum oxide, aluminum nitride, titanium oxide, tantalum dioxide, tantalum pentoxide, indium tin oxide, tin oxide, vanadium oxide, barium titanate and bismuth titanate; and organic insulating layers, such as polyethylene terephthalate, polyethylene naphthalate, polymethyl methacrylate, polymethyl acrylate, polyethylene, polypropylene, polystyrene, cyclic polyolefin, polyimide, polycarbonate, polyvinyl phenol, polyvinyl alcohol, poly(diisopropyl fumarate), poly(diethyl fumarate), poly(diisopropyl maleate), polyether sulfone, polyphenylene sulfide, cellulose triacetate, polycyclopentane, polycyclohexane-ethylene copolymers, polyfluorinated cyclopentane, CYTOP^{™}, polyfluorinated cyclohexane, polyfluorinated cyclohexane-ethylene copolymers, Parylene N^{™}, Parylene C^{™}, Parylene D^{™}, Parylene HT^{™} and Parylene C-UVF^{™}. Of these, organic insulating layers are preferred because they have good insulating properties, and Parylene C^{™} is more preferred. Surfaces of these insulating layers, furthermore, may be subjected to modification treatment, for example with a silane, such as octadecyltrichlorosilane, decyltrichlorosilane, decyltrimethoxysilane, octyltrichlorosilane, octadecyltrimethoxysilane, β-phenethyltrichlorosilane, β-phenethyltrimethoxysilane, phenyltrichlorosilane or phenyltrimethoxysilane; a phosphonic acid, such as octadecylphosphonic acid, decylphosphonic acid or octylphosphonic acid; or a silylamine, such as hexamethyldisilazane. Of these, modification treatment with octadecyltrichlorosilane, octyltrichlorosilane, β-phenethyltrichlorosilane, octadecylphosphonic acid, octylphosphonic acid or hexamethyldisilazane is preferred because it leads to improved carrier mobility and current on-off ratio and a reduced threshold voltage of the organic thin-film transistor element in this embodiment.

For the source electrode and the drain electrode, examples are the same electrodes as those listed by way of example for the gate electrode. Of these, inorganic electrodes are preferred because they have good conductivity, and gold is more preferred. To increase injection efficiency for carriers, furthermore, these electrodes may be subjected to surface treatment using a surface treatment material. Examples of such surface treatment materials include benzenethiol and pentafluorobenzenethiol.

### [Recapitulation]

A compound according to aspect 1 of the present invention is a compound represented by general formula (1) below.

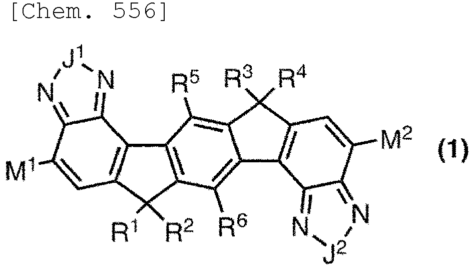

(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom. M¹ and M² each independently represent one group selected from the group consisting of a hydrogen atom, a halogen atom, a boron-containing group and a tin-containing group.)

A compound according to aspect 2 of the present invention may be the compound in aspect 1 of the present invention, wherein, in relation to gist 1, each of R¹, R², R³ and R⁴ is independently a C1 to C34 alkyl group, R¹ and R² are optionally bound to each other to form a ring together with the carbon atom to which the R¹ and R² are bound, R³ and R⁴ are optionally bound to each other to form a ring together with the carbon atom to which the R³ and R⁴ are bound, each of R⁵ and R⁶ is independently a hydrogen atom or a fluorine atom, each of J¹ and J² is independently an oxygen atom, a sulfur atom or a selenium atom, and each of M¹ and M² is independently one group selected from the group consisting of a hydrogen atom, a bromine atom, an iodine atom, a boron-containing group and a tin-containing group.

A compound according to aspect 3 of the present invention may be the compound in aspect 1 or 2 of the present invention, wherein R⁵ and R⁶ are hydrogen atoms, J¹ and J² are sulfur atoms, and each of M¹ and M² is independently one group selected from the group consisting of a hydrogen atom, a bromine atom, a boron-containing group and a tin-containing group.

A conjugated polymer according to aspect 4 of the present invention is a conjugated polymer comprising a structural unit represented by general formula (2) below

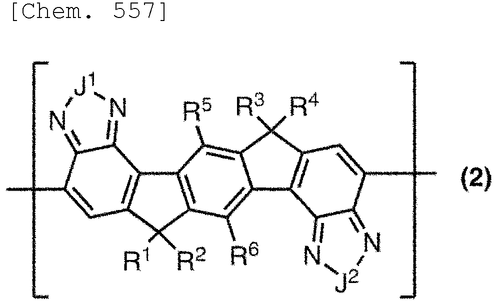

(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom.) and a structural unit represented by general formula (3) below.

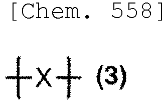

(In the formula, X represents a divalent heteroaromatic ring linking group optionally substituted with a C1 to C50 alkyl group or a C1 to C50 alkoxy group.)

A conjugated polymer according to aspect 5 of the present invention may be the conjugated polymer in aspect 4 of the present invention, wherein the conjugated polymer is a structural unit represented by general formula (24) below, which has the structural unit represented by general formula (2) and the structural unit represented by general formula (3) alternately.

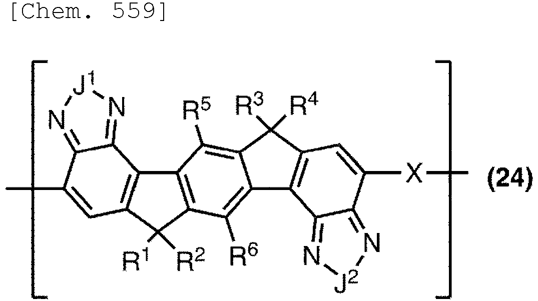

(In the formula, R¹, R², R³, R⁴, R⁵, R⁶, J¹, J² and X have the same meanings as described above.)

A conjugated polymer according to aspect 6 of the present invention may be the conjugated polymer in aspect 4 or 5 of the present invention, wherein each of R¹, R², R³ and R⁴ is independently a C1 to C34 alkyl group, R¹ and R² are optionally bound to each other to form a ring together with the carbon atom to which the R¹ and R² are bound, R³ and R⁴ are optionally bound to each other to form a ring together with the carbon atom to which the R³ and R⁴ are bound, each of R⁵ and R⁶ is independently a hydrogen atom, a fluorine atom or a C1 to C34 alkyl group, and each of J¹ and J² is independently an oxygen atom, a sulfur atom or a selenium atom.

A conjugated polymer according to aspect 7 of the present invention may be the conjugated polymer in any of aspects 4 to 6 of the present invention, wherein R⁵ and R⁶ are hydrogen atoms, and J¹ and J² are sulfur atoms.

A conjugated polymer according to aspect 8 of the present invention may be the conjugated polymer in any of aspects 4 to 7 of the present invention, wherein X is a divalent heteroaromatic ring linking group selected from the group consisting of linking groups represented by general formula (4) to general formula (23) below.

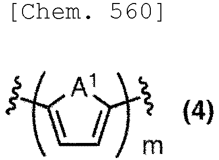

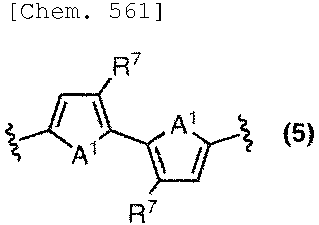

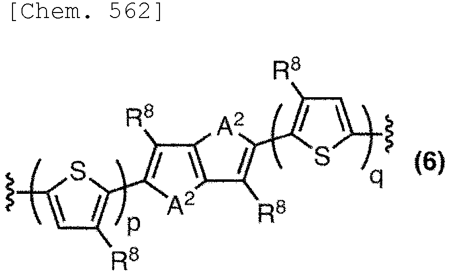

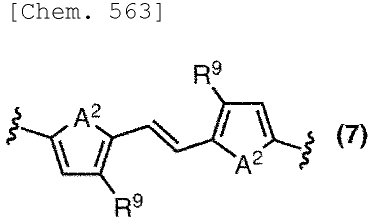

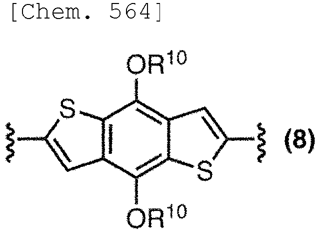

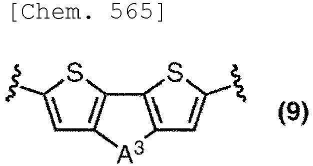

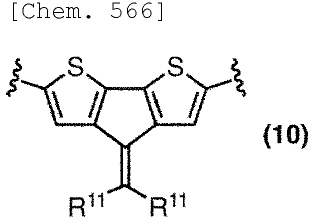

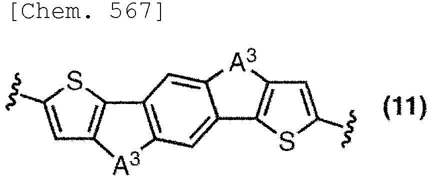

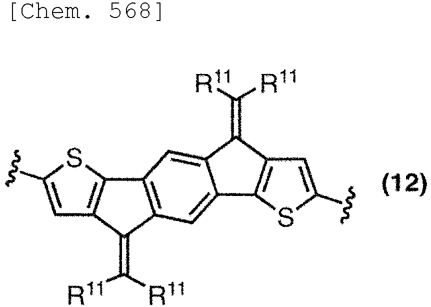

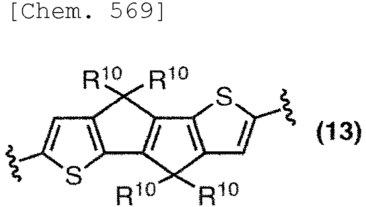

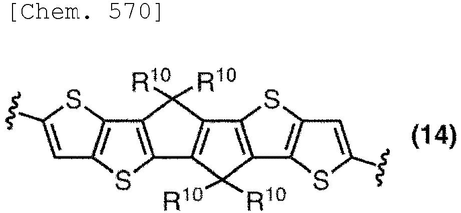

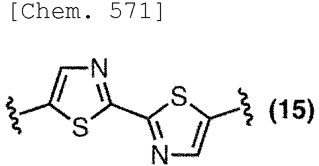

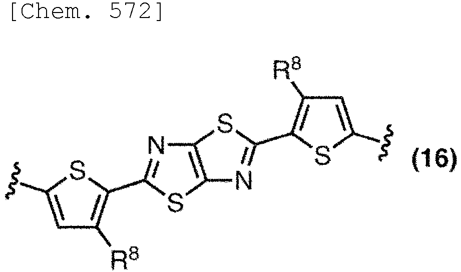

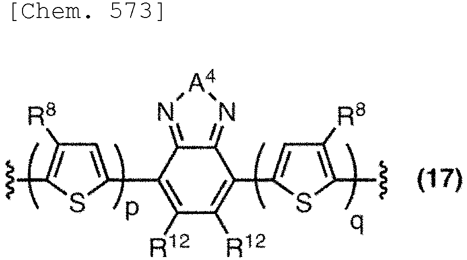

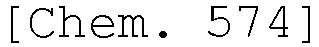

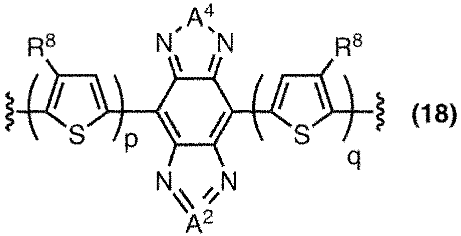

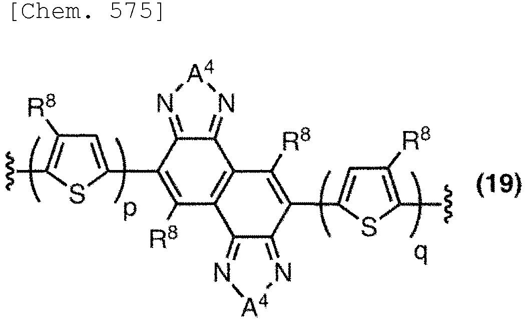

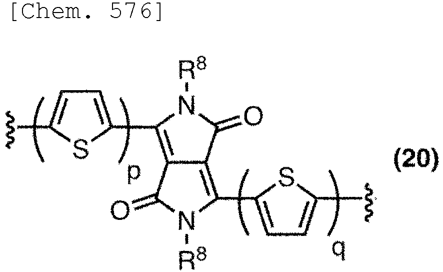

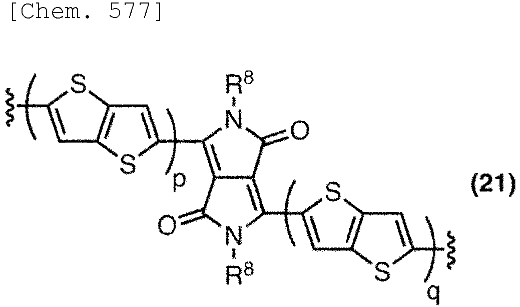

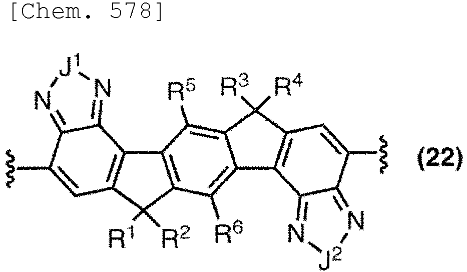

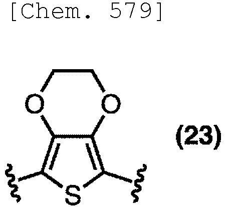

(In the formula, R¹, R², R³, R⁴, R⁵, R⁶, J¹ and J² have the same meanings as described above. A¹ and A² each independently represent a chalcogen atom. A³ represents a chalcogen atom, C(R¹⁰)₂, C(H)(R¹⁰), Si(R¹⁰)₂ or NR¹⁰, independently at each occurrence. A⁴ represents a chalcogen atom or NR¹⁰, independently at each occurrence. R⁷ represents a hydrogen atom, a fluorine atom or a C1 to C50 alkyl group. Multiple R⁷s may be the same or different from one another. R⁸ represents a hydrogen atom or a C1 to C50 alkyl group. Multiple R⁸s may be the same or different from one another. R⁹ represents a hydrogen atom, a fluorine atom or a C1 to C50 alkyl group. Multiple R⁹s may be the same or different from one another. R¹⁰ represents a C1 to C50 alkyl group. Multiple R¹⁰s may be the same or different from one another. R¹¹ represents a C1 to C50 alkyl group or a C1 to C50 thioalkyl group. R¹² represents a hydrogen atom, a fluorine atom, a C1 to C50 alkyl group or a C1 to C50 alkoxy group. m represents 1 to 3. p and q each independently represent 0 or 1.)

A conjugated polymer according to aspect 9 of the present invention may be the conjugated polymer in any of aspects 4 to 8 of the present invention, wherein A¹ is an oxygen atom, a sulfur atom or a selenium atom, independently at each occurrence, A² is a sulfur atom or a selenium atom, independently at each occurrence, A³ is C(R¹⁰)₂, C(H)(R¹⁰), Si(R¹⁰)₂ or NR¹⁰, independently at each occurrence, A⁴ is an oxygen atom, a sulfur atom, a selenium atom or NR¹⁰, independently at each occurrence, R⁷ is a fluorine atom or a C1 to C34 alkyl group, independently at each occurrence, R⁸ is a hydrogen atom or a C1 to C34 alkyl group, independently at each occurrence, R⁹ is a hydrogen atom, a fluorine atom or a C1 to C34 alkyl group, independently at each occurrence, R¹⁰ is a C1 to C34 alkyl group, independently at each occurrence, R¹¹ is a C1 to C34 alkyl group or a C1 to C34 thioalkyl group, independently at each occurrence, and R¹² is a hydrogen atom, a fluorine atom, a C1 to C34 alkyl group or a C1 to C34 alkoxy group, independently at each occurrence.

A conjugated polymer according to aspect 10 of the present invention may be the conjugated polymer in any of aspects 4 to 9 of the present invention, wherein A¹, A² and A⁴ are sulfur atoms, A³ is C(R¹⁰)₂ or C(H)(R¹⁰), R⁷ is a fluorine atom or a C1 to C20 alkyl group, independently at each occurrence, R⁸ is a hydrogen atom or a C1 to C20 alkyl group, independently at each occurrence, R⁹ is a hydrogen atom, a fluorine atom or a C1 to C20 alkyl group, independently at each occurrence, R¹⁰ is a C1 to C20 alkyl group, independently at each occurrence, R¹¹ is a C1 to C20 alkyl group or a C1 to C20 thioalkyl group, independently at each occurrence, and R¹² is a hydrogen atom, a fluorine atom, a C1 to C20 alkyl group or a C1 to C20 alkoxy group, independently at each occurrence.

A method according to aspect 11 of the present invention for producing a conjugated polymer is a method for producing a conjugated polymer composed of a structural unit represented by general formula (2) below and a structural unit represented by general formula (3) below, the method comprising:
allowing a monomer represented by general formula (mono-hal) below to react with a monomer represented by general formula (mono-X-B) below in the presence of a transition metal catalyst.

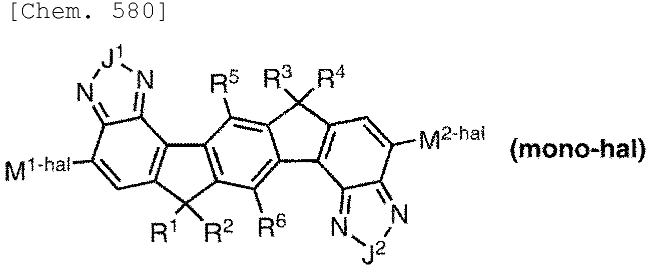

(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom. M^{1-Hal} and M^{2-hal} each independently represent a halogen atom.)

[Chem. 581] **M^{3-B}-X-M^{4-B}** (**mono-X-B**)

(In the formula, X represents a divalent heteroaromatic ring linking group optionally substituted with a C1 to C50 alkyl group or a C1 to C50 alkoxy group. M^{3-B} and M^{4-B} each independently represent a boron-containing group.)

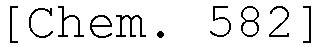

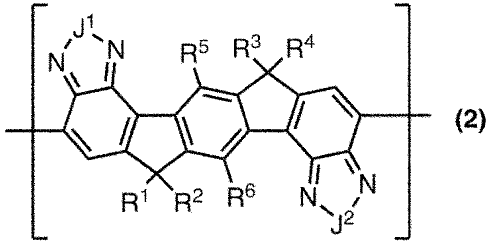

(In the formula, R¹, R², R³, R⁴, R⁵, R⁶, J¹ and J² have the same meanings as described above.)

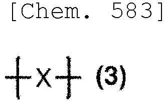

(In the formula, X has the same meaning as described above.)

A method according to aspect 12 of the present invention for producing a conjugated polymer is a method for producing a conjugated polymer composed of a structural unit represented by general formula (2) below and a structural unit represented by general formula (3) below, the method comprising:
allowing a monomer represented by general formula (mono-hal) below to react with a monomer represented by general formula (mono-X-Sn) below in the presence of a transition metal catalyst.

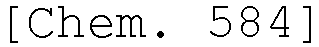

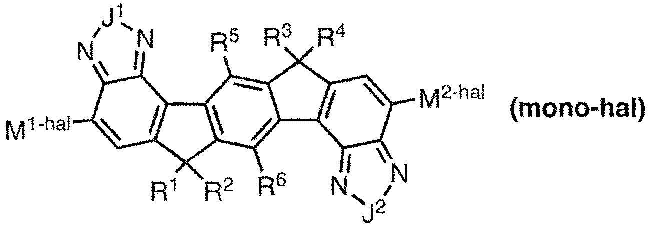

(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom. M^{1-Hal} and M^{2-hal} each independently represent a halogen atom.)

[Chem. 585] **M^{3-Sn}-X-M^{4-Sn}** (**mono-X-Sn**)

(In the formula, X represents a divalent heteroaromatic ring linking group optionally substituted with a C1 to C50 alkyl group or a C1 to C50 alkoxy group. M^{3-Sn} and M^{4-Sn} each independently represent a tin-containing group.)

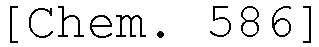

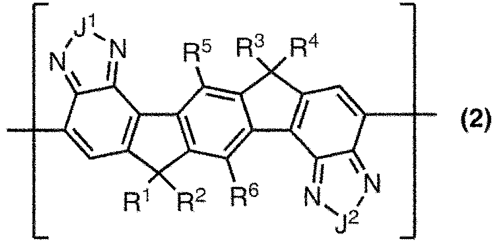

(In the formula, R¹, R², R³, R⁴, R⁵, R⁶, J¹ and J² have the same meanings as described above.)

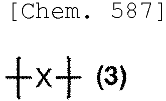

(In the formula, X has the same meaning as described above.)

A method according to aspect 13 of the present invention for producing a conjugated polymer is a method for producing a conjugated polymer composed of a structural unit represented by general formula (2) below and a structural unit represented by general formula (3) below, the method comprising:
allowing a monomer represented by general formula (mono-B) below to react with a monomer represented by general formula (mono-X-hal) below in the presence of a transition metal catalyst.

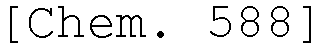

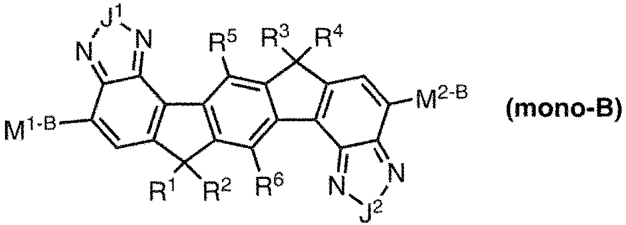

(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom. M^{1-B} and M^{2-B} each independently represent a boron-containing group.)

[Chem. 589] **M^{3-hal}-X-M^{4-hal}** (**mono-X-hal**)

(In the formula, X represents a divalent heteroaromatic ring linking group optionally substituted with a C1 to C50 alkyl group or a C1 to C50 alkoxy group. M^{3-hal} and M^{4-hal} each independently represent a halogen atom.)

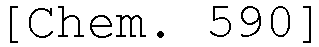

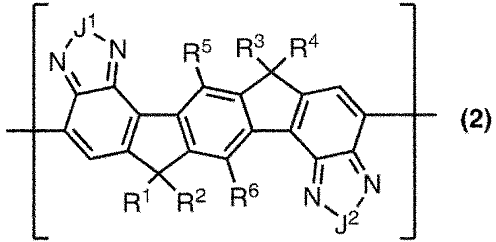

(In the formula, R¹, R², R³, R⁴, R⁵, R⁶, J¹ and J² have the same meanings as described above.)

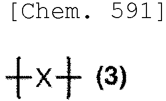

(In the formula, X has the same meaning as described above.)

A method according to aspect 14 of the present invention for producing a conjugated polymer is a method for producing a conjugated polymer composed of a structural unit represented by general formula (2) below and a structural unit represented by general formula (3) below, the method comprising:
allowing a monomer represented by general formula (mono-Sn) below to react with a monomer represented by general formula (mono-X-hal) below in the presence of a transition metal catalyst.

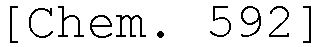

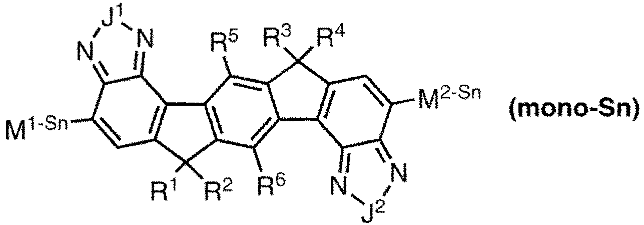

(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom. M^{1-Sn} and M^{2-Sn} each independently represent a tin-containing group.)

[Chem. 593] **M^{3-hal}-X-M^{4-hal}** (**mono-X-hal**)

(In the formula, X represents a divalent heteroaromatic ring linking group optionally substituted with a C1 to C50 alkyl group or a C1 to C50 alkoxy group. M^{3-hal} and M^{4-hal} each independently represent a halogen atom.)

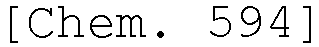

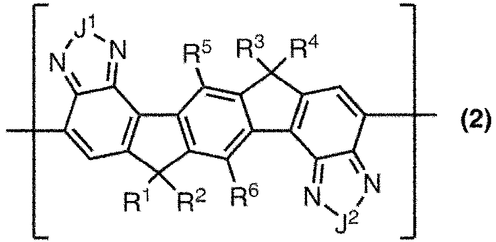

(In the formula, R¹, R², R³, R⁴, R⁵, R⁶, J¹ and J² have the same meanings as described above.)

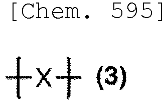

(In the formula, X has the same meaning as described above.)

A method according to aspect 15 of the present invention for producing a conjugated polymer is a method for producing a conjugated polymer composed of a divalent structural unit represented by general formula (2) below and a structural unit represented by general formula (3) below, the method comprising:
allowing a monomer represented by general formula (mono-hal) below to react with a monomer represented by general formula (mono-X-H) below in the presence of a transition metal catalyst.

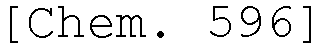

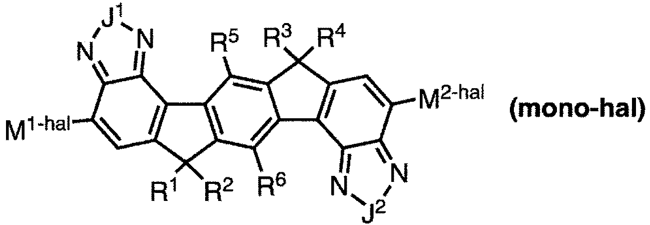

(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom. M^{1-Hal} and M^{2-hal} each independently represent a halogen atom.)

[Chem. 597] **H-X-H (mono-X-H)**

(In the formula, X represents a divalent heteroaromatic ring linking group optionally substituted with a C1 to C50 alkyl group or a C1 to C50 alkoxy group.)

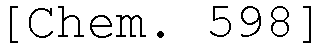

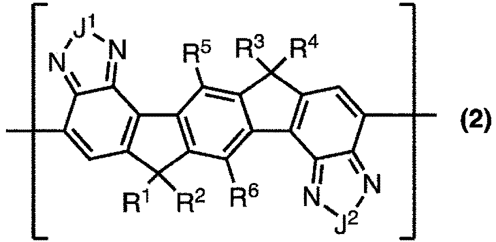

(In the formula, R¹, R², R³, R⁴, R⁵, R⁶, J¹ and J² have the same meanings as described above.)

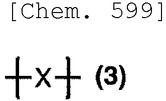

(In the formula, X has the same meaning as described above.)

A film formation composition according to aspect 16 of the present invention comprises the conjugated polymer in any of aspects 4 to 10 of the present invention.

An organic thin film according to aspect 17 of the present invention comprises the conjugated polymer in any of aspects 4 to 10 of the present invention.

An organic semiconductor element according to aspect 18 of the present invention comprises the conjugated polymer in any of aspects 4 to 10 of the present invention.

An organic thin-film transistor element according to aspect 19 of the present invention comprises the conjugated polymer in any of aspects 4 to 10 of the present invention.

### [Examples]

The present invention will now be more specifically described by providing examples, but the present invention is not limited to the following examples.

The monomers used as source materials in the examples and their precursors were structurally analyzed by ¹H-NMR measurement. The molecular weight and molecular weight distribution of the conjugated polymers obtained in the examples were estimated by Gel Permeation Chromatography (GPC) measurement. The reagents were commercially available ones.

### <NMR Measurement Conditions>

Measuring instrument: Bruker ASCEND^{™} ADVANCE III HD (400 MHz)
Measurement solvent: Deuterated chloroform (CDCl₃) or deuterated DMSO (DMSO-d₆)
Internal standard: Trimethylsilane (TMS)

### <GPC Measurement Conditions>

Measuring instrument: Tosoh Corporation HLC-8320GPC EcoSEC high-performance GPC system
Columns: TSKgel SuperH4000 + 3000 + 2000 + 1000
Measurement solvent: THF
Measurement temperature: 40°C
Calibration curve: Polystyrene standards

### <High-Temperature GPC Measurement Conditions>

Measuring instrument: Tosoh Corporation HLC-8321GPC/HT high-temperature GPC system
Column: TSKgel GMH_{HR}-H(20)HT
Measurement solvent: 1,2,4-trichlorobenzene (TCB)
Measurement temperature: 140°C
Calibration curve: Polystyrene standards

### <TGA Measurement Conditions>

Measuring instrument: SII, Inc. EXSTAR6000 TGA/DTA6200
Sample vessel: Aluminum pan
Measurement atmosphere: Nitrogen
Heating rate: 10°C/min.
T_{d3}, T_{d5} and T_{d10} represent the temperatures at 3%, 5% and 10% weight loss, respectively.

### <DSC Measurement Conditions>

Measuring instrument: SII, Inc. EXSTAR6000 DSC6220
Sample vessel: Aluminum pan
Measurement conditions: Nitrogen atmosphere, 10°C/min, 30°C to 300°C; the results were taken from the third Heating Scan.

### <CV Measurement Conditions>

Measuring instrument: BioLogic VSP-300
Working electrode: Glassy carbon (Φ = 3 mm)
Counter electrode: Platinum wire (Φ = 0.5 mm)
Reference electrode: Platinum wire (Φ = 0.5 mm)
Internal standard: Ferrocene/ferrocenium (Fc/Fc⁺)
Electrolyte solution: 0.10 M tetrabutylammonium hexafluorophosphate (Bu₄NPF₆)/acetonitrile solution
Scan speed: 50 mV/s

The redox potential Eₛₐₘₚₗₑ (V vs. Fc/Fc⁺) of the compound for measurement was determined from the half-end potential of the oxidation peak and the reduction peak in a voltammogram, with the horizontal axis corrected for Fc/Fc⁺. The HOMO level E_{HOMO} was calculated according to equation (1), assuming that the redox potential of Fc/Fc⁺ was -5.07 eV with respect to the vacuum level. ${E}_{HOMO} = - 5.07 - {E}_{sample}$

### <Ionization Potential Measurement Conditions>

Measuring instrument: Riken Keiki Co., Ltd. AC-5 atmospheric photoelectron spectrometer
Measurement conditions: Measured in air; light intensity, 3 nW

### [Synthesis Reference Example 1]

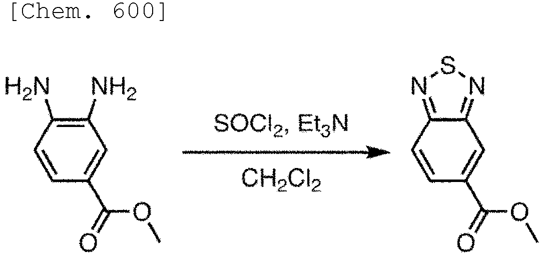

To a mixture of methyl 3,4-diaminobenzoate (6.00 g, 36.1 mmol) and triethylamine (20.0 mL, 143 mmol) and dichloromethane (180 mL), thionyl chloride (5.30 mL, 72.6 mmol) was added, and the resulting mixture was refluxed for 4 hours. After the resulting mixture was allowed to cool to room temperature, a saturated aqueous solution of sodium hydrogencarbonate was added, and extraction with chloroform was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give methyl benzo-2,1,3-thiadiazole-5-carboxylate (4.49 g, 64%) as a white solid.

¹H-NMR (CDCl₃, 400 MHz) δ (ppm): 8.76 (dd, J = 1.6, 0.8 Hz, 1H), 8.23 (dd, J = 9.2, 1.6 Hz, 1H), 8.06 (dd, J = 9.2, 0.8 Hz, 1H), 4.02 (s, 3H).

### [Synthesis Reference Example 2]

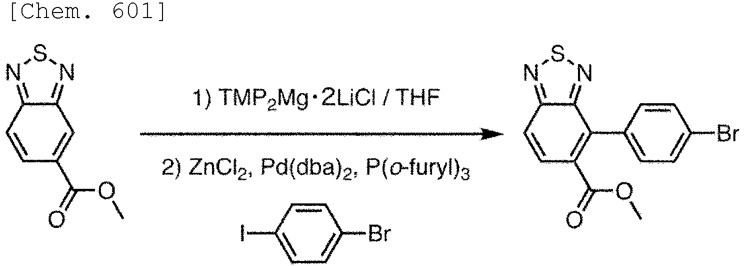

To a mixture of 2,2,6,6-tetramethylpiperidine (880 µL, 5.17 mmol) and tetrahydrofuran (5.1 mL), 3.3 mL of a hexane solution of n-butyllithium (1.6 mol/L, 5.1 mmol) was added at -40°C, and the resulting mixture was stirred for 30 minutes at 0°C. After that, a 2,2,6,6-tetramethylpiperidinyl magnesium chloride lithium chloride complex solution (5.1 mL, 5.1 mmol) was added, and the resulting mixture was stirred for 30 minutes at 0°C and 1 hour at room temperature. By distilling the resulting mixture under reduced pressure to remove the solvent and adding tetrahydrofuran (7.4 mL), a TMP₂Mg·2LiCl solution was prepared.

To a mixture of the methyl benzo-2,1,3-thiadiazole-5-carboxylate obtained in Synthesis Reference Example 1 (1.50 g, 7.72 mmol) and tetrahydrofuran (34 mL), the TMP₂Mg·2LiCl solution was added at -40°C, and the resulting mixture was stirred for 3.5 hours. After that, in an argon gas stream, a zinc chloride solution (6.2 mL, 6.2 mmol) and bis(dibenzylideneacetone)palladium(0) (59.0 mg, 103 µmol), tri(2-furyl)phosphine (48.0 mg, 207 µmol) and 1-bromo-4-iodobenzene (2.19 g, 7.74 mmol) were added, and the resulting mixture was stirred for 15.5 hours at 70°C. After the resulting mixture was allowed to cool to room temperature, a saturated aqueous solution of ammonium chloride was added, and extraction with ethyl acetate was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give methyl 4-(4-bromo-phenyl)-2,1,3-benzothiadiazole-5-carboxylate (1.81 g, 69%) as a light yellow solid.

¹H-NMR (CDCl₃, 400 MHz) δ (ppm): 8.08-8.03 (m, 2H), 7.65-7.63 (m, 2H), 7.36-7.26 (m, 2H), 3.72 (s, 3H).

### [Synthesis Reference Example 3]

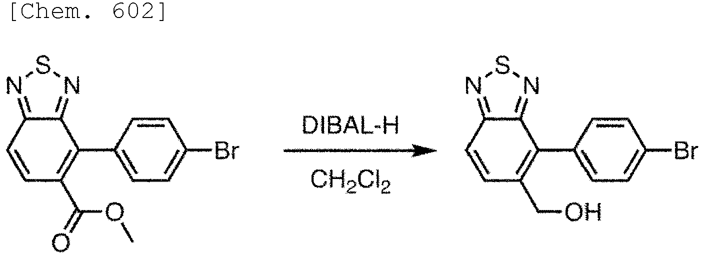

To a mixture of the methyl 4-(4-bromo-phenyl)-2,1,3-benzothiadiazole-5-carboxylate obtained in Synthesis Reference Example 2 (900 mg, 2.58 mmol) and dichloromethane (26 mL), diisobutylaluminum hydride (6.40 mL, 6.40 mmol) was added at -10°C, and the resulting mixture was stirred for 3 hours. Methanol and an aqueous solution of Rochelle salt were added to the resulting mixture, and the resulting mixture was stirred for 30 minutes at room temperature. The resulting mixture was subjected to extraction with chloroform, and the collected organic layer was dried with anhydrous magnesium sulfate and separated by filtration. The solvent was distilled away under reduced pressure to give 4-(4-bromo-phenyl)-5-hydroxymethyl-2,1,3-benzothiadiazole (750 mg, 91%) as a light yellow solid.

¹H-NMR (CDCl₃, 400 MHz) δ (ppm): 8.04 (d, J = 9.2 Hz, 1H), 7.91 (d, J = 9.2 Hz, 1H), 7.68-7.66 (m, 2H), 7.37-7.35 (m, 2H), 4.74 (d, J = 5.6 Hz, 2H), 1.77 (t, J = 5.6 Hz, 1H).

### [Synthesis Reference Example 4]

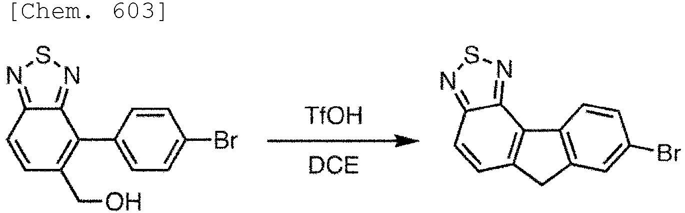

To a mixture of the 4-(4-bromo-phenyl)-5-hydroxymethyl-2,1,3-benzothiadiazole obtained in Synthesis Reference Example 3 (750 mg, 2.34 mmol) and 1,2-dichloroethane (23 mL), trifluoromethanesulfonic acid (3.10 mL, 35.0 mmol) was added, and the resulting mixture was stirred for 3 hours at 90°C. After the resulting mixture was allowed to cool to room temperature, a saturated aqueous solution of sodium hydrogencarbonate was added, and extraction with chloroform was performed. The collected organic layer was washed with water and saturated saline solution, then dried with anhydrous magnesium sulfate and then filtered, and the solvent was distilled away under reduced pressure to give 8-bromo-6H-fluoreno[3,4-c][1,2,5]thiadiazole (591 mg, 84%) as a light yellow solid.

¹H-NMR (CDCl₃, 400 MHz) δ (ppm): 8.40 (d, J = 8.0 Hz, 1H), 7.98 (d, J = 8.4 Hz, 1H), 7.81-7.78 (m, 2H), 7.66 (dd, J = 8.0, 1.6 Hz, 1H), 4.05 (s, 2H).

### [Synthesis Reference Example 5]

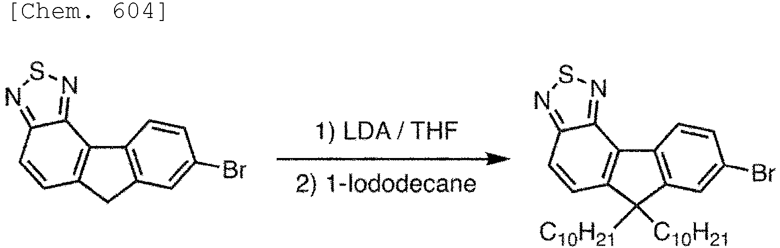

To a mixture of the 8-bromo-6H-fluoreno[3,4-c][1,2,5]thiadiazole obtained in Synthesis Reference Example 4 (700 mg, 2.31 mmol) and tetrahydrofuran (12 mL), lithium diisopropylamide (5.10 mL, 5.10 mmol) was added at -10°C, and the resulting mixture was stirred for 1.5 hours. After that, 1-iododecane (1.50 mL, 7.05 mmol) was added, and the resulting mixture was stirred for 13 hours at room temperature. Water was added to the reaction solution, and extraction with hexane was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give 8-bromo-6,6-didecyl-6H-fluoreno[3,4-c][1,2,5]thiadiazole (971 mg, 72%) as a light yellow solid.

¹H-NMR (CDCl₃, 400 MHz) δ (ppm): 8.31 (d, J = 8.0 Hz, 1H), 7.98 (d, J = 8.8 Hz, 1H), 7.63-7.58 (m, 2H), 7.54 (d, J = 1.6 Hz, 1H), 2.05 (t, J = 8.4 Hz, 4H), 1.25-0.99 (m, 28H), 0.84 (t, J = 7.0 Hz, 6H), 0.58-0.53 (m, 4H).

### [Synthesis Reference Example 6]

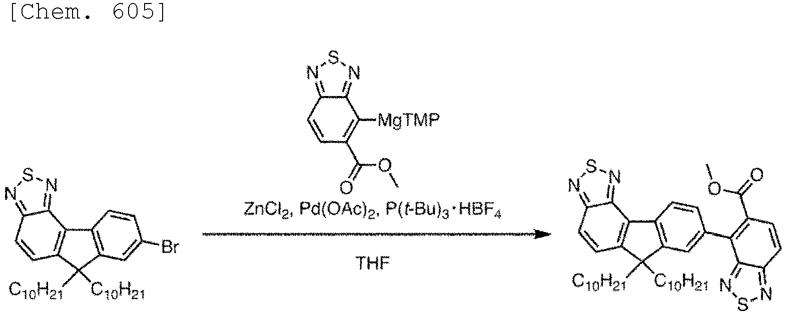

To a mixture of 2,2,6,6-tetramethylpiperidine (340 µL, 2.00 mmol) and tetrahydrofuran (2.0 mL), 1.3 mL of a hexane solution of n-butyllithium (1.6 mol/L, 2.0 mmol) was added at -40°C, and the resulting mixture was stirred for 30 minutes at 0°C. After that, a 2,2,6,6-tetramethylpiperidinyl magnesium chloride lithium chloride complex solution (2.0 mL, 2.0 mmol) was added, and the resulting mixture was stirred for 30 minutes at 0°C and 1 hour at room temperature. By distilling the resulting mixture under reduced pressure to remove the solvent and adding tetrahydrofuran (2.8 mL), a TMP₂Mg·2LiCl solution was prepared.

To a mixture of the methyl benzo-2,1,3-thiadiazole-5-carboxylate obtained in Synthesis Reference Example 1 (387 mg, 1.99 mmol) and tetrahydrofuran (11 mL), the TMP₂Mg·2LiCl solution was added at -40°C, and the resulting mixture was stirred for 2 hours. After that, in an argon gas stream, a zinc chloride solution (2.0 mL, 2.0 mmol) and palladium(II) acetate (7.5 mg, 33.4 µmol), tri-t-butylphosphonium tetrafluoroborate (19.0 mg, 65.5 µmol) and the 8-bromo-6,6-didecyl-6H-fluoreno[3,4-c][1,2,5]thiadiazole obtained in Synthesis Reference Example 5 (970 mg, 1.66 mmol), and the resulting mixture was stirred for 135 hours at 70°C. After the resulting mixture was allowed to cool to room temperature, a saturated aqueous solution of ammonium chloride was added, and extraction with ethyl acetate was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give methyl (6,6-didecyl-6H-fluoreno[3,4-c][1,2,5]thiadiazo-8-yl)-2,1,3-benzothiadiazole-5-carboxylate (897 mg, 77%) as a light yellow solid.

¹H-NMR (CDCl₃, 400 MHz) δ (ppm): 8.60 (d, J = 8.0 Hz, 1H), 8.08-8.00 (m, 3H), 7.68 (d, J = 8.8 Hz, 1H), 7.62 (dd, J = 8.0, 1.6 Hz, 1H), 7.53 (d, J = 1.2 Hz, 1H), 3.63 (s, 3H), 2.16-2.04 (m, 4H), 1.23-1.01 (m, 28H), 0.84-0.60 (m, 10H).

### [Synthesis Reference Example 7]

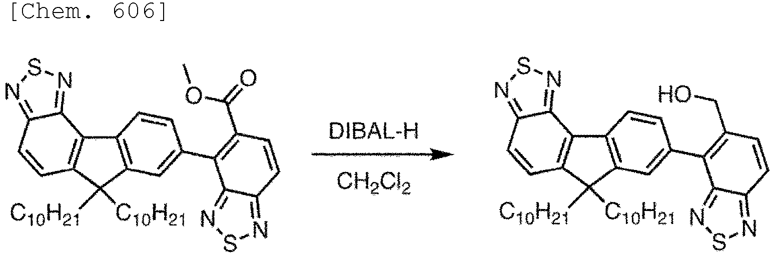

To a mixture of the methyl (6,6-didecyl-6H-fluoreno[3,4-c][1,2,5]thiadiazo-8-yl)-2,1,3-benzothiadiazole-5-carboxylate obtained in Synthesis Reference Example 6 (130 mg, 187 µmol) and dichloromethane (2.0 mL), diisobutylaluminum hydride (500 µL, 500 µmol) was added at -10°C, and the resulting mixture was stirred for 2 hours. Methanol and an aqueous solution of Rochelle salt were added to the resulting mixture, and the resulting mixture was stirred for 30 minutes at room temperature. The resulting mixture was subjected to extraction with chloroform, and the collected organic layer was dried with anhydrous magnesium sulfate and filtered. The solvent was distilled away under reduced pressure. The resulting residue was purified by silica gel column chromatography (hexane/ethyl acetate) to give 8-(5-hydroxymethyl-2,1,3-benzothiadiazo-4-yl)-6,6-didecyl-6H-fluoreno[3,4-c][1,2,5]thiadiazole (103 mg, 83%) as a light yellow solid.

¹H-NMR (CDCl₃, 400 MHz) δ (ppm): 8.62 (dd, J = 8.0, 0.4 Hz, 1H), 8.05 (d, J = 9.2 Hz, 1H), 8.01 (d, J = 8.8 Hz, 1H), 7.95 (d, J = 9.2 Hz, 1H), 7.69 (d, J = 8.8 Hz, 1H), 7.61 (dd, J = 7.6, 1.6 Hz, 1H), 7.54 (d, J = 0.8 Hz, 1H), 4.83 (d, J = 5.6 Hz, 2H), 2.17-2.04 (m, 4H), 1.77 (t, J = 5.6 Hz, 1H), 1.30-1.05 (m, 28H), 0.84-0.65 (m, 10H).

### [Synthesis Reference Example 8]

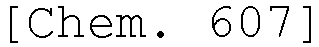

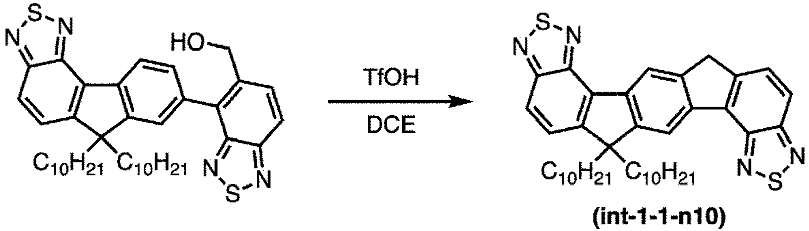

To a mixture of the 8-(5-hydroxymethyl-2,1,3-benzothiadiazo-4-yl)-6,6-didecyl-6H-fluoreno[3,4-c][1,2,5]thiadiazole obtained in Synthesis Reference Example 7 (140 mg, 209 µmol) and 1,2-dichloroethane (2.1 mL), trifluoromethanesulfonic acid (280 µL, 3.16 mmol) was added, and the resulting mixture was stirred for 2 hours at room temperature. After the resulting mixture was allowed to cool to room temperature, a saturated aqueous solution of sodium hydrogencarbonate was added, and extraction with chloroform was performed. The collected organic layer was washed with water and saturated saline solution, then dried with anhydrous magnesium sulfate and filtered, and the solvent was distilled away under reduced pressure. The resulting residue was purified by silica gel column chromatography (hexane/ethyl acetate) to give (int-1-1-n10) (85 mg, 63%) as a light yellow solid.

¹H-NMR (CDCl₃, 400 MHz) δ (ppm): 8.75 (d, J = 0.4 Hz, 1H), 8.59 (d, J = 0.4 Hz, 1H), 8.00-7.96 (m, 2H), 7.86 (d, J = 9.2 Hz, 1H), 7.70 (d, J = 8.8 Hz, 1H), 4.21 (s, 2H), 2.35-2.27 (m, 2H), 2.21-2.14 (m, 2H), 1.18-1.00 (m, 28H), 0.78 (t, J = 7.0 Hz, 6H), 0.69-0.53 (m, 4H).

### [Synthesis Reference Example 9]

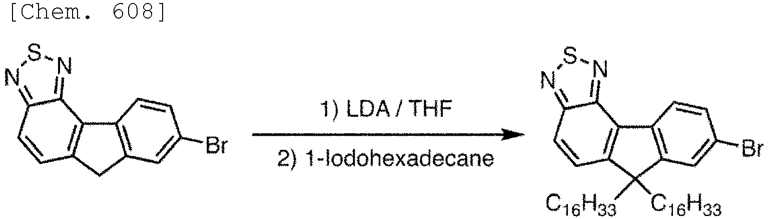

To a mixture of the 8-bromo-6H-fluoreno[3,4-c][1,2,5]thiadiazole obtained in Synthesis Reference Example 4 (1.68 g, 5.54 mmol) and tetrahydrofuran (28 mL), lithium diisopropylamide (11 mL, 11 mmol) was added at -10°C, and the resulting mixture was stirred for 1.5 hours. After that, 1-iodohexadecane (5.2 mL, 16.6 mmol) was added, and the resulting mixture was stirred for 24 hours at room temperature. Water was added to the reaction solution, and extraction with hexane was performed. The collected organic layer was washed with water and saturated saline solution, then dried with anhydrous magnesium sulfate and separated by filtration, and the solvent was distilled away under reduced pressure. The resulting residue was purified by silica gel column chromatography (hexane/chloroform) to give 8-bromo-6,6-dihexadecyl-6H-fluoreno[3,4-c][1,2,5]thiadiazole (2.38 g, 57%) as a light yellow solid.

¹H-NMR (CDCl₃, 400 MHz) δ (ppm): 8.31 (d, J = 8.0 Hz, 1H), 7.98 (d, J = 8.8 Hz, 1H), 7.63-7.54 (m, 3H), 2.05 (t, J = 8.2 Hz, 4H), 1.31-1.02 (m, 52H), 0.87 (t, J = 7.0 Hz, 6H), 0.64-0.47 (m, 4H).

### [Synthesis Reference Example 10]

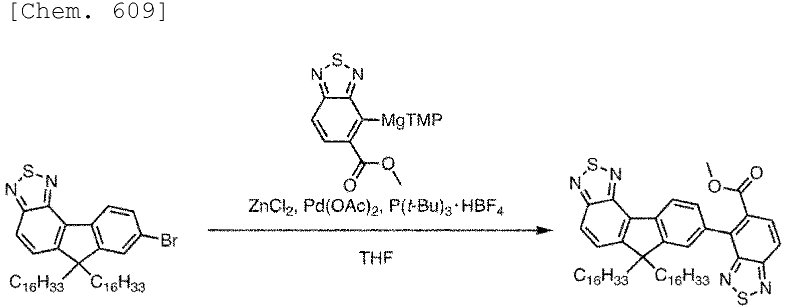

To a mixture of 2,2,6,6-tetramethylpiperidine (0.62 mL, 3.7 mmol) and tetrahydrofuran (3.7 mL), 2.4 mL of a hexane solution of n-butyllithium (1.6 mol/L, 3.7 mmol) was added at -40°C, and the resulting mixture was stirred for 30 minutes at 0°C. After that, a 2,2,6,6-tetramethylpiperidinyl magnesium chloride lithium chloride complex solution (3.7 mL, 3.7 mmol) was added, and the resulting mixture was stirred for 30 minutes at 0°C and 1 hour at room temperature. By distilling the resulting mixture under reduced pressure to remove the solvent and adding tetrahydrofuran (5.2 mL), a TMP₂Mg·2LiCl solution was prepared.

To a mixture of the methyl benzo-2,1,3-thiadiazole-5-carboxylate obtained in Synthesis Reference Example 1 (713 mg, 3.67 mmol) and tetrahydrofuran (11 mL), the TMP₂Mg·2LiCl solution was added at -40°C, and the resulting mixture was stirred for 3 hours. After that, in an argon gas stream, a zinc chloride solution (8.0 mL, 4.0 mmol) and palladium(II) acetate (14 mg, 60 µmol),tri-t-butylphosphonium tetrafluoroborate (36 mg, 120 µmol) and the 8-bromo-6,6-dihexadecyl-6H-fluoreno[3,4-c][1,2,5]thiadiazole obtained in Synthesis Reference Example 9 (2.30 g, 3.06 mmol) were added, and the resulting mixture was stirred for 62 hours at 70°C. After the resulting mixture was allowed to cool to room temperature, a saturated aqueous solution of ammonium chloride was added, and extraction with ethyl acetate was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give methyl (6,6-dihexadecyl-6H-fluoreno[3,4-c][1,2,5]thiadiazo-8-yl)-2,1,3-benzothiadiazole-5-carboxylate (1.57 g, 60%) as a light yellow solid.

¹H-NMR (CDCl₃, 400 MHz) δ (ppm): 8.61 (d, J = 7.2 Hz, 1H), 8.08-7.99 (m, 3H), 7.98 (d, J = 8.8 Hz, 1H), 7.62 (dd, J = 8.0, 1.6 Hz, 1H), 7.53 (d, J = 1.6 Hz, 1H), 3.63 (s, 3H), 2.13-2.08 (m, 4H), 1.30-1.10 (m, 52H), 0.87 (t, J = 7.0 Hz, 6H), 0.82-0.60 (m, 4H).

### [Synthesis Reference Example 11]

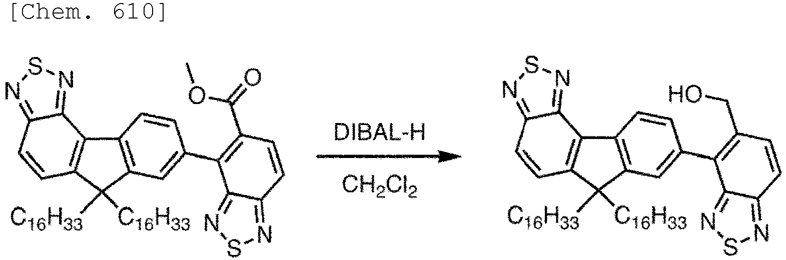

To a mixture of the methyl (6,6-dihexadecyl-6H-fluoreno[3,4-c][1,2,5]thiadiazo-8-yl)-2,1,3-benzothiadiazole-5-carboxylate obtained in Synthesis Reference Example 10 (1.50 g, 1.73 mmol) and dichloromethane (17 mL), diisobutylaluminum hydride (4.3 mL, 4.3 mmol) was added at -10°C, and the resulting mixture was stirred for 3.5 hours. Methanol and an aqueous solution of Rochelle salt were added to the resulting mixture, and the resulting mixture was stirred for 30 minutes at room temperature. The resulting mixture was subjected to extraction with chloroform, and the collected organic layer was dried with anhydrous magnesium sulfate and separated by filtration. The solvent was distilled away under reduced pressure to give 8-(5-hydroxymethyl-2,1,3-benzothiadiazo-4-yl)-6,6-dihexadecyl-6H-fluoreno[3,4-c][1,2,5]thiadiazole (1.30 g, 90%) as a light yellow solid.

¹H-NMR (CDCl₃, 400 MHz) δ (ppm): 8.62 (d, J = 7.6 Hz, 1H), 8.06 (d, J = 8.8 Hz, 1H), 8.01 (d, J = 8.8 Hz, 1H), 7.96 (d, J = 9.2 Hz, 1H), 7.69 (d, J = 9.2 Hz, 1H), 7.61 (dd, J = 7.6, 1.2 Hz, 1H), 7.54 (d, J = 1.2 Hz, 1H), 4.83 (d, J = 5.6 Hz, 2H), 2.17-2.04 (m, 4H), 1.76 (t, J = 5.6 Hz, 1H), 1.30-1.05 (m, 52H), 0.90-0.85 (m, 6H), 0.78-0.63 (m, 4H) .

### [Synthesis Reference Example 12]

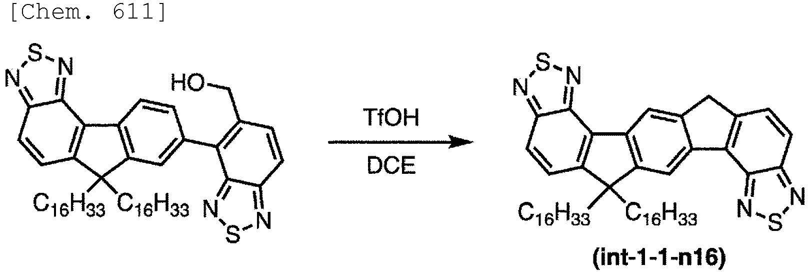

To a mixture of the 8-(5-hydroxymethyl-2,1,3-benzothiadiazo-4-yl)-6,6-dihexadecyl-6H-fluoreno[3,4-c][1,2,5]thiadiazole obtained in Synthesis Reference Example 11 (1.30 g, 1.55 mmol) and 1,2-dichloroethane (16 mL), trifluoromethanesulfonic acid (2.0 mL, 23 mmol) was added, and the resulting mixture was stirred for 3 hours at room temperature. After the resulting mixture was allowed to cool to room temperature, a saturated aqueous solution of sodium hydrogencarbonate was added, and extraction with chloroform was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/ethyl acetate) to give (int-1-1-n16) (951 mg, 75%) as a light yellow solid.

¹H-NMR (CDCl₃, 400 MHz) δ (ppm): 8.75 (s, 1H), 8.59 (s, 1H), 8.00-7.96 (m, 2H), 7.87 (d, J = 8.8 Hz, 1H), 7.71 (d, J = 8.8 Hz, 1H), 4.21 (s, 2H), 2.35-2.28 (m, 2H), 2.21-2.14 (m, 2H), 1.32-1.05 (m, 52H), 1.00-0.84 (m, 6H), 0.72-0.56 (m, 4H).

### [Synthesis Reference Example 13]

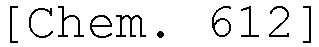

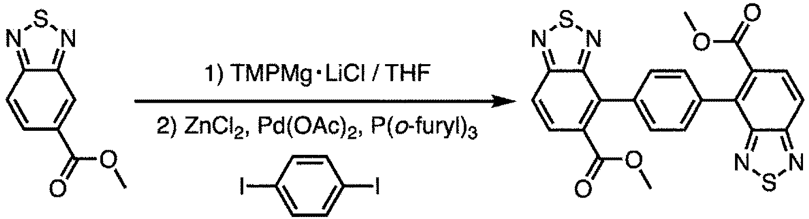

To a mixture of the methyl benzo-2,1,3-thiadiazole-5-carboxylate obtained in Synthesis Reference Example 1 (3.00 g, 15.4 mmol) and tetrahydrofuran (100 mL), a 2,2,6,6-tetramethylpiperidinyl magnesium chloride lithium chloride complex solution (19 mL, 17 mmol) was added at -15°C, and the resulting mixture was stirred for 2 hours. After that, in an argon gas stream, a zinc chloride solution (21 mL, 19 mmol) and palladium acetate (69.4 mg, 309 µmol),tri(2-furyl)phosphine (143 mg, 616 µmol) and p-diiodobenzene (2.29 g, 6.94 mmol) were added, and the resulting mixture was stirred for 5 hours at 80°C. After the resulting mixture was allowed to cool to room temperature, a saturated aqueous solution of ammonium chloride was added, and extraction with ethyl acetate was performed. The collected organic layer was washed with water and saturated saline solution, then dried with anhydrous magnesium sulfate and then filtered, and the solvent was distilled away under reduced pressure. The resulting solid was washed with methanol to give 4,4'-(1,4-phenylene)-bis(2,1,3-benzothiadiazole-5-carboxylic acid) dimethyl ester (2.45 g, 76%) as a brown solid.

¹H-NMR (CDCl₃, 400 MHz) δ (ppm): 8.10-8.04 (m, 4H), 7.64 (s, 4H), 3.75 (s, 6H).

### [Synthesis Reference Example 14]

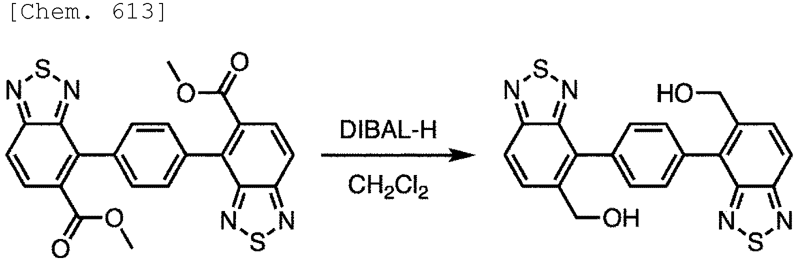

To a mixture of the 4,4'-(1,4-phenylene)-bis(2,1,3-benzothiadiazole-5-carboxylic acid) dimethyl ester obtained in Synthesis Reference Example 13 (2.44 g, 5.28 mmol) and dichloromethane (53 mL), diisobutylaluminum hydride (26 mL, 26 mmol) was added at -15°C, and the resulting mixture was stirred for 3 hours. Methanol and an aqueous solution of Rochelle salt were added to the resulting mixture, and the resulting mixture was stirred for 30 minutes at room temperature. The solid that separated out was filtered and washed with water to give 4,4'-(1,4-phenylene)-bis(5-hydroxymethyl-2,1,3-benzothiadiazole) (1.84 g, 86%) as a brown solid.

¹H-NMR (DMSO-d₆, 400 MHz) δ (ppm): 8.13 (d, J = 9.2 Hz, 2H), 8.02 (d, J = 9.2 Hz, 2H), 7.65 (s, 4H), 5.53 (t, J = 5.6 Hz, 2H), 4.62 (d, J = 5.6 Hz, 4H).

### [Synthesis Reference Example 15]

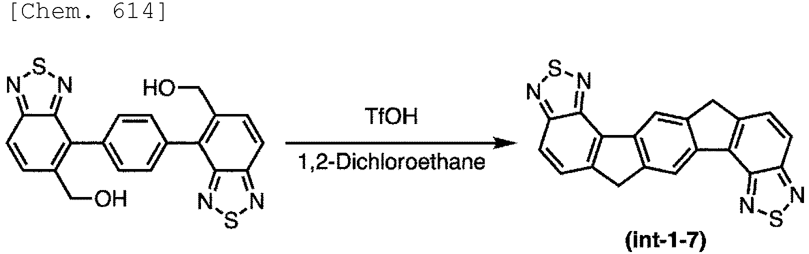

To a mixture of the 4,4'-(1,4-phenylene)-bis(5-hydroxymethyl-2,1,3-benzothiadiazole) obtained in Synthesis Reference Example 14 (1.89 g, 4.65 mmol) and dichloromethane (93 mL), trifluoromethanesulfonic acid (8.2 mL, 93 mmol) was added, and the resulting mixture was stirred for 17 hours. To the resulting mixture, a saturated aqueous solution of sodium hydrogencarbonate and methanol were added, and the resulting mixture was stirred at room temperature. The solid that separated out was filtered and washed with water and methanol to give (int-1-7) (1.42 g) as an ocherous solid. The resulting compound was used in the next reaction without purification.

¹H-NMR (CDCl₃, 400 MHz) δ (ppm): 8.75 (s, 2H), 7.97 (d, J = 9.2 Hz, 2H), 7.86 (d, J = 9.2 Hz, 2H), 4.19 (s, 4H).

### [Example 1]

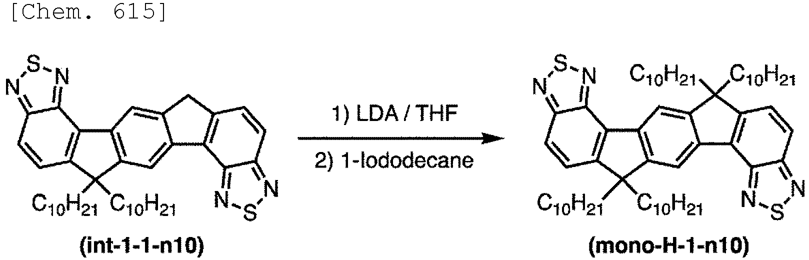

To a mixture of the (int-1-1-n10) obtained in Synthesis Reference Example 8 (194 mg, 298 µmol) and tetrahydrofuran (6.0 mL), lithium diisopropylamide (600 µL, 654 µmol) was added at -10°C, and the resulting mixture was stirred for 1.5 hours. After that, 1-iododecane (200 µL, 940 µmol) was added, and the resulting mixture was stirred for 17 hours at room temperature. Water was added to the reaction solution, and extraction with ethyl acetate was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give (mono-H-1-n10) (196 mg, 71%) as a light yellow solid.

¹H-NMR (CDCl₃, 400 MHz) δ (ppm): 8.49 (s, 2H), 7.97 (d, J = 8.8 Hz, 2H), 7.69 (d, J = 8.8 Hz, 2H), 2.34-2.26 (m, 4H), 2.21-2.14 (m, 4H), 1.16-0.99 (m, 56H), 0.77 (t, J = 7.0 Hz, 12H), 0.70-0.58 (m, 8H).

### [Example 2]

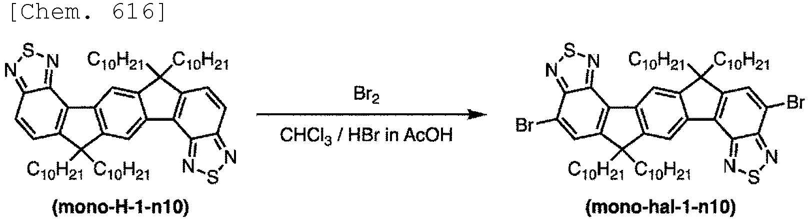

To a mixture of the (int-H-1-n10) obtained in Example 1 (140 mg, 150 µmol) and chloroform (2.0 mL) and hydrogen bromide (30% solution in acetic acid) (1.0 mL), bromine (50.0 µL, 970 µmol) was added, and the resulting mixture was refluxed for 1.5 hours. After the resulting mixture was allowed to cool to room temperature, a saturated aqueous solution of sodium hydrogencarbonate and a saturated aqueous solution of sodium thiosulfate were added, and extraction with chloroform was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give (mono-hal-1-n10) (145 mg, 89%) as a yellow solid.

¹H-NMR (CDCl₃, 400 MHz) δ (ppm): 8.43 (s, 2H), 7.92 (s, 2H), 2.32-2.26 (m, 4H), 2.18-2.11 (m, 4H), 1.15-1.00 (m, 56H), 0.78 (t, J = 7.0 Hz, 12H), 0.66-0.58 (m, 8H).

### [Example 3]

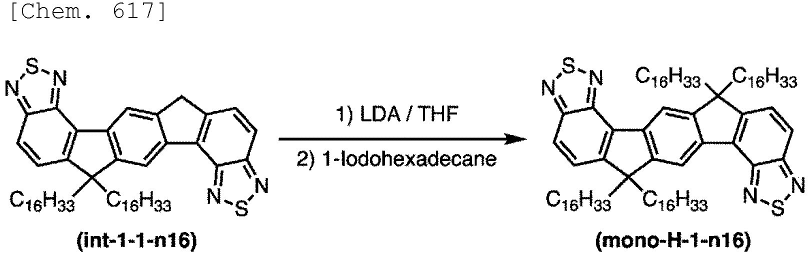

To a mixture of the (int-1-1-n16) obtained in Synthesis Reference Example 12 (900 mg, 1.10 mmol) and tetrahydrofuran (22 mL), lithium diisopropylamide (2.2 mL, 2.4 mmol) was added at -10°C, and the resulting mixture was stirred for 2.5 hours. After that, 1-iodohexadecane (0.76 mL, 2.4 mmol) was added, and the resulting mixture was stirred for 17 hours at room temperature. Water was added to the reaction solution, and extraction with ethyl acetate was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give (mono-H-1-n16) (835 mg, 60%) as a light yellow solid.

¹H-NMR (CDCl₃, 400 MHz) δ (ppm): 8.49 (s, 2H), 7.98 (d, J = 8.8 Hz, 2H), 7.70 (d, J = 8.8 Hz, 2H), 2.33-2.26 (m, 4H), 2.21-2.14 (m, 4H), 1.29-0.98 (m, 104H), 0.88 (t, J = 7.0 Hz, 12H), 0.71-0.58 (m, 8H).

### [Example 4]

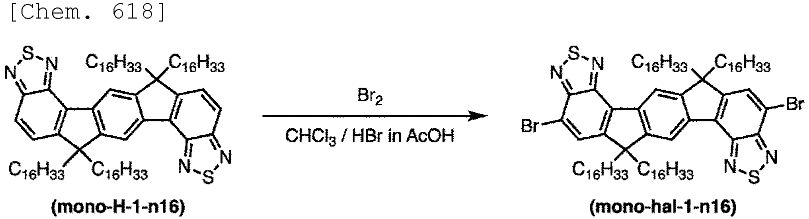

To a mixture of the (mono-H-1-n16) obtained in Example 3 (800 mg, 0.631 mmol) and chloroform (8.4 mL) and hydrogen bromide (30% solution in acetic acid) (4.2 mL), bromine (0.20 mL, 3.8 mmol) was added, and the resulting mixture was refluxed for 3 hours. After the resulting mixture was allowed to cool to room temperature, a saturated aqueous solution of sodium hydrogencarbonate and a saturated aqueous solution of sodium thiosulfate were added, and extraction with chloroform was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give (mono-hal-1-n16) (832 mg, 92%) as a yellow solid.

¹H-NMR (CDCl₃, 400 MHz) δ (ppm): 8.43 (s, 2H), 7.92 (s, 2H), 2.32-2.24 (m, 4H), 2.18-2.10 (m, 4H), 1.29-1.00 (m, 104H), 0.86 (t, J = 7.0 Hz, 12H), 0.66-0.58 (m, 8H).

### [Example 5]

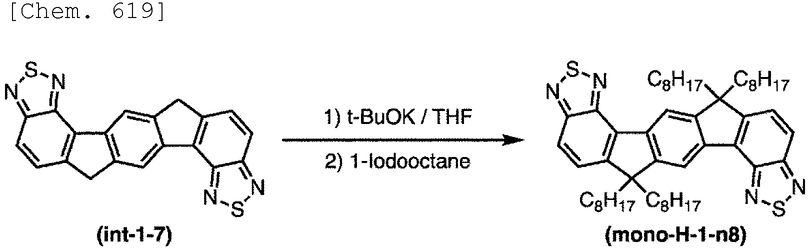

To a mixture of the (int-1-7) obtained in Synthesis Reference Example 15 (500 mg, 1.35 mmol) and tetrahydrofuran (27 mL), potassium tert-butoxide (666 mg, 5.94 mmol) was added at 0°C, and the resulting mixture was stirred for 2.5 hours. After that, 1-iodooctane (1.5 mL, 8.1 mmol) was added, and the resulting mixture was stirred for 3 hours at room temperature. A saturated aqueous solution of ammonium chloride was added to the reaction solution, and extraction with ethyl acetate was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give (mono-H-1-n8) (467 mg, 42%) as a yellow solid.

¹H-NMR (CDCl₃, 400 MHz) δ (ppm): 8.49 (s, 2H), 7.97 (d, J = 8.8 Hz, 2H), 7.69 (d, J = 8.8 Hz, 2H), 2.34-2.26 (m, 4H), 2.22-2.14 (m, 4H), 1.09-1.00 (m, 40H), 0.70 (t, J = 6.8 Hz, 12H), 0.66-0.57 (m, 8H).

### [Example 6]

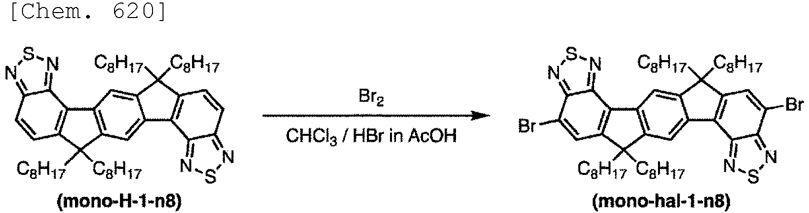

To a mixture of the (mono-H-1-n8) obtained in Example 5 (466 mg, 0.569 mmol) and chloroform (11 mL) and hydrogen bromide (30% solution in acetic acid) (5.7 mL), bromine (0.18 mL, 3.4 mmol) was added, and the resulting mixture was refluxed for 3.5 hours. After the resulting mixture was allowed to cool to room temperature, a saturated aqueous solution of sodium hydrogencarbonate and a saturated aqueous solution of sodium thiosulfate were added, and extraction with chloroform was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give (mono-hal-1-n8) (486 mg, 87%) as a yellow solid.

¹H-NMR (CDCl₃, 400 MHz) δ (ppm): 8.43 (s, 2H), 7.92 (s, 2H), 2.32-2.24 (m, 4H), 2.18-2.11 (m, 4H), 1.29-1.00 (m, 40H), 0.71 (t, J = 7.0 Hz, 12H), 0.65-0.58 (m, 8H).

### [Example 7]

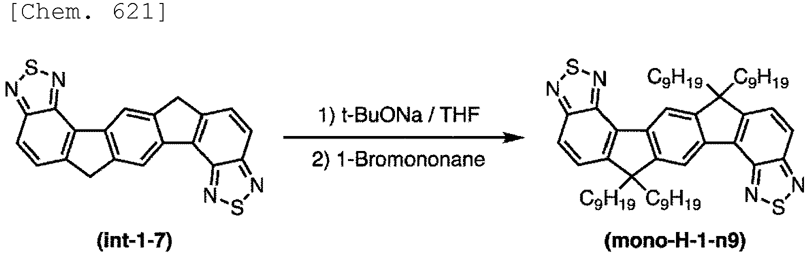

To a mixture of the (int-1-7) obtained in Synthesis Reference Example 15 (703 mg, 1.90 mmol) and tetrahydrofuran (38 mL), sodium tert-butoxide (766 mg, 7.97 mmol) was added at 0°C, and the resulting mixture was stirred for 3 hours. After that, 1-bromononane (2.2 mL, 11 mmol) was added, and the resulting mixture was stirred for 40 hours at room temperature. A saturated aqueous solution of ammonium chloride was added to the reaction solution, and extraction with ethyl acetate was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give (mono-H-1-n9) (446 mg, 27%) as a yellow solid.

¹H-NMR (CDCl₃, 400 MHz) δ (ppm): 8.49 (s, 2H), 7.97 (d, J = 8.8 Hz, 2H), 7.69 (d, J = 8.8 Hz, 2H), 2.34-2.26 (m, 4H), 2.22-2.14 (m, 4H), 1.12-0.99 (m, 48H), 0.73 (t, J = 7.0 Hz, 12H), 0.68-0.58 (m, 8H).

### [Example 8]

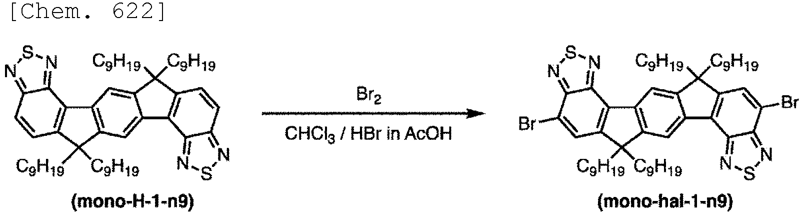

To a mixture of the (mono-H-1-n9) obtained in Example 7 (402 mg, 0.459 mmol) and chloroform (9.2 mL) and hydrogen bromide (30% solution in acetic acid) (4.6 mL), bromine (0.14 mL, 2.8 mmol) was added, and the resulting mixture was refluxed for 2.5 hours. After the resulting mixture was allowed to cool to room temperature, a saturated aqueous solution of sodium hydrogencarbonate and a saturated aqueous solution of sodium thiosulfate were added, and extraction with chloroform was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give (mono-hal-1-n9) (454 mg, 96%) as a yellow solid.

¹H-NMR (CDCl₃, 400 MHz) δ (ppm): 8.43 (s, 2H), 7.92 (s, 2H), 2.32-2.24 (m, 4H), 2.18-2.12 (m, 4H), 1.13-1.00 (m, 48H), 0.73 (t, J = 7.0 Hz, 12H), 0.66-0.60 (m, 8H).

### [Example 9]

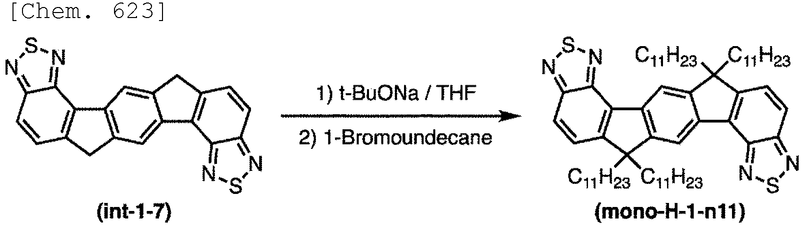

To a mixture of the (int-1-7) obtained in Synthesis Reference Example 15 (703 mg, 1.90 mmol) and tetrahydrofuran (38 mL), sodium tert-butoxide (766 mg, 7.97 mmol) was added at 0°C, and the resulting mixture was stirred for 3 hours. After that, 1-bromoundecane (2.5 mL, 11 mmol) was added, and the resulting mixture was stirred for 39 hours at room temperature. A saturated aqueous solution of ammonium chloride was added to the reaction solution, and extraction with ethyl acetate was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give (mono-H-1-n11) (479 mg, 26%) as a yellow solid.

¹H-NMR (CDCl₃, 400 MHz) δ (ppm): 8.49 (s, 2H), 7.98 (d, J = 8.8 Hz, 2H), 7.69 (d, J = 8.8 Hz, 2H), 2.33-2.26 (m, 4H), 2.22-2.14 (m, 4H), 1.25-0.99 (m, 64H), 0.80 (t, J = 7.2 Hz, 12H), 0.69-0.57 (m, 8H).

### [Example 10]

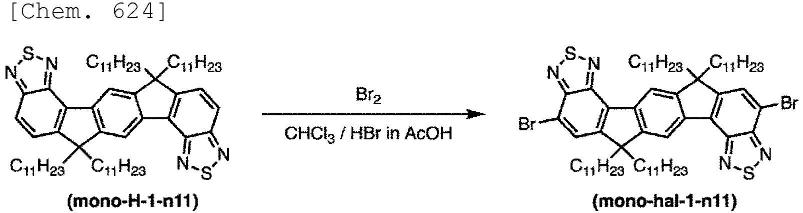

To a mixture of the (mono-H-1-n11) obtained in Example 9 (405 mg, 0.410 mmol) and chloroform (8.2 mL) and hydrogen bromide (30% solution in acetic acid) (4.1 mL), bromine (0.13 mL, 2.5 mmol) was added, and the resulting mixture was refluxed for 4 hours. After the resulting mixture was allowed to cool to room temperature, a saturated aqueous solution of sodium hydrogencarbonate and a saturated aqueous solution of sodium thiosulfate were added, and extraction with chloroform was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give (mono-hal-1-n11) (432 mg, 92%) as a yellow solid.

¹H-NMR (CDCl₃, 400 MHz) δ (ppm): 8.43 (s, 2H), 7.92 (s, 2H), 2.32-2.24 (m, 4H), 2.18-2.11 (m, 4H), 1.21-1.00 (m, 64H), 0.81 (t, J = 7.2 Hz, 12H), 0.66-0.58 (m, 8H).

### [Example 11]

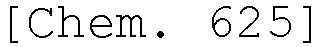

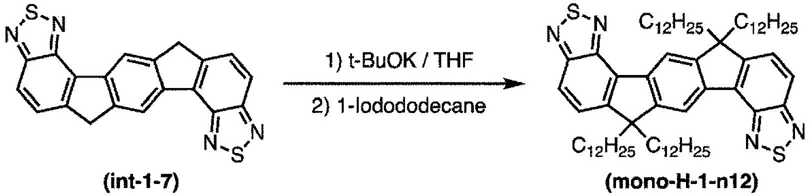

To a mixture of the (int-1-7) obtained in Synthesis Reference Example 15 (500 mg, 1.35 mmol) and tetrahydrofuran (27 mL), potassium tert-butoxide (666 mg, 5.94 mmol) was added at 0°C, and the resulting mixture was stirred for 2.5 hours. After that, 1-iodododecane (2.0 mL, 8.1 mmol) was added, and the resulting mixture was stirred for 3 hours at room temperature. A saturated aqueous solution of ammonium chloride was added to the reaction solution, and extraction with ethyl acetate was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give (mono-H-1-n12) (605 mg, 43%) as a yellow solid.

¹H-NMR (CDCl₃, 400 MHz) δ (ppm): 8.49 (s, 2H), 7.98 (d, J = 8.8 Hz, 2H), 7.69 (d, J = 8.8 Hz, 2H), 2.33-2.26 (m, 4H), 2.22-2.14 (m, 4H), 1.24-0.99 (m, 72H), 0.83 (t, J = 7.2 Hz, 12H), 0.69-0.56 (m, 8H).

### [Example 12]

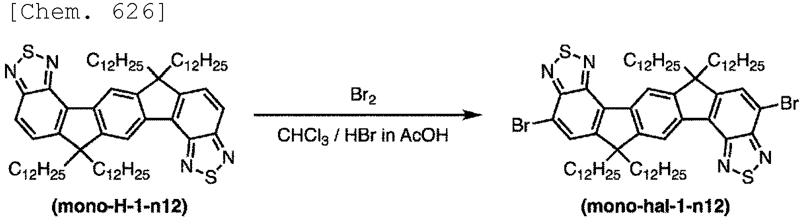

To a mixture of the (mono-H-1-n12) obtained in Example 11 (600 mg, 0.575 mmol) and chloroform (11 mL) and hydrogen bromide (30% solution in acetic acid) (5.7 mL), bromine (0.18 mL, 3.4 mmol) was added, and the resulting mixture was refluxed for 3 hours. After the resulting mixture was allowed to cool to room temperature, a saturated aqueous solution of sodium hydrogencarbonate and a saturated aqueous solution of sodium thiosulfate were added, and extraction with chloroform was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give (mono-hal-1-n12) (638 mg, 92%) as a yellow solid.

¹H-NMR (CDCl₃, 400 MHz) δ (ppm): 8.43 (s, 2H), 7.92 (s, 2H), 2.32-2.24 (m, 4H), 2.18-2.11 (m, 4H), 1.25-1.00 (m, 72H), 0.83 (t, J = 7.2 Hz, 12H), 0.66-0.58 (m, 8H).

### [Example 13]

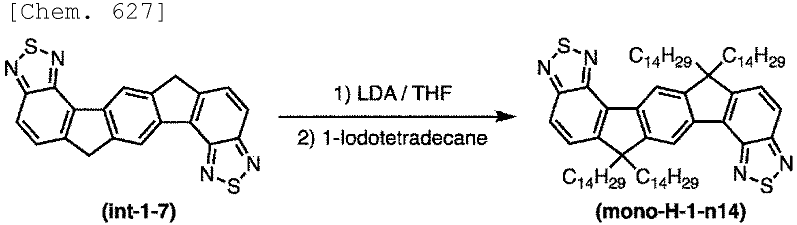

To a mixture of the (int-1-7) obtained in Synthesis Reference Example 15 (300 mg, 0.810 mmol) and tetrahydrofuran (16 mL), lithium diisopropylamide (3.2 mL, 3.6 mmol) was added at -15°C, and the resulting mixture was stirred for 4 hours. After that, 1-iodotetradecane (1.57 g, 4.86 mmol) was added, and the resulting mixture was stirred for 22 hours at room temperature. Water was added to the reaction solution, and extraction with ethyl acetate was performed. The collected organic layer was washed with water and saturated saline solution, then dried with anhydrous magnesium sulfate and separated by filtration, and the solvent was distilled away under reduced pressure. The resulting residue was purified by silica gel column chromatography (hexane/chloroform) to give (mono-H-1-n14) (398 mg, 43%) as a light yellow solid.

¹H-NMR (CDCl₃, 400 MHz) δ (ppm): 8.49 (s, 2H), 7.98 (d, J = 8.8 Hz, 2H), 7.69 (d, J = 8.8 Hz, 2H), 2.33-2.26 (m, 4H), 2.22-2.14 (m, 4H), 1.26-0.99 (m, 88H), 0.85 (t, J = 7.0 Hz, 12H), 0.70-0.56 (m, 8H).

### [Example 14]

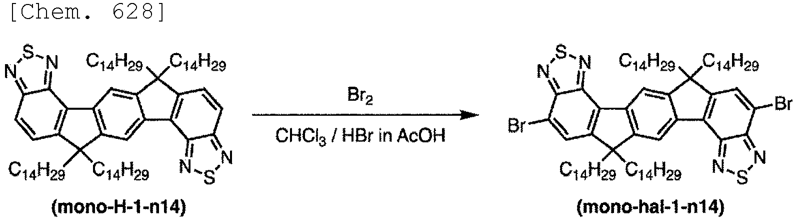

To a mixture of the (mono-H-1-n14) obtained in Example 13 (350 mg, 0.303 mmol) and chloroform (6.1 mL) and hydrogen bromide (30% solution in acetic acid) (3.0 mL), bromine (0.094 mL, 1.8 mmol) was added, and the resulting mixture was refluxed for 4 hours. After the resulting mixture was allowed to cool to room temperature, a saturated aqueous solution of sodium hydrogencarbonate and a saturated aqueous solution of sodium thiosulfate were added, and extraction with chloroform was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give (mono-hal-1-n14) (363 mg, 91%) as a yellow solid.

¹H-NMR (CDCl₃, 400 MHz) δ (ppm): 8.43 (s, 2H), 7.92 (s, 2H), 2.32-2.24 (m, 4H), 2.18-2.11 (m, 4H), 1.28-1.00 (m, 88H), 0.85 (t, J = 7.0 Hz, 12H), 0.66-0.59 (m, 8H).

### [Example 15]

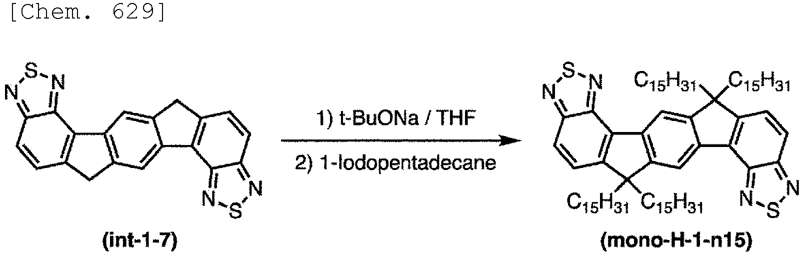

To a mixture of the (int-1-7) obtained in Synthesis Reference Example 15 (703 mg, 1.90 mmol) and tetrahydrofuran (38 mL), sodium tert-butoxide (766 mg, 7.97 mmol) was added at -10°C, and the resulting mixture was stirred for 3.5 hours. After that, 1-iodopentadecane (3.85 g, 11.4 mmol) was added, and the resulting mixture was stirred for 18 hours at room temperature. A saturated aqueous solution of ammonium chloride was added to the reaction solution, and extraction with ethyl acetate was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give (mono-H-1-n15) (479 mg, 21%) as a yellow solid.

¹H-NMR (CDCl₃, 400 MHz) δ (ppm): 8.49 (s, 2H), 7.98 (d, J = 8.8 Hz, 2H), 7.69 (d, J = 8.8 Hz, 2H), 2.33-2.26 (m, 4H), 2.21-2.14 (m, 4H), 1.29-0.99 (m, 96H), 0.86 (t, J = 7.0 Hz, 12H), 0.68-0.56 (m, 8H).

### [Example 16]

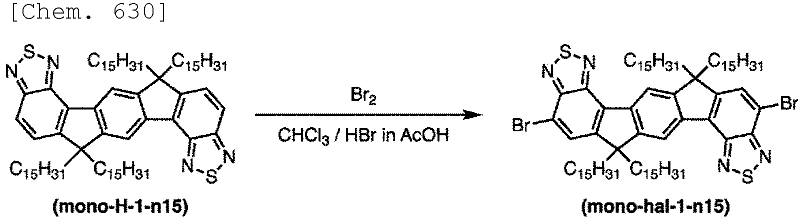

To a mixture of the (mono-H-1-n15) obtained in Example 15 (470 mg, 0.388 mmol) and chloroform (7.8 mL) and hydrogen bromide (30% solution in acetic acid) (3.9 mL), bromine (0.12 mL, 2.3 mmol) was added, and the resulting mixture was refluxed for 2.5 hours. After the resulting mixture was allowed to cool to room temperature, a saturated aqueous solution of sodium hydrogencarbonate and a saturated aqueous solution of sodium thiosulfate were added, and extraction with chloroform was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give (mono-hal-1-n15) (502 mg, 94%) as a yellow solid.

¹H-NMR (CDCl₃, 400 MHz) δ (ppm): 8.43 (s, 2H), 7.92 (s, 2H), 2.32-2.24 (m, 4H), 2.18-2.11 (m, 4H), 1.29-1.00 (m, 96H), 0.86 (t, J = 7.2 Hz, 12H), 0.66-0.58 (m, 8H).

### [Example 17]

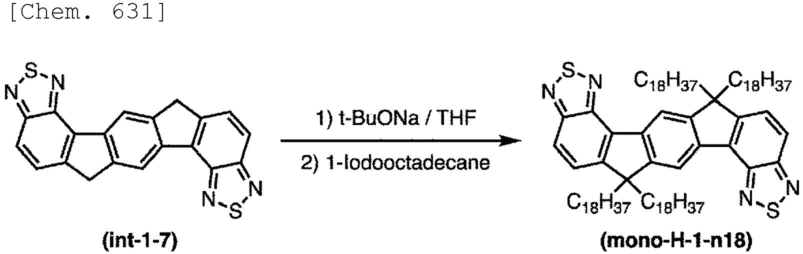

To a mixture of the (int-1-7) obtained in Synthesis Reference Example 15 (500 mg, 1.35 mmol) and tetrahydrofuran (27 mL), sodium tert-butoxide (571 mg, 5.94 mmol) was added at 0°C, and the resulting mixture was stirred for 3.5 hours. After that, 1-iodooctadecane (3.08 g, 8.10 mmol) was added, and the resulting mixture was stirred for 3.5 hours at room temperature. A saturated aqueous solution of ammonium chloride was added to the reaction solution, and extraction with ethyl acetate was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give (mono-H-1-n18) (749 mg, 40%) as a yellow solid.

¹H-NMR (CDCl₃, 400 MHz) δ (ppm): 8.49 (s, 2H), 7.98 (d, J = 8.8 Hz, 2H), 7.69 (d, J = 8.8 Hz, 2H), 2.33-2.26 (m, 4H), 2.21-2.14 (m, 4H), 1.30-0.98 (m, 120H), 0.87 (t, J = 6.8 Hz, 12H), 0.67-0.57 (m, 8H).

### [Example 18]

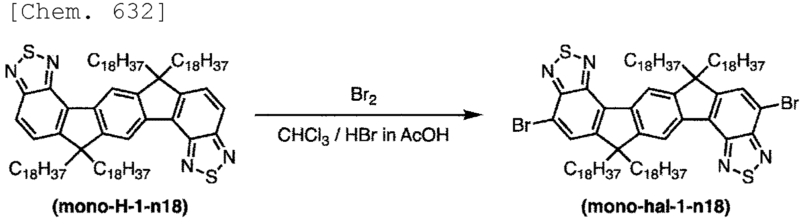

To a mixture of the (mono-H-1-n18) obtained in Example 17 (700 mg, 0.507 mmol) and chloroform (10 mL) and hydrogen bromide (30% solution in acetic acid) (5.1 mL), bromine (0.16 mL, 3.0 mmol) was added, and the resulting mixture was refluxed for 2.5 hours. After the resulting mixture was allowed to cool to room temperature, a saturated aqueous solution of sodium hydrogencarbonate and a saturated aqueous solution of sodium thiosulfate were added, and extraction with chloroform was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give (mono-hal-1-n18) (615 mg, 85%) as a yellow solid.

¹H-NMR (CDCl₃, 400 MHz) δ (ppm): 8.43 (s, 2H), 7.92 (s, 2H), 2.32-2.24 (m, 4H), 2.18-2.11 (m, 4H), 1.30-0.99 (m, 120H), 0.87 (t, J = 6.8 Hz, 12H), 0.66-0.58 (m, 8H).

### [Example 19]

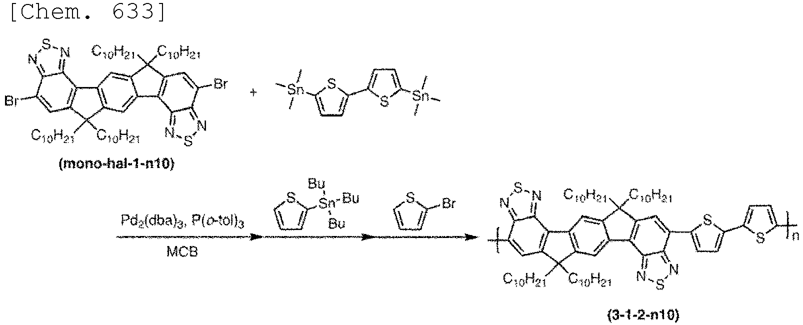

A mixture of the (mono-hal-1-n10) obtained in Example 2 (100 mg, 91.8 µmol) and 5,5'-bis(trimethylstannyl)-2,2'-bithiophene (45.2 mg, 91.8 µmol) and chlorobenzene (2.2 mL) was bubbled with argon for 30 minutes. To this solution mixture, Pd₂(dba)₃·CHCl₃ (1.9 mg, 1.8 µmol) and tri(o-tolyl)phosphine (2.2 mg, 7.3 µmol) were added, and the resulting mixture was stirred for 63 hours at 120°C. After that, 2-(tributylstannyl)thiophene (290 µL, 918 µmol) was added to the reaction solution, and the resulting mixture was stirred for 8 hours at 120°C. 2-bromothiophene (88 µL, 918 µmol),furthermore, was added, and the resulting mixture was stirred for 13 hours at 120°C. After the resulting mixture was allowed to cool to room temperature, the mixture was precipitated in a methanol/concentrated hydrochloric acid solution mixture (150 mL/15 mL), and the solid that thus separated out was filtered. The resulting solid was subjected to Soxhlet extraction using methanol, acetone, hexane, cyclohexane and n-decane, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-2-n10) (30 mg, 30%) as a black solid.

GPC (THF): Mn = 27300 g/mol, Mw = 35800 g/mol, PDI = 1.31.

GPC (TCB, 140°C): Mn = 26000 g/mol, Mw = 35000 g/mol, PDI = 1.7.
T_{d3} = 394°C, T_{d5} = 409°C, Tₐ₁₀ = 423°C.
No phase transition temperature was observed in DSC. HOMO level: -5.77 eV.

Given that no phase transfer temperature was observed, it was demonstrated that the heat resistance of (3-1-2-n10) is high.

### [Example 20]

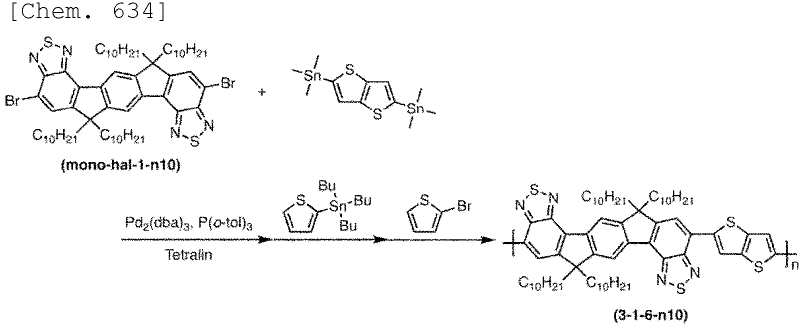

A mixture of the (mono-hal-1-n10) obtained in Example 2 (120 mg, 110 µmol) and 2,5-bis(trimethylstannyl)thieno[3,2-b]thiophene (51.3 mg, 110 µmol) and chlorobenzene (2.2 mL) was bubbled with argon for 30 minutes. To this solution mixture, Pd₂(dba)₃·CHCl₃ (2.3 mg, 2.2 µmol) and tri(o-tolyl)phosphine (2.7 mg, 8.9 µmol) were added, and the resulting mixture was stirred for 2 hours at 180°C using a microwave reactor. After that, 2-(tributylstannyl)thiophene (310 µL, 980 µmol) was added to the reaction solution, and the resulting mixture was stirred for 10 minutes at 180°C using a microwave reactor. 2-bromothiophene (110 µL, 1.15 mmol), furthermore, was added, and the resulting mixture was stirred for 10 minutes at 180°C using a microwave reactor. After the resulting mixture was allowed to cool to room temperature, the mixture was precipitated in a methanol/concentrated hydrochloric acid solution mixture (150 mL/15 mL), and the solid that thus separated out was filtered. The resulting solid was subjected to Soxhlet extraction using methanol, acetone, hexane and chloroform, through which components soluble in these solvents were removed. The filter residue, furthermore, was dissolved in chlorobenzene. The resulting mixture was concentrated under reduced pressure and precipitated in methanol, and the solid that thus separated out was filtered. The resulting solid was washed with methanol and then dried under reduced pressure at 90°C to give (3-1-6-n10) (54 mg, 54%) as a black solid.

T_{d3} = 377°C, T_{d5} = 398°C, T_{d10} = 421°C.

No phase transition temperature was observed in DSC.

### [Example 21]

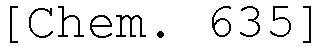

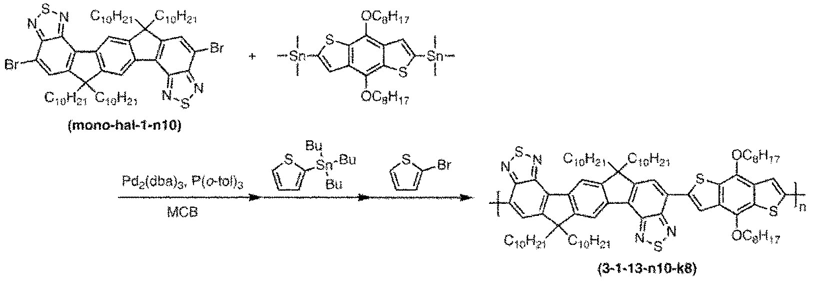

A mixture of the (mono-hal-1-n10) obtained in Example 2 (110 mg, 101 µmol) and 4,8-bis(n-octyloxy)-2,6-bis(trimethylstannyl)benzo[1,2-b:4,5-b']dithiophene (78.0 mg, 101 µmol) and chlorobenzene (2.0 mL) was bubbled with argon for 30 minutes. To this solution mixture, Pd₂ (dba)₃·CHCl₃ (2.1 mg, 2.0 µmol) and tri(o-tolyl)phosphine (2.5 mg, 8.2 µmol) were added, and the resulting mixture was stirred for 2 hours at 180°C using a microwave reactor. After that, 2-(tributylstannyl)thiophene (290 µL, 917 µmol) was added to the reaction solution, and the resulting mixture was stirred for 10 minutes at 180°C using a microwave reactor. 2-bromothiophene (100 µL, 1.04 mmol), furthermore, was added, and the resulting mixture was stirred for 10 minutes at 180°C using a microwave reactor. After the resulting mixture was allowed to cool to room temperature, the mixture was precipitated in a methanol/concentrated hydrochloric acid solution mixture (150 mL/15 mL), and the solid that thus separated out was filtered. The resulting solid was subjected to Soxhlet extraction using methanol, acetone and hexane, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-13-n10-k8) (108 mg, 77%) as a black solid.

GPC (THF): Mn = 26300 g/mol, Mw = 33500 g/mol, PDI = 1.33.

GPC (TCB, 140°C): Mn = 16000 g/mol, Mw = 28000 g/mol, PDI = 1.7.

T_{d3} = 328°C, T_{d5} = 337°C, T_{d10} = 349°C.

No phase transition temperature was observed in DSC.

### [Example 22]

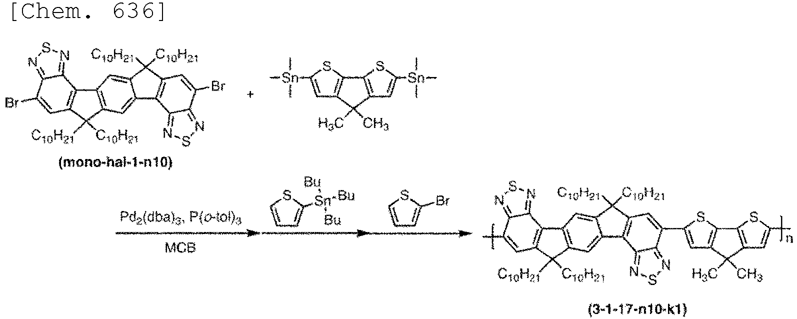

A mixture of the (mono-hal-1-n10) obtained in Example 2 (100 mg, 91.8 µmol) and 2,6-bis(trimethylstannyl)-4,4-dimethyl-4H-cyclopenta[2,1-b:3,4-b']dithiophene (48.8 mg, 91.8 µmol) and chlorobenzene (2.2 mL) was bubbled with argon for 30 minutes. To this solution mixture, Pd₂(dba)₃·CHCl₃ (1.9 mg, 1.8 µmol) and tri(o-tolyl)phosphine (2.2 mg, 7.3 µmol) were added, and the resulting mixture was stirred for 2 hours at 180°C using a microwave reactor. After that, 2-(tributylstannyl)thiophene (290 µL, 918 µmol) was added to the reaction solution, and the resulting mixture was stirred for 10 minutes at 120°C using a microwave reactor. 2-bromothiophene (88 µL, 918 µmol), furthermore, was added, and the resulting mixture was stirred for 10 minutes at 120°C using a microwave reactor. After the resulting mixture was allowed to cool to room temperature, the mixture was precipitated in a methanol/concentrated hydrochloric acid solution mixture (150 mL/15 mL), and the solid that thus separated out was filtered. The resulting solid was subjected to Soxhlet extraction using methanol, acetone and hexane, through which components soluble in these solvents were removed. The filter residue, furthermore, was dissolved in cyclohexane. The resulting mixture was concentrated under reduced pressure and precipitated in methanol, and the solid that thus separated out was filtered. The resulting solid was washed with methanol and then dried under reduced pressure at 90°C to give (3-1-17-n10-k1) (68 mg, 66%) as a black solid.

GPC (THF): Mn = 28400 g/mol, Mw = 40400 g/mol, PDI = 1.42.

GPC (TCB, 140°C): Mn = 23000 g/mol, Mw = 72000 g/mol, PDI = 3.0.

T_{d3} = 403°C, T_{d5} = 414°C, Tₐ₁₀ = 428°C.

No phase transition temperature was observed in DSC.

HOMO level: -5.78 eV.

### [Example 23]

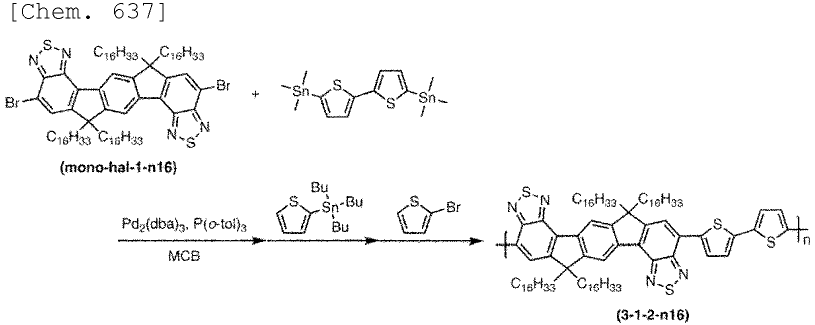

A mixture of the (mono-hal-1-n16) obtained in Example 4 (100 mg, 70.1 µmol) and 5,5'-bis(trimethylstannyl)-2,2'-bithiophene (34.5 mg, 70.1 µmol) and chlorobenzene (2.1 mL) was bubbled with argon for 30 minutes. To this solution mixture, Pd₂(dba)₃·CHCl₃ (1.9 mg, 1.8 µmol) and tri(o-tolyl)phosphine (2.2 mg, 7.3 µmol) were added, and the resulting mixture was stirred for 68 hours at 120°C. After that, 2-(tributylstannyl)thiophene (222 µL, 701 µmol) was added to the reaction solution, and the resulting mixture was stirred for 5 hours at 120°C. 2-bromothiophene (67 µL, 701 µmol),furthermore, was added, and the resulting mixture was stirred for 6 hours at 120°C. After the resulting mixture was allowed to cool to room temperature, the mixture was precipitated in a methanol/concentrated hydrochloric acid solution mixture (150 mL/15 mL), and the solid that thus separated out was filtered. The resulting solid was subjected to Soxhlet extraction using methanol, acetone, hexane and cyclohexane, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-2-n16) (70 mg, 70%) as a black solid.

GPC (THF): Mn = 37800 g/mol, Mw = 54300 g/mol, PDI = 1.44.

GPC (TCB, 140°C): Mn = 37000 g/mol, Mw = 56000 g/mol, PDI = 1.5.

T_{d3} = 369°C, T_{d5} = 386°C, T_{d10} = 412°C.

No phase transition temperature was observed in DSC.

HOMO level: -5.96 eV.

### [Example 24]

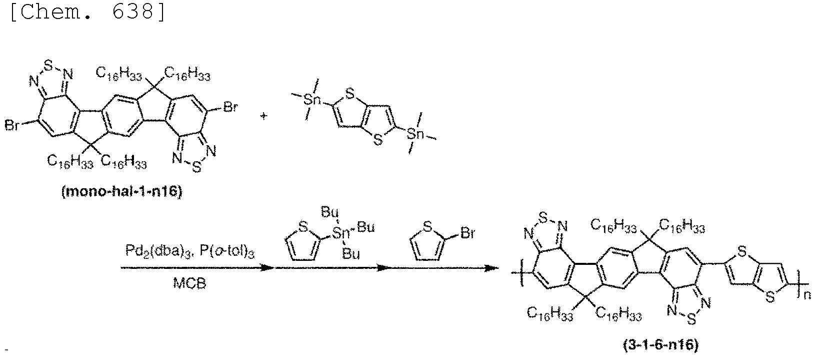

A mixture of the (mono-hal-1-n16) obtained in Example 4 (110 mg, 77.0 µmol) and 2,5-bis(trimethylstannyl)thieno[3,2-b]thiophene (35.9 mg, 77.0 µmol) and chlorobenzene (2.0 mL) was bubbled with argon for 30 minutes. To this solution mixture, Pd₂(dba)₃·CHCl₃ (1.6 mg, 1.5 µmol) and tri(o-tolyl)phosphine (1.9 mg, 6.2 µmol) were added, and the resulting mixture was stirred for 2 hours at 180°C using a microwave reactor. After that, 2-(tributylstannyl)thiophene (220 µL, 690 µmol) was added to the reaction solution, and the resulting mixture was stirred for 10 minutes at 180°C using a microwave reactor. 2-bromothiophene (70 µL, 770 µmol),furthermore, was added, and the resulting mixture was stirred for 10 minutes at 180°C using a microwave reactor. After the resulting mixture was allowed to cool to room temperature, the mixture was precipitated in a methanol/concentrated hydrochloric acid solution mixture (150 mL/15 mL), and the solid that thus separated out was filtered. The resulting solid was subjected to Soxhlet extraction using methanol, acetone, hexane and cyclohexane, through which components soluble in these solvents were removed. The filter residue, furthermore, was dissolved in THF. The resulting mixture was concentrated under reduced pressure and precipitated in methanol, and the solid that thus separated out was filtered. The resulting solid was washed with methanol and then dried under reduced pressure at 90°C to give (3-1-6-n16) (38 mg, 35%) as a black solid.

GPC (THF): Mn = 31600 g/mol, Mw = 51100 g/mol, PDI = 1.62.

GPC (TCB, 140°C): Mn = 35000 g/mol, Mw = 56000 g/mol, PDI = 1.6.

T_{d3} = 332°C, T_{d5} = 344°C, Tₐ₁₀ = 359°C.

No phase transition temperature was observed in DSC.

HOMO level: -6.03 eV.

### [Example 25]

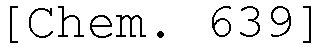

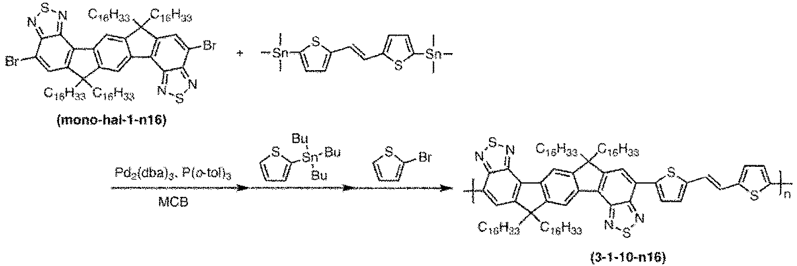

A mixture of the (mono-hal-1-n16) obtained in Example 4 (110 mg, 77.0 µmol) and trans-1,2-bis[5-(trimethylstannyl)thiophen-2-yl]ethene (40.0 mg, 77.0 µmol) and chlorobenzene (2.0 mL) was bubbled with argon for 30 minutes. To this solution mixture, Pd₂(dba)₃·CHCl₃ (1.6 mg, 1.5 µmol) and tri(o-tolyl)phosphine (1.9 mg, 6.2 µmol) were added, and the resulting mixture was stirred for 2 hours at 180°C using a microwave reactor. After that, 2-(tributylstannyl)thiophene (220 µL, 690 µmol) was added to the reaction solution, and the resulting mixture was stirred for 10 minutes at 180°C using a microwave reactor. 2-bromothiophene (70 µL, 770 µmol), furthermore, was added, and the resulting mixture was stirred for 10 minutes at 180°C using a microwave reactor. After the resulting mixture was allowed to cool to room temperature, the mixture was precipitated in a methanol/concentrated hydrochloric acid solution mixture (150 mL/15 mL), and the solid that thus separated out was filtered. The resulting solid was subjected to Soxhlet extraction using methanol, acetone, hexane and chloroform, through which components soluble in these solvents were removed. The filter residue, furthermore, was dissolved in chlorobenzene. The resulting mixture was concentrated under reduced pressure and precipitated in methanol, and the solid that thus separated out was filtered. The resulting solid was washed with methanol and then dried under reduced pressure at 90°C to give (3-1-10-n16) (89 mg, 67%) as a black solid.

GPC (TCB, 140°C): Mn = 61000 g/mol, Mw = 98000 g/mol, PDI = 1.6.

T_{d3} = 367°C, T_{d5} = 375°C, T_{d10} = 387°C.

No phase transition temperature was observed in DSC.

HOMO level: -5.97 eV.

### [Example 26]

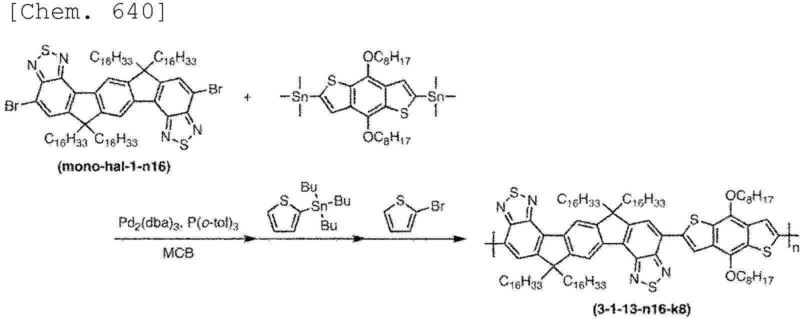

A mixture of the (mono-hal-1-n16) obtained in Example 4 (110 mg, 77.0 µmol) and 4,8-bis(n-octyloxy)-2,6-bis(trimethylstannyl)benzo[1,2-b:4,5-b']dithiophene (59.6 mg, 77.0 µmol) and chlorobenzene (2.0 mL) was bubbled with argon for 30 minutes. To this solution mixture, Pd₂(dba)₃·CHCl₃ (1.6 mg, 1.5 µmol) and tri(o-tolyl)phosphine (1.9 mg, 6.2 µmol) were added, and the resulting mixture was stirred for 2 hours at 180°C using a microwave reactor. After that, 2-(tributylstannyl)thiophene (220 µL, 690 µmol) was added to the reaction solution, and the resulting mixture was stirred for 10 minutes at 180°C using a microwave reactor. 2-bromothiophene (70 µL, 770 µmol), furthermore, was added, and the resulting mixture was stirred for 10 minutes at 180°C using a microwave reactor. After the resulting mixture was allowed to cool to room temperature, the mixture was precipitated in a methanol/concentrated hydrochloric acid solution mixture (150 mL/15 mL), and the solid that thus separated out was filtered. The resulting solid was subjected to Soxhlet extraction using methanol, acetone and hexane, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-13-n16-k8) (89 mg, 67%) as a black solid.

GPC (THF): Mn = 32500 g/mol, Mw = 45400 g/mol, PDI = 1.40.

GPC (TCB, 140°C): Mn = 35000 g/mol, Mw = 54000 g/mol, PDI = 1.5.

### [Example 27]

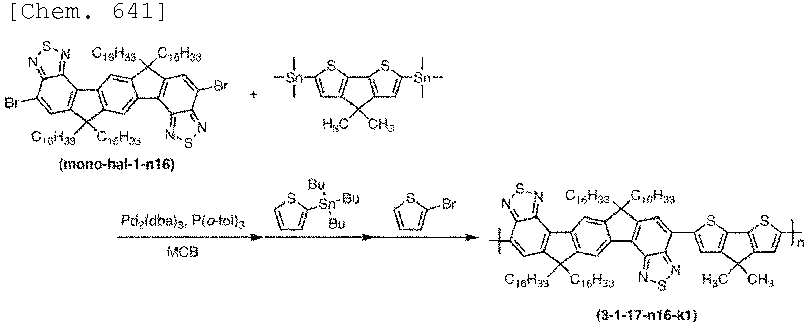

A mixture of the (mono-hal-1-n16) obtained in Example 4 (100 mg, 70.1 µmol) and 2,6-bis(trimethylstannyl)-4,4-dimethyl-4H-cyclopenta[2,1-b:3,4-b']dithiophene (37.3 mg, 70.1 µmol) and chlorobenzene (2.1 mL) was bubbled with argon for 30 minutes. To this solution mixture, Pd₂(dba)₃·CHCl₃ (1.9 mg, 1.8 µmol) and tri(o-tolyl)phosphine (2.2 mg, 7.3 µmol) were added, and the resulting mixture was stirred for 2 hours at 180°C using a microwave reactor. After that, 2-(tributylstannyl)thiophene (222 µL, 701 µmol) was added to the reaction solution, and the resulting mixture was stirred for 10 minutes at 180°C using a microwave reactor. 2-bromothiophene (67 µL, 701 µmol), furthermore, was added, and the resulting mixture was stirred for 10 minutes at 180°C using a microwave reactor. After the resulting mixture was allowed to cool to room temperature, the mixture was precipitated in a methanol/concentrated hydrochloric acid solution mixture (150 mL/15 mL), and the solid that thus separated out was filtered. The resulting solid was subjected to Soxhlet extraction using methanol, acetone and hexane, through which components soluble in these solvents were removed. The filter residue, furthermore, was dissolved in cyclohexane. The resulting mixture was concentrated under reduced pressure and precipitated in methanol, and the solid that thus separated out was filtered. The resulting solid was washed with methanol and then dried under reduced pressure at 90°C to give (3-1-17-n16-k1) (62 mg, 61%) as a black solid.

GPC (THF): Mn = 33800 g/mol, Mw = 43800 g/mol, PDI = 1.29.

GPC (TCB, 140°C): Mn = 32000 g/mol, Mw = 42000 g/mol, PDI = 1.3.

T_{d3} = 402°C, T_{d5} = 410°C, T_{d10} = 422°C.

No phase transition temperature was observed in DSC.

HOMO level: -5.72 eV.

### [Example 28]

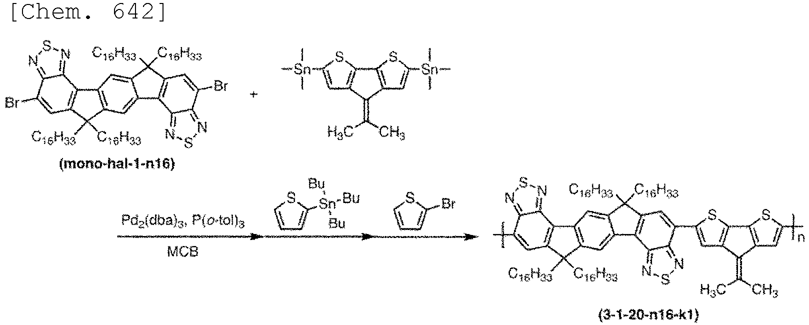

A mixture of the (mono-hal-1-n16) obtained in Example 4 (100 mg, 70.1 µmol) and 2,6-bis(trimethylstannyl)-4-(1-methylethylidene)-4H-cyclopenta[2,1-b:3,4-b']dithiophene (38.1 mg, 70.1 µmol) and chlorobenzene (2.1 mL) was bubbled with argon for 30 minutes. To this solution mixture, Pd₂(dba)₃·CHCl₃ (1.9 mg, 1.8 µmol) and tri(o-tolyl)phosphine (2.2 mg, 7.3 µmol) were added, and the resulting mixture was stirred for 2 hours at 180°C using a microwave reactor. After that, 2-(tributylstannyl)thiophene (222 µL, 701 µmol) was added to the reaction solution, and the resulting mixture was stirred for 10 minutes at 180°C using a microwave reactor. 2-bromothiophene (67 µL, 701 µmol), furthermore, was added, and the resulting mixture was stirred for 10 minutes at 180°C using a microwave reactor. After the resulting mixture was allowed to cool to room temperature, the mixture was precipitated in a methanol/concentrated hydrochloric acid solution mixture (150 mL/15 mL), and the solid that thus separated out was filtered. The resulting solid was subjected to Soxhlet extraction using methanol, acetone, hexane, cyclohexane and chloroform, through which components soluble in these solvents were removed. The filter residue, furthermore, was dissolved in chlorobenzene. The resulting mixture was concentrated under reduced pressure and precipitated in methanol, and the solid that thus separated out was filtered. The resulting solid was washed with methanol and then dried under reduced pressure at 90°C to give (3-1-20-n16-k1) (18 mg, 17%) as a black solid.

T_{d3} = 364°C, T_{d5} = 377°C, T_{d10} = 396°C.

No phase transition temperature was observed in DSC.

### [Example 29]

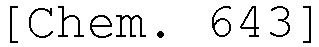

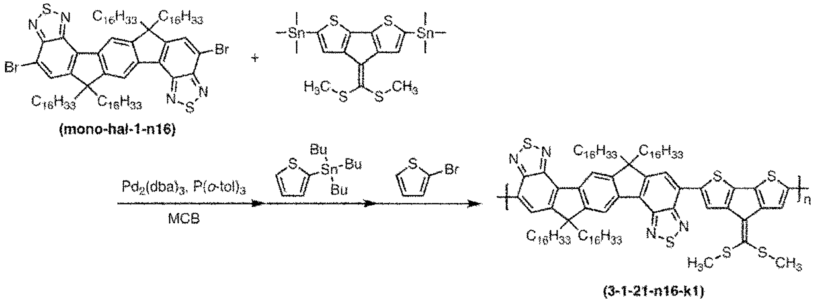

A mixture of the (mono-hal-1-n16) obtained in Example 4 (75 mg, 52.5 µmol) and 2,6-bis(trimethylstannyl)-4-[bis(methylthio)methylene]-4H-cyclopenta[2,1-b:3,4-b']dithiophene (31.9 mg, 52.5 µmol) and chlorobenzene (1.6 mL) was bubbled with argon for 30 minutes. To this solution mixture, Pd₂(dba)₃·CHCl₃ (1.1 mg, 1.0 µmol) and tri(o-tolyl)phosphine (1.3 mg, 4.2 µmol) were added, and the resulting mixture was stirred for 85 hours at 120°C. After that, 2-(tributylstannyl)thiophene (166 µL, 525 µmol) was added to the reaction solution, and the resulting mixture was stirred for 5 hours at 120°C. 2-bromothiophene (50 µL, 525 µmol), furthermore, was added, and the resulting mixture was stirred for 4 hours at 120°C. After the resulting mixture was allowed to cool to room temperature, the mixture was precipitated in a methanol/concentrated hydrochloric acid solution mixture (150 mL/15 mL), and the solid that thus separated out was filtered. The resulting solid was subjected to Soxhlet extraction using methanol, acetone, hexane, cyclohexane and chloroform, through which components soluble in these solvents were removed. The filter residue, furthermore, was dissolved in chlorobenzene. The resulting mixture was concentrated under reduced pressure and precipitated in methanol, and the solid that thus separated out was filtered. The resulting solid was washed with methanol and then dried under reduced pressure at 90°C to give (3-1-21-n16-k1) (47 mg, 57%) as a black solid.

T_{d3} = 354°C, T_{d5} = 367°C, T_{d10} = 386°C.

No phase transition temperature was observed in DSC.

### [Example 30]

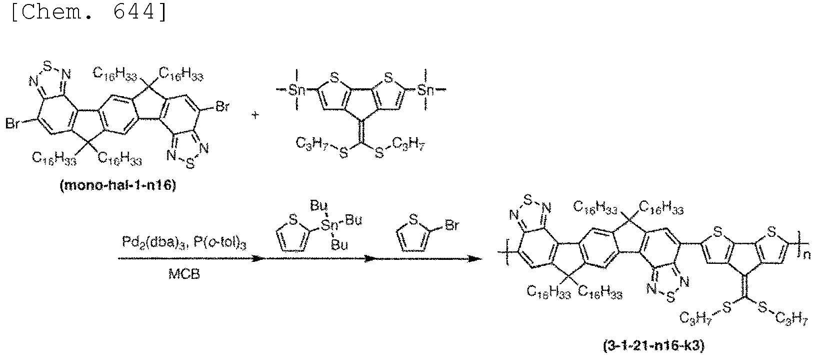

A mixture of the (mono-hal-1-n16) obtained in Example 4 (100 mg, 70.1 µmol) and 2,6-bis(trimethylstannyl)-4-[bis(propylthio)methylene]-4H-cyclopenta[2,1-b:3,4-b']dithiophene (46.6 mg, 70.1 µmol) and chlorobenzene (2.1 mL) was bubbled with argon for 30 minutes. To this solution mixture, Pd₂(dba)₃·CHCl₃ (1.9 mg, 1.8 µmol) and tri(o-tolyl)phosphine (2.2 mg, 7.3 µmol) were added, and the resulting mixture was stirred for 84 hours at 120°C. After that, 2-(tributylstannyl)thiophene (222 µL, 701 µmol) was added to the reaction solution, and the resulting mixture was stirred for 11 hours at 120°C. 2-bromothiophene (67 µL, 701 µmol), furthermore, was added, and the resulting mixture was stirred for 12 hours at 120°C. After the resulting mixture was allowed to cool to room temperature, the mixture was precipitated in a methanol/concentrated hydrochloric acid solution mixture (150 mL/15 mL), and the solid that thus separated out was filtered. The resulting solid was subjected to Soxhlet extraction using methanol, acetone, hexane and n-decane, through which components soluble in these solvents were removed. The filter residue, furthermore, was dissolved in cyclohexane. The resulting mixture was concentrated under reduced pressure and precipitated in methanol, and the solid that thus separated out was filtered. The resulting solid was washed with methanol and then dried under reduced pressure at 90°C to give (3-1-21-n16-k3) (28 mg, 25%) as a black solid.

GPC (THF): Mn = 25600 g/mol, Mw = 35000 g/mol, PDI = 1.37.

GPC (TCB, 140°C): Mn = 35000 g/mol, Mw = 58000 g/mol, PDI = 1.7.

T_{d3} = 343°C, T_{d5} = 356°C, T_{d10} = 389°C.

No phase transition temperature was observed in DSC.

HOMO level: -5.61 eV.

### [Example 31]

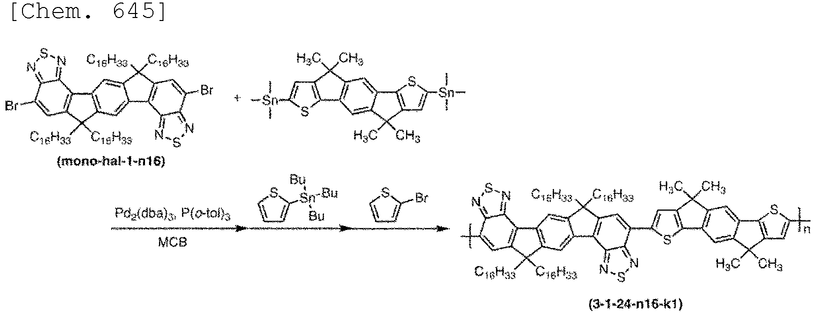

A mixture of the (mono-hal-1-n16) obtained in Example 4 (100 mg, 70.1 µmol) and 2,7-bis(trimethylstannyl)-4,4,9,9-tetramethyl-4,9-dihydro-s-indaceno[1,2-b:5,6-b']dithiophene (45.4 mg, 70.1 µmol) and chlorobenzene (2.1 mL) was bubbled with argon for 30 minutes. To this solution mixture, Pd₂(dba)₃·CHCl₃ (1.9 mg, 1.8 µmol) and tri(o-tolyl)phosphine (2.2 mg, 7.3 µmol) were added, and the resulting mixture was stirred for 2 hours at 180°C using a microwave reactor. After that, 2-(tributylstannyl)thiophene (222 µL, 701 µmol) was added to the reaction solution, and the resulting mixture was stirred for 10 minutes at 180°C using a microwave reactor. 2-bromothiophene (67 µL, 701 µmol), furthermore, was added, and the resulting mixture was stirred for 10 minutes at 180°C using a microwave reactor. After the resulting mixture was allowed to cool to room temperature, the mixture was precipitated in a methanol/concentrated hydrochloric acid solution mixture (150 mL/15 mL), and the solid that thus separated out was filtered. The resulting solid was subjected to Soxhlet extraction using methanol, acetone, hexane and cyclohexane, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-24-n16-k1) (84 mg, 75%) as a black solid.

GPC (THF): Mn = 53500 g/mol, Mw = 120000 g/mol, PDI = 2.24.

GPC (TCB, 120°C): Mn = 58000 g/mol, Mw = 100000 g/mol, PDI = 1.8.

T_{d3} = 394°C, T_{d5} = 406°C, T_{d10} = 423°C.

No phase transition temperature was observed in DSC.

HOMO level: -5.67 eV.

### [Example 32]

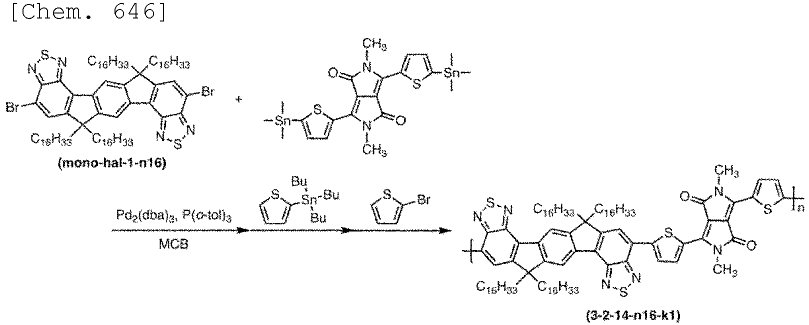

A mixture of the (mono-hal-1-n16) obtained in Example 4 (100 mg, 70.1 µmol) and 3,6-bis(5-(trimethylstannyl)-2-thienyl)-2,5-dimethyl-2,5-dihydro-pyrrolo[3,4-c]pyrrol-1,4-dione (45.9 mg, 70.1 µmol) and chlorobenzene (2.1 mL) was bubbled with argon for 30 minutes. To this solution mixture, Pd₂(dba)₃·CHCl₃ (1.9 mg, 1.8 µmol) and tri(o-tolyl)phosphine (2.2 mg, 7.3 µmol) were added, and the resulting mixture was stirred for 60 hours at 120°C. After that, 2-(tributylstannyl)thiophene (222 µL, 701 µmol) was added to the reaction solution, and the resulting mixture was stirred for 4 hours at 120°C. 2-bromothiophene (67 µL, 701 µmol), furthermore, was added, and the resulting mixture was stirred for 4 hours at 120°C. After the resulting mixture was allowed to cool to room temperature, the mixture was precipitated in a methanol/concentrated hydrochloric acid solution mixture (150 mL/15 mL), and the solid that thus separated out was filtered. The resulting solid was subjected to Soxhlet extraction using methanol, acetone, hexane and chloroform, through which components soluble in these solvents were removed. The filter residue, furthermore, was dissolved in chlorobenzene. The resulting mixture was concentrated under reduced pressure and precipitated in methanol, and the solid that thus separated out was filtered. The resulting solid was washed with methanol and then dried under reduced pressure at 90°C to give (3-2-14-n16-k1) (64 mg, 58%) as a black solid.

T_{d3} = 362°C, T_{d5} = 372°C, T_{d10} = 384°C.

No phase transition temperature was observed in DSC.

### [Example 33]

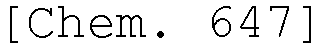

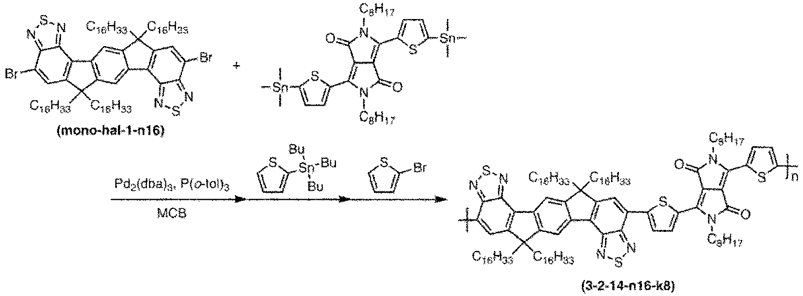

A mixture of the (mono-hal-1-n16) obtained in Example 4 (100 mg, 70.1 µmol) and 3,6-bis(5-(trimethylstannyl)-2-thienyl)-2,5-dioctyl-2,5-dihydro-pyrrolo[3,4-c]pyrrol-1,4-dione (45.9 mg, 70.1 µmol) and chlorobenzene (2.1 mL) was bubbled with argon for 30 minutes. To this solution mixture, Pd₂(dba)₃·CHCl₃ (1.9 mg, 1.8 µmol) and tri(o-tolyl)phosphine (2.2 mg, 7.3 µmol) were added, and the resulting mixture was stirred for 61 hours at 120°C. After that, 2-(tributylstannyl)thiophene (222 µL, 701 µmol) was added to the reaction solution, and the resulting mixture was stirred for 4 hours at 120°C. 2-bromothiophene (67 µL, 701 µmol), furthermore, was added, and the resulting mixture was stirred for 4 hours at 120°C. After the resulting mixture was allowed to cool to room temperature, the mixture was precipitated in a methanol/concentrated hydrochloric acid solution mixture (150 mL/15 mL), and the solid that thus separated out was filtered. The resulting solid was subjected to Soxhlet extraction using methanol, acetone, hexane and cyclohexane, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-2-14-n16-k8) (87 mg, 69%) as a black solid.

GPC (THF): Mn = 53800 g/mol, Mw = 407400 g/mol, PDI = 7.57.

### [Example 34]

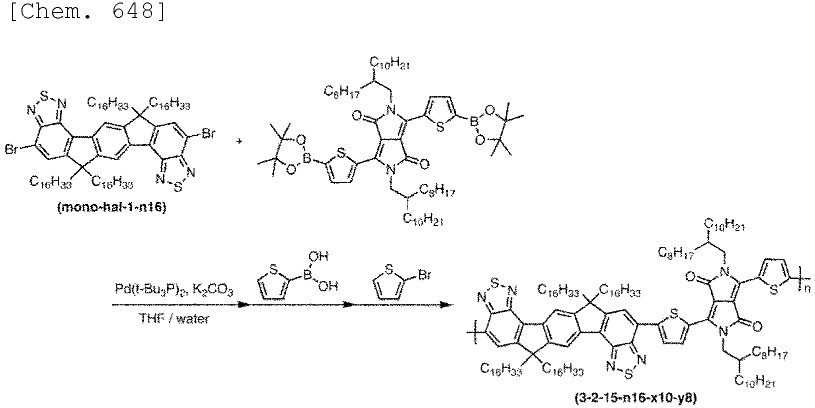

A mixture of the (mono-hal-1-n16) obtained in Example 4 (100 mg, 70.1 µmol) and 2,5-bis(a 2-octyldodecyl)-3,6-bis[5-(4,4,5,5-tetramethyl-1,3,2-dioxaborolan-2-yl)thiophen-2-yl]pyrrolo[3,4-c]pyrrol-1,4(2H,5H)-dione (78.1 mg, 70.1 µmol), THF (1.4 mL) and a 2 M aqueous solution of potassium carbonate (0.7 mL, 1.4 mmol) was bubbled with argon for 30 minutes. To this solution mixture, bis(tri-tert-butylphosphine)palladium(0) (1.8 mg, 35 µmol) was added, and the resulting mixture was stirred for 68 hours at 80°C. After that, 2-thiopheneboronic acid (80.8 mg, 631 µmol) was added to the reaction solution, and the resulting mixture was stirred for 10 hours at 120°C. 2-bromothiophene (67 µL, 701 µmol), furthermore, was added, and the resulting mixture was stirred for 13 hours at 120°C. After the resulting mixture was allowed to cool to room temperature, the mixture was precipitated in a methanol/concentrated hydrochloric acid solution mixture (150 mL/15 mL), and the solid that thus separated out was filtered. The resulting solid was subjected to Soxhlet extraction using methanol, acetone and hexane, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-2-15-n16-x10-y8) (114 mg, 77%) as a black solid.

GPC (THF): Mn = 88600 g/mol, Mw = 160000 g/mol, PDI = 1.80.

GPC (TCB, 140°C): Mn = 75000 g/mol, Mw = 170000 g/mol, PDI = 2.3.

T_{d3} = 397°C, T_{d5} = 406°C, T_{d10} = 417°C.

No phase transition temperature was observed in DSC.

HOMO level: -6.00 eV.

### [Example 35]

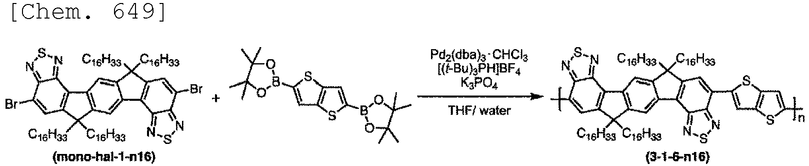

A mixture of the (mono-hal-1-n16) obtained in Example 4 (100 mg, 70.1 µmol), thieno[3,2-b]thiophene (27.5 mg, 70.1 µmol), a 2 M aqueous solution of tripotassium phosphate (0.1 mL, 200 µmol) and THF (3.0 mL) was bubbled with argon for 30 minutes. To this solution mixture, Pd₂(dba)₃·CHCl₃ (1.4 mg, 1.4 µmol) and tri-t-butylphosphonium tetrafluoroborate (1.6 mg, 5.6 µmol) were added, and the resulting mixture was stirred for 7 hours at 80°C. After the resulting mixture was allowed to cool to room temperature, the mixture was diluted with chloroform, washed with water and then precipitated in methanol, and the solid that thus separated out was filtered. The resulting solid was subjected to Soxhlet extraction using methanol, acetone and cyclohexane, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-6-n16) (83 mg, 84%) as a black solid.

GPC (THF): Mn = 27300 g/mol, Mw = 35800 g/mol, PDI = 1.31.

GPC (TCB, 140°C): Mn = 63000 g/mol, Mw = 145000 g/mol, PDI = 2.3.

HOMO level: -5.99 eV.

### [Example 36]

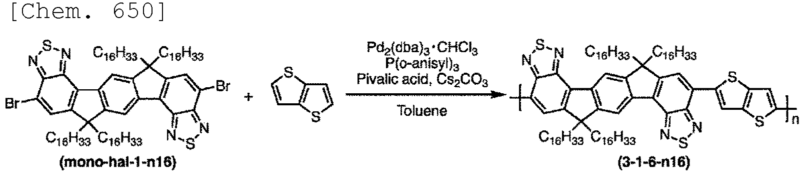

A mixture of the (mono-hal-1-n16) obtained in Example 4 (71.3 mg, 50.0 µmol), thieno[3,2-b]thiophene (7.01 mg, 50.0 µmol), Pd₂(dba)₃·CHCl₃ (1.0 mg, 1.0 µmol), tris(2-methoxyphenyl)phosphine (1.4 mg, 4.0 µmol), pivalic acid (5.1 mg, 50 µmol), cesium carbonate (48.9 mg, 150 µmol) and toluene (0.5 mL) was stirred for 42 hours at 110°C. After the resulting mixture was allowed to cool to room temperature, the mixture was diluted with chloroform, washed with water and then precipitated in methanol, and the solid that thus separated out was filtered. The resulting solid was subjected to Soxhlet extraction using methanol, acetone and cyclohexane, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-6-n16) (44 mg, 61%) as a black solid.

GPC (THF): Mn = 86700 g/mol, Mw = 215000 g/mol, PDI = 2.48.

GPC (TCB, 140°C): Mn = 93000 g/mol, Mw = 210000 g/mol, PDI = 2.2.

HOMO level: -5.95 eV.

### [Example 37]

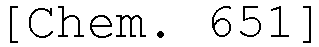

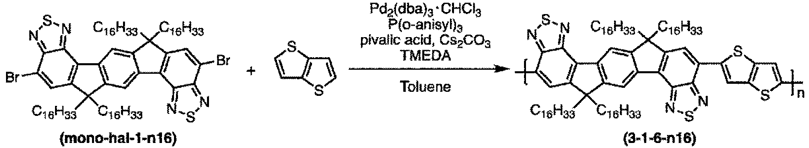

A mixture of the (mono-hal-1-n16) obtained in Example 4 (99.8 mg, 70.0 µmol), thieno[3,2-b]thiophene (9.81 mg, 70.0 µmol), Pd₂(dba)₃·CHCl₃ (1.4 mg, 1.4 µmol), tris(2-methoxyphenyl)phosphine (2.0 mg, 5.6 µmol), cesium carbonate (68.4 mg, 210 µmol), pivalic acid (7.1 mg, 70 µmol), a 0.32 M solution of N,N,N',N'-tetramethylethylenediamine in toluene (22 µL, 70 µmol) and toluene (0.68 mL) was stirred for 48 hours at 110°C. After the resulting mixture was allowed to cool to room temperature, the mixture was diluted with chloroform, washed with water and then precipitated in methanol, and the solid that thus separated out was filtered. The resulting solid was subjected to Soxhlet extraction using methanol and acetone, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-6-n16) (36 mg, 37%) as a black solid.

GPC (THF): Mn = 14000 g/mol, Mw = 34000 g/mol, PDI = 2.4.

GPC (TCB, 140°C): Mn = 15000 g/mol, Mw = 28000 g/mol, PDI = 1.9.

HOMO level: -5.95 eV.

### [Example 38]

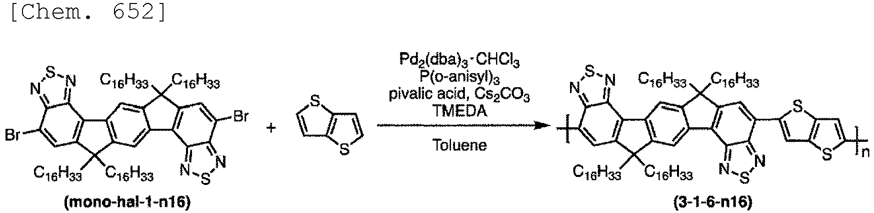

A mixture of the (mono-hal-1-n16) obtained in Example 4 (99.8 mg, 70.0 µmol), thieno[3,2-b]thiophene (9.81 mg, 70.0 µmol), Pd₂(dba)₃·CHCl₃ (1.4 mg, 1.4 µmol), tris(2-methoxyphenyl)phosphine (2.0 mg, 5.6 µmol), cesium carbonate (68.4 mg, 210 µmol), pivalic acid (7.1 mg, 70 µmol), a 0.32 M solution of N,N,N',N'-tetramethylethylenediamine in toluene (22 µL, 70 µmol) and toluene (0.45 mL) was stirred for 48 hours at 110°C. After the resulting mixture was allowed to cool to room temperature, the mixture was diluted with chloroform, washed with water and then precipitated in methanol, and the solid that thus separated out was filtered. The resulting solid was subjected to Soxhlet extraction using methanol and acetone, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-6-n16) (37 mg, 38%) as a black solid.

GPC (THF): Mn = 22000 g/mol, Mw = 99000 g/mol, PDI = 4.5.

GPC (TCB, 140°C): Mn = 22000 g/mol, Mw = 51000 g/mol, PDI = 2.3.

HOMO level: -5.81 eV.

### [Example 39]

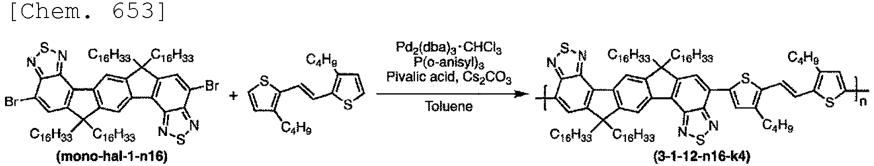

A mixture of the (mono-hal-1-n16) obtained in Example 4 (143 mg, 100 µmol), 1,2-bis(3-butylthiophen-2-yl)ethene (30.5 mg, 100 µmol), Pd₂(dba)₃·CHCl₃ (2.1 mg, 2.0 µmol), tris(2-methoxyphenyl)phosphine (2.8 mg, 8.0 µmol), pivalic acid (10.2 mg, 100 µmol), cesium carbonate (97.7 mg, 300 µmol) and toluene (0.5 mL) was stirred for 42 hours at 120°C. After the resulting mixture was allowed to cool to room temperature, the mixture was diluted with chloroform, washed with water and then precipitated in methanol, and the solid that thus separated out was filtered. The resulting solid was subjected to Soxhlet extraction using methanol, acetone and cyclohexane, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-12-n16-k4) (115 mg, 71%) as a black solid.

GPC (THF): Mn = 51300 g/mol, Mw = 93100 g/mol, PDI = 1.81.

GPC (TCB, 140°C): Mn = 47000 g/mol, Mw = 84600 g/mol, PDI = 1.8.

HOMO level: -5.66 eV.

### [Example 40]

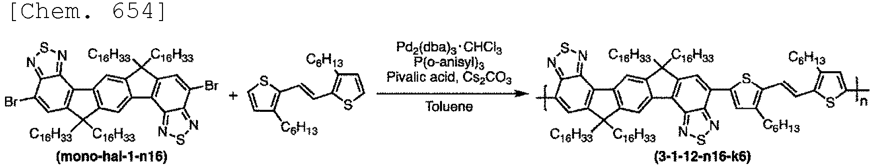

A mixture of the (mono-hal-1-n16) obtained in Example 4 (143 mg, 100 µmol), 1,2-bis(3-hexylthiophen-2-yl)ethene (36.1 mg, 100 µmol), Pd₂(dba)₃·CHCl₃ (2.1 mg, 2.0 µmol), tris(2-methoxyphenyl)phosphine (2.8 mg, 8.0 µmol), pivalic acid (10.2 mg, 100 µmol), cesium carbonate (97.7 mg, 300 µmol) and toluene (0.5 mL) was stirred for 41 hours at 120°C. After the resulting mixture was allowed to cool to room temperature, the mixture was diluted with chloroform, washed with water and then precipitated in methanol, and the solid that thus separated out was filtered. The resulting solid was subjected to Soxhlet extraction using methanol, acetone and hexane, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-12-n16-k6) (75 mg, 41%) as a black solid.

GPC (THF): Mn = 55600 g/mol, Mw = 90400 g/mol, PDI = 1.62.

GPC (TCB, 140°C): Mn = 51000 g/mol, Mw = 86700 g/mol, PDI = 1.7.

HOMO level: -5.70 eV.

### [Example 41]

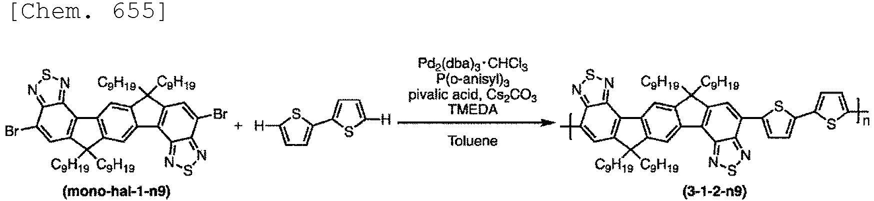

A mixture of the (mono-hal-1-n9) obtained in Example 8 (98.2 mg, 95.0 µmol), 2,2'-bithiophene (15.8 mg, 95.0 µmol), Pd₂(dba)₃·CHCl₃ (2.0 mg, 1.9 µmol), tris(2-methoxyphenyl)phosphine (2.7 mg, 7.7 µmol), cesium carbonate (92.9 mg, 285 µmol), pivalic acid (9.7 mg, 95 µmol), a 0.32 M solution of N,N,N',N'-tetramethylethylenediamine in toluene (30 µL, 96 µmol) and toluene (0.57 mL) was stirred for 24 hours at 110°C. After the resulting mixture was allowed to cool to room temperature, the mixture was diluted with chloroform, washed with water and then precipitated in methanol, and the solid that thus separated out was filtered. The resulting solid was subjected to Soxhlet extraction using methanol, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-2-n9) (98 mg, 99%) as a black solid.

GPC (THF): Mn = 25000 g/mol, Mw = 105000 g/mol, PDI = 4.2.

GPC (TCB, 140°C): Mn = 23000 g/mol, Mw = 89000 g/mol, PDI = 3.9.

HOMO level: -5.63 eV.

### [Example 42]

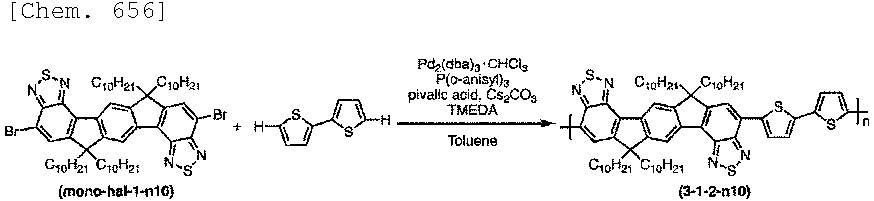

A mixture of the (mono-hal-1-n10) obtained in Example 2 (98.0 mg, 90.0 µmol), 2,2'-bithiophene (15.0 mg, 90.2 µmol), Pd₂(dba)₃·CHCl₃ (1.9 mg, 1.8 µmol), tris(2-methoxyphenyl)phosphine (2.5 mg, 7.1 µmol), cesium carbonate (87.9 mg, 270 µmol), pivalic acid (9.2 mg, 90 µmol), a 0.32 M solution of N,N,N',N'-tetramethylethylenediamine in toluene (30 µL, 96 µmol) and toluene (0.57 mL) was stirred for 24 hours at 110°C. After the resulting mixture was allowed to cool to room temperature, the mixture was diluted with chloroform, washed with water and then precipitated in methanol, and the solid that thus separated out was filtered. The resulting solid was subjected to Soxhlet extraction using methanol, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-2-n10) (78 mg, 79%) as a black solid.

GPC (THF): Mn = 26000 g/mol, Mw = 106000 g/mol, PDI = 4.1.

GPC (TCB, 140°C): Mn = 23000 g/mol, Mw = 79000 g/mol, PDI = 3.4.

HOMO level: -5.67 eV.

### [Example 43]

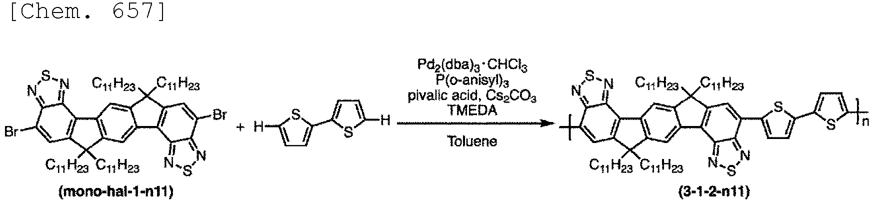

A mixture of the (mono-hal-1-n11) obtained in Example 10 (109 mg, 95.0 µmol), 2,2'-bithiophene (15.8 mg, 95.0 µmol), Pd₂(dba)₃·CHCl₃ (2.0 mg, 1.9 µmol), tris(2-methoxyphenyl)phosphine (2.7 mg, 7.7 µmol), cesium carbonate (92.9 mg, 285 µmol), pivalic acid (9.7 mg, 95 µmol), a 0.32 M solution of N,N,N',N'-tetramethylethylenediamine in toluene (30 µL, 96 µmol) and toluene (0.60 mL) was stirred for 24 hours at 110°C. After the resulting mixture was allowed to cool to room temperature, the mixture was diluted with chloroform, washed with water and then precipitated in methanol, and the solid that thus separated out was filtered. The resulting solid was subjected to Soxhlet extraction using methanol, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-2-n11) (98 mg, 89%) as a black solid.

GPC (THF): Mn = 33000 g/mol, Mw = 377000 g/mol, PDI = 11.4.

GPC (TCB, 140°C): Mn = 35000 g/mol, Mw = 160000 g/mol, PDI = 4.6.

HOMO level: -5.77 eV.

### [Example 44]

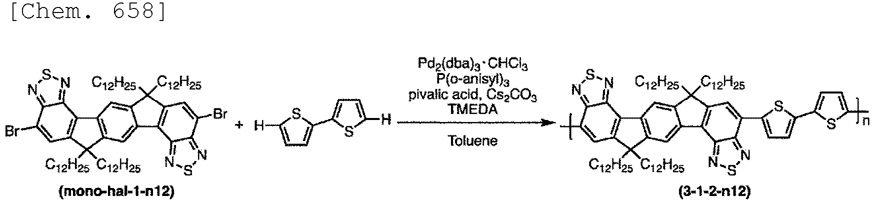

A mixture of the (mono-hal-1-n12) obtained in Example 12 (102 mg, 85.0 µmol), 2,2'-bithiophene (14.1 mg, 84.8 µmol), Pd₂(dba)₃·CHCl₃ (1.8 mg, 1.7 µmol), tris(2-methoxyphenyl)phosphine (2.4 mg, 6.8 µmol), cesium carbonate (83.0 mg, 255 µmol), pivalic acid (8.7 mg, 85 µmol), a 0.32 M solution of N,N,N',N'-tetramethylethylenediamine in toluene (27 µL, 86 µmol) and toluene (0.54 mL) was stirred for 24 hours at 110°C. After the resulting mixture was allowed to cool to room temperature, the mixture was diluted with chloroform, washed with water and then precipitated in methanol, and the solid that thus separated out was filtered. The resulting solid was subjected to Soxhlet extraction using methanol, acetone and hexane, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-2-n12) (66 mg, 65%) as a black solid.

GPC (THF): Mn = 35000 g/mol, Mw = 66000 g/mol, PDI = 1.9.

GPC (TCB, 140°C): Mn = 37000 g/mol, Mw = 79000 g/mol, PDI = 2.1.

HOMO level: -5.80 eV.

### [Example 45]

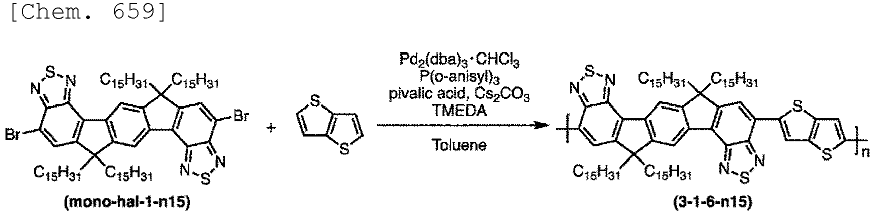

A mixture of the (mono-hal-1-n15) obtained in Example 16 (103 mg, 75.0 µmol), thieno[3,2-b]thiophene (10.5 mg, 75.0 µmol), Pd₂(dba)₃·CHCl₃ (1.5 mg, 1.5 µmol), tris(2-methoxyphenyl)phosphine (2.1 mg, 6.0 µmol), cesium carbonate (73.3 mg, 225 µmol), pivalic acid (7.1 mg, 75 µmol), a 0.32 M solution of N,N,N',N'-tetramethylethylenediamine in toluene (24 µL, 76 µmol) and toluene (0.48 mL) was stirred for 24 hours at 110°C. After the resulting mixture was allowed to cool to room temperature, the mixture was diluted with chloroform, washed with water and then precipitated in methanol, and the solid that thus separated out was filtered. The resulting solid was subjected to Soxhlet extraction using methanol and acetone, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-6-n15) (97 mg, 96%) as a black solid.

GPC (THF): Mn = 43000 g/mol, Mw = 171000 g/mol, PDI = 4.0.

### [Example 46]

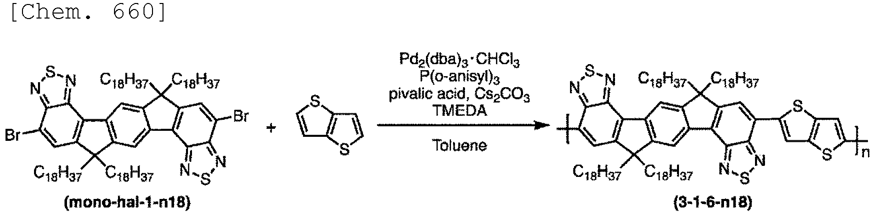

A mixture of the (mono-hal-1-n18) obtained in Example 18 (115 mg, 75.0 µmol), thieno[3,2-b]thiophene (10.5 mg, 75.0 µmol), Pd₂(dba)₃·CHCl₃ (1.5 mg, 1.5 µmol), tris(2-methoxyphenyl)phosphine (2.1 mg, 6.0 µmol), cesium carbonate (73.3 mg, 225 µmol), pivalic acid (7.1 mg, 75 µmol), a 0.32 M solution of N,N,N',N'-tetramethylethylenediamine in toluene (24 µL, 76 µmol) and toluene (0.48 mL) was stirred for 24 hours at 110°C. After the resulting mixture was allowed to cool to room temperature, the mixture was diluted with chloroform, washed with water and then precipitated in methanol, and the solid that thus separated out was filtered. The resulting solid was subjected to Soxhlet extraction using methanol, acetone and hexane, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-6-n18) (78 mg, 69%) as a black solid.

GPC (THF): Mn = 33000 g/mol, Mw = 45000 g/mol, PDI = 1.4.

GPC (TCB, 140°C): Mn = 42000 g/mol, Mw = 82000 g/mol, PDI = 1.9.

HOMO level: -5.79 eV.

### [Example 47]

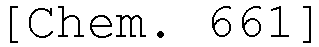

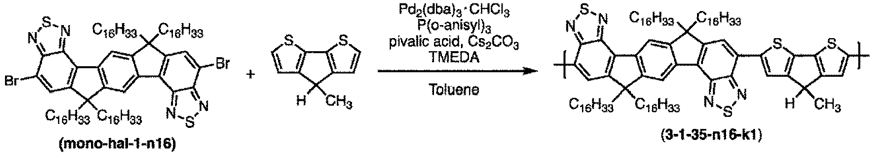

A mixture of the (mono-hal-1-n16) obtained in Example 4 (110 mg, 76.9 µmol), 4-methyl-4H-cyclopenta[2,1-b:3,4-b']dithiophene (14.8 mg, 77.0 µmol), Pd₂(dba)₃·CHCl₃ (1.6 mg, 1.6 µmol), tris(2-methoxyphenyl)phosphine (2.2 mg, 6.2 µmol), cesium carbonate (75.2 mg, 231 µmol), pivalic acid (7.9 mg, 77 µmol), a 0.32 M solution of N,N,N',N'-tetramethylethylenediamine in toluene (25 µL, 79 µmol) and toluene (0.48 mL) was stirred for 24 hours at 110°C. After the resulting mixture was allowed to cool to room temperature, the mixture was diluted with chloroform, washed with water and then precipitated in methanol, and the solid that thus separated out was filtered. The resulting solid was subjected to Soxhlet extraction using methanol, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-35-n16-k1) (107 mg, 95%) as a black solid.

GPC (THF): Mn = 17000 g/mol, Mw = 62000 g/mol, PDI = 3.6.

HOMO level: -5.67 eV.

### [Example 48]

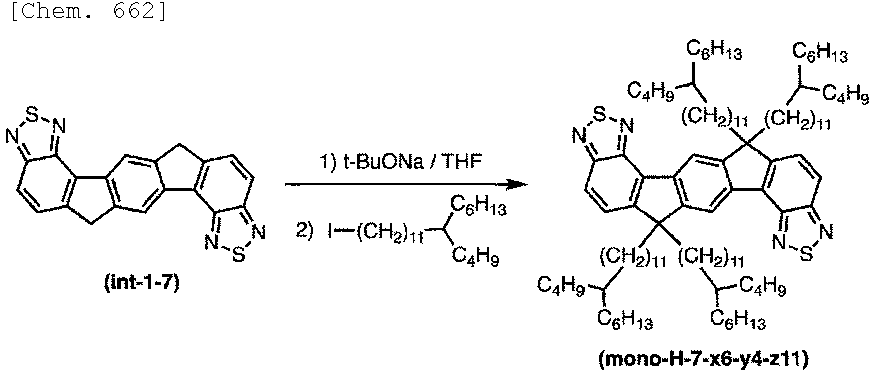

To a mixture of the (int-1-7) obtained in Synthesis Reference Example 15 (500 mg, 1.35 mmol) and tetrahydrofuran (27 mL), sodium tert-butoxide (532 mg, 5.53 mmol) was added at 0°C, and the resulting mixture was stirred for 3.5 hours. After that, 1-bromo-12-butyloctadecane (2.47 g, 5.67 mmol) was added, and the resulting mixture was stirred for 14 hours at room temperature. A saturated aqueous solution of ammonium chloride was added to the reaction solution, and extraction with ethyl acetate was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give (mono-H-7-x6-y4-z11) (1.13 g, 52%) as a yellow solid.

¹H-NMR (CDCl₃, 400 MHz) δ (ppm): 8.49 (s, 2H), 7.98 (d, J = 8.8 Hz, 2H), 7.69 (d, J = 8.8 Hz, 2H), 2.33-2.26 (m, 4H), 2.21-2.14 (m, 4H), 1.30-0.98 (m, 140H), 0.87 (t, J = 6.8 Hz, 24H), 0.68-0.58 (m, 8H).

### [Example 49]

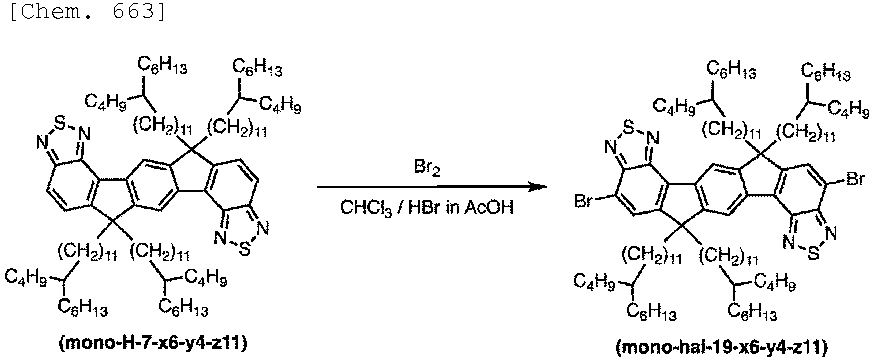

To a mixture of the (mono-H-7-x6-y4-z11) obtained in Example 48 (962 mg, 0.599 mmol) and chloroform (12 mL) and hydrogen bromide (30% solution in acetic acid) (6.0 mL), bromine (0.18 mL, 3.6 mmol) was added, and the resulting mixture was refluxed for 1.5 hours. After the resulting mixture was allowed to cool to room temperature, a saturated aqueous solution of sodium hydrogencarbonate and a saturated aqueous solution of sodium thiosulfate were added, and extraction with chloroform was performed. The collected organic layer was washed with water and saturated saline solution, then dried with anhydrous magnesium sulfate and then filtered, and the solvent was distilled away under reduced pressure. The resulting residue was purified by silica gel column chromatography (hexane/chloroform) to give (mono-hal-19-x6-y4-z11) (904 mg, 86%) as an orange viscous liquid.

¹H-NMR (CDCl₃, 400 MHz) δ (ppm): 8.43 (s, 2H), 7.92 (s, 2H), 2.32-2.24 (m, 4H), 2.18-2.11 (m, 4H), 1.30-0.99 (m, 140H), 0.87 (t, J = 6.8 Hz, 24H), 0.66-0.58 (m, 8H).

### [Example 50]

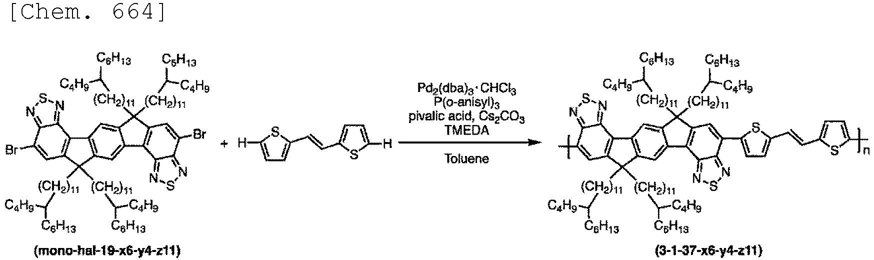

A mixture of the (mono-hal-19-x6-y4-z11) obtained in Example 49 (140 mg, 79.4 µmol), 1,2-di(2-thienyl)ethylene (15.3 mg, 79.4 µmol), Pd₂(dba)₃·CHCl₃ (1.6 mg, 1.5 µmol), tris(2-methoxyphenyl)phosphine (2.2 mg, 6.2 µmol), cesium carbonate (77.6 mg, 238 µmol), pivalic acid (8.1 mg, 79 µmol), a 0.32 M solution of N,N,N',N'-tetramethylethylenediamine in toluene (25 µL, 80 µmol) and toluene (0.38 mL) was stirred for 24 hours at 120°C. After the resulting mixture was allowed to cool to room temperature, the mixture was diluted with chloroform, washed with water and then precipitated in methanol, and the solid that thus separated out was filtered. The resulting solid was subjected to Soxhlet extraction using methanol, acetone and cyclohexane, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-37-x6-y4-z11) (66 mg, 46%) as a black solid.

GPC (THF): Mn = 34000 g/mol, Mw = 72000 g/mol, PDI = 2.1.

GPC (TCB, 140°C): Mn = 33000 g/mol, Mw = 680000 g/mol, PDI = 2.1.

HOMO level: -5.88 eV.

### [Example 51]

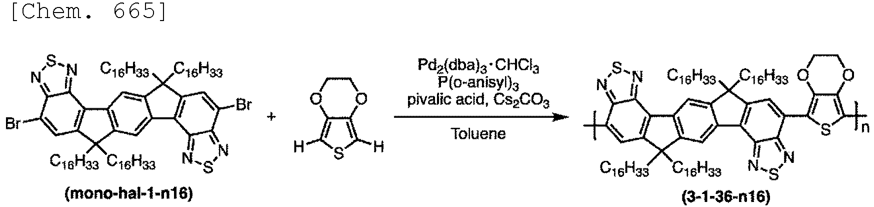

A mixture of the (mono-hal-1-n16) obtained in Example 4 (160 mg, 112 µmol), 3,4-ethylenedioxythiophene (16.0 mg, 112 µmol), Pd₂(dba)₃·CHCl₃ (2.3 mg, 2.2 µmol), tris(2-methoxyphenyl)phosphine (3.2 mg, 9.8 µmol), pivalic acid (11.5 mg, 113 µmol), cesium carbonate (110 mg, 338 µmol) and toluene (0.56 mL) was stirred for 2 hours at 120°C. After the resulting mixture was allowed to cool to room temperature, the mixture was diluted with chloroform, washed with water and then precipitated in methanol, and the solid that thus separated out was filtered. The resulting solid was subjected to Soxhlet extraction using methanol, acetone and cyclohexane, through which components soluble in these solvents were removed. The filter residue, furthermore, was dissolved in chloroform. The resulting mixture was concentrated under reduced pressure and precipitated in methanol, and the solid that thus separated out was filtered. The resulting solid was washed with methanol and then dried under reduced pressure at 90°C to give (3-1-36-n16) (81 mg, 51%) as a black solid.

GPC (THF): Mn = 12000 g/mol, Mw = 149000 g/mol, PDI = 12.4.

GPC (TCB, 140°C): Mn = 30000 g/mol, Mw = 36000 g/mol, PDI = **1.2.**

HOMO level: -5.83 eV.

### [Example 52]

### (Evaluation of Solubility)

To the conjugated polymers (1 mg) obtained in Examples 19, 21, 22, 23, 24, 26, 27, 30, 31, 34, 43 and 44, chloroform, THF, toluene and chlorobenzene were each added to give film formation compositions. The volume of each organic solvent required to completely dissolve the conjugated polymeric compound at room temperature (25°C) was measured, and the solubility (% by weight) was calculated. The determination of the point of complete dissolution was by visual confirmation. The evaluated solubility of the conjugated polymers is presented in Table **1.**

**[Table 1]**

| Solubility of Conjugated Polymers according to the Present Invention | | | | |
|---|---|---|---|---|
| Conjugated polymer | Solubility [% by weight] | | | |
| | CHCl₃ | THF | Toluene | Chlorobenzene |
| 3-1-2-n10 (Example 19) | ≥0.5 | ≥0.5 | 0.2 | ≥0.5 |
| 3-1-13-n10-k8 (Example 21) | ≥0.5 | ≥0.5 | ≥0.5 | ≥0.5 |
| 3-1-17-n10-k1 (Example 22) | ≥0.5 | ≥0.5 | ≥0.5 | ≥0.5 |
| 3-1-2-n16 (Example 23) | ≥0.5 | ≥0.5 | 0.2 | ≥0.5 |
| 3-1-6-n16 (Conduct 24) | ≥0.5 | ≥0.5 | ≥0.5 | ≥0.5 |
| 3-1-13-n16-k8 (Example 26) | ≥0.5 | ≥0.5 | ≥0.5 | ≥0.5 |
| 3-1-17-n16-k1 (Example 27) | ≥0.5 | ≥0.5 | ≥0.5 | ≥0.5 |
| 3-1-17-n21-k3 (Example 30) | ≥0.5 | ≥0.5 | ≥0.5 | ≥0.5 |
| 3-1-24-n16-k1 (Example 31) | ≥0.5 | ≥0.5 | 0.2 | ≥0.5 |
| 3-2-14-n16-x10-y8 (Example 34) | ≥0.5 | ≥0.5 | ≥0.5 | ≥0.5 |
| 3-1-2-n11 (Example 43) | ≥0.5 | ≥0.5 | 0.2 | ≥0.5 |
| 3-1-2-n12 (Example 44) | ≥0.5 | ≥0.5 | 0.2 | ≥0.5 |

### [Example 53]

A film formation composition for organic thin films was prepared by heating a 0.5 wt% solution of the conjugated polymer (3-1-2-n10) synthesized in Example 19 in o-DCB within a nitrogen atmosphere inside a glove box.

After being allowed to cool to room temperature, the composition was completely filtered through a 0.22-µm filter. Based on this, it was confirmed that the solution state was maintained and that the compound was suitable for film formation.

Subsequently, a film of Parylene C^{™} was formed as an undercoat layer on a glass substrate by CVD, and then a source electrode and a drain electrode were attached by depositing gold in a vacuum, with a shadow mask having a channel length of 100 µm and a channel width of 500 µm placed on the Parylene C layer. The solution prepared in the foregoing was applied by spin coating in a nitrogen atmosphere within a glove box. By heating it to 150°C and maintaining the temperature for 15 minutes, an organic thin film of the conjugated polymer (3-1-2-n10) was created. A film of Parylene C was formed as a gate insulating layer by CVD, and then a silver electrode was created by deposition, through which a top gate-bottom contact organic thin-film transistor element was created (the gate electrode was made of silver, the gate insulating layer was made of Parylene C, and the source electrode and drain electrode were made of gold).

In air, the organic thin-film transistor element was connected to a semiconductor parameter analyzer (manufactured by Keithley Instruments, Inc.; model 4200A-SCS), and the gate voltage (Vg) was scanned from +10 to -50 V in 1-V steps at a drain voltage (Vd = -50 V), through which the transmission characteristics were evaluated. The organic thin-film transistor element exhibited p-type properties, and its carrier mobility for holes was 0.31 cm²/Vs.

### [Example 54]

The same operations as in Example 53 were repeated, except that the conjugated polymer (3-1-17-n10-k1) synthesized in Example 22 was used. The resulting organic thin-film transistor element exhibited p-type properties, and its carrier mobility for holes was 0.13 cm²/Vs.

### [Example 55]

The same operations as in Example 53 were repeated, except that the conjugated polymer (3-1-6-n16) synthesized in Example 24 was used. The resulting organic thin-film transistor element exhibited p-type properties, and its carrier mobility for holes was 1.07 cm²/Vs.

### [Example 56]

The same operations as in Example 53 were repeated, except that the conjugated polymer (3-1-17-n16-k1) synthesized in Example 27 was used. The resulting organic thin-film transistor element exhibited p-type properties, and its carrier mobility for holes was 0.22 cm²/Vs.

### [Example 57]

The same operations as in Example 53 were repeated, except that a 0.5 wt% solution in toluene was used. The resulting organic thin-film transistor element exhibited p-type properties, and its carrier mobility for holes was 0.10 cm²/Vs.

### [Example 58]

A film formation composition for organic thin films was prepared by heating a 0.5 wt% solution of the conjugated polymer (3-1-2-n10) synthesized in Example 19 in o-DCB within a nitrogen atmosphere inside a glove box. Subsequently, a film of Parylene C was formed as an undercoat layer on a glass substrate by CVD, and then the solution prepared in the foregoing was applied by spin coating in a nitrogen atmosphere within a glove box. By heating it to 150°C and maintaining the temperature for 15 minutes, an organic thin film of the conjugated polymer (3-1-2-n10) was created. The ionization potential of the resulting organic thin film was 5.52 eV, indicating that the film was resistant to oxidation by oxygen. Based on this, it was confirmed that the film had high atmospheric stability.

### [Example 59]

The same operations as in Example 58 were repeated, except that the conjugated polymer (3-1-17-n10-k1) synthesized in Example 22 was used. The resulting organic thin film ionization potential was 5.26 eV, allowing it to be confirmed that the film had high atmospheric stability.

### [Example 60]

The same operations as in Example 58 were repeated, except that the conjugated polymer (3-1-6-n16) synthesized in Example 24 was used. The resulting organic thin film ionization potential was 5.54 eV, allowing it to be confirmed that the film had high atmospheric stability.

### [Example 61]

The same operations as in Example 58 were repeated, except that the conjugated polymer (3-1-17-n16-k1) synthesized in Example 27 was used. The resulting organic thin film ionization potential was 5.33 eV, allowing it to be confirmed that the film had high atmospheric stability.

### [Example 62]

The same operations as in Example 53 were repeated, except that the conjugated polymer (3-1-6-n16) synthesized in Example 35 was used. The resulting organic thin-film transistor element exhibited p-type properties, and its carrier mobility for holes was 0.52 cm²/Vs.

### [Example 63]

The same operations as in Example 53 were repeated, except that the conjugated polymer (3-1-6-n16) synthesized in Example 36 was used. The resulting organic thin-film transistor element exhibited p-type properties, and its carrier mobility for holes was 0.81 cm²/Vs.

### [Example 64]

The same operations as in Example 53 were repeated, except that the conjugated polymer (3-1-6-n16) synthesized in Example 37 was used. The resulting organic thin-film transistor element exhibited p-type properties, and its carrier mobility for holes was 0.35 cm²/Vs.

### [Example 65]

The same operations as in Example 53 were repeated, except that the conjugated polymer (3-1-6-n16) synthesized in Example 38 was used. The resulting organic thin-film transistor element exhibited p-type properties, and its carrier mobility for holes was 0.40 cm²/Vs.

### [Example 66]

The same operations as in Example 53 were repeated, except that the conjugated polymer (3-1-2-n9) synthesized in Example 41 was used. The resulting organic thin-film transistor element exhibited p-type properties, and its carrier mobility for holes was 0.24 cm²/Vs.

### [Example 67]

The same operations as in Example 53 were repeated, except that the conjugated polymer (3-1-2-n10) synthesized in Example 42 was used. The resulting organic thin-film transistor element exhibited p-type properties, and its carrier mobility for holes was 0.32 cm²/Vs.

### [Example 68]

The same operations as in Example 53 were repeated, except that the conjugated polymer (3-1-2-n11) synthesized in Example 43 was used. The resulting organic thin-film transistor element exhibited p-type properties, and its carrier mobility for holes was 0.34 cm²/Vs.

### [Example 69]

The same operations as in Example 53 were repeated, except that the conjugated polymer (3-1-2-n12) synthesized in Example 44 was used. The resulting organic thin-film transistor element exhibited p-type properties, and its carrier mobility for holes was 0.26 cm²/Vs.

### [Example 70]

The same operations as in Example 53 were repeated, except that the conjugated polymer (3-1-6-n18) synthesized in Example 46 was used. The resulting organic thin-film transistor element exhibited p-type properties, and its carrier mobility for holes was 0.41 cm²/Vs.

### [Example 71]

The same operations as in Example 53 were repeated, except that the conjugated polymer (3-1-35-n16-k1) synthesized in Example 47 was used. The resulting organic thin-film transistor element exhibited p-type properties, and its carrier mobility for holes was 0.17 cm²/Vs.

### [Example 72]

The same operations as in Example 58 were repeated, except that the conjugated polymer (3-1-6-n16) synthesized in Example 35 was used. The resulting organic thin film ionization potential was 5.54 eV, allowing it to be confirmed that the film had high atmospheric stability.

### [Example 73]

The same operations as in Example 58 were repeated, except that the conjugated polymer (3-1-6-n16) synthesized in Example 36 was used. The resulting organic thin film ionization potential was 5.55 eV, allowing it to be confirmed that the film had high atmospheric stability.

### [Example 74]

The same operations as in Example 58 were repeated, except that the conjugated polymer (3-1-12-n16-k4) synthesized in Example 39 was used. The resulting organic thin film ionization potential was 5.45 eV, allowing it to be confirmed that the film had high atmospheric stability.

### [Example 75]

The same operations as in Example 58 were repeated, except that the conjugated polymer (3-1-12-n16-k6) synthesized in Example 40 was used. The resulting organic thin film ionization potential was 5.39 eV, allowing it to be confirmed that the film had high atmospheric stability.

### [Example 76]

The same operations as in Example 58 were repeated, except that the conjugated polymer (3-1-2-n9) synthesized in Example 41 was used. The resulting organic thin film ionization potential was 5.53 eV, allowing it to be confirmed that the film had high atmospheric stability.

### [Example 77]

The same operations as in Example 58 were repeated, except that the conjugated polymer (3-1-2-n10) synthesized in Example 42 was used. The resulting organic thin film ionization potential was 5.53 eV, allowing it to be confirmed that the film had high atmospheric stability.

### [Example 78]

The same operations as in Example 58 were repeated, except that the conjugated polymer (3-1-2-n11) synthesized in Example 43 was used. The resulting organic thin film ionization potential was 5.60 eV, allowing it to be confirmed that the film had high atmospheric stability.

### [Example 79]

The same operations as in Example 58 were repeated, except that the conjugated polymer (3-1-2-n12) synthesized in Example 44 was used. The resulting organic thin film ionization potential was 5.41 eV, allowing it to be confirmed that the film had high atmospheric stability.

### [Example 80]

The same operations as in Example 58 were repeated, except that the conjugated polymer (3-1-6-n18) synthesized in Example 46 was used. The resulting organic thin film ionization potential was 5.45 eV, allowing it to be confirmed that the film had high atmospheric stability.

### [Example 81]

The same operations as in Example 58 were repeated, except that the conjugated polymer (3-1-35-n16-k1) synthesized in Example 47 was used. The resulting organic thin film ionization potential was 5.25 eV, allowing it to be confirmed that the film had high atmospheric stability.

### [Comparative Example 1]

A film formation composition for organic thin films was prepared by heating a 0.5 wt% solution of poly 2,5-bis(3-tetradecylthiophen-2-yl)thieno[3,2-b thiophene] (Sigma-Aldrich) in o-DCB within a nitrogen atmosphere inside a glove box.

After the composition was allowed to cool to room temperature, it was found that a solid had separated out. Based on this, it was confirmed that the compound was not suitable for film formation.

### [Comparative Example 2]

The same operations as in Example 27 were repeated, except that poly 2,5-bis(3-tetradecylthiophen-2-yl)thieno[3,2-b thiophene] (Sigma-Aldrich) was used. The ionization potential of the resulting organic thin film was 4.93 eV, allowing it to be confirmed that the film was inferior in atmospheric stability.

## Claims

1. A compound represented by general formula (1) below. 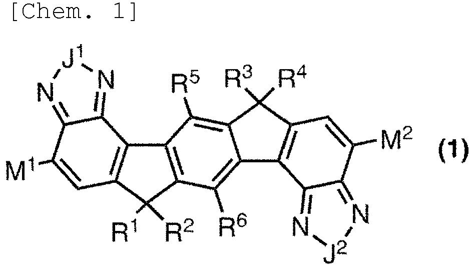 (In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom. M¹ and M² each independently represent one group selected from the group consisting of a hydrogen atom, a halogen atom, a boron-containing group and a tin-containing group.)

2. The compound according to Claim 1, wherein each of R¹, R², R³ and R⁴ is independently a C1 to C34 alkyl group;
R¹ and R² are optionally bound to each other to form a ring together with the carbon atom to which the R¹ and R² are bound;
R³ and R⁴ are optionally bound to each other to form a ring together with the carbon atom to which the R³ and R⁴ are bound;
each of R⁵ and R⁶ is independently a hydrogen atom or a fluorine atom;
each of J¹ and J² is independently an oxygen atom, a sulfur atom or a selenium atom; and
each of M¹ and M² is independently one group selected from the group consisting of a hydrogen atom, a bromine atom, an iodine atom, a boron-containing group and a tin-containing group.

3. The compound according to Claim 1 or 2, wherein R⁵ and R⁶ are hydrogen atoms;
J¹ and J² are sulfur atoms; and
each of M¹ and M² is independently one group selected from the group consisting of a hydrogen atom, a bromine atom, a boron-containing group and a tin-containing group.

4. A conjugated polymer comprising a structural unit represented by general formula (2) below 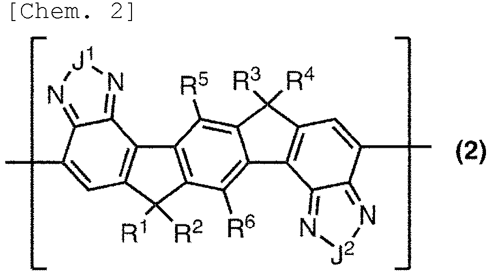
(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom.); and
a structural unit represented by general formula (3) below. 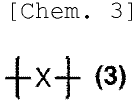
(In the formula, X represents a divalent heteroaromatic ring linking group optionally substituted with a C1 to C50 alkyl group or a C1 to C50 alkoxy group.)

5. The conjugated polymer according to Claim 4, wherein the conjugated polymer is composed of a structural unit represented by general formula (24) below, which has the structural unit represented by general formula (2) and the structural unit represented by general formula (3) alternately. 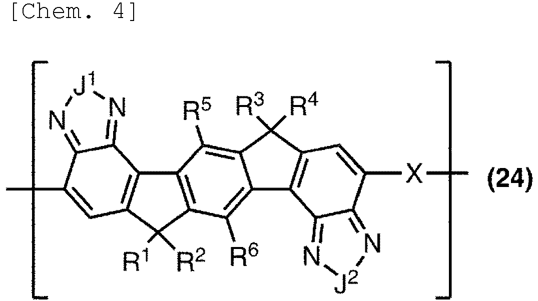 (In the formula, R¹, R², R³, R⁴, R⁵, R⁶, J¹, J² and X have the same meanings as described above.)

6. The conjugated polymer according to Claim 4, wherein each of R¹, R², R³ and R⁴ is independently a C1 to C34 alkyl group;
R¹ and R² are optionally bound to each other to form a ring together with the carbon atom to which the R¹ and R² are bound;
R³ and R⁴ are optionally bound to each other to form a ring together with the carbon atom to which the R³ and R⁴ are bound;
each of R⁵ and R⁶ is independently a hydrogen atom, a fluorine atom or a C1 to C34 alkyl group; and
each of J¹ and J² is independently an oxygen atom, a sulfur atom or a selenium atom.

7. The conjugated polymer according to Claim 4, wherein R⁵ and R⁶ are hydrogen atoms; and
J¹ and J² are sulfur atoms.

8. The conjugated polymer according to Claim 4, wherein X is a divalent heteroaromatic ring linking group selected from the group consisting of linking groups represented by general formula (4) to general formula (23) below. 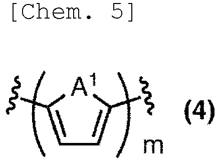 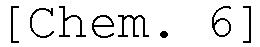 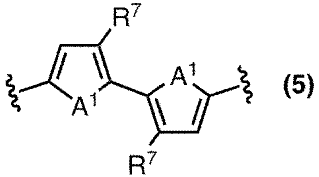 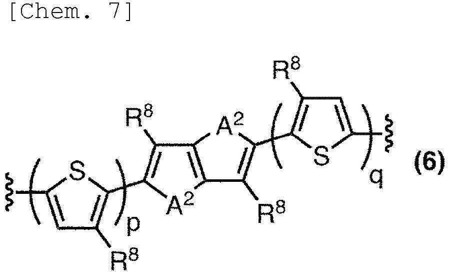 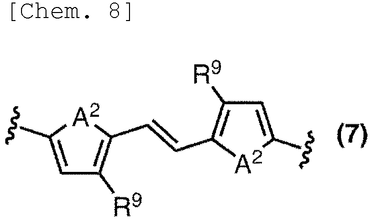 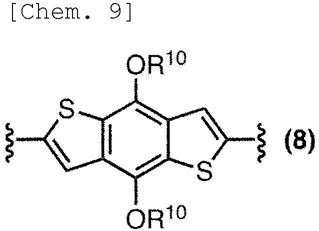 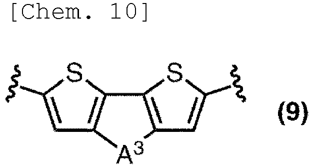 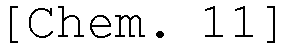 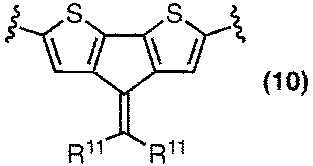 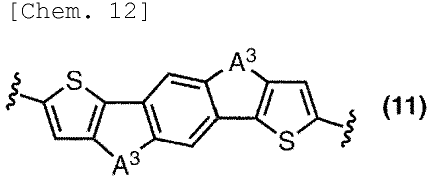 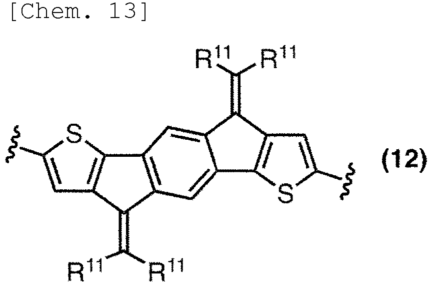 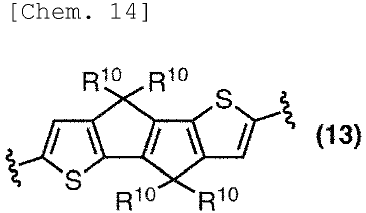 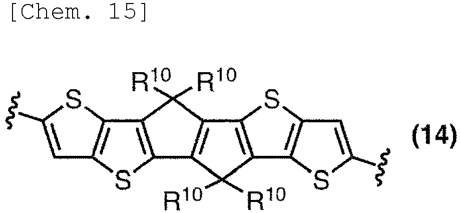 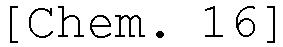 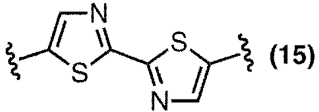 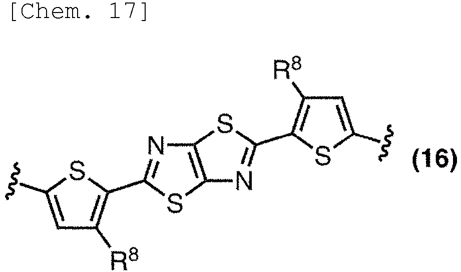 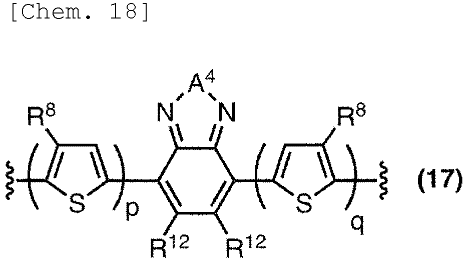 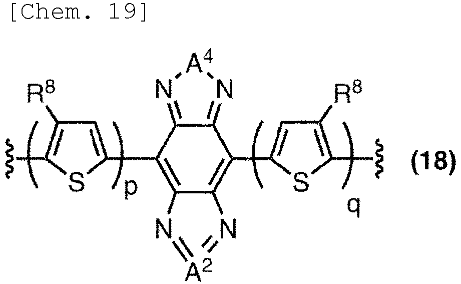 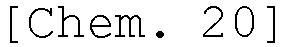 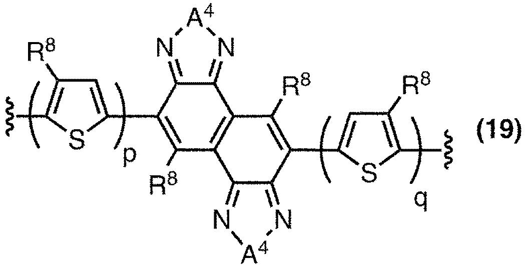 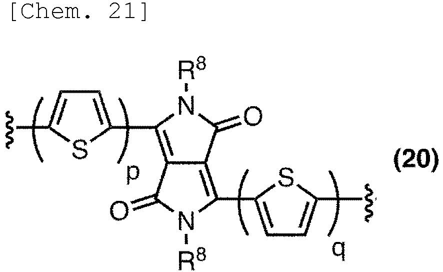 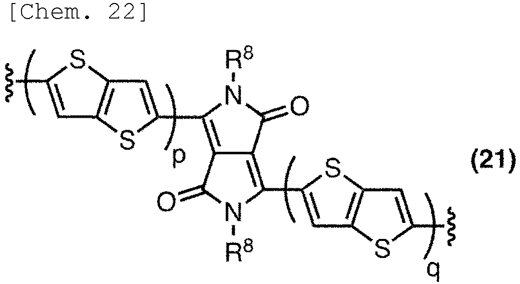 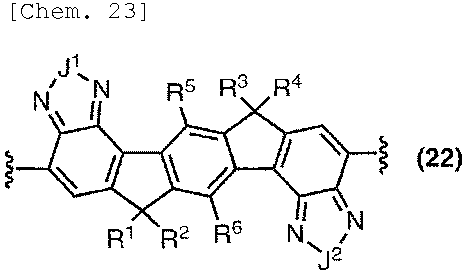 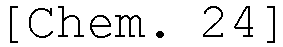 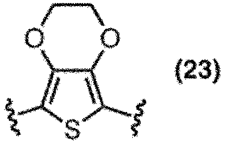 (In the formula, R¹, R², R³, R⁴, R⁵, R⁶, J¹ and J² have the same meanings as described above. A¹ and A² each independently represent a chalcogen atom. A³ represents a chalcogen atom, C(R¹⁰)₂, C(H)(R¹⁰), Si(R¹⁰)₂ or NR¹⁰, independently at each occurrence. A⁴ represents a chalcogen atom or NR¹⁰, independently at each occurrence. R⁷ represents a hydrogen atom, a fluorine atom or a C1 to C50 alkyl group. A plurality of R⁷s may be the same or different from one another. R⁸ represents a hydrogen atom or a C1 to C50 alkyl group. A plurality of R⁸s may be the same or different from one another. R⁹ represents a hydrogen atom, a fluorine atom or a C1 to C50 alkyl group. A plurality of R⁹s may be the same or different from one another. R¹⁰ represents a C1 to C50 alkyl group. A plurality of R¹⁰s may be the same or different from one another. R¹¹ represents a C1 to C50 alkyl group or a C1 to C50 thioalkyl group. R¹² represents a hydrogen atom, a fluorine atom, a C1 to C50 alkyl group or a C1 to C50 alkoxy group. m represents 1 to **3.** p and q each independently represent 0 or 1.)

9. The conjugated polymer according to Claim 8, wherein A¹ is an oxygen atom, a sulfur atom or a selenium atom, independently at each occurrence;
A² is a sulfur atom or a selenium atom, independently at each occurrence;
A³ is C(R¹⁰)₂, C(H)(R¹⁰), Si(R¹⁰)₂ or NR¹⁰, independently at each occurrence;
A⁴ is an oxygen atom, a sulfur atom, a selenium atom or NR¹⁰, independently at each occurrence;
R⁷ is a hydrogen atom, a fluorine atom or a C1 to C34 alkyl group, independently at each occurrence;
R⁸ is a hydrogen atom or a C1 to C34 alkyl group, independently at each occurrence;
R⁹ is a hydrogen atom, a fluorine atom or a C1 to C34 alkyl group, independently at each occurrence;
R¹⁰ is a C1 to C34 alkyl group, independently at each occurrence;
R¹¹ is a C1 to C34 alkyl group or a C1 to C34 thioalkyl group, independently at each occurrence; and
R¹² is a hydrogen atom, a fluorine atom, a C1 to C34 alkyl group or a C1 to C34 alkoxy group, independently at each occurrence.

10. The conjugated polymer according to Claim 8, wherein A¹, A² and A⁴ are sulfur atoms;
A³ is C(R¹⁰)₂ or C(H)(R¹⁰);
R⁷ is a hydrogen atom, a fluorine atom or a C1 to C20 alkyl group, independently at each occurrence;
R⁸ is a hydrogen atom or a C1 to C20 alkyl group, independently at each occurrence;
R⁹ is a hydrogen atom, a fluorine atom or a C1 to C20 alkyl group, independently at each occurrence;
R¹⁰ is a C1 to C20 alkyl group, independently at each occurrence;
R¹¹ is a C1 to C20 alkyl group or a C1 to C20 thioalkyl group, independently at each occurrence; and
R¹² is a hydrogen atom, a fluorine atom, a C1 to C20 alkyl group or a C1 to C20 alkoxy group, independently at each occurrence.

11. A method for producing a conjugated polymer composed of a structural unit represented by general formula (2) below and a structural unit represented by general formula (3) below, the method comprising:
allowing a monomer represented by general formula (mono-hal) below to react with a monomer represented by general formula (mono-X-B) below in the presence of a transition metal catalyst. 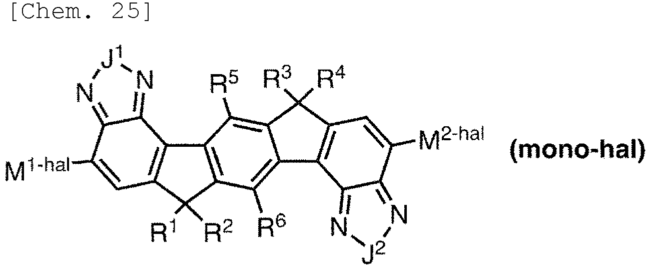
(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom. M^{1-hal} and M^{2-hal} each independently represent a halogen atom.)
[Chem. 26] M^{3-B}-X-M^{4-B} (mono-X-B)
(In the formula, X represents a divalent heteroaromatic ring linking group optionally substituted with a C1 to C50 alkyl group or a C1 to C50 alkoxy group. M^{3-B} and M^{4-B} each independently represent a boron-containing group.) 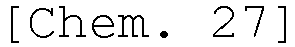 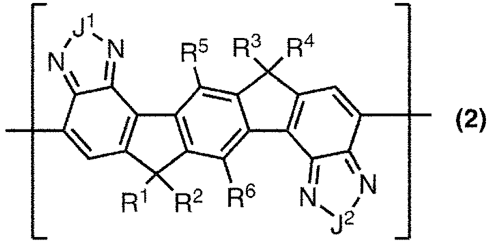
(In the formula, R¹, R², R³, R⁴, R⁵, R⁶, J¹ and J² have the same meanings as described above.) 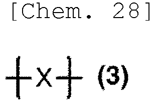
(In the formula, X has the same meaning as described above.)

12. A method for producing a conjugated polymer composed of a structural unit represented by general formula (2) below and a structural unit represented by general formula (3) below, the method comprising:
allowing a monomer represented by general formula (mono-hal) below to react with a monomer represented by general formula (mono-X-Sn) below in the presence of a transition metal catalyst. 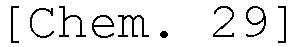 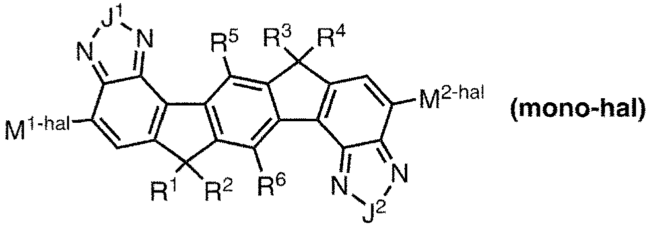
(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom. M^{1-hal} and M^{2-hal} each independently represent a halogen atom.)
[Chem. 30] M^{3-Sn}-X-M^{4-Sn} (mono-X-Sn)
(In the formula, X represents a divalent heteroaromatic ring linking group optionally substituted with a C1 to C50 alkyl group or a C1 to C50 alkoxy group. M^{3-Sn} and M^{4-Sn} each independently represent a tin-containing group.) 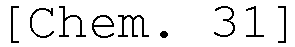 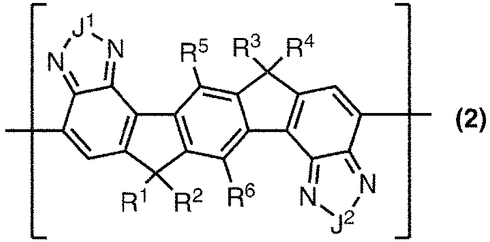
(In the formula, R¹, R², R³, R⁴, R⁵, R⁶, J¹ and J² have the same meanings as described above.) 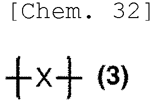
(In the formula, X has the same meaning as described above.)

13. A method for producing a conjugated polymer composed of a structural unit represented by general formula (2) below and a structural unit represented by general formula (3) below, the method comprising:
allowing a monomer represented by general formula (mono-B) below to react with a monomer represented by general formula (mono-X-hal) below in the presence of a transition metal catalyst. 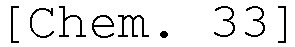 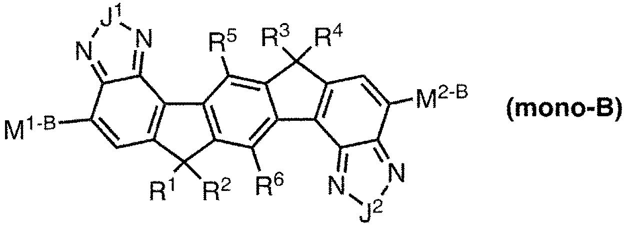
(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom. M^{1-B} and M^{2-B} each independently represent a boron-containing group.)
[Chem. 34] M^{3-hal}-X-M^{4-hal} (mono-X-hal)
(In the formula, X represents a divalent heteroaromatic ring linking group optionally substituted with a C1 to C50 alkyl group or a C1 to C50 alkoxy group. M^{3-hal} and M^{4-hal} each independently represent a halogen atom.) 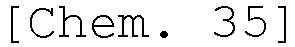 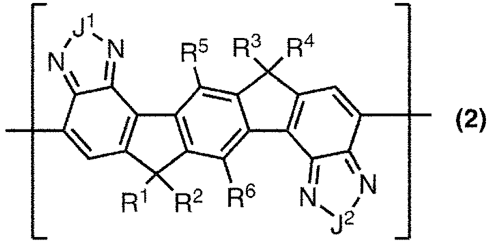
(In the formula, R¹, R², R³, R⁴, R⁵, R⁶, J¹ and J² have the same meanings as described above.) 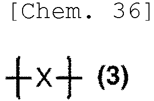
(In the formula, X has the same meaning as described above.)

14. A method for producing a conjugated polymer composed of a structural unit represented by general formula (2) below and a structural unit represented by general formula (3) below, the method comprising:
allowing a monomer represented by general formula (mono-Sn) below to react with a monomer represented by general formula (mono-X-hal) below in the presence of a transition metal catalyst. 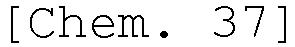 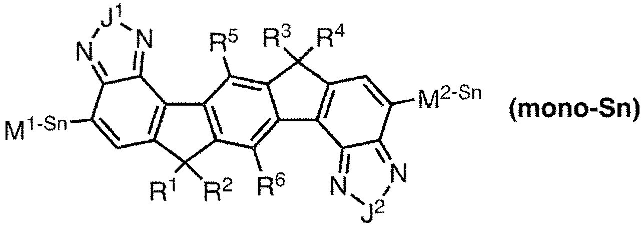
(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom. M^{1-Sn} and M^{2-Sn} each independently represent a tin-containing group.)
[Chem. 38] M^{3-hal}-X-M^{4-hal} (mono-X-hal)
(In the formula, X represents a divalent heteroaromatic ring linking group optionally substituted with a C1 to C50 alkyl group or a C1 to C50 alkoxy group. M^{3-hal} and M^{4-hal} each independently represent a halogen atom.) 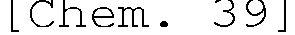 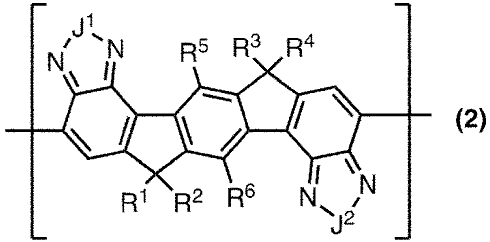
(In the formula, R¹, R², R³, R⁴, R⁵, R⁶, J¹ and J² have the same meanings as described above.) 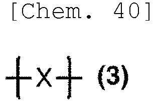
(In the formula, X has the same meaning as described above.)

15. A method for producing a conjugated polymer composed of a divalent structural unit represented by general formula (2) below and a structural unit represented by general formula (3) below, the method comprising:
allowing a monomer represented by general formula (mono-hal) below to react with a monomer represented by general formula (mono-X-H) below in the presence of a transition metal catalyst. 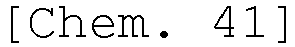 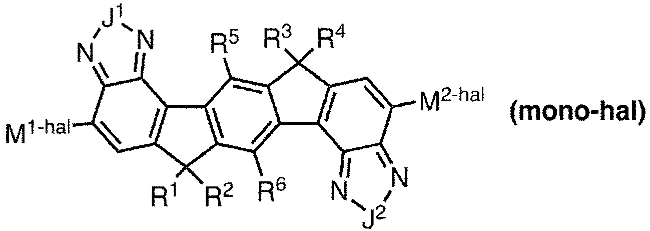
(In the formula, R¹, R², R³ and R⁴ each independently represent a C1 to C50 alkyl group. R¹ and R² are optionally bound to each other to form a ring together with the carbon atom to which the R¹ and R² are bound. R³ and R⁴ are optionally bound to each other to form a ring together with the carbon atom to which the R³ and R⁴ are bound. R⁵ and R⁶ each independently represent a hydrogen atom, a fluorine atom or a C1 to C50 alkyl group. J¹ and J² each independently represent a chalcogen atom. M^{1-hal} and M^{2-hal} each independently represent a halogen atom.)
[Chem. 42] H-X-H (mono-X-H)
(In the formula, X represents a divalent heteroaromatic ring linking group optionally substituted with a C1 to C50 alkyl group or a C1 to C50 alkoxy group.) 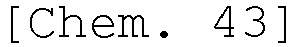 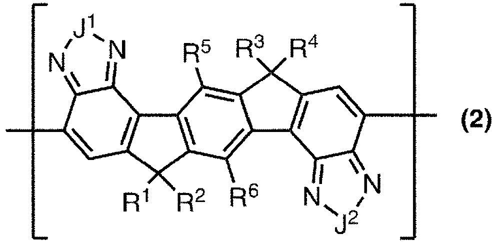
(In the formula, R¹, R², R³, R⁴, R⁵, R⁶, J¹ and J² have the same meanings as described above.) 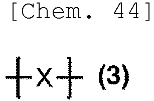
(In the formula, X has the same meaning as described above.)

16. A film formation composition comprising the conjugated polymer according to any one of Claims 4 to 10.

17. An organic thin film comprising the conjugated polymer according to any one of Claims 4 to 10.

18. An organic semiconductor element comprising the conjugated polymer according to any one of Claims 4 to 10.

19. An organic thin-film transistor element comprising the conjugated polymer according to any one of Claims 4 to 10.
